# EUROPEAN PATENT SPECIFICATION

(11) **EP 3 724 188 B1**
(45) Date of publication and mention of the grant of the patent: **07.06.2023**
(21) Application number: 18833552.5
(22) Date of filing: 12.12.2018
(51) Int. Cl.: C07D 453/02, A61K 31/439, A61P 25/28

(54) **PROGRANULIN MODULATORS AND METHODS OF USING THE SAME**
PROGRANULIN-MODULATOREN UND VERFAHREN ZU DEREN VERWENDUNG
MODULATEURS DE PROGRANULINE ET LEURS PROCÉDÉS D'UTILISATION

(30) Priority: 12.12.2017 US 201762597505 P; 25.09.2018 US 201862735946 P
(43) Date of publication of application: 21.10.2020
(73) Proprietor: Arkuda Therapeutics, Cambridge, MA 02139 (US)
(72) Inventor: KOENIG, Gerhard, Cambridge, MA 02139 (US); TEBBE, Mark, Joseph, Arlington, MA 02476 (US); BURNETT, Duane, A., Cambridge, MA 02139 (US)
(74) Representative: Mewburn Ellis LLP
(86) International application number: PCT/US2018/065063
(87) International publication number: WO 2019/118528

(56) References cited:
- EP-A1- 0 801 067
- EP-A1- 1 726 304
- WO-A1-2012/175119
- WO-A1-2015/143300
- WO-A2-02/096422
- WO-A2-2010/081036
- JP-A- 2003 267 977
- GANALA NAGA TRINADHACHARI ET AL: "An Improved Process for the Preparation of Highly Pure Solifenacin Succinate via Resolution through Diastereomeric Crystallisation", ORGANIC PROCESS RESEARCH AND DEVELOPMENT, vol. 18, no. 8, 11 July 2014 (2014-07-11), pages 934-940, XP55575040, US ISSN: 1083-6160, DOI: 10.1021/op500083y
- NAVNATH C. NIPHADE ET AL: "Efficient and single pot process for the preparation of enantiomerically pure solifenacin succinate, an antimuscarinic agent", MONATSHEFTE FÜR CHEMIE = CHEMICAL MONTHLY, vol. 142, no. 11, 1 November 2011 (2011-11-01), pages 1181-1186, XP055551830, Vienna ISSN: 0026-9247, DOI: 10.1007/s00706-011-0610-7
- NAITO RYO ET AL: "Synthesis and Antimuscarinic Properties of Quinuclidin-3-yl 1,2,3,4-Tetrahydroisoquinoline-2-carboxyla te Derivatives as Novel Muscarinic Receptor Antagonists", JOURNAL OF MEDICINAL CHEMISTRY, AMERICAN CHEMICAL SOCIETY, US, vol. 48, 20 October 2005 (2005-10-20), pages 6597-6606, XP002435582, ISSN: 0022-2623, DOI: 10.1021/JM050099Q
- OSTOPOVICI LILIANA ET AL: "Exploring the binding site of the human muscarinic M3 receptor: homology modeling and docking study", INTERNATIONAL JOURNAL OF QUANTUM CHEMISTRY, WILEY, vol. 107, no. 8, 3 January 2007 (2007-01-03), pages 1794-1802, XP009512245, ISSN: 1097-461X, DOI: 10.1002/QUA.21290 [retrieved on 2007-03-22]

## Description

### BACKGROUND

Mutations in the GRN gene cause Frontotemporal dementia (FTD), e.g., by degeneration of the frontal lobes, Frontotemporal lobar degeneration (FTLD) (see, e.g., Cruts et al., Granulin Mutations Associated with Frontotemporal Lobar Degeneration and Related Disorders: An Update, Hum Mutat, 2008 and Baker et al., Nature, 2006.) FTD-associated mutations in GRN result in a reduction of progranulin protein expression, which suggests that haploinsufficiency of progranulin is the critical pathogenic factor in FTD-GRN. Plasma and CSF progranulin levels are reduced by up to 70% in pathogenic GRN mutation carriers (Ghidoni, et al., Neurodegen Dis, 2012). More than 60 non-sense mutations in the GRN gene have been described. Plasma can be easily monitored for PGRN (see e.g., Meeter, Nature Neurology, volume 13, 2017). Thus, granulin- and/or progranulin-associated disorders can be modulated by compounds which increase progranulin levels and/or activity.

All known FTD-GRN-associated mutations cause haploinsufficiency of progranulin, suggesting that restoration of proper progranulin levels or progranulin protein function will be therapeutically beneficial for FTD-GRN patients. Several studies have shown that even subtle reductions in progranulin levels by genetic modifiers (e.g., TMEM106B, SLPI, Rs5848) have significant effects on the age-of-onset of FTD, increase the risk of developing FTD, or worsen the course of autoimmune diseases such as osteoarthritis (see, e.g., Nicholson et al., J Neurochem, 2013; Cruchaga et al., Arch Neurol, 2012; and Wei et al, Plos One, 2014). Polymorphisms that affect progranulin levels have also been identified as genetic modifiers of several other neurodegenerative diseases, such as Alzheimer's disease and C9orf72-linked FTD (see, e.g., Sheng et al., Gene, 2014 and van Blitterswijk et al., Mol Neurodegen, 2014).

Granulins are a family of secreted and glycosylated proteins. They are cleaved from a common precursor protein called progranulin (PGRN). Progranulin is a secreted glycoprotein and is expressed in neurons, neuroglia, chondrocytes, epithelial cells and leukocytes (Toh H et al. J Mol Neurosci 201-1 Nov; 45(3):538-48). It is a precursor protein with an N-terminal signal peptide and seven and one half granulin motifs. Each of these granulin motifs contains 12 cysteines, which are responsible for 6 disulfide bridges in every granulin (Bateman A et al. Bioessays 2009:1245-54). Progranulin is coded by the GRN gene. Mutations in the GRN gene have been implicated in up to 25% of frontotemporal lobar degeneration, inherited in an autosomal dominant fashion with high penetrance (see, e.g., Mackenzie, Acta Neuropathologica, 114(1): 49-54 (2007)). Thus, modulation of progranulin levels or activity is an attractive target for treating disorders associated with GRN activity or GRN-gene mutations.
WO 2015/143300 describes isoxazole treatments for frontotemporal dementia which increase progranulin expression.

### SUMMARY

Provided herein are compounds, or pharmaceutically acceptable salts thereof, having a structure as defined in claim 1.

Also provided herein are compounds as listed in Table A and Table B, or pharmaceutically acceptable salts thereof.

Further provided are compositions comprising a compound as disclosed herein and a pharmaceutically acceptable excipient.

Also provided are use of a compound as disclosed herein as a medicament for the modulation of progranulin, e.g., to increase progranulin secretion.

Compounds defined herein are also useful for modulating progranulin in a subject in need thereof.

Compounds defined herein are also useful for treating a progranulin-associated disorder in a subject in need thereof. In some cases, the the progranulin-associated disorder is Alzheimer's disease (AD), Parkinson's disease (PD), Amyotrophic lateral sclerosis (ALS), Frontotemporal dementia (FTD), Frontotemporal dementia -Granulin subtype (FTD-GRN), Lewy body dementia (LBD), Prion disease, Motor neuron diseases (MND), Huntington's disease (HD), Spinocerebellar ataxia (SCA), Spinal muscular atrophy (SMA), a lysosomal storage disease, a disease associated with inclusions and/or misfunction of C9orf72, TDP-43, FUS, UBQLN2, VCP, CHMP28, and/or MAPT, an acute neurological disorder, glioblastoma, or neuroblastoma.

### DETAILED DESCRIPTION

Provided herein are compounds that modulate progranulin levels and can be useful as therapeutics for granulin (GRN)- and/or progranulin (PGRN)-associated disorders.

### Compounds as Progranulin Modulators

Provided herein are compounds that can modulate progranulin production and/or levels. Compounds of the disclosure have a structure of Formula (IA) as defined in claim 1.

As used herein, the term "alkyl" refers to straight chained and branched saturated hydrocarbon groups containing, e.g., one to thirty carbon atoms, for example, one to twenty carbon atoms, or one to ten carbon atoms. The term Cₙ means the alkyl group has "n" carbon atoms. For example, C₄ alkyl refers to an alkyl group that has 4 carbon atoms. C₁-₇ alkyl refers to an alkyl group having a number of carbon atoms encompassing the entire range (e.g., 1 to 7 carbon atoms), as well as all subgroups (e.g., 1-6, 2-7, 1-5, 3-6, 1, 2, 3, 4, 5, 6, and 7 carbon atoms). Non-limiting examples of alkyl groups include, methyl, ethyl, n-propyl, isopropyl, n-butyl, sec-butyl (2-methylpropyl), t-butyl (1,1-dimethylethyl), 3,3-dimethylpentyl, and 2-ethylhexyl. Unless otherwise indicated, an alkyl group can be an unsubstituted alkyl group or a substituted alkyl group.

The term "alkylene" used herein refers to an alkyl group having a substituent. For example, the term "alkylenehalo" refers to an alkyl group substituted with a halo group. For example, an alkylene group can be -CH₂CH₂- or -CH₂-. The term Cₙ means the alkylene group has "n" carbon atoms. For example, C₁₋₆ alkylene refers to an alkylene group having a number of carbon atoms encompassing the entire range, as well as all subgroups, as previously described for "alkyl" groups. Unless otherwise indicated, an alkylene group can be an unsubstituted alkylene group or a substituted alkylene group.

As used herein, the term "alkoxy" refers to a straight or branched hydrocarbon group which has an oxygen atom as the point of attachment. The alkoxy group can further comprise additional oxygen atoms in the hydrocarbon backbone, e.g., 1, 2, 3, or 4 additional oxygen atoms.

As used herein, the term "cycloalkyl" refers to an aliphatic cyclic hydrocarbon group containing three to ten carbon atoms (e.g., 3, 4, 5, 6, 7, 8, 9, or 10 carbon atoms). A cycloalkyl moiety can alternatively be referred to as a carbocycle. The term Cₙ means the cycloalkyl group has "n" carbon atoms. For example, C₅ cycloalkyl refers to a cycloalkyl group that has 5 carbon atoms in the ring. C₆-C₁₀ cycloalkyl refers to cycloalkyl groups having a number of carbon atoms encompassing the entire range (e.g., 6 to 10 carbon atoms), as well as all subgroups (e.g., 6-7, 6-8, 7-8, 6-9, 6, 7, 8, 9, and 10 carbon atoms). Nonlimiting examples of cycloalkyl groups include cyclopropyl, cyclobutyl, cyclopentyl, cyclohexyl, cycloheptyl, and cyclooctyl. The cycloalkyl groups described herein can be isolated or fused to another cycloalkyl group, a heterocycloalkyl group, an aryl group and/or a heteroaryl group. When a cycloalkyl group is fused to another cycloalkyl group, then each of the cycloalkyl groups can contain three to ten carbon atoms unless specified otherwise. Unless otherwise indicated, a cycloalkyl group can be unsubstituted or substituted.

As used herein, the term "heterocycloalkyl" is defined similarly as cycloalkyl, except the ring contains one to three heteroatoms independently selected from oxygen, nitrogen, and sulfur. A heterocycloalkyl moiety can alternatively be referred to as a heterocycle. In particular, the term "heterocycloalkyl" refers to a ring containing a total of three to ten atoms (e.g., five to ten), of which 1, 2, 3 or three of those atoms are heteroatoms independently selected from the group consisting of oxygen, nitrogen, and sulfur, and the remaining atoms in the ring are carbon atoms. Nonlimiting examples of heterocycloalkyl groups include piperdine, pyrazolidine, tetrahydrofuran, tetrahydropyran, dihydrofuran, morpholine, and the like. Cycloalkyl and heterocycloalkyl groups can be saturated or partially unsaturated ring systems optionally substituted with, for example, one to three groups, independently selected alkyl, alkyleneOH, C(O)NH₂, NH₂, oxo (=O), aryl, alkylenehalo, halo, and OH. Other substituents are also contemplated, including C₀₋₃ alkylene-halo, C₀₋₃ alkylene-CN, C₀₋₃ alkylene-NH₂, C₀₋₃ alkylene-OH, and C₀₋₃ alkylene-O-C₁₋₃alkyl. Heterocycloalkyl groups optionally can be further N-substituted with alkyl, alkylene-OH, alkylenearyl, and alkyleneheteroaryl. The heterocycloalkyl groups described herein can be isolated or fused to another heterocycloalkyl group, a cycloalkyl group, an aryl group, and/or a heteroaryl group. When a heterocycloalkyl group is fused to another heterocycloalkyl group, then each of the heterocycloalkyl groups can contain three to ten total ring atoms, and one to three heteroatoms. Unless otherwise indicated, a heterocycloalkyl group can be unsubstituted or substituted.

As used herein, the term "aryl" refers to a monocyclic aromatic group, such as phenyl. Unless otherwise indicated, an aryl group can be unsubstituted or substituted with one or more, and in particular one to four groups independently selected from, for example, halo, alkyl, alkenyl, OCF₃, NO₂, CN, NC, OH, alkoxy, amino, CO₂H, CO₂alkyl, aryl, and heteroaryl. Other substituents are also contemplated, including C₀₋₃ alkylene-halo, C₀₋₃ alkylene-CN, C₀₋₃ alkylene-NH₂, C₀₋₃ alkylene-OH, and C₀₋₃ alkylene-O-C₁₋₃alkyl. Aryl groups can be isolated (e.g., phenyl) or fused to another aryl group (e.g., naphthyl, anthracenyl), a cycloalkyl group (e.g., tetraydronaphthyl), a heterocycloalkyl group, and/or a heteroaryl group. Exemplary aryl groups include, but are not limited to, phenyl, chlorophenyl, fluorophenyl, methylphenyl, methoxyphenyl, trifluoromethylphenyl, nitrophenyl, 2,4-methoxychlorophenyl, and the like.

As used herein, the term "heteroaryl" refers to a monocyclic aromatic ring having 5 to 6 total ring atoms, and containing one to four heteroatoms selected from nitrogen, oxygen, and sulfur atom in the aromatic ring. Unless otherwise indicated, a heteroaryl group can be unsubstituted or substituted with one or more, and in particular one to four, substituents selected from, for example, halo, alkyl, alkenyl, OCF₃, NO₂, CN, NC, OH, alkoxy, amino, CO₂H, CO₂alkyl, aryl, and heteroaryl. Other substituents are also contemplated, including C₀₋₃ alkylene-halo, C₀₋₃ alkylene-CN, C₀₋₃ alkylene-NH₂, C₀₋₃ alkylene-OH, and C₀₋₃ alkylene-O-C₁₋₃alkyl. In some cases, the heteroaryl group is substituted with one or more of alkyl and alkoxy groups. Examples of heteroaryl groups include, but are not limited to, thienyl, furyl, pyridyl, pyrrolyl, oxazolyl, triazinyl, triazolyl, isothiazolyl, isoxazolyl, imidazolyl, pyrazinyl, pyrimidinyl, thiazolyl, and thiadiazolyl.

R² can be C₆₋₁₀aryl, 5-10 membered heteroaryl comprising 1-4 ring heteroatoms selected from N, O, and S, or 5-12 membered monocyclic or bicyclic carbocycle or heterocycle, wherein the heterocycle comprises 1-4 ring heteroatoms selected from N, O, and S, and R² is optionally substituted with 1-3 R³ groups. In various cases, R² is phenyl, e.g., phenyl substituted with 1-2 fluoro and/or chloro groups. In some cases, R² is pyridyl, pyrazine, pyrimidine, thiophene, benzothiophene, benzofuranyl, piperidinone, indole, indazole, benzodiazole, benzoimidazole, benzoxazole, benzothiazole, indolizine, imidazo-[1,2-a]pyridine, imidazo-[1,5-a]pyridine, or imidazo-[1,2-a]pyrimidine. In various cases, R² is unsubstituted. In some cases, R² is substituted with 1 R³. In some cases, R² is substituted with 2 R³. In some cases, R² is substituted with 3 R³.

In some specific cases, R² is selected from the group consisting of : 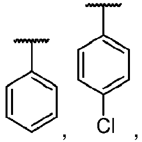 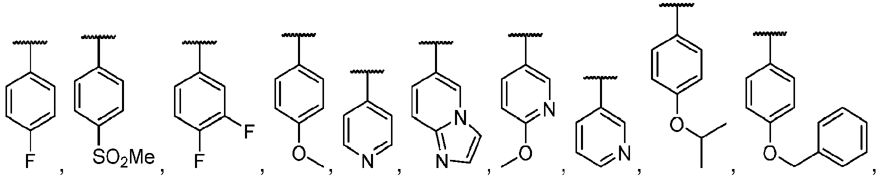 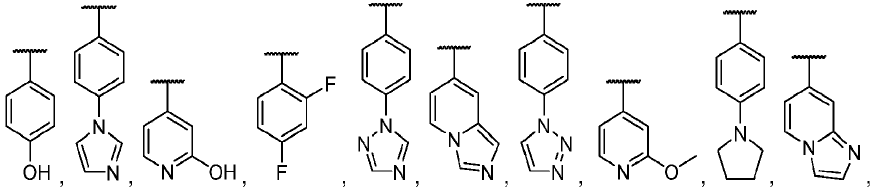 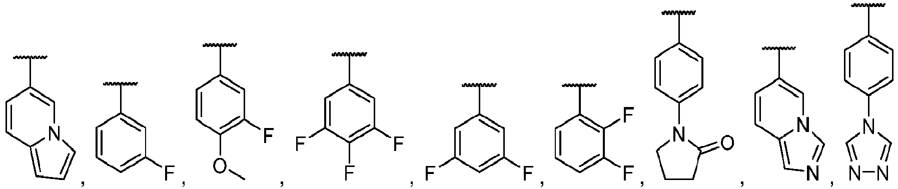 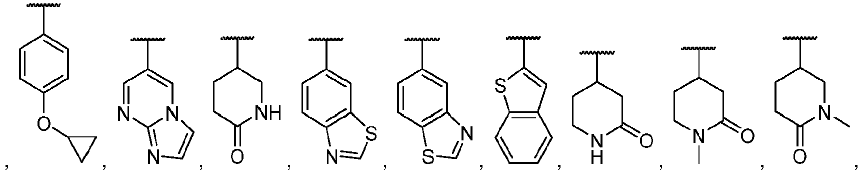 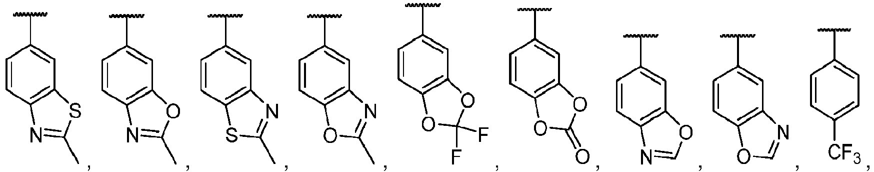 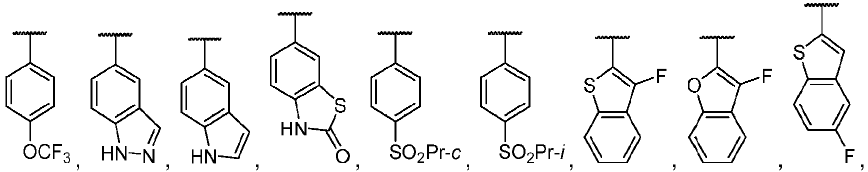 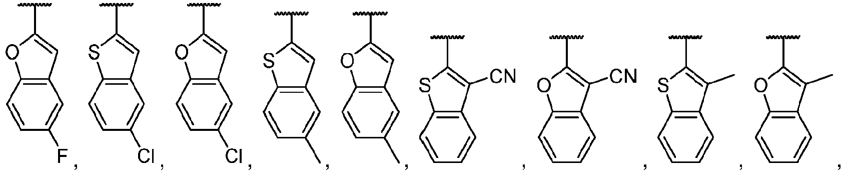 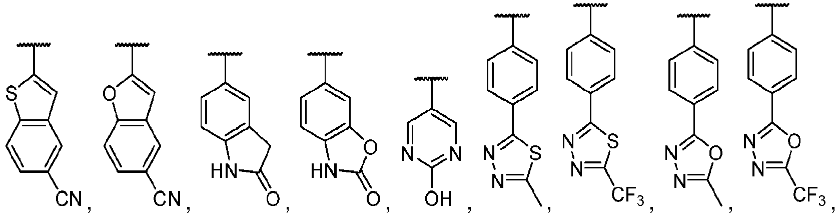 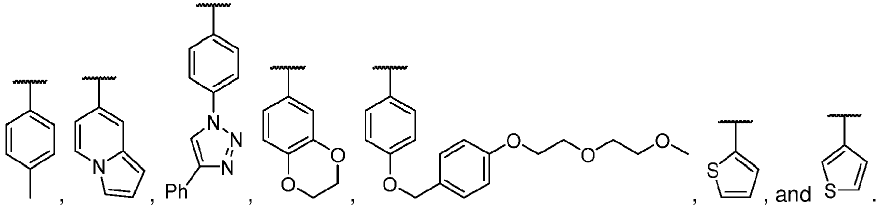 In some cases, R² is:

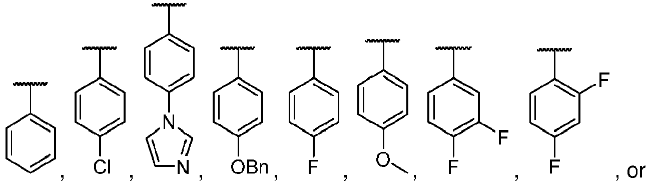

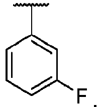

Compounds of the disclosure are of a structure of Formula (IA) 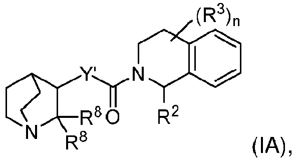 wherein Y' is CH₂ or NH and each R⁸ is independently H or Me, or both R⁸ taken together with the carbon to which they are attached form a cyclopropyl ring. In some cases, the structure of Formula (IA) can be 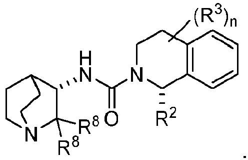 The structure can alternatively be 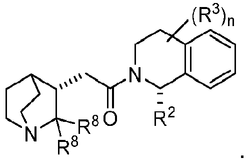 In various cases, the structure of Formula (IA) can be wherein one R⁸ is H and the other Me. In some cases, each R⁸ is Me. In various cases, both R⁸ together with the carbon to which they are attached form a cyclopropyl ring.

Some specifically contemplated compounds of the disclosure include those as shown in Table A below, or a pharmaceutically acceptable salt thereof.

**Table A**

| Strucutre | Comp. ID # |
|---|---|
| 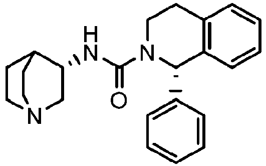 | 1047 |
| 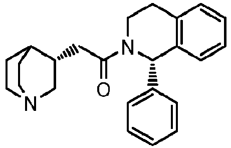 | 1048 |
| 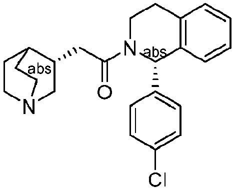 | 1049 |
| 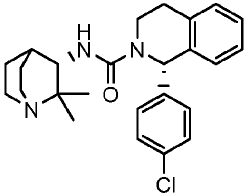 | 1050 |
| 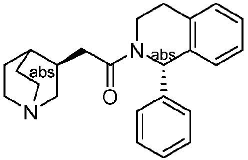 | 1051 |
| 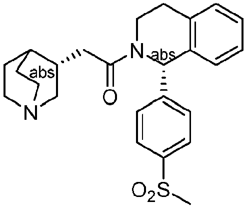 | 1052 |
| 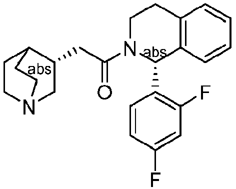 | 1053 |
| 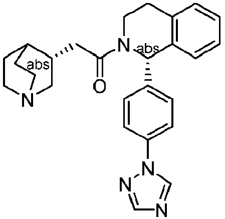 | 1054 |
| 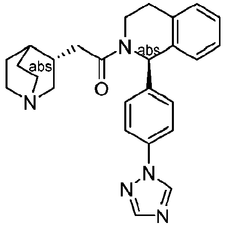 | 1055 |
| 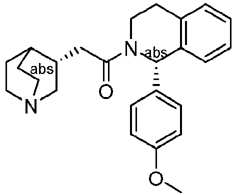 | 1056 |
| 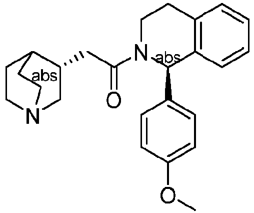 | 1057 |
| 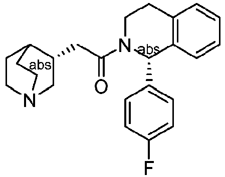 | 1058 |
| 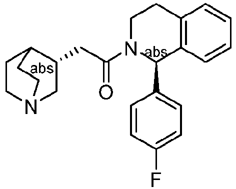 | 1059 |
| 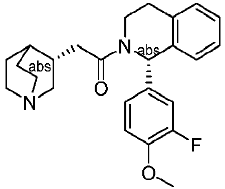 | 1060 |
| 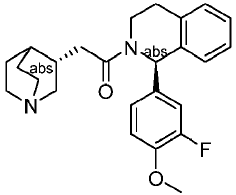 | 1061 |
| 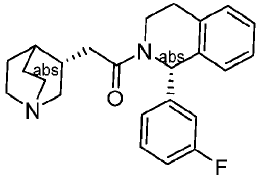 | 1062 |
| 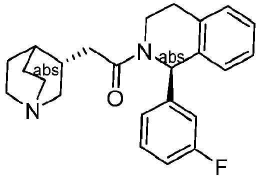 | 1063 |
| 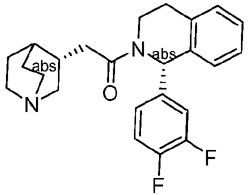 | 1064 |
| 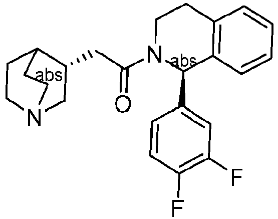 | 1065 |
| 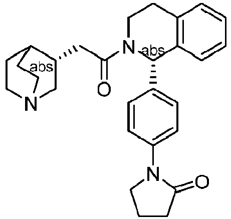 | 1066 |
| 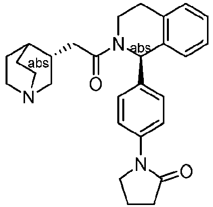 | 1067 |
| 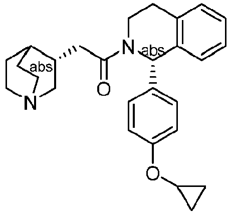 | 1068 |
| 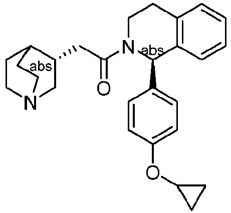 | 1069 |
| 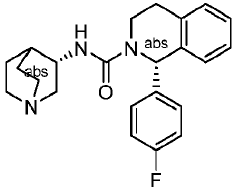 | 1070 |
| 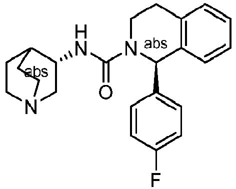 | 1071 |
| 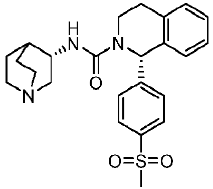 | 1072 |
| 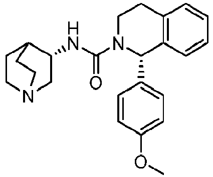 | 1073 |
| 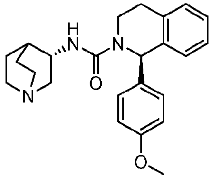 | 1074 |
| 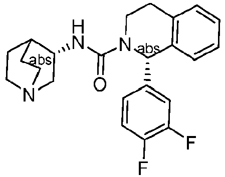 | 1075 |
| 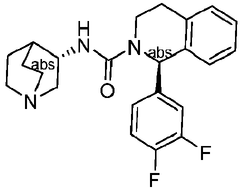 | 1076 |
| 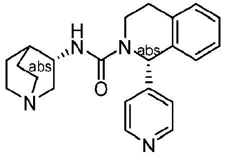 | 1077 |
| 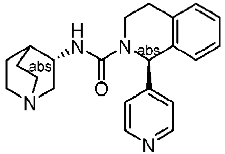 | 1078 |
| 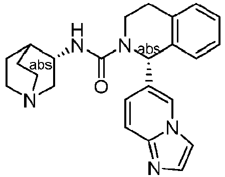 | 1079 |
| 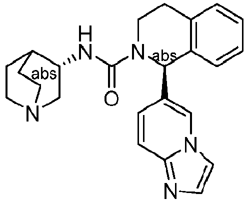 | 1080 |
| 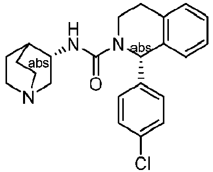 | 1081 |
| 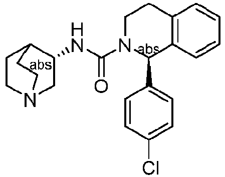 | 1082 |
| 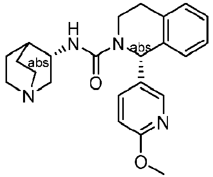 | 1083 |
| 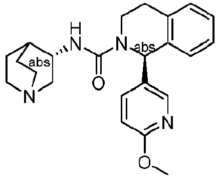 | 1084 |
| 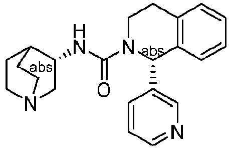 | 1085 |
| 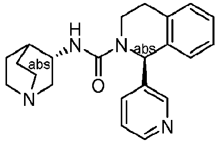 | 1086 |
| 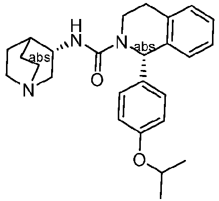 | 1087 |
| 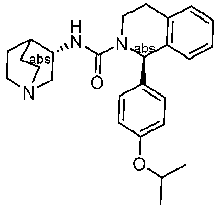 | 1088 |
| 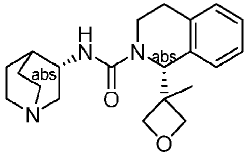 | 1089 |
| 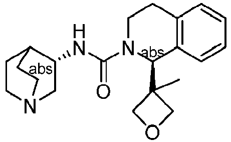 | 1090 |
| 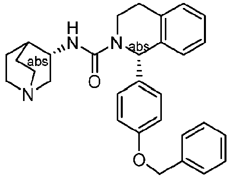 | 1091 |
| 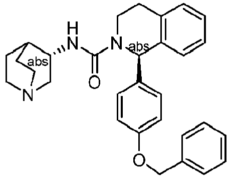 | 1092 |
| 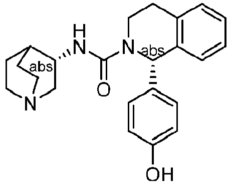 | 1093 |
| 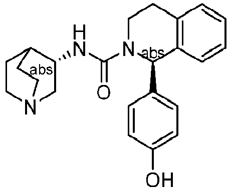 | 1094 |
| 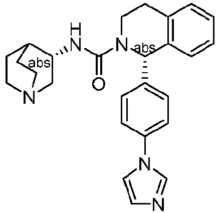 | 1095 |
| 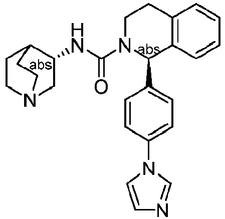 | 1096 |
| 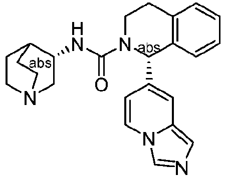 | 1097 |
| 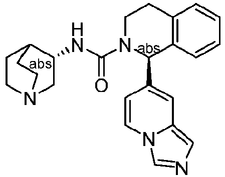 | 1098 |
| 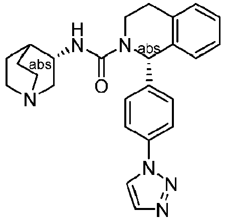 | 1099 |
| 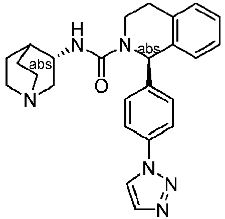 | 1100 |
| 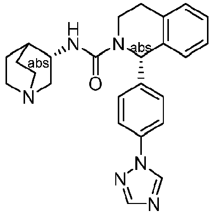 | 1101 |
| 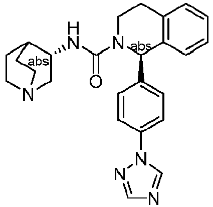 | 1102 |
| 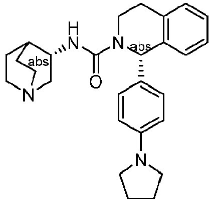 | 1103 |
| 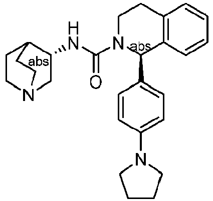 | 1104 |
| 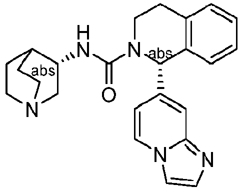 | 1105 |
| 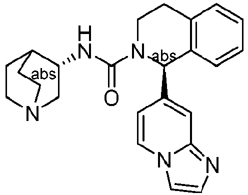 | 1106 |
| 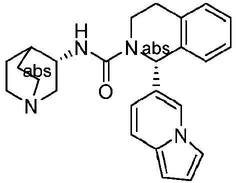 | 1107 |
| 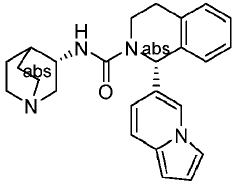 | 1108 |
| 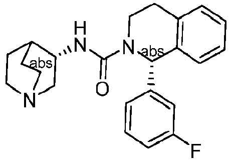 | 1109 |
| 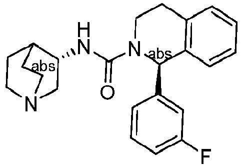 | 1110 |
| 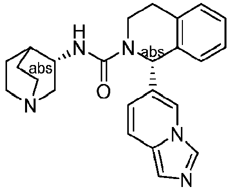 | 1111 |
| 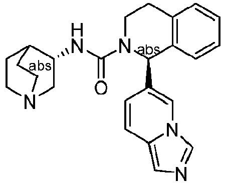 | 1112 |
| 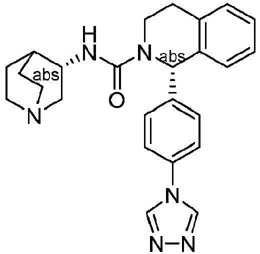 | 1113 |
| 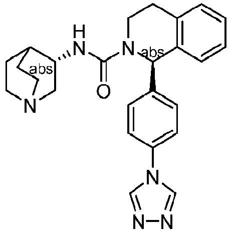 | 1114 |
| 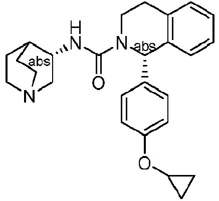 | 1115 |
| 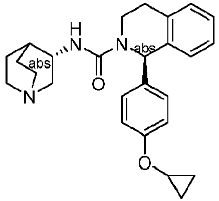 | 1116 |
| 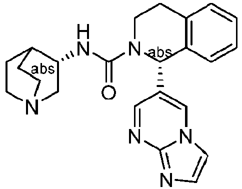 | 1117 |
| 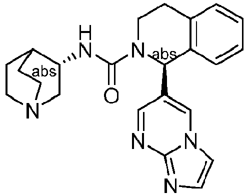 | 1118 |
| 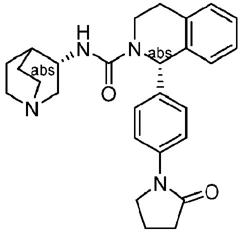 | 1119 |
| 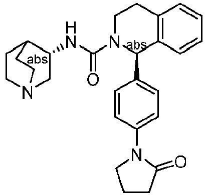 | 1120 |
| 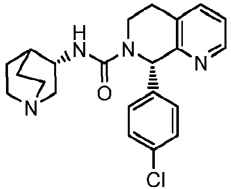 | 1121 |
| 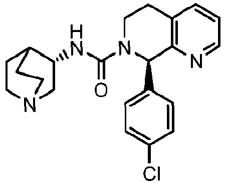 | 1122 |
| 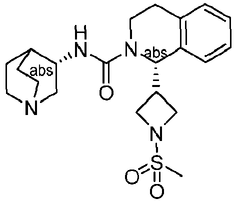 | 1123 |
| 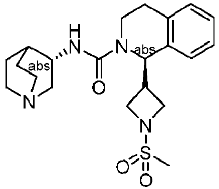 | 1124 |
| 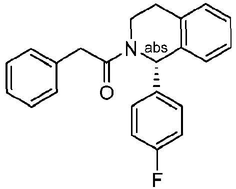 | 1125 |
| 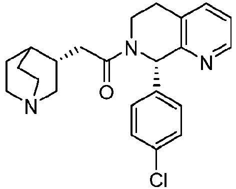 | 1151 |
| 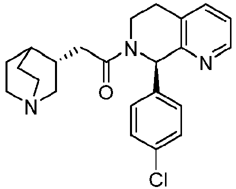 | 1152 |

In various cases, a compound of the disclosure is one as listed in Table B, or a pharmaceutically acceptable salt thereof.

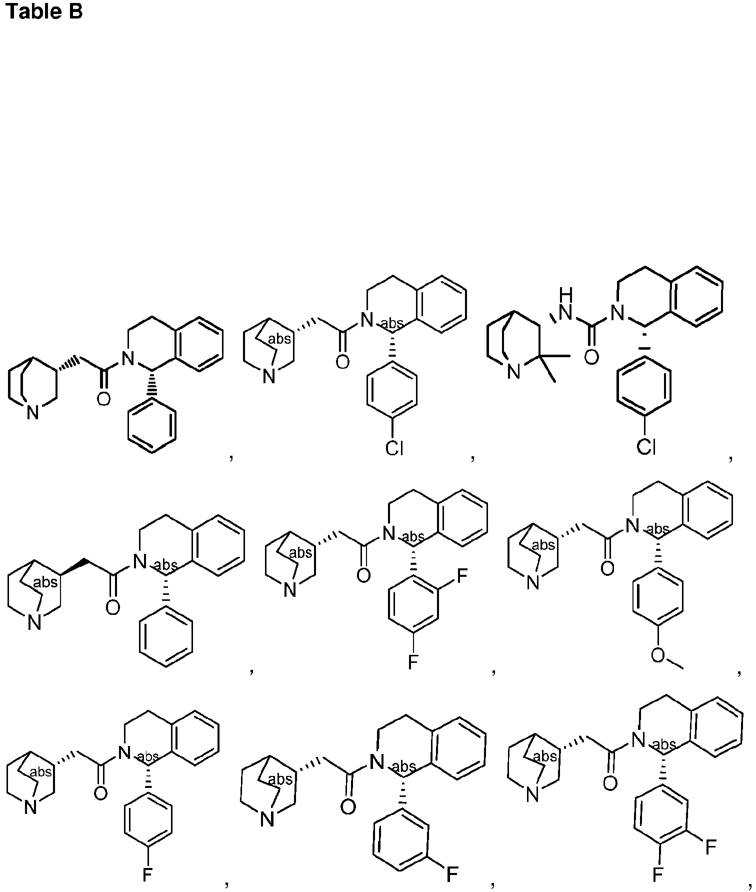

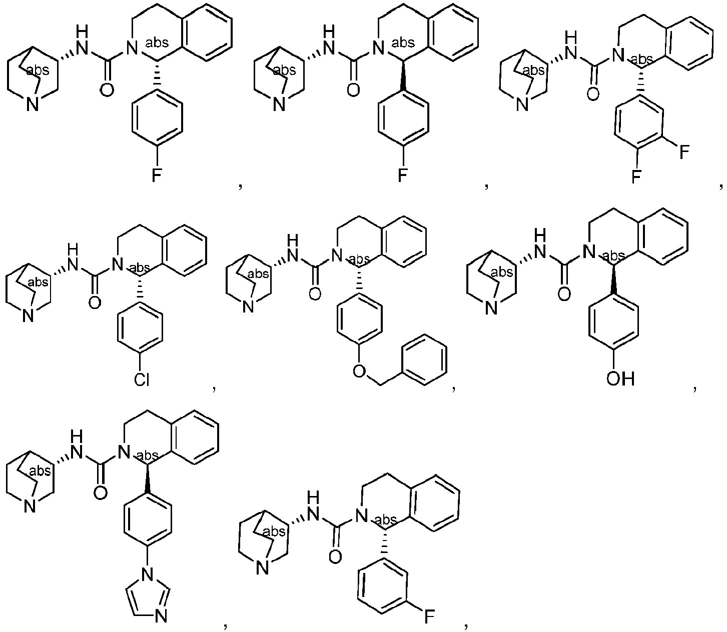

In various cases, the compound of the disclosure is a compound, or pharmaceutically acceptable salt thereof, selected from the group consisting of

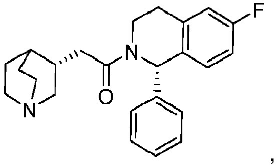

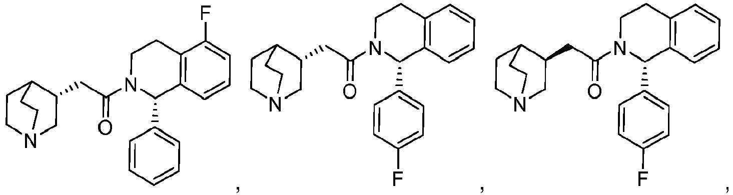

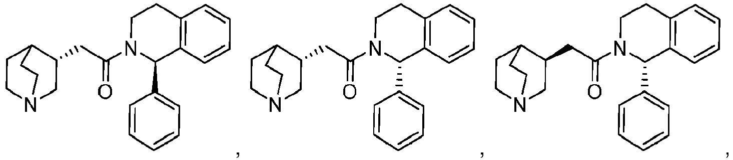

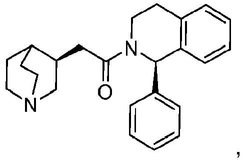

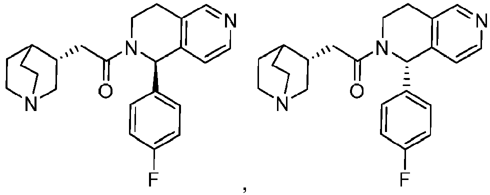

As used herein, the term "substituted," when used to modify a chemical functional group, refers to the replacement of at least one hydrogen radical on the functional group with a substituent. Substituents can include, but are not limited to, alkyl, cycloalkyl, alkenyl, cycloalkenyl, alkynyl, heterocycloalkyl, aryl, heteroaryl, hydroxyl, oxy, alkoxy, heteroalkoxy, ester, thioester, carboxy, cyano, nitro, amino, amido, acetamide, and halo (e.g., fluoro, chloro, bromo, or iodo). When a chemical functional group includes more than one substituent, the substituents can be bound to the same carbon atom or to two or more different carbon atoms.

The compounds disclosed herein include all pharmaceutically acceptable isotopically-labeled compounds wherein one or more atoms of the compounds disclosed herein are replaced by atoms having the same atomic number, but an atomic mass or mass number different from the atomic mass or mass number usually found in nature, examples of which include isotopes of hydrogen, such as ²H and ³H. In some cases, one or more hydrogen atoms of the compounds disclosed herein are specifically deuterium (²H).

As used herein, the term "pharmaceutically acceptable" means that the referenced substance, such as a compound of the present disclosure, or a formulation containing the compound, or a particular excipient, are safe and suitable for administration to a patient or subject. The term "pharmaceutically acceptable excipient" refers to a medium that does not interfere with the effectiveness of the biological activity of the active ingredient(s) and is not toxic to the host to which it is administered.

The compounds disclosed herein can be as a pharmaceutically acceptable salt. As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al. describe pharmaceutically acceptable salts in detail in J. Pharmaceutical Sciences, 1977, 66, 1-19, which is incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, glutamate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts of compounds containing a carboxylic acid or other acidic functional group can be prepared by reacting with a suitable base. Such salts include, but are not limited to, alkali metal, alkaline earth metal, aluminum salts, ammonium, N+(C₁₋₄alkyl)₄ salts, and salts of organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, N,N'-bisdehydroabietylamine, glucamine, N-methylglucamine, collidine, quinine, quinoline, and basic amino acids such as lysine and arginine. This invention also envisions the quaternization of any basic nitrogen-containing groups of the compounds disclosed herein. Water or oil-soluble or dispersible products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

### Pharmaceutical Formulations, Dosing, and Routes of Administration

Further provided are pharmaceutical formulations comprising a compound as described herein or pharmaceutically acceptable salt thereof, and a pharmaceutically acceptable excipient.

The compounds described herein can be administered to a subject in a therapeutically effective amount, alone or as part of a pharmaceutically acceptable composition or formulation. In addition, the compounds can be administered all at once, multiple times, or delivered substantially uniformly over a period of time. It is also noted that the dose of the compound can be varied over time.

A particular administration regimen for a particular subject will depend, in part, upon the compound, the amount of compound administered, the route of administration, and the cause and extent of any side effects. The amount of compound administered to a subject (e.g., a mammal, such as a human) in accordance with the disclosure should be sufficient to affect the desired response over a reasonable time frame. Dosage typically depends upon the route, timing, and frequency of administration. Accordingly, the clinician titers the dosage and modifies the route of administration to obtain the optimal therapeutic effect, and conventional range-finding techniques are known to those of ordinary skill in the art.

Purely by way of illustration, the method comprises administering, for example, from about 0.1 mg/kg up to about 100 mg/kg of compound or more, depending on the factors mentioned above. In other embodiments, the dosage ranges from 1 mg/kg up to about 100 mg/kg; or 5 mg/kg up to about 100 mg/kg; or 10 mg/kg up to about 100 mg/kg. Some conditions require prolonged treatment, which may or may not entail administering lower doses of compound over multiple administrations. If desired, a dose of the compound is administered as two, three, four, five, six or more sub-doses administered separately at appropriate intervals throughout the day, optionally, in unit dosage forms. The treatment period will depend on the particular condition, and may last one day to several months.

Suitable methods of administering a physiologically-acceptable composition, such as a pharmaceutical composition comprising the compounds disclosed herein are well known in the art. Although more than one route can be used to administer a compound, a particular route can provide a more immediate and more effective reaction than another route. Depending on the circumstances, a pharmaceutical composition comprising the compound is applied or instilled into body cavities, absorbed through the skin or mucous membranes, ingested, inhaled, and/or introduced into circulation. For example, in certain circumstances, it will be desirable to deliver a pharmaceutical composition comprising the agent orally, through injection by intravenous, intraperitoneal, intracerebral (intra-parenchymal), intracerebroventricular, intramuscular, intra-ocular, intraarterial, intraportal, intralesional, intramedullary, intrathecal, intraventricular, transdermal, subcutaneous, intraperitoneal, intranasal, enteral, topical, sublingual, urethral, vaginal, or rectal means, by sustained release systems, or by implantation devices. If desired, the compound is administered regionally via intrathecal administration, intracerebral (intra-parenchymal) administration, intracerebroventricular administration, or intraarterial or intravenous administration feeding the region of interest. Alternatively, the composition is administered locally via implantation of a membrane, sponge, or another appropriate material onto which the desired compound has been absorbed or encapsulated. Where an implantation device is used, the device is, in one aspect, implanted into any suitable tissue or organ, and delivery of the desired compound is, for example, via diffusion, timed-release bolus, or continuous administration.

To facilitate administration, the compound is, in various aspects, formulated into a physiologically-acceptable composition comprising a carrier (e.g., vehicle, adjuvant, or diluent). The particular carrier employed is limited only by physico-chemical considerations, such as solubility and lack of reactivity with the compound, and by the route of administration. Physiologically- acceptable carriers are well known in the art. Illustrative pharmaceutical forms suitable for injectable use include sterile aqueous solutions or dispersions and sterile powders for the extemporaneous preparation of sterile injectable solutions or dispersions (for example, see U.S. Patent No. 5,466,468). Injectable formulations are further described in, e.g., Pharmaceutics and Pharmacy Practice, J. B. Lippincott Co., Philadelphia. Pa., Banker and Chalmers, eds., pages 238-250 (1982), and ASHP Handbook on Injectable Drugs, Toissel, 4th ed., pages 622-630 (1986)). A pharmaceutical composition comprising the compound is, in one aspect, placed within containers, along with packaging material that provides instructions regarding the use of such pharmaceutical compositions. Generally, such instructions include a tangible expression describing the reagent concentration, as well as, in certain embodiments, relative amounts of excipient ingredients or diluents (e.g., water, saline or PBS) that may be necessary to reconstitute the pharmaceutical composition.

Compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents, or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Microorganism contamination can be prevented by adding various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents delaying absorption, for example, aluminum monostearate and gelatin.

Solid dosage forms for oral administration include capsules, tablets, powders, and granules. In such solid dosage forms, the active compound is admixed with at least one inert customary excipient (or carrier) such as sodium citrate or dicalcium phosphate or (a) fillers or extenders, as for example, starches, lactose, sucrose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (a) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, and tablets, the dosage forms may also comprise buffering agents. Solid compositions of a similar type may also be used as fillers in soft and hard filled gelatin capsules using such excipients as lactose or milk sugar, as well as high molecular weight polyethylene glycols, and the like.

Solid dosage forms such as tablets, dragees, capsules, pills, and granules can be prepared with coatings and shells, such as enteric coatings and others well known in the art. The solid dosage forms may also contain opacifying agents. Further, the solid dosage forms may be embedding compositions, such that they release the active compound or compounds in a certain part of the intestinal tract in a delayed manner. Examples of embedding compositions that can be used are polymeric substances and waxes. The active compound can also be in micro-encapsulated form, optionally with one or more excipients.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1 ,3-butylene glycol, dimethylformamide, oils, in particular, cottonseed oil, groundnut oil, corn germ oil, olive oil, castor oil, and sesame seed oil, glycerol, tetrahydrofurfuryl alcohol, polyethylene glycols and fatty acid esters of sorbitan, or mixtures of these substances, and the like.

Besides such inert diluents, the composition can also include adjuvants, such as wetting agents, emulsifying and suspending agents, sweetening, flavoring, and perfuming agents. Suspensions, in addition to the active compound, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal administration are preferably suppositories, which can be prepared by mixing the compounds of the disclosure with suitable non-irritating excipients or carriers such as cocoa butter, polyethylene glycol or a suppository wax, which are solid at ordinary room temperature, but liquid at body temperature, and therefore, melt in the rectum or vaginal cavity and release the active component.

The compositions used in the methods of the invention may be formulated in micelles or liposomes. Such formulations include sterically stabilized micelles or liposomes and sterically stabilized mixed micelles or liposomes. Such formulations can facilitate intracellular delivery, since lipid bilayers of liposomes and micelles are known to fuse with the plasma membrane of cells and deliver entrapped contents into the intracellular compartment.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration.

The frequency of dosing will depend on the pharmacokinetic parameters of the agents and the routes of administration. The optimal pharmaceutical formulation will be determined by one of skill in the art depending on the route of administration and the desired dosage. See, for example, Remington's Pharmaceutical Sciences, 18th Ed. (1990) Mack Publishing Co., Easton, PA, pages 1435-1712, incorporated herein by reference. Such formulations may influence the physical state, stability, rate of in vivo release and rate of in vivo clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein, as well as the pharmacokinetic data observed in animals or human clinical trials.

The precise dosage to be employed depends upon several factors including the host, whether in veterinary medicine or human medicine, the nature and severity of the condition, e.g., disease or disorder, being treated, the mode of administration and the particular active substance employed. The compounds may be administered by any conventional route, in particular enterally, and, in one aspect, orally in the form of tablets or capsules. Administered compounds can be in the free form or pharmaceutically acceptable salt form as appropriate, for use as a pharmaceutical, particularly for use in the prophylactic or curative treatment of a disease of interest. These measures will slow the rate of progress of the disease state and assist the body in reversing the process direction in a natural manner.

It will be appreciated that the pharmaceutical compositions and treatment methods of the invention are useful in fields of human medicine and veterinary medicine. Thus, the subject to be treated is in one aspect a mammal. In another aspect, the mammal is a human.

In jurisdictions that forbid the patenting of methods that are practiced on the human body, the meaning of "administering" of a composition to a human subject shall be restricted to prescribing a controlled substance that a human subject will self-administer by any technique *(e.g.,* orally, inhalation, topical application, injection, insertion, etc.). The broadest reasonable interpretation that is consistent with laws or regulations defining patentable subject matter is intended. In jurisdictions that do not forbid the patenting of methods that are practiced on the human body, the "administering" of compositions includes both methods practiced on the human body and also the foregoing activities.

### Methods of Use

Compounds as disclosed herein can affect cells to increase secretion of progranulin. Solifenacin is a drug currently used for urinary incontinence. It has been found that this compound also causes the secretion of progranulin from mouse BV2 cells. As such, compounds disclosed herein can be useful in treating disorders associated with aberrant (e.g., reduced) progranulin secretion or activity. In various cases, the compound disclosed herein increases progranulin secretion. In some cases, the compound used is solifenacin or a pharmaceutically acceptable salt thereof,

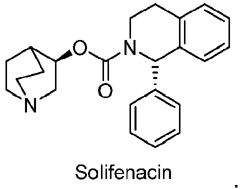

Specifically contemplated are therapeutically effective amounts of a compound disclosed herein to modulate progranulin (e.g., to increase secretion of progranulin), for use as a therapeutic in a subject. As used herein, the term "therapeutically effective amount" means an amount of a compound or combination of therapeutically active compounds (e.g., a progranulin modulator or combination of modulators) that ameliorates, attenuates or eliminates one or more symptoms of a particular disease or condition (e.g., progranulin- or granulin-associated), or prevents or delays the onset of one of more symptoms of a particular disease or condition.

As used herein, the terms "patient" and "subject" may be used interchangeably and mean animals, such as dogs, cats, cows, horses, and sheep (e.g., non-human animals) and humans. Particular patients or subjects are mammals (e.g., humans). The terms patient and subject include males and females.

Contemplated disorders associated with aberrant progranulin activity include Alzheimer's disease (AD), Parkinson's disease (PD) and PD-related disorders, Amytrophic lateral sclerosis (ALS), Frontotemperal dementia (FTD), Lewy body dementia (LBD), Prion disease, Motor neurone diseases (MND), Huntington's disease (HD), Spinocerebellar ataxia (SCA), Spinal muscular atrophy (SMA) and other neurodegenerative diseases. Other disorders contemplated include lysosomal dys-or misfunction disorders, such as lysosomal storage diseases (e.g., Paget disease, Gaucher's disease, Nieman's Pick disease, Tay-Sachs Disease, Fabry Disease, Pompes disease, and Naso-Hakula disease). Other diseases contemplated include those associated with inclusions and/or misfunction of C9orf72, TDP-43, FUS, UBQLN2, VCP, CHMP28, and/or MAPT. Other diseases include acute neurological disorders such as stroke, cerebral hemorrhage, traumatic brain injury and other head traumas as well as diseases of the brain such as glioblastoma and neuroblastomas. In some cases, the disorder is Alzheimer's disease (AD), Parkinson's disease (PD), Amyotrophic lateral sclerosis (ALS), Frontotemporal dementia (FTD), Frontotemporal dementia -Granulin subtype (FTD-GRN), Lewy body dementia (LBD), Prion disease, Motor neuron diseases (MND), Huntington's disease (HD), Spinocerebellar ataxia (SCA), Spinal muscular atrophy (SMA), a lysosomal storage disease, a disease associated with inclusions and/or misfunction of C9orf72, TDP-43, FUS, UBQLN2, VCP, CHMP28, and/or MAPT, an acute neurological disorder, glioblastoma, or neuroblastoma. In some cases, the lysosomal storage disease is Paget disease, Gaucher's disease, Nieman's Pick disease, Tay-Sachs Disease, Fabry Disease, Pompes disease, or Naso-Hakula disease. In various cases, the acute neurological disorder is stroke, cerebral hemorrhage, traumatic brain injury or head trauma. In various cases, the progranulin-associated disorder is Frontotemporal dementia (FTD). In some cases, the progranulin-associated disorder is Frontotemporal dementia -Granulin subtype (FTD-GRN).

### Synthesis of Compounds disclosed herein

Compounds can be synthesized in using typical synthetic chemistry techniques, well within the skill of the ordinarily skilled synthetic chemist. Generally, the synthesis of the disclosed compounds can be achieved following similar synthesis as detailed in WO 96/20194 (Yamanouchi Pharma) and WO 2012/001481 (Aurobindo).

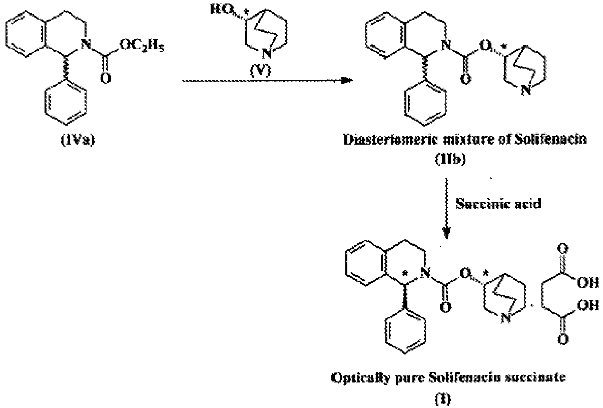

Various reference compounds having a carbamate linkage can be prepared via the following general synthetic scheme:

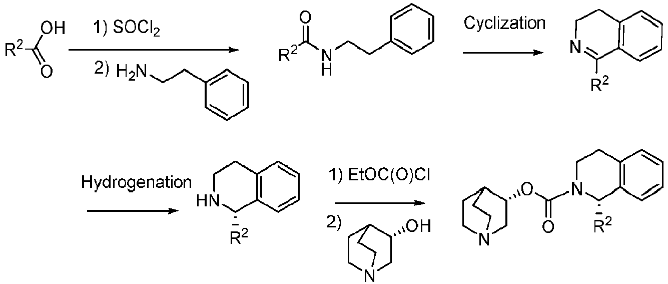

An acid having the desired R² is reacted with phenethylamine then cyclized to form a substituted dihydroisoquinoline, which is hydrogenated and reacted with, e.g., chloroformate or a carbonate, and then a hydroxy-quinuclidine to form the desired carbamate compound.

Various compounds disclosed herein having an amide linkage can be prepared via the following general synthetic scheme:

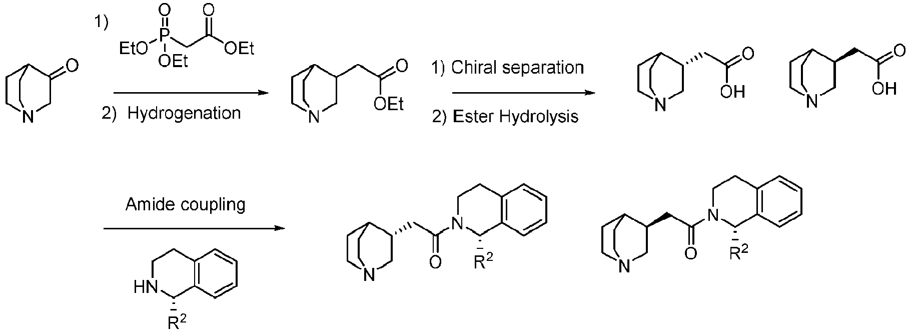

A quinuclidinone is reacted in a Horner-Wadsworth Emmons homologation then the resulting exocyclic alkene reduced. After chiral separation of the two enantiomers, the ester is hydrolyzed and reacted with an appropriate tetrahydroquinoline to form the desired amide compound.

Various compounds disclosed herein having a urea linkage can be prepared via the following general synthetic scheme:

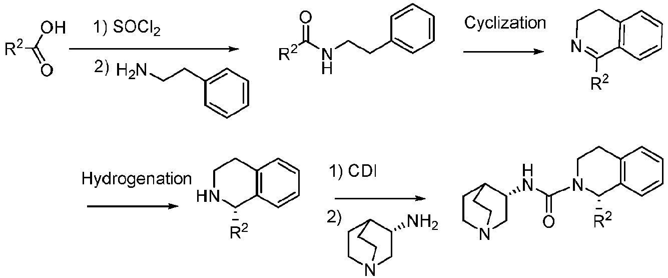

An acid having the desired R² is reacted with phenethylamine then cyclized to form a substituted dihydroisoquinoline, which is hydrogenated and reacted with, e.g., a coupling reagent such as CDI, and then an amino-quinuclidine to form the desired urea compound.

Other general synthetic guidelines are provided in view of the specific syntheses discussed in the Examples section below.

### EXAMPLES

General Methods: All ¹HNMR experiments were run in Bruker Avance III 400, at 25 °C.

Analytic methods: All Analytical-SFC experiments were run on SFC Method Station (Thar, Waters), Column temperature: 40 ºC, Mobile phase: CO₂/ Methanol (0.2% Methanol Ammonia) = Flow: 4.0 mL/min, Back Pressure: 120 Bar, Detection wavelength: 214 nm.

Preparative methods: All Preparative-SFC experiments were run on SFC-80 (Thar, Waters), Column temperature: 35 ºC, Mobile phase (example): CO₂/ Methanol (0.2% Methanol Ammonia) = Flow rate: 80 g/min, Back pressure: 100 bar, Detection wavelength: 214 nm.

Preparative Method B: Acidic reversed phase MPLC: Instrument type: Reveleris^{™} prep MPLC; Column: Phenomenex LUNA C18(3) (150x25 mm, 10 µ); Flow: 40 mL/min; Column temp: room temperature; Eluent A: 0.1% (v/v) Formic acid in water, Eluent B: 0.1% (v/v) Formic acid in acetonitrile; using the indicated gradient and wavelength.

LCMS experiments: All LCMS experiments were run on an Agilent 1200 with a column temperature of 40 °C, monitor UV absorption at 214 nm and observe a mass range from 100-1000. Individual conditions vary slightly as described in the methods below:
LCMS Method A: Column: ZORBAX SB-C18 3.0 x 50 mm, 3.5 µm; Mobile Phase: A: Water (0.1% TFA), B: ACN (0.1% TFA); Gradient: 5% B increase to 95% B over 1.3 min, stop at 3 min. Flow Rate: 1.8 mL/min
LCMS Method A1: Column: XBridge SB-C18 3.0 x 50 mm, 3.5 µm; Mobile Phase: A: Water (0.01% TFA), B: ACN (0.01% TFA); Gradient: 5% B increase to 95% B over 1.3 min, stop at 3 min. Flow Rate: 2.0 mL/min
LCMS Method A2: Column: SunFire-C18 3.0 × 50 mm, 3.5 µm; Mobile Phase: A: Water (0.01% TFA), B: ACN (0.01% TFA); Gradient: 5% B increase to 95% B over 1.3 min, stop at 3 min. Flow Rate: 2.0 mL/min
LCMS Method B: Column: XBridge C18 50 × 4.6 mm, 3.5 µm; Mobile Phase: A: Water (0.1% TFA), B: ACN (0.1% TFA); Gradient: 5% B increase to 95% B over 1.2 min, stop at 3min. Flow Rate: 2.0 mL/min
LCMS Method C: Column: XBridge SB-C18 3.0 × 50 mm, 3.5 µm; Mobile Phase: A: Water (10 mM NH₄HCO₃), B: ACN; Gradient: 5% B increase to 95% B over 1.2 min. Flow Rate: 2.0 mL/min;
LCMS Method C1: Column: XBridge SB-C18 3.0 × 50 mm, 3.5 µm; Mobile Phase: A: Water (10 mM NH₄HCO₃), B: ACN; Gradient: 5% B increase to 95% B over 1.4 min. Flow Rate: 2.0 mL/min;
LCMS Method C2: Column: XBridge C18 4.6 × 50 mm, 3.5 µm; Mobile Phase: A: Water (10 mM NH₄HCO₃), B: ACN; Gradient: 5% B increase to 95% B over 1.3 min. Flow Rate: 2.0 mL/min;
LCMS Method D: Column: XBridge SB-C18 3.0 × 50 mm, 3.5µm; Mobile Phase: A: Water (0.1% TFA), B: ACN (0.1% TFA); Gradient: 5% B increase to 95% B over 3.1 min. Flow Rate: 1.8 mL/min;
LCMS Method E: Column: XBridge SB-C18 3.0 × 50 mm, 3.5 µm; Mobile Phase: A: Water (0.1% TFA), B: ACN (0.1% TFA); Gradient: 5% B increase to 95% B over 1.8 min, stop at 3 min. Flow Rate: 1.8 mL/min;
LCMS Method F: Column: XBridge SB-C18 3.0 × 50mm, 3.5 µm; Mobile Phase: A: Water (0.1% TFA), B: ACN (0.1% TFA); Gradient: 5% B increase to 95% B over 2 min, stop at 3 min. Flow Rate: 1.8 mL/min;
LCMS Method G: Apparatus: Agilent 1260 Bin. Pump: G1312B, degasser; autosampler, ColCom, DAD: Agilent G1315D, 220-320 nm, MSD: Agilent LC/MSD G6130B ESI, pos/neg 100-1000, ELSD Alltech 3300 gas flow 1.5 mL/min, gas temp: 40°C; column: Waters XSelectTM C18, 50x2.1 mm, 3.5 µm, Temp: 35 ºC, Flow: 0.8 mL/min, Gradient: t0 = 5% A, t3.5 min = 98% A, t6 min = 98% A, Posttime: 2 min; Eluent A: 0.1% formic acid in acetonitrile, Eluent B: 0.1% formic acid in water.

### Reference Example 1: Compound 1001 Synthesis

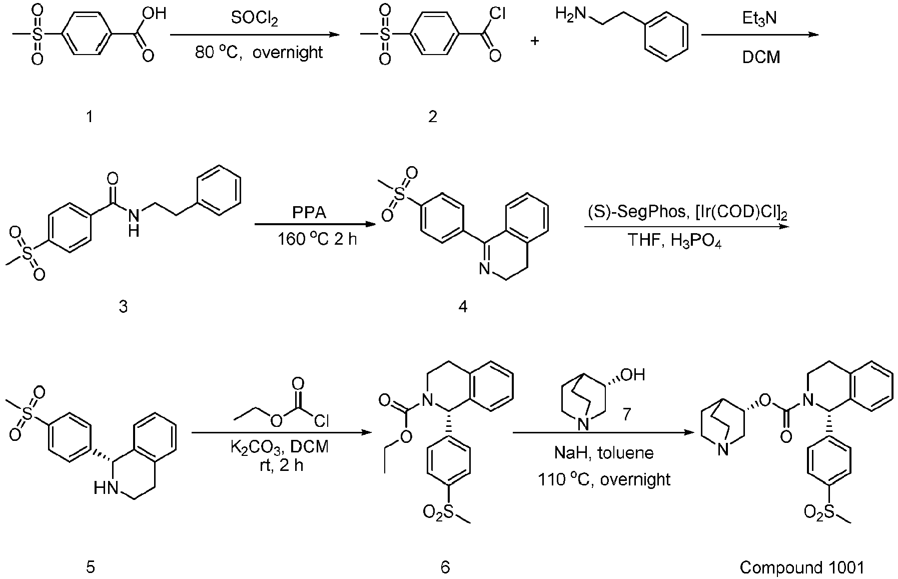

Step 1: To a solution of 4-(methylsulfonyl)benzoic acid **1** (6.0 g, 30.0 mmol) in tetrahydrofuran (20 mL) was added 5 mL of sulfurous dichloride. The reaction was stirred at 60 °C until TLC analysis indicated the total consumption of the starting material. The solvent was evaporated to give 6.54 g of 4-(methylsulfonyl)benzoyl chloride **2.**

Step 2: A mixture of 2-phenylethanamine (3.63 g, 30 mmol) and triethylamine (6.06 g, 60 mmol) in tetrahydrofuran (40 mL) was cooled to 0 °C, and then 4-(methylsulfonyl)benzoyl chloride **2** (6.54 g, 30 mmol) was added dropwise. The mixture was stirred at room temperature for 2h. The tetrahydrofuran was removed *in vacuo* and the mixture was diluted with 40 mL of water. It was extracted with three 50 mL portions of dichloromethane/methanol (20/1), then dried with Na₂SO₄. The mixture was filtered and the solvent was evaporated to give 6.1 g of 4-(methylsulfonyl)-N-phenethylbenzamide 3. LCMS: (M+H)⁺ =304 (UV 214 nm); Retention time =1.33 min. Method A

Step 3: To 4-(methylsulfonyl)-N-phenethylbenzamide **3** (6.1 g, 20 mmol) was added polyphosphoric acid (5 mL). The mixture was stirred overnight at 160 °C. The mixture was cooled to 25 °C and quenched with ice water (100 mL), alkalized with NaOH (10% aq.) to pH=11 and extracted with three 100 mL portions of dichloromethane/methanol (20/1). The combined organic layers were washed with brine (100 mL), dried and concentrated *in vacuo* to give 4.5 g of 1-(4-(methylsulfonyl)phenyl)-3,4-dihydroisoquinoline **4**. LCMS: (M+H)⁺ = 286 (254 nm); retention time = 0.99 min. Method A

Step 4: To a solution of 1-(4-(methylsulfonyl)phenyl)-3,4-dihydroisoquinoline **4** (1 g, 3.5 mmol) in THF (200 mL) was added (S)-SegPhos (100 mg, 0.16 mmol), [Ir(COD)Cl]₂ (50 mg, 0.16 mmol) and H₃PO₄ (85%)(0.5 mL) under N₂. The mixture was stirred at 60 °C for 12 hours under 60 bar H₂. The mixture was cooled to 25 °C and concentrated *in vacuo* to remove THF, alkalized to pH = 11 with NaOH (10% aq.) and extracted with three 50 mL portions of dichloromethane/methanol (20/1). The combine organic layers were washed with brine (50 mL), dried and concentrated *in vacuo* to give 733 mg of (S)-1-(4-(methylsulfonyl)phenyl)-1,2,3,4-tetrahydroisoquinoline **5 .** LCMS: (M+H)⁺ = 288 (214 nm); retention time = 1.512 min. Method C

Step 5: To a solution of (S)-1-(4-(methylsulfonyl)phenyl)-1,2,3,4-tetrahydroisoquinoline **5** (200 mg, 0.7 mmol) in DCM (5 mL) was added ethyl carbonochloridate (153 mg, 1.4 mmol),and potassium carbonate (290 mg, 2.1 mmol). The mixture was stirred at room temperature for 2 h. The mixture was diluted with 40 mL of water and extracted by three 50 mL portions of DCM. The combined organic layers were dried with Na₂SO₄ and concentrated to give (S)-ethyl 1-(4-(methylsulfonyl)phenyl)-3,4-dihydroisoquinoline-2(1H)-carboxylate **6** (251 mg). LCMS: (M+H)⁺ = 360 (254 nm); retention time = 1.902 min. Method D

Step 6: To a mixture of (S)-ethyl 1-(4-(methylsulfonyl)phenyl)-3,4-dihydroisoquinoline-2(1H)-carboxylate **6** (251 mg, 0.7 mmol) in toluene (10 mL) was added (S)-quinuclidin-3-ol (178 mg, 1.4 mmol) and NaH (112 mg, 2.8 mmol). The mixture was stirred at 110 °C for 2 h under N₂. The mixture was diluted with water (20 mL) and extracted with three 20 mL portions of dichloromethane/methanol (20/1). The combined organic layers were washed with brine (20 mL), dried and concentrated *in vacuo* to give crude product. The crude product was purified by HPLC method (Mobile Phase: A:H₂O (10 mM NH₄HCO₃) B:MeCN Gradient: 5%-95% B in 1.2min, Flow Rate: 2.0 mL/min Column : XBridge C18 50 × 4.6 mm, 3.5 µm Oven Temperature: 40 °C UV214 nm, MASS: 100-1000) to give 6.1 mg of (S)-((S)-quinuclidin-3-yl)-1-(4-(methylsulfonyl)phenyl)-3,4-dihydroisoquinoline-2(1H)-carboxylate **(Compound 1001).** LCMS: (M+H)⁺ = 441; purity = 98.4% (214 nm); retention time = 1.580 min. Method C ¹H NMR(400 MHz, CD₃OD) δ 7.79 (d, J = 8.4 Hz, 2H), 7.44 (s, 2H), 7.15 - 7.07 (m, 4H), 6.72 (s, 1H), 3.87 - 3.84 (m, 1H), 3.54 - 3.22 (m, 1H), 3.17 - 3.07 (m, 1H), 2.99 (s, 3H), 2.90 - 2.76 (m, 2H), 2.75 - 2.62 (m, 4H), 2.03 - 1.65 (m, 2H), 1.64 - 1.46 (m, 2H), 1.45 -1.11 (m, 3H). Chiral SFC: CO₂/MeOH containing 0.2% methanolic ammonia over an ENANTIOPAK^{®} AS column (4.6 × 100 mm 5µm), retention time = 1.80 min).

Compounds 1002 and 1003 were prepared analogously to 1001. The diastereomers were separated by chiral SFC eluting with CO₂/MeOH containing 0.2% methanolic ammonia over an EnantioPak^{®} AS column (4.6 × 100 mm 5µm) to give **Compound 1002** (5.7 mg, retention time = 2.0 min) and **Compound 1003** (7.5 mg, retention time = 2.82 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1002:** LCMS: (M+H)⁺ = 380; purity = 100% (214 nm); retention time = 1.328 min. Method C ¹H NMR (400 MHz, DMSO-*d₆*) δ 7.28 (d, J = 22.6 Hz, 4H), 6.11-6.12 (br, 3H), 5.33 (s, 1H), 4.65 (s, 1H), 3.85 - 3.73 (m, 1H), 3.10-3.09 (br, 1H), 2.77 (d, J = 71.4 Hz, 2H), 2.74 - 2.56 (m, 2H), 2.04 - 1.85 (m, 2H), 1.52 (d, J = 47.7 Hz, 2H), 1.24 (s, 6H), 0.85 (d, J = 6.9 Hz, 2H).

**Compound 1003:** LCMS: (M+H)⁺ = 380; purity = 100% (214 nm); retention time = 1.323 min. Method C ¹H NMR (400 MHz, DMSO-*d₆*) δ 7.53 - 7.05 (m, 4H), 6.47 - 5.64 (m, 3H), 5.32 (t, J = 4.8 Hz, 1H), 4.61 (d, J = 42.4 Hz, 1H), 3.65 (d, J = 49.6 Hz, 1H), 3.07-3.06 (br, 1H), 2.86-2.85 (br, 2H), 2.64 (d, J = 23.1 Hz, 2H), 2.11 - 1.81 (m, 2H), 1.82 - 1.42 (m, 3H), 1.41 - 1.02 (m, 5H).

Compounds 1004 and 1005 were prepared analogously to 1001. The diastereomers were separated by chiral SFC eluting with CO₂/MeOH containing 0.2% methanolic ammonia over a CHIRALPAK^{®} IG column (4.6 × 100 mm 5µm) to give **Compound 1004** (7.5 mg, retention time = 1.47 min) and **Compound 1005** (6.7 mg, retention time 2.07 min). Stereochemical assignment of (S) at quinuclidine is absolute based on starting materials from the chiral pool, stereochemical assignment at 1 position of the tetrahydroisoquinoline is assigned based on chromatographic elution order as compared to diastereomers of related analogues of known configuration.

**Compound 1004:** LCMS: (M+H)⁺ =380; purity = 100% (214 nm); retention time = 1.837 min. Method C ¹H NMR (400 MHz, DMSO-*d₆*) δ 7.24 (d, *J=* 3.1 Hz, 3H), 7.20 (d, *J* = 7.5 Hz, 1H), 7.16 (s, 1H), 7.13 (d, *J=* 8.8 Hz, 2H), 6.24 (s, 1H), 4.63 (s, 1H), 3.89 (d*, J* = 13.1 Hz, 1H), 3.08 (dd, *J=* 14.2, 8.4 Hz, 1H), 2.86 (dd, *J* = 8.8, 5.9 Hz, 2H), 2.69-2.68 (br, 2H), 2.61 (d, *J* = 15.9 Hz, 3H), 1.91-1.90 (br, 1H), 1.63- 1.52 (m, 1H), 1.46-1.45 (br, 2H), 1.23-1.22 (br, 2H).

**Compound 1005:** LCMS: (M+H)⁺ =380; purity = 100% (214 nm); retention time= 1.838 min. Method C ¹H NMR (400 MHz, DMSO-*d₆*) δ 7.23 (s, 3H), 7.21 - 7.09 (m, 4H), 6.24 (s, 1H), 4.71 - 4.58 (m, 1H), 3.93- 3.80 (m, 1H), 3.05-3.04 (br, 1H), 2.85 (d, *J* = 22.5 Hz, 2H), 2.74 - 2.54 (m, 3H), 1.91-1.90 (br, 1H), 1.69-1.68 (br, 1H), 1.58 (dd, *J* = 13.5, 9.2 Hz, 1H), 1.47-1.46 (br, 1H), 1.37 - 1.20 (m, 1H).

Compounds 1126 and 1127 were prepared analogously to 1001. The diastereomers were separated by chiral SFC eluting with CO₂/MeOH containing 0.2% methanolic ammonia over an EnantioPak^{®} IG column (4.6 × 100 mm 5µm) to give **Compound 1126** (63.7 mg, retention time = 1.26 min) and **Compound 1127** (45.7 mg, retention time = 1.99 min). Stereochemical assignment of (S) at quinuclidine is absolute based on starting materials, stereochemical assignment at the 1-position of the tetrahydroisoquinoline is assigned based on chromatographic elution order as compared to diastereomers of related analogues of known configuration.

**Compound 1126:** LCMS: (M+H)⁺ = 431; purity = 100% (214 nm); retention time = 1.677 min. Method E ¹H NMR (400 MHz, DMSO-*d₆*) δ 7.69 (d, J = 8.2 Hz, 2H), 7.48-7.47 (brs, 2H), 7.23 (d, J = 8.4 Hz, 4H), 6.31-6.30 (brs, 1H), 4.63-4.62 (brs, 1H), 3.92- 3.77 (m, 1H), 3.49-3.48 (brs, 1H), 3.06-3.05 (brs, 1H), 2.89 (d, J = 5.8 Hz, 2H), 2.78-2.54 (m, 5H), 2.01-1.80 (m, 1H), 1.56-1.55 (brs, 1H), 1.45-1.45 (brs, 1H), 1.38-1.05 (m, 2H).

**Compound 1127:** LCMS: (M+H)⁺ = 431; purity = 100% (214 nm); retention time = 1.645 min. Method E ¹H NMR (400 MHz, DMSO-*d₆*) δ 7.70 (d, J = 8.0 Hz, 2H), 7.48 (d, J = 43.6 Hz, 2H), 7.34-7.12 (m, 4H), 6.30 (s, 1H), 4.67-4.66 (brs, 1H), 3.59-3.58 (brs, 1H), 3.51-3.50 (brs, 1H), 3.09-3.08 (brs, 1H), 2.99-2.72 (m, 3H), 2.70- 2.55 (m, 3H), 2.33-2.32 (brs, 1H), 2.04-1.85 (m, 1H), 1.82-1.53 (m, 2H), 1.49 (brs, 1H), 1.39-1.22 (m, 1H).

Compounds 1128 and 1129 were prepared analogously to 1001. The diastereomers were separated by chiral SFC eluting with CO₂/MeOH containing 0.2% methanolic ammonia over an EnantioPak^{®} IG column (4.6 × 100 mm 5µm) to give **Compound 1128** (92.5 mg, retention time = 1.17 min) and **Compound1129** (102.5 mg, retention time = 1.65 min). Stereochemical assignment of (S) at quinuclidine is absolute based on starting materials, stereochemical assignment at 1-position of the tetrahydroisoquinoline is assigned based on chromatographic elution order as compared to diastereomers of related analogues of known configuration.

**Compound 1128:** LCMS: (M+H)⁺ = 447; purity = 100% (214 nm); retention time = 1.712 min. Method E ¹H NMR (400 MHz, DMSO-*d₆*) δ 7.49-7.29(m, 4H), 7.25-7.14 (m, 4H), 6.27-6.26 (brs, 1H), 4.63 (s, 1H), 3.86 (dt, J = 12.6, 5.4 Hz, 1H), 3.44-3.43 (brs, 1H), 3.16-2.98 (m, 1H), 2.89 (d, J = 5.2 Hz, 2H), 2.77-2.54 (m, 5H), 1.95-1.94 (brs, 1H), 1.56-1.55 (brs, 1H), 1.45-1.44 (brs, 1H), 1.37-1.03 (m, 2H).

**Compound 1129:** LCMS: (M+H)⁺ = 447; purity = 100% (214 nm); retention time = 1.673 min. Method E ¹H NMR (400 MHz, DMSO-*d₆*) δ 8.26-8.25 (brs, 1H), 7.48-7.30(m, 3H), 7.28-7.16 (m, 4H), 6.28-6.27 (brs, 1H), 4.72-4.71 (brs, 1H), 3.82-3.82 (brs, 1H), 3.45 (d, J = 47.4 Hz, 1H), 3.27-3.11 (m, 1H), 3.01 - 2.59 (m, 5H), 2.00-1.98 (brs, 1H), 1.86-1.61 (m, 2H), 1.56-1.55 (brs, 1H), 1.39-1.38 (brs, 1H), 1.27-1.20 (m, 1H).

Compounds 1132 and 1133 were made analogously to 1001. The diastereomers were separated by chiral SFC eluting with CO₂/MeOH containing 0.2% methanolic ammonia over an EnantioPak^{®} IG column (4.6 x 100 mm 5µm) to give **Compound 1132** (27 mg, retention time = 4.16 min) and **Compound 1133** (31.8 mg, retention time = 5.61 min). Stereochemical assignment of (S) at quinuclidine is absolute based on starting materials, stereochemical assignment at 1-position of the tetrahydroisoquinoline is assigned based on chromatographic elution order as compared to diastereomers of related analogues of known configuration.

**Compound 1132:** LCMS: (M+H)⁺ = 434; purity = 100% (214 nm); retention time = 1.502 min. Method E ¹H NMR (400 MHz, DMSO-*d₆*) δ 7.96 (d, J = 8.3 Hz, 1H), 7.71-7.70 (brs, 1H), 7.35-7.13 (m, 5H), 6.40-6.38 (brs, 1H), 4.67-4.66 (brs, 1H), 3.89-3.88 (brs, 1H), 3.13 (dd, J = 13.9, 8.3 Hz, 1H), 2.95-2.83 (m, 2H), 2.77 (s, 3H), 2.76- 2.57 (m, 5H), 1.98-1.85 (m, 1H), 1.59 (brs, 1H), 1.54- 1.38 (m, 2H), 1.36-1.29 (m, 1H), 1.26- 1.22 (m, 1H).

**Compound 1133:** LCMS: (M+H)⁺ = 434; purity = 100% (214 nm); retention time = 1.502 min. Method E ¹H NMR (400 MHz, DMSO-*d₆*) δ 7.97 (d, J = 8.3 Hz, 1H), 7.66 (d, J = 72.2 Hz, 1H), 7.40-7.15 (m, 5H), 6.37-6.36 (brs, 1H), 4.74- 4.58 (m, 1H). 3.93- 3.80 (m, 1H), 3.43-3.42 (brs, 1H), 3.05-3.05 (brs, 1H), 2.95-2.82 (m, 2H), 2.77 (s, 3H), 2.59-2.58 (brs, 3H), 2.33-2.32 (brs, 1H), 1.97-1.88 (m, 1H), 1.76-1.65 (m, 1H), 1.65-1.57 (m, 1H), 1.53-1.40 (m, 1H), 1.37-1.28 (m, 1H), 1.27 - 1.20 (m, 1H).

Compounds 1134 and 1135 were made analogously to 1001. The diastereomers were separated by chiral SFC eluting with CO₂/MeOH containing 0.2% methanolic ammonia over an EnantioPak^{®} AS column (4.6 × 100 mm 5µm) to give **Compound 1134** (30 mg, retention time = 1.67 min) and **Compound 1135** (31 mg, retention time = 2.92 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1134:** LCMS: (M+H)⁺ = 411; purity = 100% (214 nm); retention time = 1.87 min. Method C ¹H NMR (400 MHz, DMSO-*d₆*) δ 7.53- 7.31 (m, 1H), 7.21 (d, J = 2.9 Hz, 3H), 7.09 (t, J = 8.3 Hz, 2H), 6.99- 6.87 (m, 2H), 5.52- 5.32 (m, 1H), 4.63-4.62 (brs, 1H), 4.29 (dd, J = 12.4, 6.2 Hz, 1H), 4.24- 4.13 (m, 1H), 4.07 (d, J = 12.4 Hz, 1H), 3.93 (d, J = 12.6 Hz, 1H), 3.61- 3.46 (m, 1H), 3.18- 2.99 (m, 1H), 2.84 (d, J = 10.2 Hz, 2H), 2.63-2.62 (m, 5H), 1.89-1.88 (m, 1H), 1.73-1.72 (brs, 1H), 1.58 (dd, J = 13.5, 9.0 Hz, 1H), 1.47 (dt, J = 13.0, 10.0 Hz, 1H), 1.31-1.30 (m, 1H).

**Compound 1135:** LCMS: (M+H)⁺ = 411; purity = 100% (214 nm); retention time = 1.88 min. Method C ¹H NMR (400 MHz, DMSO-*d₆*) δ 7.42 (dd, J = 20.1, 17.1 Hz, 1H), 7.27-7.16 (m, 3H), 7.16 -7.04 (m, 2H), 7.02- 6.87 (m, 2H), 5.51- 5.32 (m, 1H), 4.63-4.62 (brs, 1H), 4.35- 4.26 (m, 1H), 4.25- 4.14 (m, 1H), 4.00-3.98 (m, 1H), 3.60- 3.44 (m, 1H), 3.05 (dd, J = 14.4, 7.5 Hz, 1H), 2.85 (dd, J = 14.8, 7.7 Hz, 2H), 2.64-2.63 (m, 4H), 2.00- 1.82 (m, 1H), 1.79- 1.52 (m, 2H), 1.47-1.46 (brs, 1H), 1.34- 1.25 (m, 1H).

Compounds 1138 and 1139 were made analogously to 1001. The diastereomers were separated by chiral SFC eluting with n-hexane/EtOH containing 0.1% DEA over an EnantioPak^{®} IG column (4.6 × 250 mm 5µm) to give **compound 1138** (18.4 mg, retention time = 20.391 min) and **compound 1139** (14.5 mg, retention time = 32.554 min). Stereochemical assignment of (S) at quinuclidine is absolute based on starting materials, stereochemical assignment at 1-position of the tetrahydroisoquinoline is assigned based on chromatographic elution order as compared to diastereomers of related analogues of known configuration.

**Compound 1138:** LCMS: (M+H)⁺ = 418; purity = 100% (254 nm); retention time = 1.419 min. Method E ¹H NMR (400 MHz, DMSO-*d₆*) δ 7.58 (d, J = 8.2 Hz, 1H), 7.51- 7.31 (m, 1H), 7.30-7.06 (m, 5H), 6.35 (s, 1H), 4.64-4.63 (brs, 1H), 3.89-3.88 (brs, 1H), 3.44-3.42 (brs, 1H), 3.10 (dd, J = 13.9, 8.7 Hz, 1H), 2.97-2.83 (m, 2H), 2.75- 2.54 (m, 7H), 1.97-1.83 (m, 1H), 1.57-1.55 (brs, 1H), 1.47-1.45 (brs, 1H), 1.36-1.29 (m, 1H), 1.27- 1.20 (m, 2H).

**Compound 1139:** LCMS: (M+H)⁺ = 418; purity = 100% (254 nm); retention time = 1.412 min. Method E ¹H NMR (400 MHz, DMSO-*d₆*) δ 7.59 (d, J = 8.2 Hz, 1H), 7.54-7.32 (m, 1H), 7.23 (d, J = 13.0 Hz, 5H), 6.34 (s, 1H), 4.72- 4.58 (m, 1H), 3.93- 3.80 (m, 1H), 3.60-3.40 (m, 1H), 3.03-3.02 (brs, 1H), 2.90-2.88 (brs, 2H), 2.74- 2.54 (m, 6H), 1.92 (d, J = 2.7 Hz, 1H), 1.70-1.68 (brs, 1H), 1.58 (dd, J = 13.5, 9.2 Hz, 1H), 1.47-1.45 (brs, 1H), 1.37-1.28 (m, 2H), 1.27- 1.22 (m, 2H).

**Compound 1173** was prepared analogously to Compound 1001, and isolated as a mixture of the diastereomers having the (S) stereochemistry at the quinuclidine 3-position and a mix at the 1-position of the tetrahydroisoqiuinoline fragment. LCMS: (M+H)⁺ = 420.0; purity = 100.00% (214 nm); retention time = 1.514 min. Method C ¹H NMR (400 MHz, DMSO-*d₆*) δ 7.21 (d, *J* = 21.4 Hz, 5H), 7.03 (d, *J* = 6.6 Hz, 2H), 6.27 (s, 1H), 4.68 - 4.63 (m, 1H), 3.95 - 3.80 (m, 2H), 3.07 (m, 2H), 2.86 (m, 2H), 2.65 (m, 5H), 1.92 (m, 1H), 1.74 (m, 2H), 1.64 - 1.54 (m, 1H), 1.50-1.46 (m, 1H), 1.35-1.32 (m, 1H), 1.25-1.21 (m, 1H).

### Reference Example 2: Compound 1004 Scale Up Synthesis

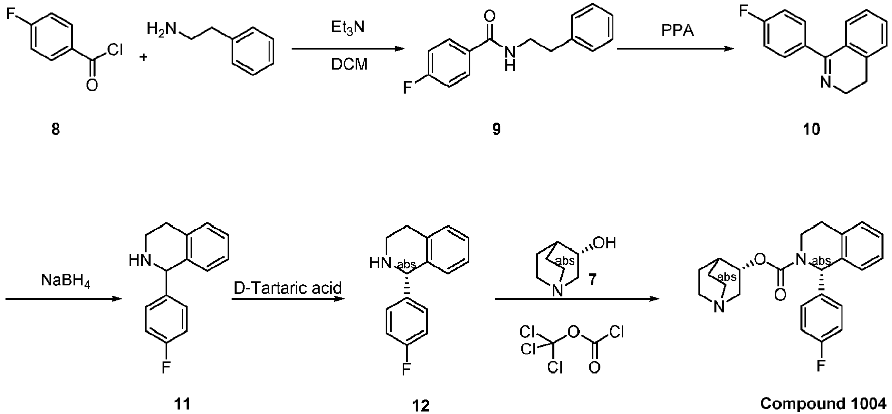

Step 1: To a solution of 2-phenylethanamine (400 g, 3.3 mol) in dichloromethane (4 L) was added 4-fluorobenzoyl chloride (522 g, 3.3 mol) at 0 °C. Triethylamine (434 g, 4.3mol) was added to the white reaction suspension at 0 °C. The mixture was stirred for 2 hours at room temperature. Water (4 L) was added and the phases were separated. The organic phase was washed with brine (2 L) and dried over Na₂SO₄. The solvent was evaporated to give 760 g of 4-fluoro-N-phenethylbenzamide **9.**

Step 2: A round bottomed flask was charged with 500 mL of PPA. The material was heated to 160 °C, then 4-fluoro-N-phenethylbenzamide **9** (350 g, 1.44 mol) was added. The mixture was stirred at 160 °C for 3 hours. The mixture was cooled to 25 °C and 3 L of water was added. The mixture was alkalized with NaOH (20% aq.) to pH 11 and extracted with three 1 L portions of ethyl acetate. The combine organic layers were washed three times with with brine, dried and concentrated *in vacuo* to give crude product. The crude product was purified by column chromatography eluting with Petroleum Ether/Ethyl Acetate (3:1) to give 273 g of 1-(4-fluorophenyl)-3,4-dihydroisoquinoline **10.**

Step 3: To a solution of 1-(4-fluorophenyl)-3,4-dihydroisoquinoline **10** (273 g, 1.2 mol) in MeOH (3000 mL) was added NaBH₄ (138 g, 3.6 mol) at room temperature. The mixture was stirred at room temperature for 0.5 h. The mixture was concentrated *in vacuo* and the solid was dissolved in ethyl acetate (1000 mL). The mixture was washed with water (1000 mL) and brine (500 mL), dried over Na₂SO₄ and concentrated to give 232 g of 1-(4-fluorophenyl)-1,2,3,4-tetrahydroisoquinoline **11** .

Note: Additional related racemic 1-aryl tetrahydroisoquinolines were prepared analogously.

Step 4: To a solution of racemic 1-(4-fluorophenyl)-1,2,3,4-tetrahydroisoquinoline (232 g, 1022 mmol) in isopropanol (2 L) was added dropwise a solution of D-Tartaric acid (200 g, 1329 mmol) in isopropanol (1 L) at room temperature. The mixture was stirred at room temperature overnight. The precipitate was filtered and the cake was washed with isopropanol (200 mL) to give a solid (370 g). The solid was added into isopropanol (2 L) and heated to 100 °C. Water was added dropwise (0.6 L) at 100 °C until the solid was dissolved. The mixture was allowed to crystallize at room temperature overnight. The precipitate was isolated by filtration and the cake was washed with isopropanol (200 mL) to give a solid (190 g). A second recrystallization from isopropanol and water (~3/1, 100 °C to room temperature overnight) afforded 150 g of a solid. The solid was dissolved in water (500 mL), alkalized with NaOH (20% aq.) to pH 11 and extracted with three 200 mL portions of ethyl acetate. The combined organic layers were washed with brine (0.5 L), dried and concentrated *in vacuo* to give 70 g of (S)-1-(4-fluorophenyl)-1,2,3,4-tetrahydroisoquinoline **12.**

Chiral SFC: CO₂/MeOH containing 0.2% ammonia over CHIRALPAK^{®} IG column (4.6 × 100 mm 5 µm), retention time = 6.08 min), 100% ee.

Step 5: To a solution of (S)-quinuclidin-3-ol **7** (11.2 g, 88 mmol) in MeCN (200 mL) was added trichloromethyl carbonochloridate (17.3 g, 88 mmol) and the mixture was stirred at room temperature for 2 h. The mixture was concentrated to give a white solid. The white solid was dissolved in 200 mL of DMF and (S)-1-(4-fluorophenyl)-1,2,3,4-tetrahydroisoquinoline **12** (20 g, 88 mmol), TEA (26.7 g, 264 mmol) was added. The mixture was stirred at 90 °C overnight. The mixture was cooled to 25 °C and water (1 L) was added. The mixture was extracted with three 300 mL portions of ethyl acetate. The combine organic was washed with three 300 mL portions of brine, dried and concentrated *in vacuo* to give crude product. The crude product was purified by column chromatography eluting with petroleum ether:ethyl acetate (3:1) to give 5 g of (S)-((S)-quinuclidin-3-yl) 1-(4-fluorophenyl)-3,4-dihydroisoquinoline-2(1H)-carboxylate **compound 1004.**

**Compound 1004:** LCMS: (M+H)⁺ =380; purity = 100% (214 nm); retention time = 1.655 min. Method E ¹H NMR (400 MHz, DMSO-*d₆*) δ 7.23 (t, J = 5.6 Hz, 4H), 7.20 - 7.10 (m, 4H), 6.26 (s, 1H), 4.64-4.62 (br, 1H), 3.98 - 3.83 (m, 1H), 3.37 - 3.29 (m, 1H), 3.08 (dd, J = 14.0, 8.3 Hz, 1H), 2.93 - 2.79 (m, 2H), 2.70-2.68 (br, 2H), 2.65 - 2.53 (m, 3H), 1.91-1.90 (br, 1H), 1.79 - 1.52 (m, 2H), 1.51 - 1.39 (m, 1H), 1.25-1.24 (br,1H).

Compounds 1005 and 1006 were prepared analogously to 1004. The two diastereomers were separated by chiral SFC eluting with CO₂/MeOH containing 0.2% methanolic ammonia over a CHIRALPAK^{®} IG column (4.6 × 100 mm 5µm) to give **compound 1006** (28.8 mg, retention time = 3.21 min) and **compound 1007** (7.5 mg, retention time = 5.68 min).

**Compound 1006:** LCMS: (M+H)⁺ = 429; purity = 100% (214 nm); retention time = 1.589 min. Method C ¹H NMR (400 MHz, DMSO-*d₆*) δ 8.21 (s, 1H), 7.75 - 7.65 (m, 1H), 7.60 (d, J = 6.7 Hz, 2H), 7.44 - 7.16 (m, 6H), 7.16 - 7.04 (m, 1H), 6.29 (s, 1H), 4.74 - 4.57 (m, 1H), 3.95 - 3.78 (m, 1H), 3.06-3.05 (br, 1H), 2.97 - 2.77 (m, 2H), 2.74 - 2.45 (m, 6H), 1.93 (d, J = 2.6 Hz, 1H), 1.72-1.70 (br, 1H), 1.66 - 1.52 (m, 1H), 1.43-1.42 (br, 1H), 1.35 (d, J = 5.9 Hz, 1H).

**Compound 1007:** LCMS: (M+H)⁺ = 429; purity = 100% (214 nm); retention time = 1.585 min. Method C ¹H NMR (400 MHz, DMSO-*d₆*): δ 8.20 (s, 1H), 7.69 (t, J = 1.2 Hz, 1H), 7.59 (d, J = 8.5 Hz, 2H), 7.27 (t, J = 25.0 Hz, 6H), 7.09 (s, 1H), 6.30 (s, 1H), 4.65-4.64 (br, 1H), 3.90-3.88 (br, 1H), 3.09 (dd, J = 13.6, 8.4 Hz, 1H), 2.90 (dd, J = 17.9, 11.9 Hz, 2H), 2.77 - 2.51 (m, 6H), 1.92-1.90 (br, 1H), 1.65 (d, J = 35.1 Hz, 1H), 1.63 - 1.51 (m, 1H), 1.47-1.46 (br, 1H), 1.30-1.28 (br, 1H).

Compounds 1008 and 1009 were prepared analogously to 1004. The two diastereomers were separated by chiral SFC eluting with CO₂/MeOH containing 0.2% methanolic ammonia over a Cellulose-SC column (4.6 × 100 mm 5µm) to give **compound 1008** (5.5 mg, retention time = 1.97 min) and **compound 1009** (7.2 mg, retention time = 2.72 min).

**Compound 1008:** LCMS: (M+H)⁺ = 394; purity = 100% (214 nm); retention time = 1.432 min. Method C ¹H NMR (400 MHz, DMSO-*d₆*) δ 8.09 (d, J = 5.2 Hz, 1H), 7.29 (d, J = 41.0 Hz, 3H), 6.74 (dd, J = 110.3, 67.5 Hz, 2H), 6.13 (d, J = 30.4 Hz, 1H), 4.65 (s, 1H), 3.80 (s, 3H), 3.04-3.03 (br, 2H), 2.97 - 2.52 (m, 5H), 2.06-2.05 (br, 3H), 1.89 - 1.09 (m, 5H).

**Compound 1009:** LCMS: (M+H)⁺ = 394; purity = 100% (214 nm); retention time = 1.455 min. Method C ¹H NMR (400 MHz, DMSO-*d₆*) δ 8.08 (d, J = 5.3 Hz, 1H), 7.24 (s, 3H), 6.91 (s, 1H), 6.61 (d, J = 74.3 Hz, 1H), 6.15 (s, 1H), 4.63-4.62 (br, 1H), 3.86 - 3.70 (m, 4H), 3.64 - 3.43 (m, 1H), 3.07-3.06 (br, 1H), 2.69-2.68 (br, 7H), 2.04 - 1.65 (m, 2H), 1.66 - 1.24 (m, 5H).

Compounds 1010 and 1011 were prepared analogously to 1004. The two diastereomers were separated by chiral SFC eluting with CO₂/MeOH containing 0.2% methanolic ammonia over a CHIRALPAK^{®} IG column (4.6 × 100 mm 5µm) to give **compound 1010** (6.3 mg, retention time = 2.16 min) and **compound 1011** (7.0 mg, retention time = 2.81 min).

**Compound 1010:** LCMS: (M+H)⁺ =399; purity = 100% (214 nm); retention time = 1.754 min. Method F ¹H NMR (400 MHz, CD₃OD): δ 7.27 - 7.12 (m, 7H), 6.32 (s, 1H), 4.04 - 3.99 (m, 1H), 3.45 (br, 1H), 3.33 - 3.24 (m, 8H), 2.08-2.06 (br, 1H), 1.91 - 1.87 (m, 2H), 1.68-1.67 (br, 1H), 1.49-1.48 (br, 1H).

**Compound 1011:** LCMS: (M+H) =399; purity = 100% (214 nm); retention time = 1.760 min. Method F ¹H NMR (400 MHz, CD₃OD): δ 7.15 - 6.90 (m, 7H), 6.18 (s, 1H), 3.87-3.86 (br, 1H), 3.23 - 3.01 (m, 1H), 2.88 - 2.59 (m, 8H), 1.97-1.96 (br, 1H), 1.88 - 1.62 (m, 2H), 1.52-1.51 (br, 1H), 1.45-1.44 (br, 1H).

Compounds 1012 and 1013 were prepared analogously to 1004. The two diastereomers were separated by chiral SFC eluting with CO₂/EtOH containing 1% methanolic ammonia over a CHIRALPAK^{®} IG column (4.6 × 100 mm 5µm) to give **compound 1012** (25.2 mg, retention time = 4.01 min) and **compound 1013** (40.9 mg, retention time = 4.88 min).

**Compound 1012:** LCMS: (M+H)⁺ =399; purity = 100% (214 nm); retention time = 1.839 min. Method C ¹H NMR (400 MHz, DMSO-*d₆*) δ 7.37 - 7.12 (m, 5H), 7.10 - 6.95 (m, 2H), 6.38 (s, 1H), 4.73 - 4.56 (m, 1H), 3.95 (dt, J = 13.0, 5.0 Hz, 1H), 3.05-3.04 (br, 1H), 2.92-2.91 (br, 2H), 2.80 - 2.54 (m, 4H), 1.92-1.91 (br, 1H), 1.68 (d, J = 24.3 Hz, 1H), 1.59 (dd, J = 13.6, 9.1 Hz, 1H), 1.44 (d, J = 29.6 Hz, 1H), 1.40 - 1.27 (m, 2H), 1.27 - 1.17 (m, 1H).

**Compound 1013:** LCMS: (M+H)⁺ =399; purity = 100% (214 nm); retention time = 1.859 min. Method C ¹H NMR (400 MHz, DMSO-*d₆*) δ 7.35 - 7.10 (m, 5H), 7.01 (ddd, J = 14.5, 8.3, 2.3 Hz, 2H), 6.37 (s, 1H), 4.59-4.58 (br, 1H), 4.11 - 3.86 (m, 1H), 3.08 (dd, J = 14.4, 8.4 Hz, 1H), 2.91 (s, 2H), 2.62 (dd, J = 28.8, 12.3 Hz, 4H), 1.96 - 1.75 (m, 1H), 1.55 (s, 1H), 1.43 (d, J = 18.7 Hz, 1H), 1.38 - 1.32 (m, 2H), 1.25 (d, J = 4.0 Hz, 1H), 1.23 (d, J = 3.0 Hz, 2H).

Compounds 1014 and 1015 were prepared analogously to 1004. The two diastereomers were separated by chiral SFC eluting with CO₂/MeOH containing 1% methanolic ammonia over a CHIRALPAK^{®} IG column (4.6 × 100 mm 5µm) to give **compound 1014** (13.4 mg, retention time = 2.33 min) and **compound 1015** (15.0 mg, retention time = 2.55 min).

**Compound 1014:** LCMS: (M+H)⁺ =399; purity = 96% (214 nm); retention time = 1.704 min. Method F ¹H NMR (400 MHz, CDCl₃) δ 7.28 (s, 3H), 7.26 - 7.16 (m, 2H), 7.08 - 7.07 (m, 1H), 7.98 - 7.97 (m, 1H), 6.49 (s, 1H), 4.79 - 4.77 (m, 1H), 4.15 (br, 1H), 3.49 (br, 1H), 3.21 (br, 1H), 3.02 - 2.70 (m, 6H), 2.51 (br, 1H), 1.75 - 1.64 (m, 3H), 1.57 - 1.41 (m, 2H).

**Compound 1015:** LCMS: (M+H)⁺ =399; purity = 95% (214 nm); retention time = 1.707 min. Method F ¹H NMR (400 MHz, CDCl₃) δ 7.26 (s, 3H), 7.21 - 7.20 (m, 2H), 7.19 - 7.17 (m, 1H), 7.08 - 6.97 (m, 1H), 6.49 (s, 1H), 4.62 (br, 1H), 4.21 (br, 1H), 3.51 - 3.47 (m, 1H), 3.28 - 3.18 (m, 1H), 3.05 - 2.71 (m, 7H), 2.05 - 1.69 (m, 3H), 1.67 - 1.48 (m, 2H).

Compounds 1016 and 1017 were prepared analogously to 1004. The two diastereomers were separated by chiral SFC eluting with CO₂ and methanol:acetonitrile (3:2) containing 0.2% ammonia over a CHIRALPAK^{®} IG column (4.6 x 100 mm 5µm) to give **compound 1016** (11.0 mg, retention time = 4.62 min) and **compound 1017** (9.8 mg, retention time = 7.19 min).

**Compound 1016:** LCMS: (M+H)⁺ = 446; purity = 100% (214 nm); retention time = 1.622 min. Method C ¹H NMR (400 MHz, CD₃OD) δ 7.44 (d, J = 8.6 Hz, 2H), 7.19 - 7.09 (m, 4H), 6.99 (d, J = 7.5 Hz, 1H), 6.28(s, 1H), 3.90 (dt, J = 13.1, 5.1 Hz, 1H), 3.79 (t, J = 7.1 Hz, 2H), 3.37 (dd, J = 13.8, 12.3 Hz, 1H), 3.17 - 3.12 (m, 1H), 2.89 - 2.63 (m, 7H), 2.48 (t, J = 8.1 Hz, 2H), 2.06 (dt, J = 15.4, 7.5 Hz, 2H), 1.72 - 1.53 (m, 3H), 1.47 - 1.17 (m, 3H).

**Compound 1017:** LCMS: (M+H)⁺ = 446; purity = 100% (214 nm); retention time = 1.624 min. Method C ¹H NMR (400 MHz, CD₃OD) δ 7.45 (s, 2H), 7.24 - 6.98 (m, 6H), 6.19 (s, 1H), 3.80 (dd, J = 21.1, 14.1 Hz, 3H), 3.39 (d, J = 116.8 Hz, 1H), 3.19 - 3.07 (m, 1H), 2.91 - 2.41 (m, 9H), 2.11 - 2.01 (m, 2H), 1.96 (s, 1H), 1.83 - 1.61 (m, 2H), 1.62 - 0.99 (m, 3H).

Compounds 1018 and 1019 were prepared analogously to 1004. The two diastereomers were separated by chiral SFC eluting with CO₂ and 0.1% DEA in ethanol over a CHIRALPAK^{®} IG column (4.6 x 100 mm 5 µm) to give **compound 1018** (5.5 mg, retention time = 25.52 min) and **compound 1019** (7.2 mg, retention time = 42.2 min).

**Compound 1018:** LCMS: (M+H)⁺ = 430; purity = 100% (214 nm); retention time = 1.566 min. Method C ¹H NMR (400 MHz, DMSO-*d₆*) δ 9.24 (s, 1H), 8.22 (s, 1H), 7.80 (d, J = 8.6 Hz, 2H), 7.40 (s, 2H), 7.23 (d, J = 15.4 Hz, 4H), 6.30 (s, 1H), 4.64-4.63 (br, 1H), 3.91 (d, J = 12.3 Hz, 1H), 3.45-3.44 (br, 1H), 3.09 (dd, J = 14.0, 8.4 Hz, 1H), 2.97 - 2.79 (m, 2H), 2.79 - 2.54 (m, 5H), 1.91-1.90 (br, 1H), 1.57-1.56 (br, 1H), 1.47 -1.46(br, 1H), 1.27-1.26 (m, 2H).

**Compound 1019:** LCMS: (M+H)⁺ = 430; purity = 100% (214 nm); retention time = 1.562 min. Method C ¹H NMR (400 MHz, DMSO-*d₆*) δ 9.25 (s, 1H), 8.22 (s, 1H), 7.81 (d, J = 7.6 Hz, 2H), 7.41 (d, J = 37.8 Hz, 2H), 7.25-7.24 (m, 4H), 6.29 (s, 1H), 4.74 - 4.56 (m, 1H), 3.96 - 3.79 (m, 1H), 3.47-3.46 (m, 1H), 3.06-3.05 (br, 1H), 2.91-2.90 (br, 2H), 2.55-2.44 (m, 5H), 1.93-1.92 (br, 1H), 1.71-1.70 (br, 1H), 1.58 (dd, J = 13.1, 8.8 Hz, 1H), 1.48-1.47 (br, 1H), 1.35 (d, J = 6.7 Hz, 1H).

Compounds 1020 and 1021 were prepared analogously to 1004. The two diastereomers were separated by chiral SFC eluting with CO₂ and hexane:EtOH (60:40) containing 0.1% DEA over a CHIRALPAK^{®} AY-H. column (4.6 × 100 mm 5 µm) to give **compound 1020** (37.8 mg, retention time = 7.29 min) and **compound 1021** (35.3 mg, retention time = 5.69 min).

**Compound 1020:** LCMS: (M+H)⁺ = 381; purity = 97.34% (214 nm); retention time = 1.431 min. Method A1 ¹H NMR(400 MHz, DMSO-*d₆*): δ 7.36 (t, *J=* 6.4 Hz, 1H), 7.24-7.21 (m, 4H), 7.09-7.07 (m, 3H), 6.23 (s, 1H), 4.64-4.63 (m, 1H), 3.88-3.83 (m, 1H), 3.11-3.06 (m, 1H), 2.87-2.60 (m, 8H), 1.97-1.82 (m, 2H), 1.58-1.46 (m, 2H), 1.37-1.30 (m, 1H). Chiral SFC: CO₂/MeOH containing 0.2% ammonia over a CHIRALPAK^{®} IG column (4.6 × 100 mm 5 µm), retention time = 2.62 min

**Compound 1021:** LCMS: (M+H)⁺ = 381; purity = 100% (214 nm); retention time = 1.430 min. Method A1 ¹H NMR(400 MHz, DMSO-*d₆*) δ 7.37 (t, J = 6.4Hz, 1H), 7.30-7.21 (m, 4H), 7.14 -7.07(m, 3H), 6.23 (s, 1H), 4.66-4.64 (m, 1H), 3.83-3.79 (m, 1H), 3.10-3.01 (m, 1H), 2.87-2.60 (m, 8H), 1.98-1.89 (m, 1H), 1.72-1.47 (m, 3H), 1.37-1.30 (m, 1H). Chiral SFC: CO₂/MeOH containing 0.2% ammonia over a CHIRALPAK^{®} IG column (4.6 × 100 mm 5 µm), retention time = 3.08 min

Compounds 1022 and 1023 were prepared analogously to 1004. The two diastereomers were separated by chiral SFC eluting with CO₂/EtOH containing 1% methanolic ammonia over a CHIRALPAK^{®} AD-H column (20 × 250 mm 10 µm) to give **compound 1022** (31.8 mg) and **compound 1023** (19.1 mg).

**Compound 1022:** LCMS: (M+H)⁺ = 419; purity = 100% (214 nm); retention time = 1.71 min. Method E ¹H NMR: (400 MHz, CDCl₃) δ 7.24-7.14 (m, 5H), 7.07 (d, *J=* 7.2 Hz, 1H), 6.97 (d, *J=* 8.8 Hz, 2H), 6.33 (s, 1H), 4.83-4.81 (m, 1H), 4.15-4.03 (m, 1H), 3.73-3.69 (m, 1H), 3.33-3.26 (m, 2H), 3.01-2.74 (m, 7H), 2.14-2.13 (m, 1H), 1.85-1.79 (m, 1H), 1.70-1.60 (m, 2H), 1.48-1.41 (m, 1H), 0.77-0.76 (m, 4H). Chiral SFC: CO₂/EtOH containing 1% methanolic ammonia over a CHIRALPAK^{®} AD-H column (4.6 × 100 mm 5 µm), retention time = 2.18 min. Chiral SFC: CO₂/MeOH containing 0.2% methanolic ammonia over a CHIRALPAK^{®} IG column (4.6 × 100 mm 5 µm), retention time = 3.06 min.

**Compound 1023:** LCMS: (M+H) = 419; purity = 97% (214 nm); retention time = 1.71 min. Method E ¹H NMR: (400 MHz, CDCl₃) δ 7.24-7.14 (m, 5H), 7.08 (d, *J=* 7.2 Hz, 1H), 6.97 (d, *J=* 8.4 Hz, 2H), 6.33 (s, 1H), 4.82 (bs, 1H), 4.13-4.03 (m, 1H), 3.73-3.69 (m, 1H), 3.34-3.25 (m, 2H), 3.05-2.97 (m, 1H), 2.92-2.73 (m, 6H), 2.06-2.05 (m, 1H), 1.87 (bs, 1H), 1.70-1.67 (m, 1H), 1.63-1.55 (m, 1H), 1.48-1.40 (m, 1H), 0.77-0.76 (m, 4H). Chiral SFC: CO₂/EtOH containing 1% methanolic ammonia over a CHIRALPAK^{®} AD-H column (4.6 × 100 mm 5 µm), retention time = 1.70 min). Chiral SFC: CO₂/MeOH containing 0.2% methanolic ammonia over a CHIRALPAK^{®} IG column (4.6 × 100 mm 5 µm), retention time = 4.35 min.

Compounds 1024 and 1025 were prepared analogously to 1004. The two diastereomers were separated by chiral SFC eluting with CO₂/EtOH containing 1% methanolic ammonia over a CHIRALPAK^{®} AD-H column (20 × 250 mm 10 µm) to give **compound 1024** (21.9 mg) and **compound 1025** (23 mg).

**Compound 1024:** LCMS: (M+H)⁺ = 393; purity = 100% (214 nm); retention time = 1.800 min. Method C ¹H NMR (400 MHz, CD₃OD) δ 7.30 - 6.92 (m, 4H), 6.86 - 6.83 (m, 1H), 6.72 (d, *J=* 8.0 Hz, 2H), 6.17 (s, 1H), 3.89-3.88 (br, 1H), 3.64 (s, 3H), 3.20 - 3.06 (m, 2H), 2.82 - 2.64 (m, 8H), 1.98-1.97 (br, 1H), 1.69-1.68 (br, 2H), 1.54-1.53 (br, 1H), 1.30-1.29 (br, 1H). Chiral SFC: CO₂/MeOH containing 0.2% methanolic ammonia over a CHIRALPAK^{®} IG column (4.6 × 100 mm 5 µm), retention time = 2.51 min.

**Compound 1025:** LCMS: (M+H)⁺ = 393; purity = 95% (214 nm); retention time = 1.802 min. Method C ¹H NMR (400 MHz, CD₃OD) δ 7.22 - 7.09 (m, 6H), 6.86 (s, 2H), 6.25 (s, 1H), 3.99 - 3.94 (m, 1H), 3.75 (s, 3H), 3.43 - 3.22 (m, 2H), 2.95 - 2.72 (m, 8H), 2.06-2.65 (br, 1H), 1.85-1.84 (br, 1H), 1.75-1.74 (br, 1H), 1.65-1.64 (br, 1H), 1.55-1.54 (br, 1H). Chiral SFC: CO₂/MeOH containing 0.2% methanolic ammonia over a CHIRALPAK^{®} IG column (4.6 × 100 mm 5 µm), retention time = 3.44 min.

Compounds 1026 and 1027 were prepared analogously to 1004. Two diastereomers were separated by chiral SFC eluting with CO₂/MeOH containing 0.2% methanolic ammonia over a CHIRALPAK^{®} IG column (4.6 × 100 mm 5 µm) to give **compound 1026** (24.8 mg) and **compound 1027** (19.2 mg).

**Compound 1026:** LCMS: (M+H)⁺ = 384; purity = 100% (214 nm); retention time = 1.353 min. Method C ¹H NMR (400 MHz, DMSO-*d₆*) δ 7.45 (s, 1H), 7.20 (d, J = 10.1 Hz, 4H), 4.90 (dd, J = 27.2, 9.5 Hz, 1H), 4.59 (s, 1H), 3.88 - 3.56 (m, 2H), 3.13 (s, 1H), 3.09 - 2.99 (m, 2H), 2.97 - 2.90 (m, 1H), 2.84 (dd, J = 22.1, 12.1 Hz, 1H), 2.79 - 2.66 (m, 2H), 2.66 - 2.54 (m, 2H), 2.43 (d, J = 14.0 Hz, 1H), 2.30 - 1.92 (m, 4H), 1.86 (s, 1H), 1.71 (d, J = 26.6 Hz, 1H), 1.53 (d, J = 27.4 Hz, 3H), 1.46 (s, 1H), 1.31 (dd, J = 28.8, 19.0 Hz, 1H). Chiral SFC: CO₂ and hexane/ethanol (1/4) containing 0.1% DEA over a CHIRALPAK^{®} IG column (4.6 × 250 mm 5 µm), retention time = 29.02 min.

**Compound 1027:** LCMS: (M+H)⁺ = 384; purity = 100% (214 nm); retention time = 1.345 min. Method C ¹H NMR (400 MHz, DMSO-*d₆*) δ 7.48 (d, J = 19.2 Hz, 1H), 7.22 (dd, J = 12.7, 7.3 Hz, 4H), 4.84 (dd, J = 19.8, 9.8 Hz, 1H), 4.59 (s, 1H), 4.08 - 3.54 (m, 3H), 3.25 - 2.81 (m, 5H), 2.74 (d, J = 17.9 Hz, 1H), 2.58 (dd, J = 18.6, 9.7 Hz, 1H), 2.41 (t, J = 23.7 Hz, 1H), 2.05 (ddd, J = 36.3, 29.4, 18.6 Hz, 5H), 1.70 (d, J = 22.6 Hz, 1H), 1.67 - 1.34 (m, 4H), 1.27 (dd, J = 30.1, 14.6 Hz, 1H). Chiral SFC: CO₂ and hexane/ethanol (1/4) containing 0.1% DEA over a CHIRALPAK^{®} IG column (4.6 × 250 mm 5 µm), retention time = 34.59 min.

Compounds 1028 and 1029 were prepared analogously to 1004. The two diastereomers were separated by chiral SFC eluting with CO₂/MeOH containing 0.2% methanolic ammonia over a CHIRALPAK^{®} IG column (4.6 × 100 mm 5 µm) to give **compound 1028** (46.3 mg) and **compound 1029** (34.7 mg).

**Compound 1028:** LCMS: (M+H)⁺ = 394; purity = 100% (254 nm); retention time = 1.381 min. Method E ¹H NMR(400 MHz, CD₃OD) δ 8.01 (s, 1H), 7.54 (dd, *J* = 11.2 Hz, 1H), 7.27 - 7.25 (m, 3H), 7.21 (d, *J* = 4.0 Hz, 1H), 6.75 (d, *J* = 8.4 Hz, 1H), 6.31 (br, 1H), 4.85 - 4.81 (m, 1H), 4.08 - 4.01 (m, 1H), 3.85 (s, 3H), 3.45 - 3.25 (m, 2H), 3.01 - 2.62 (m, 7H), 2.11 (br, 1H), 1.82 - 1.71 (m, 2H), 1.68 - 1.55 (m, 1H), 1.54 - 1.48 (m, 1H). Chiral SFC: CO₂/MeOH containing 0.2% methanolic ammonia over a CHIRALPAK^{®} IG column (4.6 × 100 mm 5 µm), retention time = 2.15 min.

**Compound 1029:** LCMS: (M+H)⁺ =394; purity = 100% (214 nm); retention time = 1.430 min. Method E ¹H NMR(400 MHz, CD₃OD) δ 8.01 (s, 1H), 7.54 (dd, *J* = 11.2 Hz, 1H), 7.27 - 7.25 (m, 3H), 7.21 (d, *J* = 4.0 Hz, 1H), 6.75 (d, *J* = 8.4 Hz, 1H), 6.33 (br, 1H), 5.11 - 5.01 (m, 1H), 4.08 - 4.01 (m, 1H), 3.85 (s, 3H), 3.79 - 3.76 (m, 1H), 3.45 - 2.22 (m, 6H), 3.01 (br, 1H), 2.90 - 2.85 (m, 1H), 2.44 (s, 1H), 2.22 - 1.88 (m, 4H). Chiral SFC: CO₂/MeOH containing 0.2% methanolic ammonia over a CHIRALPAK^{®} IG column (4.6 × 100 mm 5 µm), retention time = 3.47 min.

Compounds 1030 and 1031 were prepared analogously to 1004. The two diastereomers were separated by chiral SFC eluting with CO₂/MeOH containing 0.2% methanolic ammonia over a CHIRALPAK^{®} IG column (4.6 × 100 mm 5 µm) to give **compound 1030** (29.3 mg) and **compound 1031** (29.0 mg).

**Compound 1030:** LCMS: (M+H)⁺ =441; purity = 100% (214 nm); retention time = 1.617 min. Method C ¹H NMR (400 MHz, DMSO-*d₆*) δ 7.92 (t, J = 7.3 Hz, 2H), 7.72 (dd, J = 13.6, 6.8 Hz, 1H), 7.62 (dd, J = 17.9, 7.8 Hz, 2H), 7.31 - 7.15 (m, 3H), 7.13 - 7.07 (m, 1H), 5.68 (dd, J = 79.0, 7.5 Hz, 1H), 4.52 (dd, J = 12.2, 8.4 Hz, 1H), 4.29 - 4.15 (m, 1H), 3.73 - 3.47 (m, 2H), 3.17 - 2.85 (m, 3H), 2.79 - 2.54 (m, 5H), 1.83 (s, 1H), 1.67 - 1.38 (m, 3H), 1.37- 1.28 (m, 1H). Chiral HPLC: hexane/ethanol (1/4) containing 0.1% DEA over a CHIRALPAK^{®} IG column (4.6 × 250 mm 5 µm), retention time = 22.08 min.

**Compound 1031:** LCMS: (M+H)⁺ = 441; purity = 100% (214 nm); retention time = 1.608 min. Method C ¹H NMR (400 MHz, DMSO-*d₆*) δ 7.91 (dd, J = 13.8, 7.6 Hz, 2H), 7.72 (q, J = 7.0 Hz, 1H), 7.63 (t, J = 7.6 Hz, 2H), 7.29 - 7.14 (m, 3H), 7.11 (d, J = 6.4 Hz, 1H), 5.65 (dd, J = 51.6, 8.0 Hz, 1H), 4.51 (d, J = 8.4 Hz, 1H), 4.23 (ddd, J = 30.8, 15.0, 10.2 Hz, 1H), 3.69 - 3.48 (m, 2H), 3.17 - 2.97 (m, 2H), 2.78 - 2.54 (m, 6H), 2.39 (dd, J = 32.9, 14.7 Hz, 1H), 2.10 - 1.76 (m, 2H), 1.66 - 1.39 (m, 3H). Chiral HPLC: hexane:ethanol (1:4) containing 0.1% DEA over a CHIRALPAK^{®} IG column (4.6 × 250 mm 5 µm), retention time = 21.07 min.

Compounds 1032 and 1033 were prepared analogously to 1004. The two diastereomers were separated by chiral SFC eluting with CO₂/MeOH containing 0.2% methanolic ammonia over a Cellulose SC column (20 × 250 mm 10 µm) to give **compound 1032** (19.3 mg) and **compound 1033** (21.3 mg).

**Compound 1032:** LCMS: (M+H)⁺ = 420.2; purity = 100% (214 nm); retention time = 1.478 min. Method C2 ¹H NMR: (400 MHz, CD₃OD) δ 9.13 (s, 1H), 7.90 (d, *J=* 8.4 Hz, 1H), 7.78 (br, 1H), 7.48-7.35 (m, 1H), 7.20-7.08 (m, 3H), 7.07-7.00 (m, 1H), 6.38 (s, 1H), 4.73-4.72 (br, 1H), 3.92 (dt, *J=* 13.2, 4.2 Hz, 1H), 3.43-3.42 (br, 1H), 3.19-3.13 (m, 1H), 2.93-2.62 (m, 7H), 1.97-1.96 (br, 1H), 1.88-1.60 (m, 2H), 1.27-1.26 (br, 1H), 1.18-1.17 (br, 1H). Chiral SFC: CO₂/MeOH containing 0.2% methanolic ammonia over a CHIRALPAK^{®} IG column (4.6 × 100 mm 5 µm), retention time = 4.08 min. Chiral SFC: CO₂/MeOH containing 0.2% methanolic ammonia over a Cellulose SC column (4.6 × 100 mm 5 µm), retention time = 5.11 min.

**Compound 1033:** LCMS: (M+H)⁺ = 420.1; purity = 96% (214 nm); retention time = 1.419 min. Method A1 ¹H NMR: (400 MHz, CD₃OD) δ 9.11 (s, 1H), 7.90 (d, *J* = 8.0 Hz, 1H), 7.74 (br, 1H), 7.40 (br, 1H), 7.21-7.00 (m, 4H), 6.39 (s, 1H), 4.73-4.72 (br, 1H), 3.80-3.78 br, 1H), 3.63-3.26 (m, 1H), 3.15-3.14 (br, 1H), 2.95-2.42 (m, 7H), 1.98-1.97 (br, 1H), 1.85-1.60 (m, 2H), 1.55-1.54 (br, 1H), 1.42-1.40 (br, 1H). Chiral SFC: CO₂/MeOH containing 0.2% methanolic ammonia over a CHIRALPAK^{®} IG column (4.6 × 100 mm 5 µm), retention time = 5.01 min. Chiral SFC: CO₂/MeOH containing 0.2% methanolic ammonia over a Cellulose SC column (4.6 × 100 mm 5 µm), retention time = 4.05 min.

Compounds 1034 and 1035 were prepared analogously to 1004. The two diastereomers were separated by chiral SFC eluting with CO₂ and *n*-hexane:EtOH (1:1) containing 0.1% DEA over a CHIRALPAK^{®} IC column (4.6 × 250 mm 5 µm) to give **compound 1034** (53.8 mg, retention time = 11.02 min) and **compound 1035** (56.0 mg, retention time 13.24 min).

**Compound 1034:** LCMS: (M+H)⁺ = 420.1; purity = 100% (214 nm); retention time = 1.517 min. Method C1 ¹H NMR: (400 MHz, CD₃OD) δ 9.12 (s, 1H), 7.91 (d, *J=* 7.6 Hz, 1H), 7.86-7.64 (m, 1H), 7.36 (s, 1H), 7.21-7.02 (m, 4H), 6.33-6.32 (br, 1H), 4.73-4.72 (br, 1H), 3.87-3.86 (br, 1H), 3.57-3.26 (m, 1H), 3.12-3.11 (br, 1H), 2.93-2.46 (m, 7H), 1.96-1.95 (br, 1H), 1.76-1.75(br, 1H), 1.64-1.63 (br, 1H), 1.52-1.51 (br, 1H), 1.38-1.37 (br, 1H). Chiral SFC: CO₂ and hexane:ethanol (1:1) containing 0.1% DEA over a CHIRALPAK^{®} IC column (4.6 × 250 mm 5 µm), retention time = 11.02 min.

**Compound 1035:** LCMS: (M+H)⁺ = 420.1; purity = 100% (214 nm); retention time = 1.505 min. Method C1 ¹H NMR: (400 MHz, CD₃OD) δ 9.12 (s, 1H), 7.90 (d, *J=* 8.4 Hz, 1H), 7.77 (br, 1H), 7.35 (d, *J=* 8.0 Hz, 1H), 7.20-7.08 (m, 3H), 7.05 (d, *J=* 7.2 Hz, 1H), 6.40 (s, 1H), 4.72-4.71 (s, 1H), 3.93 (dt, *J* = 13.2, 4.2 Hz, 1H), 3.36-3.35 (br, 1H), 3.19-3.10 (m, 1H), 2.94-2.58 (m, 7H), 1.96-1.95 (br, 1H), 1.84-1.60 (m, 2H), 1.52-1.50 (br, 1H), 1.35-1.34 (br, 1H). Chiral SFC: CO₂ and hexane:ethanol (1:1) containing 0.1% DEA over a CHIRALPAK^{®} IC column (4.6 × 250 mm 5 µm), retention time = 19.41 min.

Compounds 1036 and 1037 were prepared analogously to 1004. The two diastereomers were separated by chiral SFC eluting with CO₂ and n-hexane:EtOH (1:1) containing 0.1% DEA over a CHIRALPAK^{®} IG column (4.6 × 250 mm 5 µm) to give **compound 1036** (41.5 mg) and **compound 1037** (50.0 mg).

**Compound 1036:** LCMS: (M+H)⁺ = 419; purity = 100% (214 nm); retention time = 1.731 min. Method E ¹H NMR (400 MHz, CD₃OD) δ 7.78 - 7.76 (m, 1H), 7.65 (d, *J=* 8.4 Hz, 1H), 7.32 - 7.23 (m, 6H), 6.96 - 6.92 (m, 1H), 6.56 (s, 1H), 4.92 - 4.84 (m, 1H), 4.10 - 4.07 (m, 1H), 3.44-3.43 (br, 1H), 3.32-3.30 (br, 1H), 3.05 - 2.65 (m, 7H), 2.10-2.09 (br, 1H), 1.88-1.87 (br, 1H), 1.85 - 1.75 (m, 1H), 1.74 - 1.65 (m, 1H), 1.49-1.48 (br, 1H). Chiral SFC: CO₂/MeOH containing 0.2% methanolic ammonia over a CHIRALPAK^{®} IG column (4.6 × 100 mm 5 µm), retention time = 3.71 min.

**Compound 1037:** LCMS: (M+H)⁺ = 419; purity = 100% (214 nm); retention time = 1.720 min. Method E ¹H NMR (400 MHz, CD₃OD) δ 7.90 (s, 1H), 7.76 (d, *J=* 7.6 Hz, 1H), 7.38 (d, *J=* 6.8 Hz, 1H), 7.35 - 7.28 (m, 5H), 7.02 - 6.95 (m, 1H), 6.52 (s, 1H), 4.71 - 4.69 (m, 1H), 3.99 - 3.96 (m, 1H), 3.14 - 3.08 (m, 1H), 2.93 - 2.51 (m, 8H), 1.97-1.96 (br, 1H), 1.63-1.62 (br, 1H), 1.62 - 1.52 (m, 1H), 1.51 -1.48 (m, 1H), 1.47 - 1.41 (m, 1H). Chiral SFC: CO₂/MeOH containing 0.2% methanolic ammonia over a CHIRALPAK^{®} IG column (4.6 x 100 mm 5 µm), retention time = 4.55 min.

Compounds 1038 and 1039 were prepared analogously to 1004. The two diastereomers were separated by chiral SFC eluting with CO₂ and n-hexane:EtOH (1:1) containing 0.1% DEA over a CHIRALPAK^{®} IC column (20 × 250 mm 10 µm) to give **compound 1038** (39.8 mg) and **compound 1039** (41.0 mg).

**Compound 1038:** LCMS: (M+H)⁺ = 411; purity = 100% (214 nm); retention time = 1.60 min. Method E ¹H NMR: (400 MHz, CDCl₃) δ 7.26-7.19 (m, 3H), 7.06 (d, *J* = 5.6 Hz, 1H), 6.97-6.86 (m, 3H), 6.40-6.19 (m, 1H), 4.85-4.83 (m, 1H), 4.17-4.00 (m, 1H), 3.88 (s, 3H), 3.34-3.25 (m, 2H), 3.00-2.92 (m, 3H), 2.84-2.80 (m, 4H), 2.13-2.03 (m, 1H), 1.85-1.73 (m, 2H), 1.72-1.62 (m, 1H), 1.61-1.48 (m, 1H). Chiral SFC: CO₂ and n-hexane:EtOH (1:4) containing 0.1% DEA over a CHIRALPAK^{®} IG column (4.6 × 250 mm 5 µm), retention time = 10.11 min.

**Compound 1039:** LCMS: (M+H)⁺ = 411; purity = 100% (214 nm); retention time = 1.59 min. Method E ¹H NMR: (400 MHz, CDCl₃) δ 7.26-7.20 (m, 3H), 7.07 (d, *J* = 6.8 Hz, 1H), 6.96-6.88 (m, 3H), 6.38-6.17 (m, 1H), 4.92-4.90 (m, 1H), 4.09-3.98 (m, 1H), 3.88 (s, 3H), 3.41-3.28 (m, 2H), 3.02-2.80 (m, 7H), 2.20-1.95 (m, 2H), 1.85-1.71 (m, 1H), 1.70-1.63 (m, 1H), 1.61-1.53 (m, 1H). Chiral SFC: CO₂ and *n*-hexane:EtOH (1:4) containing 0.1% DEA over a CHIRALPAK^{®} IG column (4.6 x 250 mm 5 µm), retention time = 11.99 min.

Compounds 1040 and 1041 were prepared analogously to 1004. The two diastereomers were separated by chiral SFC eluting with CO₂ and MeOH containing 0.2% methanolic ammonia over a CHIRALPAK^{®} IG column (4.6 x 100 mm 5 µm) to give **compound 1040** (11.7 mg) and **compound 1041** (12.1 mg).

**Compound 1040:** LCMS: (M+H)⁺ = 399; purity = 98.88% (214 nm); retention time = 1.882 min. Method C ¹H NMR (400 MHz, DMSO-*d₆*) δ 7.24-7.23 (m, 4H), 7.14 (t, J = 9.2 Hz, 1H), 7.04-7.03 (m, 1H), 6.89-6.88 (br, 1H), 6.21-6.20 (br, 1H), 4.63-4.62 (br, 1H), 3.87 - 3.73 (m, 1H), 3.58 -3.57(br, 1H), 3.06-3.05 (br, 1H), 2.87-2.86 (m, 2H), 2.60-2.58 (m, 5H), 2.06 - 1.66 (m, 2H), 1.56-1.55 (br, 1H), 1.44-1.42 (br, 1H), 1.36- 1.27 (m, 1H). Chiral SFC: CO₂ and MeOH containing 0.2% methanolic ammonia over a CHIRALPAK^{®} IG column (4.6 x 100 mm 5 µm), retention time = 2.42 min.

**Compound 1041:** LCMS: (M+H)⁺ = 399; purity = 100% (214 nm); retention time = 1.892 min. Method C ¹H NMR (400 MHz, DMSO-*d₆*) δ 7.28 (d, J = 32.0 Hz, 3H), 7.15 (tt, J = 9.2, 2.2 Hz, 1H), 7.05-7.04 (m, 1H), 6.87-6.86 (m, 1H), 6.17 (s, 1H), 4.65-4.64 (br, 1H), 3.74-3.73 (br, 1H), 3.42-3.40 (br, 1H), 3.04-3.03 (br, 1H), 2.88-2.86 (br, 2H), 2.63 (d, J = 32.3 Hz, 3H), 2.27 (d, J = 45.9 Hz, 1H), 1.96 (d, J = 25.7 Hz, 1H), 1.58-1.57 (br, 2H), 1.47-1.46 (br, 1H). Chiral SFC: CO₂ and MeOH containing 0.2% methanolic ammonia over a CHIRALPAK^{®} IG column (4.6 x 100 mm 5 µm), retention time = 2.86 min.

Compounds 1042 and 1043 were prepared analogously to 1004. The two diastereomers were separated by chiral SFC eluting with CO₂and EtOH containing 1% methanolic ammonia over a CHIRALPAK^{®} AD column (20 x 250 mm 10 µm) to give **compound 1042** (19.8 mg) and **compound 1043** (13.6 mg).

**Compound 1042:** LCMS: (M+H)⁺ = 430.2; purity = 100% (214 nm); retention time = 1.285 min. Method A1 ¹H NMR: (400 MHz, CD₃OD) δ 8.87-8.86 (m, 2H), 7.49 (d, *J=* 8.8 Hz, 2H), 7.36-7.35 (m, 2H), 7.19-7.13 (m, 2H), 7.13-7.09 (m, H), 7.05-7.01 (m, 1H), 6.29 (s, 1H), 4.75-4.67 (m, 1H), 3.90 (dt, *J* = 13.2, 4.2 Hz, 1H), 3.80-3.79 (br, 1H), 3.19-3.13 (m, 1H), 2.91-2.82 (m, 1H), 2.82-2.59 (m, 6H), 1.97-1.96 (br, 1H), 1.86-1.61 (m, 2H), 1.58-1.49 (m, 1H), 1.19-1.18 (br, 1H). Chiral SFC: CO₂ and MeOH containing 0.2% methanolic ammonia over a CHIRALPAK^{®} IG column (4.6 x 100 mm 5 µm), retention time = 4.02 min.

**Compound 1043:** LCMS: (M+H)⁺ = 430.2; purity = 100% (214 nm); retention time = 1.284 min. Method A1 ¹H NMR: (400 MHz, CD₃OD) δ 8.87-8.86 (m, 2H), 8.41 (s, 1H), 7.57-7.38 (m, 2H), 7.37-7.28 (m, 1H), 7.22-7.09 (m, 4H), 6.35-6.17 (m, 1H), 4.93-4.92 (br, 1H), 3.88-3.87 (br, 1H), 3.61-3.34 (m, 2H), 3.18-3.00 (m, 4H), 2.97-2.70 (m, 3H), 2.25-2.24 (br, 1H), 2.06-2.05 (br, 1H), 1.98-1.61 (m, 3H). Chiral SFC: CO₂ and MeOH containing 0.2% methanolic ammonia over a CHIRALPAK^{®} IG column (4.6 x 100 mm 5 µm), retention time = 9.64 min.

Compounds 1044 and 1045 were prepared analogously to 1004. The two diastereomers were separated by chiral SFC eluting with CO₂and EtOH containing 1% methanolic ammonia over a CHIRALPAK^{®} AD column (4.6 x 100 mm 5 µm) to give **compound 1044** (67.9 mg) and **compound 1045** (72.0 mg).

**Compound 1044:** LCMS: (M+H)⁺ = 349.1; Purity = 100% (214 nm); retention time = 1.499 min. Method E ¹H NMR (400 MHz, DMSO-*d₆*) δ 7.41 (d, J = 7.5 Hz, 1H), 7.34 (d, J = 4.3 Hz, 3H), 7.25 (ddd, J = 19.0, 14.2, 7.3 Hz, 4H), 7.06 (t, J = 8.5 Hz, 1H), 6.04 - 5.96 (m, 1H), 4.89 (dd, J = 59.0, 13.6 Hz, 2H), 4.60 - 4.50 (m, 1H), 2.83 (dd, J = 14.3, 7.9 Hz, 1H), 2.76-2.55(m, 1H), 2.48 - 2.31 (m, 2H), 1.93 - 1.73 (m, 3H), 1.63 - 1.45 (m, 1H), 1.44-1.27 (m, 2H), 1.18-1.02 (m, 1H). Chiral SFC: CO₂ and MeOH containing 0.2% methanolic ammonia over a CHIRALPAK^{®} IG column (4.6 x 100 mm 5 µm), retention time = 1.81 min. Chiral SFC: CO₂ and EtOH containing 1% methanolic ammonia over a CHIRALPAK^{®} AD-H column (4.6 x 100 mm 5 µm), retention time = 1.35 min.

**Compound 1045:** LCMS: (M+H)⁺ = 349.2; Purity = 100% (214 nm); retention time = 1.501 min. Method E ¹H NMR (400 MHz, DMSO-*d₆*) δ 7.41 (d, J = 7.4 Hz, 1H), 7.35 - 7.25 (m, 5H), 7.25 - 7.18 (m, 2H), 7.03 (dd, J = 31.6, 7.7 Hz, 1H), 6.00 (d, J = 16.1 Hz, 1H), 5.05 - 4.77 (m, 2H), 4.52 (dd, J = 18.5, 14.2 Hz, 1H), 3.10 - 2.92 (m, 1H), 2.87 - 2.55 (m, 1H), 2.48-2.42 (m, 1H), 2.37-2.24 (m, 1H), 2.08 - 1.76 (m, 1H), 1.62-1.52 (m, 1H), 1.50 - 1.26 (m, 2H), 0.89-0.80 (m, 1H), 0.71-0.62 (m, 1H). Chiral SFC: CO₂and MeOH containing 0.2% methanolic ammonia over a CHIRALPAK^{®} IG column (4.6 x 100 mm 5 µm), retention time = 1.91 min. Chiral SFC: CO₂and EtOH containing 1% methanolic ammonia over a CHIRALPAK^{®} AD-H column (4.6 x 100 mm 5 µm), retention time = 1.63 min.

### Reference Example 3: Synthesis of Compound 1046

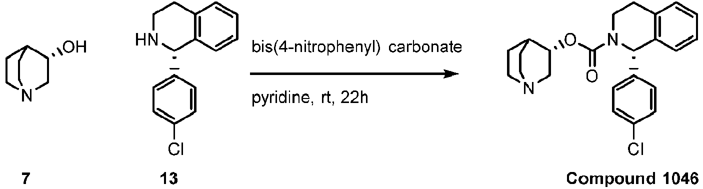

Under an argon atmosphere, a solution of (S)-(+)-3-quinuclidinol **7** (57 mg, 0.451 mmol) and bis(4-nitrophenyl) carbonate (137 mg, 0.451 mmol) in pyridine (5 mL) was stirred at room temperature for 4 hours. Then, (S)-1-(4-chlorophenyl)-1,2,3,4-tetrahydroisoquinoline **10** (110 mg, 0.451 mmol) was added and stirring was continued for 18 hours. The mixture was poured into a 1:1 mixture of ice and saturated potassium carbonate (30 mL) and extracted with two 25 mL portions of ethyl acetate. The combined organic layers were dried over sodium sulfate and concentrated under reduced pressure. The residue was purified by flash silica gel chromatography eluting with a gradient of chloroform:7M NH₃ in methanol = 1:0 to 9:1 to obtain **compound 1046** (82 mg, 0.207 mmol) after lyophilisation from acetonitrile/water. **Compound 1046:** LCMS: (M+H)⁺ = 397.1, retention time = 2.80 min., Method G. ¹H NMR (400 MHz, Chloroform-*d*), observed as a mixture of rotamers, δ 7.26 - 7.11 (m, 7H), 7.08 - 6.98 (m, 1H), 6.57 - 6.09 (m, 1H), 4.90 - 4.70 (m, 1H), 4.28 - 3.87 (m, 1H), 3.44 -3.15 (m, 2H), 3.07-2.68 (m, 7H), 2.14- 2.04 (m, 1H), 1.76- 1.65 (m, 1H), 1.65 - 1.51 (m, 1H), 1.48-1.34(m, 1H).

### Example 1: Synthesis of Compound 1047

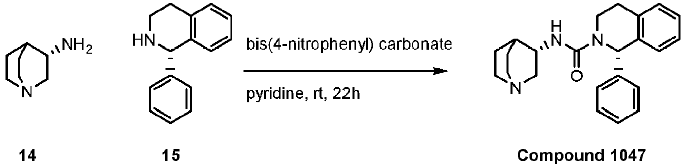

Under an argon atmosphere, a suspension of (S)-quinuclidin-3-amine **14** (120 mg, 0.951 mmol) and bis(4-nitrophenyl) carbonate (289 mg, 0.951 mmol) in pyridine (5 mL) was stirred at room temperature for 4 hours. Then, (1*S*)-1-phenyl-1,2,3,4-tetrahydroisoquinoline **15** (199 mg, 0.951 mmol) was added and stirring was continued for 18 hours. The mixture was poured into a 1:1 mixture of ice and saturated aqueous potassium carbonate (30 mL) and extracted with two 20 mL portions of ethyl acetate. The combined organic layers were dried over sodium sulfate, concentrated under reduced pressure and purified by flash silica gel chromatography eluting with a gradient of chloroform:7M NH₃ in methanol = 1:0 to 9:1 to obtain **compound 1047** (235 mg, 0.650 mmol). **Compound 1047:** LCMS: (M+H)⁺ = 362.2, retention time = 2.54 min., Method G. ¹H NMR (400 MHz, Chloroform-*d*) δ 7.34 - 7.27 (m, 4H), 7.26 - 7.13 (m, 5H), 6.17 (s, 1H), 4.58 (d, J = 6.7 Hz, 1H), 3.92 - 3.79 (m, 1H), 3.77 - 3.66 (m, 2H), 3.40 - 3.29 (m, 1H), 2.98 - 2.84 (m, 2H), 2.84 - 2.63 (m, 4H), 2.50 - 2.35 (m, 1H), 1.85 - 1.78 (m, 1H), 1.65 - 1.54 (m, 2H), 1.35 - 1.23 (m, 2H).

### Example 2: Synthesis of Compound 1048

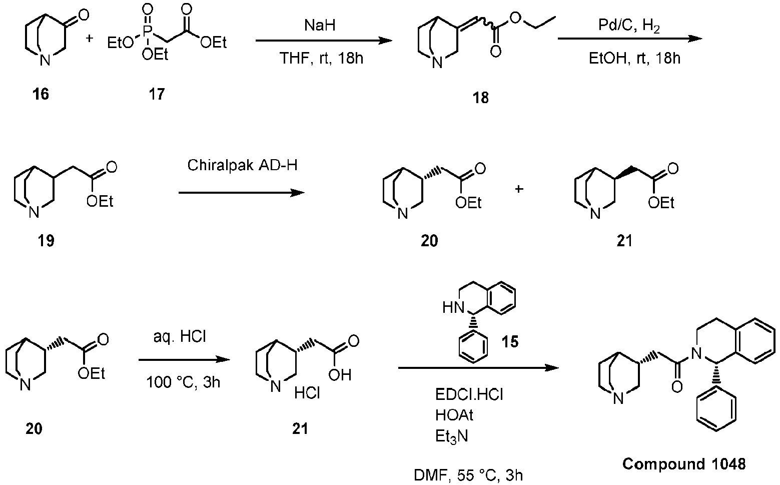

Step 1. Horner-Wadsworth-Emmons homologation: To an ice cold suspension of NaH (60% in oil, 0.96 g, 24 mmol) in THF (30 mL) was added ethyl 2-(diethoxyphosphoryl)acetate **17** (4.7 g, 21mmol). The mixture was stirred for 30 min. Then quinuclidinone **16** (2.5g, 20 mmol) in THF (10 mL) was added, the mixture was stirred at room temperature overnight. Water (20 mL) was added to quench the reaction, and the mixture was extracted with three 30 mL portions of DCM. The combined organic layers were dried, filtered, and concentrated to give the desired product **18**. LCMS: (M+H)⁺ = 196; Purity 100% (UV 254 nm); Retention time = 1.057 min. Method G

Step 2 Reduction of the double bond A suspension of **18** (1.5g, 7.7mol) and Pd/C (10%, 150 mg) in EtOH (15 ml) was stirred under hydrogen atmosphere, and the mixture was stirred at room temperature overnight. LCMS showed the reaction was complete. The mixture was filtered; the solvent was removed *in vacuo* to give the crude product. The crude product was purified by preparative HPLC to give the product 1.2 g of the desired ester **19**. LCMS: (M+H)⁺ = 198; Purity 100% (UV 214 nm); Retention time = 1.086 min Method G

Step 3 Chiral separation of enantiomers of ester **19**: Racemic ethyl 2-(quinuclidin-3-yl)acetate **19** (3.82 g, 19.3 mmol) was resolved by preparative chiral HPLC (Chiralpak AD-H, 20 x 250 mm, 5 µm, Flow: 18 mL/min, isocratic 90/10, time: 30 min, Eluent A: heptane (+ 0.1% DEA), Eluent B: ethanol) to give: Ethyl (*S*)-2-(quinuclidin-3-yl)acetate **20** (1.03 g, 5.22 mmol) as a waxy solid: chiral HPLC: 100%, retention time = 10.66 min (Chiralcel AD-H (250 x 4.6 mm, 5 µm); mobile phase heptane (+0.1% DEA):ethanol (90:10); flow rate: 1.0 mL/min). Optical rotation [α]_{D}^{23.3}: -70.2 (c = 0.74, methanol). Ethyl (*R*)-2-(quinuclidin-3-yl)acetate **21** (1.16 g, 5.22 mmol): chiral HPLC: 100%, retention time = 18.50 min (Chiralcel AD-H (250 x 4.6 mm, 5 µm); mobile phase heptane (+0.1% DEA):ethanol (90:10); flow rate: 1.0 mL/min). Optical rotation [α]_{D}^{23.4}: 70.3 (c = 0.74, methanol).

Step 4. Hydrolysis of the ester A solution of ethyl (*S*)-2-(quinuclidin-3-yl)acetate **20** (0.95 g, 4.82 mmol) in 3M aqueous hydrochloric acid (50 mL, 150 mmol) was heated under reflux for 3 hours. The mixture was concentrated under reduced pressure and co-evaporated with diethyl ether twice to obtain (*S*)-2-(quinuclidin-3-yl)acetic acid hydrochloride **21** (0.95 g, 4.62 mmol). Optical rotation [α]_{D}^{23.9}: -66.8 (c = 0.54, methanol). ¹H NMR (400 MHz, deuterium oxide) δ 3.57 - 3.45 (m, 1H), 3.32 - 3.08 (m, 4H), 2.90 - 2.79 (m, 1H), 2.62 - 2.39 (m, 3H), 1.99 - 1.84 (m, 4H), 1.84 - 1.69 (m, 1H).

Step 5. A suspension of (1*S*)-1-phenyl-1,2,3,4-tetrahydroisoquinoline **15** (76 mg, 0.365 mmol), (*S*)-2-(quinuclidin-3-yl)acetic acid hydrochloride **21** (75 mg, 0.365 mmol), *N-*Ethyl-*N'*-(3-dimethylaminopropyl)carbodiimide hydrochloride (77 mg, 0.401 mmol), 1-Hydroxy-7-azabenzotriazole (5 mg, 0.04 mmol) and triethylamine (0.152 mL, 1.09 mmol) in *N*,*N*-dimethylformamide (5 mL) was heated at 55 °C for 3 hours. The mixture was poured into a 1:1 mixture of ice and saturated potassium carbonate (30 mL) and extracted with two 25 mL portions of ethyl acetate. The combined organic layers were dried over sodium sulfate and concentrated under reduced pressure. The residue was purified by silica flash chromatography eluting with a gradient of chloroform:7M NH₃ in methanol = 1:0 to 9:1 to obtain **compound 1048.** Further purification by acidic preparative MPLC Method B (Linear Gradient: t=0 min 5% A, t=1 min 5% A, t=2 min 20% A; t=17 min 60% A; t=18 min 100%; t=23 min 100% A; detection: 220/254 nm) followed by lyophilisation afforded **compound 1048** as its formic acid salt. The formic acid salt was dissolved in 10 mL 1:1 chloroform and saturated sodium bicarbonate and stirred vigorously for 30 minutes. The layers were separated, the organic layer was dried over sodium sulfate and concentrated under reduced pressure, followed by lyophilisation (acetonitrile/water) to obtain **compound 1048** (85 mg, 0.236 mmol). **Compound 1048:** LCMS: 100%, retention time = 2.59 min., (M+H)⁺ = 361.2 (method G). ¹H NMR (400 MHz, Chloroform-*d*), observed as a mixture of rotamers, δ 7.34 - 7.14 (m, 8.23H), 7.10 (d, J = 7.7 Hz, 0.77H), 6.96 (s, 0.77H), 6.03 (s, 0.23H), 4.30 - 4.15 (m, 0.23H), 3.85 - 3.74 (m, 0.77H), 3.53 - 3.42 (m, 0.77H), 3.38 (s, 0.23H), 3.29 - 3.17 (m, 1H), 3.04 - 2.92 (m, 1H), 2.91 - 2.66 (m, 5H), 2.66 - 2.54 (m, 0.46H), 2.53 - 2.40 (m, 1.54H), 2.40 -2.29 (m, 1H), 2.29 - 2.18 (m, 1H), 1.69- 1.49 (m, 4H), 1.48- 1.33 (m, 1H).

Compound 1049 was prepared analogously. **Compound 1049** was purified by acidic preparative MPLC, Method B (Linear Gradient: t=0 min 5% A, t=1 min 5% A, t=2 min 20% A; t=17 min 60% A; t=18 min 100%; t=23 min 100% A; detection: 220/254 nm) followed by lyophilisation as its formic acid salt. This material was desalted by SCX-2 chromatography (product eluted with 2.5 M ammonia in methanol). Product fractions were concentrated under reduced pressure and the residue was lyophilised (acetonitrile/water) to obtain **Compound 1049.** LCMS: 100%, retention time = 2.76 min., (M+H)⁺ = 395.2 (method G). ¹H NMR (400 MHz, Chloroform-d), observed as a mixture of rotamers, δ 7.32 - 7.16 (m, 5H), 7.16-7.02 (m, 3H), 6.90 (s, 0.85H), 5.99 (s, 0.15H), 4.27-4.14 (m, 0.15H), 3.86-3.73 (m, 0.85H), 3.49 - 3.38 (m, 0.85H), 3.37 - 3.28 (m, 0.15H), 3.28 - 3.17 (m, 1H), 3.04 - 2.91 (m, 1H), 2.91 - 2.73 (m, 5H), 2.65 - 2.53 (m, 0.3H), 2.52 - 2.40 (m, 1.7H), 2.40 - 2.28 (m, 1H), 2.27 - 2.14 (m, 1H), 1.71 - 1.50 (m, 4H), 1.48 - 1.32 (m, 1H).

Compound 1051 was prepared analogously. **Compound 1051**was prepared using (*R*)-2-(quinuclidin-3-yl)acetic acid hydrochloride and (1*S*)-1-phenyl-1,2,3,4-tetrahydroisoquinoline according to the procedure above. LCMS: 98%, retention time = 2.60 min, (M+H)⁺ = 361.2 (method G). ¹H NMR (400 MHz, Chloroform-d), observed as a mixture of rotamers, δ 7.36 - 7.15 (m, 8.23H), 7.13 - 7.05 (m, 0.77H), 6.95 (s, 0.77H), 6.02 (s, 0.23H), 4.30 - 4.16 (m, 0.23H), 3.84 - 3.71 (m, 0.77H), 3.53 - 3.35 (m, 1H), 3.28 - 3.14 (m, 1H), 3.07 - 2.90 (m, 1H), 2.90 - 2.68 (m, 5H), 2.63 - 2.55 (m, 0.46H), 2.56 - 2.42 (m, 1.54H), 2.42 - 2.30 (m, 1H), 2.30 - 2.15 (m, 1H), 1.71 - 1.51 (m, 4H), 1.48 - 1.33 (m, 1H).

Compound 1052 was prepared analogously. **Compound 1052** was prepared using (*S*)-2-(quinuclidin-3-yl)acetic acid hydrochloride **22** and (S)-1-(4-(methylsulfonyl)phenyl)-1 ,2,3,4-tetrahydroisoquinoline **5** according to the procedure above. LCMS: (M+H)⁺ = 439; purity = 100% (214 nm); retention time = 1.352 min. Method A2 ¹H NMR(400 MHz, DMSO-*d₆*) δ 9.04 (br, 1H), 7.85 (d, J = 8.4 Hz, 2H), 7.44 (d, J = 8.4 Hz, 2H), 7.28 - 7.24 (m, 4H), 6.72 (s, 1H), 3.82 - 3.77 (m, 1H), 3.60 - 3.49 (m, 2H), 3.19 (s, 4H), 2.85 - 2.77 (m, 4H), 2.67 - 2.61 (m, 1H), 2.34 - 2.32 (m, 2H), 2.01 - 1.96 (m, 1H), 1.88 - 1.80 (m, 4H), 1.69 - 1.66 (m, 1H), 1.24 (br, 1H).

Compound 1053 was prepared analogously. **Compound 1053** was prepared using (*S*)-2-(quinuclidin-3-yl)acetic acid hydrochloride **22** and racemic 1-(2,4-difluorophenyl)-1,2,3,4-tetrahydroisoquinoline according to the procedure above. The diastereomers were purified by SFC eluting with MeOH containing 0.2% methanolic ammonia, ChiralPak IG (4.6 x 100 mm 5 µm). **Compound 1053:** LCMS: (M+H)⁺ = 397; purity = 100% (214 nm); retention time = 1.774 min. Method C ¹H NMR (400 MHz, DMSO-*d₆*) δ 7.20 (dd, J = 20.6, 9.9 Hz, 5H), 7.08 (d, J = 7.9 Hz, 1H), 6.99 (d, J = 8.6 Hz, 1H), 6.73 (s, 1H), 3.91 (d, J = 13.3 Hz, 1H), 3.57 (s, 1H), 2.92 (d, J = 5.6 Hz, 2H), 2.73 - 2.54 (m, 6H), 2.42 (dd, J = 16.0, 6.3 Hz, 1H), 2.14 (d, J = 13.2 Hz, 1H), 1.65-1.64 (brs, 1H), 1.48 (d, J = 19.4 Hz, 3H). Chiral SFC: CO₂ and EtOH containing 1% methanolic ammonia over a CHIRALPAK^{®} AD- column (4.6 x 100 mm 5 µm), retention time = 1.85 min.

Compound 1054 and 1055 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1054** (11.7 mg) and **compound 1055** (12.1 mg).

**Compound 1054** LCMS: (M+H)⁺ = 428; purity = 100% (214 nm); retention time = 1.481 min. Method C ¹H NMR (400 MHz, CDCl₃) δ 8.53 (d, J = 11.5 Hz, 2H), 8.09 (s, 1H), 7.69 - 7.52 (m, 2H), 7.36 (dd, J = 17.9, 8.4 Hz, 2H), 7.24 (d, J = 6.6 Hz, 2H), 7.11 (d, J = 7.2 Hz, 1H), 6.91 (s, 1H), 3.77 (d, J = 13.3 Hz, 1H), 3.53 (ddd, J = 17.8, 17.2, 7.6 Hz, 2H), 3.24-3.11 m, 2H), 2.98 (dd, J = 10.1, 5.4 Hz, 1H), 2.90 (d, J = 16.0 Hz, 1H), 2.75 (d, J = 12.4 Hz, 2H), 2.56 (d, J = 9.0 Hz, 1H), 2.25 - 2.19 (m, 1H), 1.94-1.93 (brs, 2H), 1.70 - 1.57 (m, 1H), 1.28-1.27 (brs, 3H). Chiral SFC: CO₂ and EtOH containing 1% methanolic ammonia over an EnantioPak^{®} AS column (4.6 x 100 mm 5 µm), retention time = 1.38 min.

**Compound 1055:** LCMS: (M+H)⁺ = 428; purity = 100% (214 nm); retention time = 1.444 min. Method C ¹H NMR (400 MHz, CDCl₃) δ 8.53 (d, J = 11.5 Hz, 2H), 8.09 (s, 1H), 7.69 - 7.52 (m, 2H), 7.36 (dd, J = 17.9, 8.4 Hz, 2H), 7.24 (d, J = 6.6 Hz, 2H), 7.11 (d, J = 7.2 Hz, 1H), 6.91 (s, 1H), 3.77 (d, J = 13.3 Hz, 1H), 3.53 (ddd, J = 17.8, 17.2, 7.6 Hz, 2H), 3.24-3.11 m, 2H), 2.98 (dd, J = 10.1, 5.4 Hz, 1H), 2.90 (d, J = 16.0 Hz, 1H), 2.75 (d, J = 12.4 Hz, 2H), 2.56 (d, J = 9.0 Hz, 1H), 2.25 - 2.19 (m, 1H), 1.94-1.93 (brs, 2H), 1.70 - 1.57 (m, 1H), 1.28-1.27 (brs, 3H). Chiral SFC: CO₂ and MeOH containing 0.2% methanolic ammonia over an EnantioPak^{®} AS column (4.6 x 100 mm 5 µm), retention time = 2.67 min.

Compound 1056 and 1057 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1056** (5.8 mg) and **compound 1057** (6.0 mg).

**Compound 1056** LCMS: (M+H)⁺ = 391; purity = 98.4% (214 nm); retention time = 1.655 min. Method F ¹H NMR(400 MHz, CD₃OD) δ 7.15 - 7.04 (m, 3H), 6.95 (d, *J=* 8.0 Hz, 2H), 6.81 - 6.67 (m, 3H), 3.82 - 3.79 (m, 1H), 3.65 (s, 3H), 3.43 - 3.36 (m, 1H), 3.20 - 3.09 (m, 1H), 2.93 - 2.82 (m, 1H), 2.81 - 2.69 (m, 2H), 2.60 - 2.40 (m, 3H), 2.21 - 2.07 (m, 2H), 1.96 - 1.92(m, 1H), 1.79 - 1.76 (m, 1H), 1.64 - 1.58 (m, 2H), 1.50 - 1.47 (m, 2H), 1.20 (br, 2H). Chiral SFC: CO₂ and EtOH containing 1% methanolic ammonia over a CHIRALPAK^{®} AD-H column (4.6 x 100 mm 5 µm), retention time = 2.12 min.

**Compound 1057** LCMS: (M+H)⁺ = 391; purity = 100% (214 nm); retention time = 1.662 min. Method F ¹H NMR(400 MHz, CD₃OD) δ 7.17-6.97 (m, 3H), 6.78 (d, *J* = 12.0 Hz, 2H), 6.72 - 6.68 (m, 3H), 3.80 - 3.66 (m, 1H), 3.62 (s, 3H), 3.44 - 3.36 (m, 1H), 3.20 - 3.14 (m, 1H), 2.93 - 2.86 (m, 3H), 2.62 - 2.46 (m, 2H), 2.41 - 2.37 (m, 1H), 2.21 - 2.07 (m, 2H), 1.96 - 1.92 (m, 1H), 1.82 - 1.76 (m, 1H), 1.63 (br, 3H), 1.53 - 1.40 (m, 1H), 1.19 (br, 2H). Chiral SFC: CO₂ and EtOH containing 1% methanolic ammonia over a CHIRALPAK^{®} AD-H column (4.6 x 100 mm 5 µm), retention time = 3.36 min.

Compound 1058 and 1059 were prepared analogously. The two diastereomers were separated by chiral SFC eluting with CO₂ and MeOH containing 0.2% methanolic ammonia over a CHIRALPAK^{®} OD-H column (4.6 x 100 mm 5 µm) to give **compound 1059** (16.2 mg) and **compound 1058** (12.4 mg).

**Compound 1059** LCMS: (M+H)⁺ = 379; purity = 100% (214 nm); retention time = 1.718 min. Method C ¹H NMR (400 MHz, DMSO-*d₆*) δ 7.25 (d, J = 3.9 Hz, 2H), 7.21 (dd, J = 10.0, 5.8 Hz, 2H), 7.17 (s, 1H), 7.14-7.10 (m, 3H), 6.70 (s, 1H), 3.84 (dd, J = 11.6, 6.6 Hz, 1H), 3.47 -3.40 (m, 1H), 3.03 -2.90 (m, 2H), 2.78 (dt, J = 16.2, 4.5 Hz, 1H), 2.68 (d, J = 7.0 Hz, 4H), 2.23 (dd, J = 13.6, 6.0 Hz, 1H), 2.02 (d, J = 7.0 Hz, 2H), 1.66-1.65 (brs, 1H), 1.57 - 1.45 (m, 3H), 1.26 (d, J = 18.3 Hz, 2H). Chiral SFC: CO₂ and MeOH containing 0.2% methanolic ammonia over a CHIRALPAK^{®} OD-H column (4.6 x 100 mm 5 µm), retention time = 2.10 min.

**Compound 1058** LCMS: (M+H)⁺ = 379; purity = 100% (214 nm); retention time = 1.718 min. Method C ¹H NMR (400 MHz, DMSO-*d₆*) δ 7.25 (d, J = 3.6 Hz, 2H), 7.22 (d, J = 7.4 Hz, 1H), 7.20 -7.15 (m, 2H), 7.15-7.11 (m, 3H), 6.70 (s, 1H), 3.91 -3.79 (m, 1H), 3.46 - 3.37 (m, 1H), 3.03 - 2.90 (m, 2H), 2.78 (dd, J = 11.8, 4.4 Hz, 1H), 2.70 - 2.57 (m, 5H), 2.45 (dd, J = 15.9, 6.4 Hz, 1H), 2.20 (dd, J = 13.4, 5.9 Hz, 1H), 1.99-1.98 (brs, 1H), 1.67-1.65 (brs, 1H), 1.57 - 1.43 (m, 3H), 1.25 (d, J = 13.0 Hz, 1H). Chiral SFC: CO₂ and MeOH containing 0.2% methanolic ammonia over a CHIRALPAK^{®} OD-H column (4.6 x 100 mm 5 µm), retention time = 3.02 min.

Compound 1060 and 1061 were prepared analogously. The two diastereomers were separated by chiral SFC eluting with CO₂ and MeOH containing 0.2% methanolic ammonia over a CHIRALPAK^{®} AD column (20 x 250 mm 10 µm) to give **compound 1060** (4.5 mg) and **compound 1061** (4.5 mg).

**Compound 1060** LCMS: (M+H)⁺ = 409.2; purity = 99% (214 nm); retention time = 1.659 min. Method C ¹H NMR: (400 MHz, CD₃OD) δ 7.19-7.09 (m, 3H), 6.98 (d, *J=* 7.6 Hz, 1H), 6.88 (t, *J* = 8.8 Hz, 1H), 6.83-6.68 (m, 2H), 6.65 (s, 1H), 3.81 (dt, *J* = 13.6, 4.8 Hz, 1H), 3.73 (s, 3H), 3.43-3.33 (m, 1H), 3.08-3.00 (m, 1H), 2.97-2.86 (m, 1H), 2.84-2.63 (m, 5H), 2.60-2.45 (m, 2H), 2.33 (dd, *J* = 13.6, 6.8 Hz, 1H), 2.19-2.09 (m, 1H), 1.71-1.70 (brs, 1H), 1.66-1.48 (m, 3H), 1.41-1.40 (brs, 1H). Chiral SFC: CO₂ and EtOH containing 1% methanolic ammonia over a CHIRALPAK^{®} AD-H column (4.6 x 100 mm 5 µm), retention time = 2.34 min.

**Compound 1061** LCMS: (M+H)⁺ = 409.2; purity = 98% (214 nm); retention time = 1.657 min. Method C ¹H NMR: (400 MHz, CD₃OD) δ 7.19-7.09 (m, 3H), 6.98 (d, *J* = 7.6 Hz, 1H), 6.88 (t, *J* = 8.8 Hz, 1H), 6.83-6.68 (m, 2H), 6.65 (s, 1H), 3.82 (dt, *J* = 13.6, 4.8 Hz, 1H), 3.73 (s, 3H), 3.45-3.35 (m, 1H), 3.15-3.04 (m, 1H), 2.95-2.83 (m, 1H), 2.81-2.62 (m, 6H), 2.61-2.53 (m, 1H), 2.51-2.43 (m, 1H), 2.35-2.25 (m, 1H), 2.15-2.07 (m, 1H), 1.78-1.67 (m, 1H), 1.64-1.52 (m, 3H), 1.44-1.34 (m, 1H). Chiral SFC: CO₂ and EtOH containing 1% methanolic ammonia over a CHIRALPAK^{®} AD-H column (4.6 x 100 mm 5 µm), retention time = 3.21 min.

Compounds 1062 and 1063 were prepared analogously. The two diastereomers were separated by chiral SFC eluting with CO₂ and MeOH containing 0.2% methanolic ammonia over a CHIRALPAK^{®} AD-H column (4.6 x 100 mm 5 µm) to give **compound 1063** (18.1 mg) and **compound 1062** (17.3 mg).

**Compound 1062** LCMS: (M+H)⁺ = 379; purity = 100% (214 nm); retention time = 1.479 min. Method A2 ¹H NMR(400 MHz, CDCl₃) δ 7.25-7.20 (m, 4H), 7.10 (dd, *J* = 7.2, 3.2 Hz, 1H), 7.0(d, J = 8.0Hz, 1H), 6.95-6.86(m, 3H), 3.79 (dt, *J* = 9.2, 4.4 Hz, 1H), 3.47 (td, *J*= 8.4, 4.4Hz, 1H), 3.27-3.21 (m, 1H), 3.00-2.96 (m, 1H), 2.89-2.81 (m, 5H), 2.50-2.32 (m, 3H), 2.26-2.21 (m, 1H), 1.67-1.63 (m, 4H), 1.47-1.43 (m, 1H). Chiral SFC: CO₂ and EtOH containing 1% methanolic ammonia over a CHIRALPAK^{®} AD-H column (4.6 x 100 mm 5 µm), retention time = 3.29 min.

**Compound 1063** LCMS: (M+H)⁺ = 379; purity = 100% (214 nm); retention time = 1.480 min. Method A2 ¹H NMR(400 MHz, CDCl₃) δ 7.25-7.20 (m, 4H), 7.09 (dd, *J* = 7.2, 3.3 Hz, 1H), 7.00 (d, *J* = 8.0Hz, 1H), 6.95-6.84(m, 3H), 3.78 (dt, J = 9.2, 4.4 Hz, 1H), 3.47 (td, *J* = 10.0, 4.4 Hz, 1H), 3.29-3.23 (m, 1H), 3.01-2.92 (m, 1H), 2.88-2.82 (m, 5H), 2.49 (dd, J = 7.6, 4.0 Hz, 2H), 2.27-2.23 (m, 1H), 1.70-1.63 (m, 4H), 1.49-1.43 (m, 1H). Chiral SFC: CO₂ and EtOH containing 1% methanolic ammonia over a CHIRALPAK^{®} AD-H column (4.6 x 100 mm 5 µm), retention time = 2.72 min.

Compounds 1064 and 1065 were prepared analogously. The two diastereomers were separated by chiral SFC eluting with CO₂ and EtOH containing 1% methanolic ammonia over a CHIRALPAK^{®} IG column (4.6 x 100 mm 5 µm) to give **compound 1064** (4.8 mg) and **compound 1065** (4.6 mg).

**Compound 1064** LCMS: (M+H) = 397; purity = 100% (214 nm); retention time = 1.722 min. Method F ¹H NMR (400 MHz, CDCl₃): δ 7.28 - 7.20 (m, 3H), 7.08 - 6.93 (m, 4H), 6.85 (s, 1H), 3.78 - 3.75 (m, 1H), 3.50 - 3.28 (m, 2H), 2.99 - 2.71 (m, 6H), 2.51 - 2.21 (m, 4H), 1.81 -1.58 (m, 4H), 1.51-1.48 (m, 1H). Chiral SFC: CO₂ and EtOH containing 1% methanolic ammonia over a CHIRALPAK^{®} AD-H column (4.6 x 100 mm 5 µm), retention time = 2.64 min.

**Compound 1065** LCMS: (M+H)⁺ = 397; purity = 95% (214 nm); retention time = 1.727 min. Method F ¹H NMR(400 MHz, CDCl₃): δ 7.26- 7.21 (m, 3H), 7.11- 6.88 (m, 4H), 6.85 (s, 1H), 3.78 -3.74 (m, 1H), 3.50- 3.27 (m, 2H), 3.02- 2.71 (m, 6H), 2.51- 2.20 (m, 4H), 1.81-1.55 (m, 4H), 1.51-1.48 (m, 1H). Chiral SFC: CO₂ and EtOH containing 1% methanolic ammonia over a CHIRALPAK^{®} AD-H column (4.6 x 100 mm 5 µm), retention time = 2.08 min.

Compounds 1066 and 1067 were prepared analogously. The two diastereomers were separated by chiral SFC eluting with CO₂ and EtOH containing 1% methanolic ammonia over a CHIRALPAK^{®} IG column (4.6 x 100 mm 5 µm) to give **compound 1066** (19 mg) and **compound 1067** (10 mg).

**Compound 1066:** LCMS: (M+H)⁺ = 444; purity = 100% (214 nm); retention time = 1.531 min. Method C ¹H NMR (400 MHz, DMSO-*d₆*) δ 7.54 (d, J = 8.7 Hz, 2H), 7.27 - 7.20 (m, 4H), 7.12 (dd, J = 20.0, 7.9 Hz, 2H), 6.68 (s, 1H), 3.78 (t, J = 7.0 Hz, 2H), 2.99 - 2.90 (m, 2H), 2.78 - 2.64 (m, 4H), 2.44 (s, 1H), 2.30 - 2.14 (m, 2H), 2.03 (dt, J = 15.2, 7.6 Hz, 4H), 1.66- 1.49 (m, 4H), 1.31 - 1.15 (m, 6H). Chiral HPLC: eluting with EtOH containing 0.1% DEA over a CHIRALPAK^{®} IG column (4.6 x 100 mm 5 µm), retention time = 52.95 min.

**Compound 1067:** LCMS: (M+H)⁺ = 444; purity = 100% (214 nm); retention time = 1.529 min. Method C ¹H NMR (400 MHz, DMSO-*d₆*) δ 7.54 (d, J = 8.6 Hz, 2H), 7.29 - 7.19 (m, 4H), 7.13 (dd, J = 20.7, 7.9 Hz, 2H), 6.68 (s, 1H), 3.78 (t, J = 7.0 Hz, 2H), 3.07 - 2.90 (m, 2H), 2.79 (dd, J = 14.1, 6.2 Hz, 4H), 2.44 (s, 1H), 2.36 - 2.17 (m, 2H), 2.04 (dd, J = 15.0, 7.5 Hz, 4H), 1.76 - 1.52 (m, 4H), 1.29 (d, J = 48.6 Hz, 4H). Chiral HPLC: eluting with EtOH containing 0.1% DEA over a CHIRALPAK^{®} IG column (4.6 x 100 mm 5 µm), retention time = 69.95 min.

Compounds 1068 and 1069 were prepared analogously. The two diastereomers were separated by chiral SFC eluting with CO₂ and EtOH containing 0.5% methanolic ammonia over a CHIRALPAK^{®} AD column (20 x 250 mm 10 µm) to give **compound 1068** (9.9 mg) and **compound 1069** (11.8 mg).

**Compound 1068** LCMS: (M+H)⁺ = 417; purity = 99% (214 nm); retention time = 1.44 min. Method A2 ¹H NMR: (400 MHz, CD₃OD) δ 7.26 (d, *J=* 4.0 Hz, 3H), 7.07 (d, *J* = 8.4 Hz, 3H), 6.95 (d, *J=* 8.4 Hz, 2H), 6.80 (s, 1H), 3.96-3.90 (m, 1H), 3.77-3.73 (m, 1H), 3.55-3.48 (m, 1H), 3.22-3.16 (m, 1H), 3.09-3.01 (m, 1H), 2.92-2.78 (m, 7H), 2.69-2.57 (m, 2H), 2.51-2.46 (m, 1H), 2.30-2.26 (m, 2H), 1.86-1.84 (m, 1H), 1.57-1.51 (m, 1H), 0.79-0.75 (m, 2H), 0.68-0.64 (m, 2H). Chiral SFC: CO₂ and EtOH containing 1% methanolic ammonia over a CHIRALPAK^{®} AD-H column (4.6 x 100 mm 5 µm), retention time = 5.00 min.

**Compound 1069** LCMS: (M+H)⁺ = 417; purity = 99% (214 nm); retention time = 1.44 min. Method A2 ¹H NMR: (400 MHz, CD₃OD) δ 7.26 (d, *J=* 4.0 Hz, 3H), 7.08 (d, *J* = 8.8 Hz, 3H), 6.95 (d, *J=* 8.8 Hz, 2H), 6.80 (s, 1H), 3.96-3.92 (m, 1H), 3.77-3.73 (m, 1H), 3.56-3.48 (m, 1H), 3.34-3.32 (m, 1H), 3.24-3.18 (m, 1H), 3.06-2.81 (m, 7H), 2.72-2.55 (m, 2H), 2.45-2.21 (m, 3H), 1.86-1.85 (m, 1H), 1.56-1.53 (m, 1H), 0.77 (d, *J=* 6.0 Hz, 2H), 0.66 (d, *J=* 1.6 Hz, 2H). Chiral SFC: CO₂ and EtOH containing 1% methanolic ammonia over a CHIRALPAK^{®} AD-H column (4.6 x 100 mm 5 µm), retention time = 2.41 min.

### Example 3: Synthesis of Compound 1070 and 1071

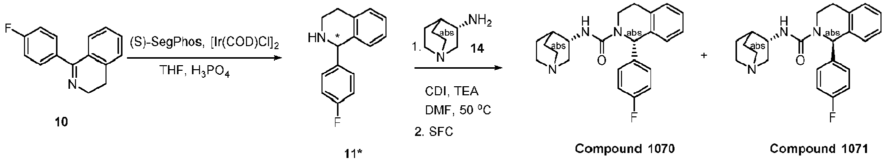

Step 1: To a solution of 1-(4-fluorophenyl)-3,4-dihydroisoquinoline **10** (2 g, 8.8 mmol) in THF (200 mL) was added (S)-SegPhos (100 mg, 0.16 mmol), [Ir(COD)Cl]₂ (50 mg, 0.16 mmol) and H₃PO₄ (85%)(0.5 mL) under N₂. The mixture was stirred at 60 °C for 12 hours under 60 bar H₂. The mixture was cooled to 25°C and concentrated *in vacuo* to remove THF, alkalized with NaOH (10% aq.) to pH = 11 and extracted with three 50 mL portions of ethyl acetate. The combined organic layers were washed three times with 50 mL of brine, dried and concentrated *in vacuo* to give 1-(4-fluorophenyl)-1 ,2,3,4-tetrahydroisoquinoline **11*** (800 mg) enriched with the (S)-enantiomer (~88% ee). This material was used directly in subsequent reactions without further purification. LCMS: (M+H)⁺ = 226; purity = 42% (214 nm); retention time = 1.757 min.

Step 2: To a solution of 1-(4-fluorophenyl)-1,2,3,4-tetrahydroisoquinoline **11*** (350 mg, 1.54 mmol) in DMF (2 mL) was added (S)-quinuclidin-3-amine **14** (291 mg, 2.31 mmol), CDI (162 mg, 3.08 mmol) and TEA (312 mg, 3.08 mmol). The mixture was stirred at 70 °C for 10 min. The mixture was cooled to 25 °C and 20 mL of water was added. The mixture was extracted with three 20 mL portions of ethyl acetate. The combined organic layers were washed three times with 20 mL of brine, dried and concentrated *in vacuo* to give crude product. The crude product was purified by Preparative HPLC (Mobile Phase : A: H₂O (10mM NH₄HCO₃) B:MeCN Gradient: 5%-95% B in 1.2min Flow Rate: 2.0 mL/min Column : XBridge C18 50 x 4.6 mm, 3.5 µm Oven Temperature: 40 °C UV214 nm,MASS 100-1000) to give 1-(4-fluorophenyl)-N-((S)-quinuclidin-3-yl)-3,4-dihydroisoquinoline-2(1H)-carboxamide (90 mg). LCMS: (M+H) = 380; purity = 100% (214 nm); retention time = 1.648 min.

Step 3: The two diastereomers were separated by chiral SFC eluting with CO₂ and MeOH containing 0.2% methanolic ammonia over a CHIRALPAK^{®} IG column (4.6 x 100 mm 5 µm) to give **compound 1070** (57.8 mg) and **compound 1071** (6.1 mg).

**Compound 1070** LCMS: (M+H)⁺ = 380; purity = 100% (214 nm); retention time = 1.632 min. Method C ¹H NMR(400 MHz, DMSO-*d₆*) δ 7.23 (t, J = 4.2 Hz, 2H), 7.19 (dd, J = 8.3, 3.3 Hz, 1H), 7.14 (td, J = 8.8, 2.6 Hz, 4H), 6.44 (s, 1H), 6.25 (d, J = 6.2 Hz, 1H), 3.84 (dt, J = 12.7, 5.1 Hz, 1H), 3.68 (d, J = 6.2 Hz, 1H), 3.28-3.17 (m, 1H), 3.06-2.96 (m, 1H), 2.92-2.75 (m, 2H), 2.70 (dt, J = 16.4, 4.7 Hz, 1H), 2.66-2.53 (m, 4H), 1.72 (d, J = 2.7 Hz, 1H), 1.66 (d, J = 3.0 Hz, 1H), 1.58-1.42 (m, 2H), 1.30-1.14 (m, 1H). Chiral SFC: CO₂ and MeOH containing 0.2% methanolic ammonia over a CHIRALPAK^{®} IG column (4.6 x 100 mm 5 µm), retention time = 2.36 min.

**Compound 1071** LCMS: (M+H)⁺ = 380; purity = 100% (214 nm); retention time = 1.597 min. Method C ¹H NMR: (400 MHz, DMSO-*d₆*) δ 7.23 (d, *J* = 3.8 Hz, 2H), 7.20 (dd, *J* = 8.0, 4.4 Hz, 1H), 7.18-7.08 (m, 5H), 6.45 (s, 1H), 6.28 (d, *J* = 5.9 Hz, 1H), 3.90-3.77 (m, 1H), 3.65 (s, 1H), 3.29-3.17 (m, 1H), 3.05-2.94 (m, 1H), 2.87 (ddd, *J* = 15.4, 9.2, 5.9 Hz, 1H), 2.81-2.66 (m, 2H), 2.66-2.54 (m, 3H), 2.47 (d, *J* = 5.4 Hz, 1H), 1.75 (d, *J* = 2.7 Hz, 2H), 1.53 (ddd, *J* = 15.8, 7.9, 4.9 Hz, 2H), 1.25-1.23 (brs, 2H). Chiral SFC: CO₂ and MeOH containing 0.2% methanolic ammonia over a CHIRALPAK^{®} IG column (4.6 x 100 mm 5 µm), retention time = 4.41 min.

### Example 4: Synthesis of Compound 1072

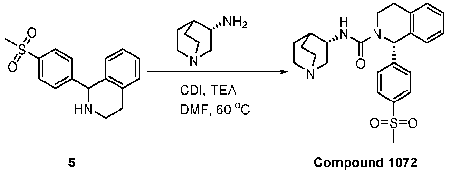

To a solution of 1-(S)-(4-(methylsulfonyl)phenyl)-1,2,3,4-tetrahydroisoquinoline **5** (287 mg, 1.0 mmol) in DMF (2 mL) was added (S)-quinuclidin-3-amine (400 mg, 2.0 mmol), CDI (400 mg, 2.0 mmol) and TEA (606 mg, 6.0 mmol). The mixture was stirred at 60 °C for 2 h. The mixture was cooled to 25°C and 20 mL of water was added. The mixture was extracted with three 20 mL portions of dichloromethane:methanol (20:1). The combined organic layers were washed with brine (20 mL), dried and concentrated *in vacuo* to give crude product. The crude product was purified by preparative HPLC (Mobile Phase : A:H₂O (10mM NH₄HCO₃) B:MeCN Gradient: 5%-95% B in 1.2min Flow Rate: 2.0 mL/min Column: XBridge C18 50 x 4.6 mm, 3.5 µm Oven Temperature: 40 °C UV214 nm,MASS100-1000) to give (S)-1-(4-(methylsulfonyl)phenyl)-N-((S)-quinuclidin-3-yl)-3,4-dihydroisoquinoline-2(1H)-carboxamide, **compound 1072** (25 mg). **Compound 1072:** LCMS: (M+H)⁺ = 440; purity = 100% (214 nm); retention time = 1.388 min. Method C ¹H NMR(400 MHz, DMSO-*d₆*) δ 7.85 (d, J = 8.0 Hz, 2H), 7.41 (d, J = 12.0 Hz, 2H), 7.26 - 7.22 (m, 4H), 6.51 (s, 1H), 6.28 (d, J = 8.0 Hz, 1H), 3.81 - 3.78 (m, 1H), 3.68 - 3.65 (m, 1H), 3.38 - 3.36 (m, 1H), 3.17 (s, 3H), 3.03 - 2.97 (m, 1H), 2.88 - 2.86 (m, 1H), 2.80 - 2.56 (m, 6H), 1.74 - 1.72 (m, 1H), 1.66 - 1.63 (m, 1H), 1.55 - 1.46 (m, 2H), 1.24 - 1.18 (m, 1H). Chiral SFC: CO₂ and MeOH containing 0.2% methanolic ammonia over a CHIRALPAK^{®} IG column (4.6 x 100 mm 5 µm), retention time = 3.69 min.

### Example 5: Synthesis of Compound 1073 and 1074

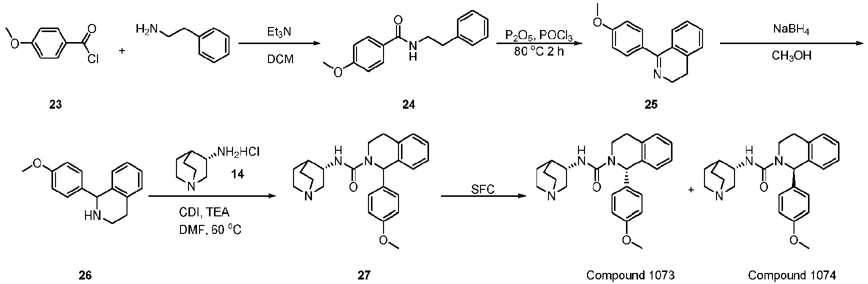

Step 1: A mixture of 2-phenylethanamine (3.63 g, 30 mmol) and triethylamine (6.06 g, 60 mmol) in tetrahydrofuran (40 mL) was cooled to 0 °C, and then 4-methoxybenzoyl chloride **23** (5.1 g, 30 mmol) was added dropwise. The mixture was stirred at room temperature for 2h. The tetrahydrofuran was removed *in vacuo,* and 40 mL of water was added. The mixture was extracted with three 50 mL portions of dichloromethane:methanol (20:1), then dried with Na₂SO₄. The solvent was evaporated to give 4-methoxy-N-phenethylbenzamide **24** (3.5 g). LCMS: (M+H)⁺ =256; Purity 26% (UV 254 nm); Retention time = 1.181min.

Step 2: To the 4-methoxy-N-phenethylbenzamide **24** (2.55 g, 10 mmol) was added polyphosphoric acid (5 mL). The mixture was stirred overnight at 160 °C. The mixture was cooled to 25 °C and quenched with ice water (100 mL), alkalized with NaOH (10% aq.) to pH = 11 and extracted with three 100 mL portions of dichloromethane:methanol (20:1). The combined organic layers were washed with brine (100 mL), dried and concentrated *in vacuo* to give 1-(4-methoxyphenyl)-3,4-dihydroisoquinoline **25** (1.78 g). LCMS: (M+H)⁺ = 238; purity = 75 % (254 nm); retention time = 1.321 min.

Step 3: 1-(4-methoxyphenyl)-3,4-dihydroisoquinoline (1.78 g, 7.5 mmol) in methanol (20 mL) was cooled to 0 °C, and then sodium borohydride (855 mg, 22.5 mmol) was added dropwise. The mixture was stirred at room temperature for 2h. The methanol was removed *in vacuo.* Water (40 mL) was added to the mixture and it was extracted with three 50 mL portions of dichloromethane. The combined organixc layers were dried with Na₂SO₄ and the solvent was evaporated to give 1-(4-methoxyphenyl)-1 ,2,3,4-tetrahydroisoquinoline **26** (1.75 g). LCMS: (M+H)⁺ = 240; purity = 98% (254 nm); retention time = 1.365 min.

Step 4: To a solution of 1-(4-methoxyphenyl)-1,2,3,4-tetrahydroisoquinoline **26** (239 mg, 1.0 mmol) in DMF (2 mL) was added (S)-quinuclidin-3-amine **14** (400 mg, 2.0 mmol), CDI (400 mg, 2.0 mmol) and TEA (606 mg, 6.0 mmol). The mixture was stirred at 60 °C for 2 h. The mixture was cooled to 25 °C and 20 mL of water was added. The mixture was extracted with three 20 mL portions of dichloromethane:methanol (20:1). The combined organic layers were washed with brine (20 mL), dried and concentrated *in vacuo* to give crude product. The crude product was purified by preparative HPLC (Mobile Phase : A: H₂O (10 mM NH₄HCO₃) B:MeCN Gradient: 5%-95% B in 1.2 min Flow Rate: 2.0 mL/min Column: XBridge C18 50 x 4.6 mm, 3.5 µm Oven Temperature: 40 °C UV214 nm,MASS 100-1000) to give 1-(4-methoxyphenyl)-N-((S)-quinuclidin-3-yl)-3,4-dihydroisoquinoline-2(1H)-carboxamide (100 mg). LCMS: (M+H)⁺ = 392; purity = 100% (214 nm); retention time = 1.574 min.

Step 5: The two diastereomers were separated by chiral SFC eluting with CO₂ and MeOH containing 0.2% methanolic ammonia over a CHIRALPAK^{®} IG column (4.6 x 100 mm 5 µm) to give **compound 1073** (58.6 mg) and **compound 1074** (64.7 mg).

**Compound 1073:** LCMS: (M+H)⁺ = 392; purity = 100% (214 nm); retention time = 1.559 min. Method C ¹H NMR(400 MHz, CD₃OD) δ 7.23 - 7.21 (m, 3H), 7.19 - 7.11 (m, 2H), 6.85 (d, J = 8.0 Hz, 2H), 6.40 (s, 1H), 3.93 - 3.89 (m, 1H), 3.85 - 3.79 (m, 1H), 3.77 (s, 3H), 3.51 - 3.44 (m, 1H), 3.30 - 3.15 (m, 2H), 2.98 - 2.87 (m, 2H), 2.83-2.72 (m, 3H), 2.71 - 2.66 (m, 1H), 2.19 - 2.15 (m, 1H), 1.96 - 1.88 (m, 1H), 1.77 - 1.69 (m, 2H), 1.48 - 1.31 (m, 2H). Chiral SFC: CO₂ and MeOH containing 0.2% methanolic ammonia over a CHIRALPAK^{®} IG column (4.6 x 100 mm 5 µm), retention time = 2.93 min.

**Compound 1074:** LCMS: (M+H)⁺ = 392; purity = 100% (214 nm); retention time = 1.565 min. Method C ¹H NMR(400 MHz, CD₃OD) δ 7.23 - 7.20 (m, 3H), 7.16 - 7.10 (m, 3H), 6.85 (d, J = 8.0 Hz, 2H), 6.41 (s, 1H), 3.87 - 3.79 (m, 2H), 3.77 (s, 3H), 3.50 - 3.43 (m, 1H), 3.26 - 3.20 (m, 1H), 2.95 - 2.92 (m, 2H), 2.87 - 2.72 (m, 4H), 2.62 - 2.57 (m, 2H), 1.96 - 1.82 (m, 3H), 1.77 - 1.71 (m, 2H). Chiral SFC: CO₂ and MeOH containing 0.2% methanolic ammonia over a CHIRALPAK^{®} IG column (4.6 x 100 mm 5 µm), retention time = 4.90 min.

Compounds 1075 and 1076 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1075** (32 mg) and **compound 1076** (28 mg).

**Compound 1075:** LCMS: (M+H)⁺ = 398.1; purity = 100% (214 nm); retention time = 1.655 min. Method C ¹H NMR (400 MHz, DMSO-*d₆*) δ 7.40 - 7.31 (m. 1H), 7.27 - 7.17 (m, 3H), 7.13 (dd, J = 19.4, 11.2 Hz, 1H), 6.95-6.94 (brs, 1H), 6.41 (s, 1H), 6.28 (d, J = 6.1 Hz, 1H), 3.85 - 3.78 (m, 1H), 3.67-3.66 (brs, 1H), 3.28 (dd, J = 8.7, 4.8 Hz, 1H), 3.05 - 2.97 (m, 1H), 2.83 (dd, J = 14.6, 9.0 Hz, 2H), 2.67-2.65 (m, 5H), 1.76 - 1.62 (m, 2H), 1.58 - 1.42 (m, 2H), 1.23 (s, 2H). Chiral SFC: CO₂ and MeOH containing 0.2% methanolic ammonia over a CHIRALPAK^{®} IG column (4.6 x 100 mm 5 µm), retention time = 2.41 min.

**Compound 1076:** LCMS: (M+H)⁺ = 398.1; purity = 100% (214 nm); retention time = 1.670 min. Method C ¹H NMR (400 MHz, DMSO-*d₆*) δ 7.36 (dd, J = 19.2, 8.5 Hz, 1H), 7.22 (dt, J = 12.7, 5.7 Hz, 3H), 7.11 (d, J = 10.1 Hz, 1H), 6.94-6.93 (brs, 1H), 6.42 (s, 1H), 6.30 (d, J = 6.0 Hz, 1H), 3.83 - 3.76 (m, 1H), 3.66 (s, 1H), 3.30 (s, 1H), 3.03 - 2.97 (m, 1H), 2.80 (dd, J = 49.6, 6.9 Hz, 3H), 2.71 - 2.56 (m, 4H), 1.75-1.74 (m, 2H), 1.52-1.50 (m, 2H), 1.23-1.22 (m, 2H). Chiral SFC: CO₂ and MeOH containing 0.2% methanolic ammonia over a CHIRALPAK^{®} IG column (4.6 x 100 mm 5 µm), retention time = 4.48 min.

Compounds 1077 and 1078 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1077** (103.9 mg) and **compound 1078** (100 mg).

**Compound 1077:** LCMS: (M+H)⁺ = 363; purity = 100% (214 nm); retention time = 1.309 min. Method C ¹H NMR (400 MHz, CD₃OD) δ 8.45 (dd, J = 4.6, 1.6 Hz, 2H), 7.34-7.28 (m, 5H), 6.47 (s, 1H), 3.96 (d, J = 36.0 Hz, 1H), 3.79 - 3.49 (m, 2H), 3.28 (dd, J = 10.7, 3.1 Hz, 1H), 3.01 - 2.71 (m, 7H), 2.10 (dd, J = 4.4, 2.2 Hz, 1H), 2.00 - 1.87 (m, 1H), 1.82 - 1.72 (m, 2H), 1.59 - 1.50 (m, 1H). Chiral SFC: CO₂and MeOH containing 0.2% methanolic ammonia over a CHIRALPAK^{®} IG column (4.6 x 100 mm 5 µm), retention time = 2.62 min.

**Compound 1078:** LCMS: (M+H)⁺ = 363; purity = 100% (214 nm); retention time = 1.272 min. Method C ¹H NMR (400 MHz, CD₃OD) δ 8.45 (dd, J = 4.6, 1.6 Hz, 2H), 7.34 - 7.26 (m, 5H), 6.48 (s, 1H), 3.90 (s, 1H), 3.78 - 3.66 (m, 1H), 3.58 (ddd, J = 31.2, 19.1, 10.7 Hz, 1H), 3.30 - 3.12 (m, 1H), 3.06 - 2.55 (m, 7H), 1.99 - 1.95 (m, 1H), 1.89 (dd, J = 5.8, 2.9 Hz, 1H), 1.75-1.74 (m, 2H), 1.54-1.52 (m, 1H). Chiral SFC: CO₂ and MeOH containing 0.2% methanolic ammonia over a CHIRALPAK^{®} IG column (4.6 x 100 mm 5 µm), retention time = 6.13 min.

Compounds 1079 and 1080 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1079** (111 mg) and **compound 1080** (87 mg).

**Compound 1079:** LCMS: (M+H)⁺ = 402; purity = 100% (214 nm); retention time = 1.466 min. Method C ¹H NMR (400 MHz, DMSO-*d₆*) δ 8.02 (s, 1H), 7.92 (s, 1H), 7.51 (dd, J = 5.2, 4.1 Hz, 2H), 7.30 - 7.20 (m, 4H), 7.14 (dd, J = 9.4, 1.7 Hz, 1H), 6.48 (s, 1H), 3.91 (dd, J = 8.3, 4.9 Hz, 1H), 3.71 (d, J = 21.4 Hz, 1H), 3.28 (d, J = 4.3 Hz, 2H), 3.05 - 2.97 (m, 1H), 2.95 - 2.74 (m, 3H), 2.59 (dd, J = 14.0, 5.6 Hz, 3H), 1.77 - 1.61 (m, 2H), 1.59 - 1.40 (m, 2H), 1.37 - 1.13 (m, 2H). Chiral SFC: CO₂ and MeOH containing 0.2% methanolic ammonia over a CHIRALPAK^{®} IG column (4.6 x 100 mm 5 µm), retention time = 3.30 min.

**Compound 1080:** LCMS: (M+H)⁺ = 402; purity = 100% (214 nm); retention time = 1.478 min. Method C ¹H NMR (400 MHz, DMSO-*d₆*) δ 8.02 (s, 2H), 7.92 (s, 1H), 7.51 - 7.48 (m, 1H), 7.30 - 7.19 (m, 3H), 7.14 (dd, J = 9.4, 1.6 Hz, 1H), 6.49 (s, 1H), 6.38 (d, J = 6.0 Hz, 1H), 3.90-3.89 (brs, 1H), 3.80 (d, J = 6.0 Hz, 2H), 3.67-3.66 (brs, 2H), 3.03 - 2.91 (m, 3H), 2.78 - 2.61 (m, 3H), 1.78 - 1.69 (m, 2H), 1.52 (d, J = 6.7 Hz, 2H), 1.24-1.22 (brs, 2H). Chiral SFC: CO₂ and MeOH containing 0.2% methanolic ammonia over a CHIRALPAK^{®} IG column (4.6 x 100 mm 5 µm), retention time = 12.07 min.

Compounds 1081 and 1082 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1081** (27.8 mg) and **compound 1082** (8.7 mg).

**Compound 1081:** LCMS: (M+H)⁺ = 395; purity = 100% (214 nm); retention time = 1.745 min. Method C ¹H NMR (400 MHz, DMSO-*d₆*) δ 7.36 (d, J = 8.5 Hz, 2H), 7.23 (t, J = 3.6 Hz, 2H), 7.20 (dd, J = 8.1, 3.6 Hz, 1H), 7.15 (t, J = 6.5 Hz, 3H), 6.43 (s, 1H), 6.28 (d, J = 6.3 Hz, 1H), 3.87 - 3.77 (m, 1H), 3.68 (d, J = 6.2 Hz, 1H), 3.27 - 3.20 (m, 1H), 3.07 - 2.96 (m, 1H), 2.92 - 2.76 (m, 2H), 2.75 - 2.54 (m, 5H), 1.73 (d, J = 2.8 Hz, 1H), 1.66-1.65 (brs, 1H), 1.59 - 1.41 (m, 2H), 1.23-1.22 (brs, 2H). Chiral SFC: CO₂and MeOH containing 0.2% methanolic ammonia over a CHIRALPAK^{®} IG column (4.6 x 100 mm 5 µm), retention time = 2.66 min.

**Compound 1082:** LCMS: (M+H)⁺ = 395; purity = 100% (214 nm); retention time = 1.759 min. Method C ¹H NMR (400 MHz, DMSO-*d₆*) δ 7.23 (t, *J =* 4.0 Hz, 1H), 7.20 (dd, *J* = 8.2, 3.6 Hz, 1H), 7.14 (t, *J=* 6.7 Hz, 1H), 6.44 (s, 1H), 6.30 (d, *J=* 6.0 Hz, 1H), 3.88 - 3.76 (m, 1H), 3.66-3.65 (s, 1H), 3.25 (ddd, *J=* 13.5, 9.0, 4.9 Hz, 1H), 3.25 (ddd, *J=* 13.5, 9.0, 4.9 Hz, 1H), 3.09 -2.94 (m, 1H), 2.87 (ddd, *J* = 14.8, 9.0, 5.7 Hz, 1H), 2.81 -2.54 (m, 3H), 1.75 (d, *J* = 2.8 Hz, 1H), 1.53 (ddd, *J* = 16.2, 8.1, 4.7 Hz, 1H), 1.23-1.22 (brs, 1H). Chiral SFC: CO₂ and MeOH containing 0.2% methanolic ammonia over a CHIRALPAK^{®} IG column (4.6 x 100 mm 5 µm), retention time = 4.79 min.

Compounds 1083 and 1084 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1083** (51.6 mg) and **compound 1084** (55.6 mg).

**Compound 1083:** LCMS: (M+H)⁺ = 393; purity = 100% (214 nm); retention time = 1.491 min. Method C ¹H NMR (400 MHz, DMSO-*d₆*) δ 7.88 (d, *J* = 2.4 Hz, 1H), 7.45 (dd, *J* = 8.6, 2.5 Hz, 1H), 7.33 -7.16 (m, 3H), 7.12 (d, *J* = 7.1 Hz, 1H), 6.75 (d, *J* = 8.6 Hz, 1H), 6.42 (s, 1H), 6.31 (d, *J* = 6.2 Hz, 1H), 3.89 (dt, *J=* 13.1, 4.9 Hz, 1H), 3.80 (s, 3H), 3.68 (d, *J* = 6.2 Hz, 1H), 3.27 - 3.16 (m, 1H), 3.08 - 2.97 (m, 1H), 2.86 (ddd, *J* = 15.6, 10.7, 5.9 Hz, 2H), 2.74 (dt, *J* = 16.3, 4.4 Hz, 1H), 2.69 - 2.55 (m, 4H), 1.79- 1.71 (m, 1H), 1.69 (dd, *J* = 5.5, 2.6 Hz, 1H), 1.61 - 1.42 (m, 2H), 1.23-1.22 (m, 1H). Chiral SFC: CO₂ and MeOH containing 0.2% methanolic ammonia over a CHIRALPAK^{®} IG column (4.6 x 100 mm 5 µm), retention time = 2.65 min.

**Compound 1084:** LCMS: (M+H)⁺ = 393; purity = 100% (214 nm); retention time = 1.479 min. Method C ¹H NMR (400 MHz, DMSO-*d₆*) δ 7.88 (d, J = 2.4 Hz, 1H), 7.44 (dd, J = 8.6, 2.5 Hz, 1H), 7.27 - 7.22 (m, 2H), 7.22 - 7.16 (m, 1H), 7.12 (d, J = 7.2 Hz, 1H), 6.75 (d, J = 8.6 Hz, 1H), 6.46 - 6.39 (m, 2H), 3.87 (dt, J = 13.0, 5.0 Hz, 1H), 3.80 (s, 3H), 3.73 (d, J = 5.7 Hz, 1H), 3.28 - 3.17 (m, 1H), 3.15 - 3.05 (m, 1H), 2.89 (ddd, J = 15.8, 9.7, 5.9 Hz, 2H), 2.79 - 2.65 (m, 4H), 2.61 (dd, J = 13.4, 5.3 Hz, 1H), 1.81 (d, J = 2.8 Hz, 2H), 1.67 - 1.49 (m, 2H), 1.39 - 1.26 (m, 1H). Chiral SFC: CO₂ and MeOH containing 0.2% methanolic ammonia over a CHIRALPAK^{®} IG column (4.6 x 100 mm 5 µm), retention time = 5.23 min.

Compounds 1085 and 1086 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1085** (7.7 mg) and **compound 1086** (8.9 mg).

**Compound 1085:** LCMS: (M+H)⁺ = 363; purity = 100% (214 nm); retention time = 1.354 min. Method C ¹H NMR (400 MHz, DMSO-*d₆*) δ 8.43 (dd, J = 4.7, 1.5 Hz, 1H), 8.40 (d, J = 2.1 Hz, 1H), 7.49 (d, J = 7.9 Hz, 1H), 7.32 (dd, J = 7.8, 4.7 Hz, 1H), 7.27 - 7.24 (m, 2H), 7.24 - 7.20 (m, 1H), 7.18 (d, J = 6.8 Hz, 1H), 6.49 (s, 1H), 6.32 (d, J = 6.2 Hz, 1H), 3.90 - 3.81 (m, 1H), 3.67 (d, J = 7.0 Hz, 1H), 3.31 - 3.23 (m, 1H), 3.07 - 2.95 (m, 1H), 2.93 - 2.85 (m, 1H), 2.82 (d, J = 17.0 Hz, 1H), 2.78 - 2.66 (m, 2H), 2.66 - 2.54 (m, 4H), 1.76 - 1.70 (m, 1H), 1.67 (d, J = 2.9 Hz, 1H), 1.51 (ddd, J = 14.9, 8.9, 2.8 Hz, 2H), 1.23 (d, J = 4.0 Hz, 2H). Chiral SFC: CO₂ and MeOH containing 0.2% methanolic ammonia over a CHIRALPAK^{®} IG column (4.6 x 100 mm 5 µm), retention time = 2.61 min.

**Compound 1086:** LCMS: (M+H)⁺ = 363; purity = 100% (214 nm); retention time = 1.328 min. Method C ¹H NMR (400 MHz, DMSO-*d₆*) δ 8.43 (dd, J = 4.7, 1.5 Hz, 1H), 8.39 (d, J = 2.1 Hz, 1H), 7.48 (d, J = 7.9 Hz, 1H), 7.32 (dd, J = 7.8, 4.8 Hz, 1H), 7.25 (dd, J = 5.0, 1.6 Hz, 1H), 7.24 - 7.20 (m, 1H), 7.18 (d, J = 6.8 Hz, 1H), 6.50 (s, 1H), 6.36 (d, J = 5.8 Hz, 1H), 3.89 - 3.79 (m, 1H), 3.67 (d, J = 5.8 Hz, 1H), 3.28 (dd, J = 8.8, 4.6 Hz, 1H), 3.07 - 2.98 (m, 1H), 2.95 - 2.85 (m, 1H), 2.79 (s, 1H), 2.77 - 2.68 (m, 1H), 2.68 - 2.52 (m, 3H), 1.77 (d, J = 2.7 Hz, 2H), 1.61 - 1.45 (m, 2H), 1.27-1.26 (m, 2H). Chiral SFC: CO₂ and MeOH containing 0.2% methanolic ammonia over a CHIRALPAK^{®} IG column (4.6 x 100 mm 5 µm), retention time = 5.59 min.

Compounds 1087 and 1088 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1087** (7.5 mg) and **compound 1088** (6.2 mg).

**Compound 1087:** LCMS: (M+H)⁺ = 420; purity = 100% (214 nm); retention time = 1.78 min. Method C ¹H NMR (400 MHz, CDCl₃) δ 7.19 (dd, J = 5.7, 3.1 Hz, 5H), 6.81 (d, J = 8.7 Hz, 2H), 6.07 (s, 1H), 5.33 (d, J = 18.4 Hz, 1H), 4.60 (d, J = 6.8 Hz, 1H), 4.50 (dt, J = 12.1, 6.0 Hz, 1H), 3.85 (s, 1H), 3.71 (ddd, J = 26.3, 12.5, 6.5 Hz, 3H), 3.43 -3.23 (m, 1H), 2.90 (s, 2H), 2.75 (dd, J = 15.3, 7.6 Hz, 4H), 2.44 (d, J = 14.9 Hz, 1H), 2.21 (dd, J = 17.0, 9.1 Hz, 1H), 2.03 (d, J = 11.7 Hz, 1H), 1.83 (d, J = 3.2 Hz, 1H), 1.26 (d, J = 1.2 Hz, 6H). Chiral SFC: CO₂ and MeOH containing 0.2% methanolic ammonia over a CHIRALPAK^{®} IG column (4.6 x 100 mm 5 µm), retention time = 2.50 min.

**Compound 1088:** LCMS: (M+H)⁺ = 420; purity = 100% (214 nm); retention time = 1.81 min. Method C ¹H NMR (400 MHz, CDCl₃) δ 7.22 - 7.11 (m, 5H), 6.81 (d, J = 8.7 Hz, 2H), 6.10 (s, 1H), 5.34 (dd, J = 14.0, 9.3 Hz, 1H), 4.71 (d, J = 6.6 Hz, 1H), 4.60 - 4.39 (m, 1H), 3.90 (s, 1H), 3.81 - 3.57 (m, 2H), 3.41 - 3.22 (m, 1H), 3.00 - 2.64 (m, 5H), 2.38 (d, J = 18.4 Hz, 1H), 2.21 (dd, J = 16.9, 9.1 Hz, 1H), 2.01-1.99 (m, 2H), 1.30 (d, J = 1.2 Hz, 6H). Chiral SFC: CO₂ and MeOH containing 0.2% methanolic ammonia over a CHIRALPAK^{®} IG column (4.6 x 100 mm 5 µm), retention time = 4.42 min.

Compounds 1089 and 1090 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1089** (15 mg) and **compound 1090** (12 mg).

**Compound 1089:** LCMS: (M+H)⁺ = 356; purity = 100% (214 nm); retention time = 1.488 min. Method F ¹H NMR (400 MHz, DMSO-*d₆*) δ 7.24 - 7.14 (m, 4H), 6.19 (d, J = 6.2 Hz, 1H), 5.35 (s, 1H), 4.76 (d, J = 5.8 Hz, 1H), 4.46 (d, J = 5.6 Hz, 1H), 4.07 (d, J = 5.8 Hz, 1H), 4.01 (d, J = 5.6 Hz, 1H), 3.97 - 3.88 (m, 1H), 3.63 (d, J = 6.6 Hz, 1H), 2.99 (dd, J = 12.6, 8.9 Hz, 2H), 2.80 (dd, J = 16.0, 9.8 Hz, 2H), 2.75 - 2.52 (m, 5H), 1.69 (d, J = 2.6 Hz, 2H), 1.57 - 1.41 (m, 2H), 1.34 (s, 3H), 1.26- 1.13 (m, 1H). Chiral SFC: CO₂ and MeOH containing 0.2% methanolic ammonia over a CHIRALPAK^{®} IG column (4.6 x 100 mm 5 µm), retention time = 1.35 min.

**Compound 1090:** LCMS: (M+H)⁺ = 356; purity = 100% (214 nm); retention time = 1.532 min. Method F ¹H NMR (400 MHz, DMSO-*d₆*) δ 7.28 - 7.08 (m, 4H), 6.20 (d, J = 5.9 Hz, 1H), 5.36 (s, 1H), 4.72 (d, J = 5.8 Hz, 1H), 4.44 (d, J = 5.6 Hz, 1H), 4.05 (d, J = 5.7 Hz, 1H), 4.01 (d, J = 5.6 Hz, 1H), 3.94 - 3.85 (m, 1H), 3.60 (s, 1H), 3.00 (dd, J = 27.0, 13.4 Hz, 2H), 2.89 - 2.54 (m, 6H), 1.73 (dd, J = 13.8, 2.9 Hz, 2H), 1.59 - 1.39 (m, 2H), 1.34 (s, 3H), 1.24-1.13 (brs, 1H). Chiral SFC: CO₂ and MeOH containing 0.2% methanolic ammonia over a CHIRALPAK^{®} IG column (4.6 x 100 mm 5 µm), retention time = 3.76 min.

Compounds 1091 and 1092 were prepared analogously. The two diastereomers were separated by chiral SFC eluting with CO₂ and MeOH containing 0.2% methanolic ammonia over a CHIRALPAK^{®} IG column (4.6 x 100 mm 5 µm) to give **compound 1091** (6.1 mg) and **compound 1092** (4.8 mg).

**Compound 1091:** LCMS: (M+H)⁺ = 468; purity = 100% (214 nm); retention time = 1.92 min. Method C ¹H NMR (400 MHz, DMSO-*d₆*) δ 7.46 - 7.36 (m, 4H), 7.32 (d, J = 7.0 Hz, 1H), 7.20 (dd, J = 13.2, 3.7 Hz, 3H), 7.09 (d, J = 7.1 Hz, 1H), 7.04 (d, J = 8.5 Hz, 2H), 6.92 (d, J = 8.6 Hz, 2H), 6.38 (s, 1H), 6.21 (d, J = 6.3 Hz, 1H), 3.85 (d, J = 12.6 Hz, 1H), 3.67 (s, 1H), 3.23 -3.16 (m, 1H), 3.06 - 2.97 (m, 1H), 2.90 -2.76 (m, 2H), 2.72-2.71 (brs, 1H), 2.61 (t, J = 24.0 Hz, 4H), 1.99-1.98 (brs, 1H), 1.69 (d, J = 25.1 Hz, 2H), 1.50-1.49 (brs, 2H), 1.24-1.23 (brs, 3H). Chiral SFC: CO₂ and MeOH containing 0.2% methanolic ammonia over a CHIRALPAK^{®} IG column (4.6 x 100 mm 5 µm), retention time = 7.73 min.

**Compound 1092:** LCMS: (M+H)⁺ = 468; purity = 100% (214 nm); retention time = 1.92 min. Method C ¹H NMR (400 MHz, DMSO-*d₆*) δ 7.45 -7.30 (m, 5H), 7.20 (dd, J = 12.2, 5.5 Hz, 3H), 7.06 (dd, J = 22.4, 7.8 Hz, 3H), 6.92 (d, J = 8.6 Hz, 2H), 6.40 (s, 1H), 6.25 (d, J = 5.8 Hz, 1H), 3.83 (s, 1H), 3.64 (s, 1H), 3.23 - 3.16 (m, 1H), 2.99 (d, J = 10.5 Hz, 1H), 2.90 - 2.55 (m, 7H), 1.75-1.74 (brs, 2H), 1.52-1.51 (brs, 3H), 1.23-1.22 (brs, 3H). Chiral SFC: CO₂ and MeOH containing 0.2% methanolic ammonia over a CHIRALPAK^{®} IG column (4.6 x 100 mm 5 µm), retention time = 13.46 min.

Compounds 1093 and 1094 were prepared analogously. The two diastereomers were separated by chiral SFC eluting with CO₂ and MeOH containing 0.2% methanolic ammonia over a CHIRALPAK^{®} AD column (4.6 x 100 mm 5 µm) to give **compound 1093** (6.0 mg) and **compound 1094** (7.7 mg).

**Compound 1093:** LCMS: (M+H)⁺ = 378; purity = 100% (214 nm); retention time = 1.399 min. Method C ¹H NMR (400 MHz, CD₃OD) δ 7.26 - 7.21 (m, 3H), 7.14 - 7.12 (m, 1H), 7.01 (d, *J =* 8.4 Hz, 2H), 6.70 (d, *J* = 11.2 Hz, 2H), 6.37 (s, 1H), 4.24 (br, 1H), 3.81 - 3.73 (m, 2H), 3.50 - 3.32 (m, 3H), 3.29 - 3.18 (m, 2H), 3.01 - 2.78 (m, 5H), 2.31 - 2.28 (m, 1H), 2.26 -2.15 (m, 2H), 2.13 -2.01 (m, 1H), 1.95- 1.85 (m, 1H). Chiral SFC: CO₂ and MeOH containing 0.2% methanolic ammonia over a CHIRALPAK^{®} AD-H column (4.6 x 100 mm 5 µm), retention time = 2.57 min.

**Compound 1094:** LCMS: (M+H)⁺ = 378; purity = 100% (214 nm); retention time = 1.373 min. Method C ¹H NMR (400 MHz, CD₃OD) δ 7.26 - 7.22 (m, 3H), 7.21 - 7.15 (m, 1H), 7.03 -6.85 (m, 2H), 6.78 - 6.61 (m, 2H), 6.39 (s, 1H), 4.24 - 4.15 (m, 1H), 3.81 - 3.63 (m, 2H), 3.50 - 3.25 (m, 6H), 3.21 - 3.01 (m, 1H), 2.97 - 2.92 (m, 1H), 2.88 - 2.75 (m, 1H), 2.32 - 2.25 (m, 1H), 2.23 - 2.11 (m, 1H), 2.10 - 1.99 (m, 2H), 1.98 - 1.85 (m, 1H). Chiral SFC: CO₂ and MeOH containing 0.2% methanolic ammonia over a CHIRALPAK^{®} IG column (4.6 x 100 mm 5 µm), retention time = 3.40 min.

Compounds 1095 and 1096 were prepared analogously. The two diastereomers were separated by chiral SFC eluting with CO₂ and MeOH containing 0.2% methanolic ammonia over a CHIRALPAK^{®} IG column (4.6 × 100 mm 5 µm) to give **compound 1095** (30 mg) and **compound 1096** (30 mg).

**Compound 1095:** LCMS: (M+H)⁺ = 428; purity = 100% (214 nm); retention time = 1.426 min. Method C ¹H NMR (400 MHz, DMSO-*d₆*) δ 8.19 (s, 1H), 7.68 (t, J = 1.3 Hz, 1H), 7.62 - 7.50 (m, 2H), 7.24 (ddd, J = 7.7, 5.4, 2.9 Hz, 5H), 7.09 (s, 1H), 6.57 - 6.40 (m, 2H), 3.94 - 3.74 (m, 2H), 3.27 (dd, J = 13.9, 9.5 Hz, 4H), 3.02-3.01 (brs, 1H), 2.86 (dd, J = 29.8, 20.7 Hz, 4H), 2.74 (dd, J = 11.5, 4.9 Hz, 1H), 1.98 (s, 1H), 1.93 - 1.85 (m, 1H), 1.67-1.66 (brs, 2H), 1.42-1.41 (brs, 1H). Chiral SFC: CO₂ and EtOH containing 1% methanolic ammonia over a CHIRALPAK^{®} AD-H column (4.6 × 100 mm 5 µm), retention time = 3.03 min.

**Compound 1096:** LCMS: (M+H)⁺ = 428; purity = 100% (214 nm); retention time = 1.410 min. Method C ¹H NMR (400 MHz, DMSO-*d₆*) δ 8.19 (s, 1H), 7.68 (t, J = 1.2 Hz, 1H), 7.56 (d, J = 8.6 Hz, 2H), 7.31 - 7.17 (m, 6H), 7.08 (s, 1H), 6.50 (s, 1H), 6.33 (d, J = 5.9 Hz, 1H), 3.91 - 3.78 (m, 1H), 3.68 (d, J = 6.4 Hz, 1H), 3.33 - 3.26 (m, 3H), 3.08 - 2.98 (m, 1H), 2.94 - 2.84 (m, 1H), 2.82 - 2.71 (m, 2H), 2.68 - 2.56 (m, 3H), 1.78 (d, J = 2.7 Hz, 2H), 1.54 (ddd, J = 15.2, 8.9, 3.1 Hz, 2H), 1.25 (d, J = 13.5 Hz, 1H). Chiral SFC: CO₂ and EtOH containing 1% methanolic ammonia over a CHIRALPAK^{®} AD-H column (4.6 × 100 mm 5 µm), retention time = 3.90 min.

Compounds 1097 and 1098 were prepared analogously. The two diastereomers were separated by chiral SFC eluting with CO₂ and MeOH containing 0.2% methanolic ammonia over a CHIRALPAK^{®} IG column (4.6 × 100 mm 5 µm) to give **compound 1097** (24.2 mg) and **compound 1098** (8.9 mg).

**Compound 1097:** LCMS: (M+H)⁺ = 402; purity = 100% (214 nm); retention time = 1.368 min. Method C ¹H NMR (400 MHz, DMSO-*d₆*) δ 8.28 (dd, J = 16.5, 6.2 Hz, 2H), 7.23 (dt, J = 11.8, 4.5 Hz, 5H), 6.91 (d, J = 15.7 Hz, 1H), 6.59 (dd, J = 7.3, 1.7 Hz, 1H), 6.41 - 6.23 (m, 2H), 3.95 - 3.78 (m, 1H), 3.68 (s, 1H), 3.28 - 3.20 (m, 1H), 3.08 - 2.95 (m, 1H), 2.86 (ddd, J = 16.5, 11.4, 6.6 Hz, 1H), 2.74 (dt, J = 16.4, 4.6 Hz, 2H), 2.67 - 2.53 (m, 4H), 1.71 (t, J = 13.3 Hz, 2H), 1.52 (dd, J = 12.7, 6.4 Hz, 2H), 1.22 (d, J = 8.0 Hz, 1H). Chiral SFC: CO₂ and EtOH containing 1% methanolic ammonia over a CHIRALPAK^{®} AD-H column (4.6 × 100 mm 5 µm), retention time = 1.94 min.

**Compound 1098:** LCMS: (M+H)⁺ = 402; purity = 100% (214 nm); retention time = 1.335 min. Method C ¹H NMR (400 MHz, DMSO-*d₆*) δ 8.42 - 8.13 (m, 2H), 7.39 - 7.09 (m, 5H), 6.88 (s, 1H), 6.58 (dd, J = 7.3, 1.6 Hz, 1H), 6.35 (d, J = 6.7 Hz, 2H), 3.87 (dt, J = 13.1, 5.0 Hz, 1H), 3.68 (d, J = 6.2 Hz, 1H), 3.28 - 3.18 (m, 1H), 3.09 - 2.95 (m, 1H), 2.89 (ddd, J = 15.4, 9.5, 5.7 Hz, 1H), 2.82 - 2.68 (m, 2H), 2.59 (ddd, J = 28.0, 14.0, 8.2 Hz, 4H), 1.76 (dd, J = 12.5, 3.0 Hz, 2H), 1.54 (ddd, J = 15.2, 9.0, 3.0 Hz, 2H), 1.24 (d, J = 6.7 Hz, 1H). Chiral SFC: CO₂ and EtOH containing 1% methanolic ammonia over a CHIRALPAK^{®} AD-H column (4.6 × 100 mm 5 µm), retention time = 4.31 min.

Compounds 1099 and 1100 were prepared analogously. The two diastereomers were separated by chiral SFC eluting with CO₂ and i-PrOH containing 0.1% DEA over a CHIRALPAK^{®} AD-H column (4.6 × 100 mm 5 µm) to give **compound 1099** (30.8 mg) and **compound 1100** (4.6 mg).

**Compound 1099:** LCMS: (M+H)⁺ = 429; purity = 100% (214 nm); retention time = 1.462 min. Method C ¹H NMR (400 MHz, DMSO-*d₆*) δ 8.75 (d, J = 1.1 Hz, 1H), 7.96 (d, J = 1.1 Hz, 1H), 7.82 (d, J = 8.6 Hz, 1H), 7.35 (d, J = 8.5 Hz, 1H), 7.26 (dd, J = 4.5, 2.2 Hz, 1H), 7.25 - 7.19 (m, 1H), 6.52 (s, 1H), 6.30 (d, J = 6.3 Hz, 1H), 3.91 - 3.80 (m, 1H), 3.70 (s, 1H), 3.07 - 2.98 (m, 1H), 2.88 (ddd, J = 26.1, 15.8, 11.3 Hz, 1H), 2.79 - 2.69 (m, 1H), 2.70 - 2.55 (m, 2H), 1.78 - 1.72 (m, 1H), 1.68 (s, 1H), 1.60 - 1.42 (m, 1H), 1.23 (s, 1H). Chiral SFC: CO₂ and MeOH containing 1% methanolic ammonia over a CHIRALPAK^{®} IG column (4.6 × 100 mm 5 µm), retention time = 1.47 min. Chiral SFC: CO₂ and i-PrOH containing 1% DEA over a CHIRALPAK^{®} AD-H column (4.6 × 100 mm 5 µm), retention time = 2.87 min.

**Compound 1100:** LCMS: (M+H)⁺ = 429; purity = 100% (214 nm); retention time = 1.446 min. Method C ¹H NMR (400 MHz, DMSO-*d₆*) δ 8.75 (d, J = 1.1 Hz, 1H), 7.95 (d, J = 1.0 Hz, 1H), 7.82 (d, J = 8.6 Hz, 2H), 7.34 (d, J = 8.5 Hz, 2H), 7.28 - 7.24 (m, 2H), 7.22 (dd, J = 7.5, 4.3 Hz, 2H), 6.53 (s, 1H), 6.33 (d, J = 5.9 Hz, 1H), 3.91 - 3.79 (m, 1H), 3.68 (s, 1H), 3.07 - 2.97 (m, 1H), 2.96 - 2.84 (m, 1H), 2.83 - 2.70 (m, 2H), 2.64 (d, J = 26.9 Hz, 3H), 1.77 (d, J = 2.9 Hz, 2H), 1.62 - 1.43 (m, 2H), 1.23 (s, 2H). Chiral SFC: CO₂ and i-PrOH containing 1% DEA over a CHIRALPAK^{®} AD-H column (4.6 × 100 mm 5 µm), retention time = 4.71 min.

Compounds 1101 and 1102 were prepared analogously. The two diastereomers were separated by chiral SFC eluting with CO₂ and MeOH containing 0.2% methanolic ammonia over a CHIRALPAK^{®} IG column (4.6 × 100 mm 5 µm) to give **compound 1101** (17.1 mg) and **compound 1102** (34.3 mg).

**Compound 1101:** LCMS: (M+H)⁺ = 429; purity = 100% (214 nm); retention time = 1.455 min. ¹H NMR (400 MHz, DMSO-*d₆*) δ 9.24 (d, J = 9.0 Hz, 1H), 8.21 (s, 1H), 7.77 (d, J = 8.6 Hz, 2H), 7.38 - 7.13 (m, 6H), 6.50 (s, 1H), 6.30 (d, J = 6.3 Hz, 1H), 3.94 - 3.78 (m, 1H), 3.70 (d, J = 6.2 Hz, 1H), 3.33 - 3.25 (m, 1H), 3.09 - 2.96 (m, 1H), 2.80 (ddd, J = 16.5, 10.1, 4.8 Hz, 2H), 2.69 - 2.54 (m, 4H), 1.76 - 1.63 (m, 2H), 1.53 (tdd, J = 10.4, 8.4, 4.7 Hz, 2H), 1.23 (s, 2H). Chiral SFC: CO₂ and MeOH:ACN (3:2) containing 0.2% methanolic ammonia over a CHIRALPAK^{®} IG column (4.6 × 100 mm 5 µm), retention time = 3.27 min.

**Compound 1102:** LCMS: (M+H)⁺ = 429; purity = 100% (214 nm); retention time = 1.429 min. ¹H NMR (400 MHz, DMSO-*d₆*) δ 9.28 (s, 1H), 8.27 (s, 1H), 7.83 (d, J = 8.6 Hz, 2H), 7.43 - 7.18 (m, 6H), 6.57 (s, 1H), 6.39 (d, J = 5.9 Hz, 1H), 4.00 - 3.83 (m, 1H), 3.73 (d, J = 6.1 Hz, 1H), 3.07 (ddd, J = 13.4, 9.6, 1.8 Hz, 1H), 2.95 (ddd, J = 14.9, 8.9, 5.7 Hz, 1H), 2.89 - 2.74 (m, 2H), 2.75 - 2.57 (m, 4H), 1.82 (dd, J = 12.3, 2.8 Hz, 2H), 1.69 - 1.47 (m, 2H), 1.42 - 1.24 (m, 2H). Chiral SFC: CO₂ and MeOH:ACN (3:2) containing 0.2% methanolic ammonia over a CHIRALPAK^{®} IG column (4.6 × 100 mm 5 µm), retention time = 11.75 min.

Compounds 1103 and 1104 were prepared analogously. The two diastereomers were separated by chiral SFC eluting with CO₂ and MeOH containing 0.2% methanolic ammonia over a CHIRALPAK^{®} IG column (4.6 × 100 mm 5 µm) to give **compound 1103** (5.8 mg) and **compound 1104** (6.0 mg).

**Compound 1103:** LCMS: (M+H)⁺ = 431; purity = 99% (214 nm); retention time = 1.432 min. Method F ¹H NMR(400 MHz, DMSO-*d₆*) δ 7.22 - 7.21 (m, 2H), 7.06 - 7.05 (m, 1H), 6.90 (d, *J* = 8.0 Hz, 1H), 6.66 - 6.51 (m, 2H), 6.43 (d, *J* = 8.0 Hz, 1H), 6.32 (s, 1H), 4.02 (br, 2H), 3.81 - 3.77 (m, 2H), 3.17 - 3.16 (m, 2H), 2.74 - 2.61 (m, 2H), 2.30 - 2.25 (m, 1H), 2.12 - 1.78 (m, 7H), 1.68 (br, 2H), 1.48 - 1.24 (m, 7H). Chiral SFC: CO₂ and MeOH containing 0.2% methanolic ammonia over a CHIRALPAK^{®} IG column (4.6 × 100 mm 5 µm), retention time = 6.31 min.

**Compound 1104:** LCMS: (M+H)⁺ = 431; purity = 100% (214 nm); retention time = 1.430 min. Method F ¹H NMR(400 MHz, DMSO-*d₆*) δ 7.22 - 7.21 (m, 2H), 7.16 - 7.10 (m, 1H), 6.90 (d, *J=* 12.0 Hz, 1H), 6.68 (br, 1H), 6.43 (d, *J=* 8.0 Hz, 1H), 6.35 - 6.24 (m, 2H), 5.30 - 5.26 (m, 1H), 3.82 - 3.59 (m, 4H), 3.35 - 3.31 (m, 1H), 3.05 - 2.52 (m, 6H), 2.25 (s, 1H), 2.15 - 1.62 (m, 6H), 1.48 - 1.20 (m, 7H). Chiral SFC: CO₂ and MeOH containing 0.2% methanolic ammonia over a CHIRALPAK^{®} IG column (4.6 × 100 mm 5 µm), retention time = 11.19 min.

Compounds 1105 and 1106 were prepared analogously. The two diastereomers were separated by chiral SFC eluting with CO₂ and MeOH containing 0.2% methanolic ammonia over a CHIRALPAK^{®} IG column (20 × 250 mm 10 µm) to give **compound 1105** (21.0 mg) and **compound 1106** (16.2 mg).

**Compound 1105:** LCMS: (M+H)⁺ = 402; purity = 99% (214 nm); retention time = 1.19 min. Method C1 ¹H NMR: (400 MHz, DMSO-*d₆*) δ 8.47 (d, *J* = 6.8 Hz, 1H), 7.89 (d, *J* = 4.8 Hz, 1H), 7.53-7.51 (m, 1H), 7.28-7.26 (m, 4H), 6.97 (s, 1H), 6.83-6.81 (m, 1H), 6.46-6.39 (m, 2H), 3.86-3.74 (m, 2H), 3.38-3.29 (m, 2H), 3.13-3.07 (m, 1H), 2.93-2.85 (m, 2H), 2.79-2.51 (m, 4H), 1.79-1.73 (m, 2H), 1.59-1.53 (m, 2H), 1.30-1.24 (m, 1H). Chiral SFC: CO₂ and MeOH containing 0.2% methanolic ammonia over a CHIRALPAK^{®} IG column (4.6 × 100 mm 5 µm), retention time = 4.55 min.

**Compound 1106:** LCMS: (M+H)⁺ = 402; purity = 98% (214 nm); retention time = 1.15 min. Method C1 ¹H NMR: (400 MHz, DMSO-*d₆*) δ 8.46 (d, *J* = 7.2 Hz, 1H), 7.88 (s, 1H), 7.51 (d, *J=* 0.8 Hz, 1H), 7.27 (d, *J=* 4.4 Hz, 4H), 6.96 (s, 1H), 6.81-6.79 (m, 1H), 6.47 (s, 1H), 6.36 (d, *J=* 6.0 Hz, 1H), 3.87-3.82 (m, 1H), 3.68 (d, *J* = 6.0 Hz, 1H), 3.33-3.29 (m, 2H), 3.04-2.98 (m, 1H), 2.94-2.86 (m, 1H), 2.78-2.73 (m, 2H), 2.68-2.55 (m, 3H), 1.79-1.75 (m, 2H), 1.57-1.50 (m, 2H), 1.29-1.24 (m, 2H). Chiral SFC: CO₂ and MeOH containing 0.2% methanolic ammonia over a CHIRALPAK^{®} IG column (4.6 × 100 mm 5 µm), retention time = 8.01 min.

Compounds 1109 and 1110 were prepared analogously. The two diastereomers were separated by chiral SFC eluting with CO₂ and EtOH containing 1% methanolic ammonia over a CHIRALPAK^{®} AD-H column (4.6 × 100 mm 5 µm) to give **compound 1109** (69.2 mg) and **compound 1110** (62.9 mg).

**Compound 1109:** LCMS: (M+H)⁺ = 380; purity = 100% (214 nm); retention time = 1.397 min. Method A2 ¹H NMR(400 MHz, DMSO-*d₆*) δ 7.33 (dt, *J* = 8.0Hz, 1.2 Hz, 1H), 7.25-7.18 (m, 4H), 7.08(td, J = 8.8, 2.4 Hz, 1H),6.98-6.90 (m, 2H), 6.43 (s, 1H), 6.26 (d, *J* = 5.2 Hz, 1H), 3.81 (dt, J = 12.4, 5.6 Hz, 1H), 3.68 (d, *J* = 6.2 Hz, 1H), 3.00(dt, *J* = 9.2Hz, 1.6 Hz, 1H), 2.86-2.56 (m, 8H), 1.73-1.70 (m, 2H), 1.53-1.47 (m, 2H), 1.24-1.21 (m, 1H). Chiral SFC: CO₂ and MeOH containing 0.2% methanolic ammonia over a CHIRALPAK^{®} IG column (4.6 × 100 mm 5 µm), retention time = 1.88 min.

**Compound 1110:** LCMS: (M+H)⁺ = 380; purity = 100% (214 nm); retention time = 1.393 min. Method A2 ¹H NMR(400 MHz, DMSO-*d₆*) δ 7.34 (dt, J = 8.0, 1.2 Hz, 1H), 7.26-7.22 (m, 4H), 7.07 (td, J = 8.8, 2.4 Hz, 1H), 6.98-6.90 (m, 2H), 6.75 (d, *J* = 5.2 Hz,, 1H), 6.47 (s, 1H), 4.09-4.02 (m, 1H), 3.79 (dt, J = 12.0, 5.6 Hz, 1H), 3.58 (td, J = 12.8 Hz, 2.4Hz, 1H), 3.37-3.34 (m, 1H), 3.26-3.17 (m, 5H), 2.94-2.87 (m, 1H), 2.72 (dt, J = 16.0, 5.2 Hz, 1H), 2.12-2.03 (m, 2H), 1.87-1.84 (m, 2H), 1.72-1.66 (m, 1H). Chiral SFC: CO₂ and MeOH containing 0.2% methanolic ammonia over a CHIRALPAK^{®} IG column (4.6 × 100 mm 5 µm), retention time = 2.66 min.

Compounds 1111 and 1112 were prepared analogously. The two diastereomers were separated by chiral SFC eluting with CO₂ and hexane:EtOH (3:7) containing 0.1% DEA over a CHIRALPAK^{®} IG column (20 × 250 mm 10 µm) to give **compound 1111** (23.8 mg) and **compound 1112** (24.1 mg).

**Compound 1111:** LCMS: (M+H)⁺ = 402.1; purity = 99% (214 nm); retention time = 1.380 min. Method C ¹H NMR: (400 MHz, CD₃OD) δ 8.21 (s, 1H), 7.74 (s, 1H), 7.49 (d, *J* = 9.6 Hz, 1H), 7.34-7.24 (m, 5H), 6.85 (dd, *J=* 9.6, 1.2 Hz, 1H), 6.45 (s, 1H), 3.96-3.83 (m, 2H), 3.53-3.45 (m, 1H), 3.33-3.19 (m, 1H), 3.04-2.68 (m, 7H), 1.97-1.91 (m, 1H), 1.89-1.79 (m, 1H), 1.78-1.68 (m, 2H), 1.55-1.43 (m, 1H). Chiral SFC: CO₂ and hexane:EtOH (3:7) containing 0.1% DEA over a CHIRALPAK^{®} IG column (4.6 × 250 mm 5 µm), retention time = 11.37 min.

**Compound 1112:** LCMS: (M+H)⁺ = 402.1; purity = 94% (214 nm); retention time = 1.352 min. Method C ¹H NMR: (400 MHz, CD₃OD) δ 8.20 (s, 1H), 7.72 (s, 1H), 7.47 (d, *J* = 9.6 Hz, 1H), 7.33-7.24 (m, 5H), 6.83 (dd, *J* = 9.6, 1.6 Hz, 1H), 6.47 (s, 1H), 3.95-3.81 (m, 2H), 3.53-3.42 (m, 1H), 3.28-3.20 (m, 1H), 3.07-2.97 (m, 1H), 2.96-2.72 (m, 5H), 2.68 (ddd, *J=* 14.0, 5.6, 2.0 Hz, 1H), 1.99-1.93 (m, 1H), 1.92-1.81 (m, 1H), 1.79-1.62 (m, 2H), 1.54-1.44 (m, 1H). Chiral SFC: CO₂ and hexane:EtOH (3:7) containing 0.1% DEA over a CHIRALPAK^{®} IG column (4.6 × 250 mm 5 µm), retention time = 16.40 min.

Compounds 1113 and 1114 were prepared analogously. The two diastereomers were separated by chiral SFC eluting with CO₂ and hexane:EtOH containing 0.1% DEA over a CHIRALPAK^{®} IG column (4.6 × 250 mm 5 µm) to give **compound 1113** (41.3 mg) and **compound 1114** (33.4 mg).

**Compound 1113:** LCMS: (M+H)⁺ = 429.0; purity = 99% (214 nm); retention time = 1.323 min. Method C ¹H NMR: (400 MHz, CD₃OD) δ 8.96 (s, 2H), 7.56 (d, *J* = 8.4 Hz, 2H), 7.40 (d, *J=* 8.8 Hz, 2H), 7.31-7.17 (m, 4H), 6.53 (s, 1H), 3.97-3.88 (m, 1H), 3.83-3.72 (m, 1H), 3.58-3.46 (m, 1H), 3.29-3.22 (m, 1H), 3.02-2.91 (m, 2H), 2.86-2.67 (m, 5H), 1.94 (q, *J* = 2.8 Hz, 1H), 1.87-1.78 (m, 1H), 1.78-1.69 (m, 2H), 1.55-1.43 (m, 1H). Chiral SFC: CO₂ and hexane:EtOH (2:8) containing 0.1% DEA over a CHIRALPAK^{®} IG column (4.6 × 250 mm 5 µm), retention time = 10.59 min.

**Compound 1114:** LCMS: (M+H)⁺ = 429.1; purity = 97% (214 nm); retention time = 1.294 min. Method C ¹H NMR: (400 MHz, CD₃OD) δ 8.96 (s, 2H), 7.56 (d, *J=* 8.4 Hz, 2H), 7.39 (d, *J=* 8.4 Hz, 2H), 7.29-7.17 (m, 4H), 6.55 (s, 1H), 3.94-3.85 (m, 1H), 3.84-3.73 (m, 1H), 3.57-3.47 (m, 1H), 3.29-3.18 (m, 1H), 3.03-2.89 (m, 2H), 2.86-2.73 (m, 4H), 2.66 (ddd, *J=* 12.0, 5.6, 2.0 Hz, 1H), 1.95 (q, *J=* 2.8 Hz, 1H), 1.91-1.81 (m, 1H), 1.80-1.67 (m, 2H), 1.56-1.44 (m, 1H). Chiral SFC: CO₂ and hexane:EtOH (2:8) containing 0.1% DEA over a CHIRALPAK^{®} IG column (4.6 × 250 mm 5 µm), retention time = 19.69 min.

Compounds 1115 and 1116 were prepared analogously. The two diastereomers were separated by chiral SFC eluting with CO₂ and MeOH containing 0.2% methanolic ammonia over a CHIRALPAK^{®} IG column (20*250mm 10µm) to give **compound 1115** (57.8 mg) and **compound 1116** (6.1 mg).

**Compound 1115:** LCMS: (M+H)⁺ = 418; purity = 100% (214 nm); retention time = 1.44 min. Method A2 ¹H NMR: (400 MHz, CDCl₃) δ 7.21-7.12 (m, 6H), 6.97 (d, *J=* 8.8 Hz, 2H), 6.10 (s, 1H), 4.60 (d, *J* = 6.4 Hz, 1H), 3.85-3.84 (m, 1H), 3.75-3.65 (m, 3H), 3.36-3.30 (m, 1H), 2.92-2.89 (m, 2H), 2.80-2.70 (m, 4H), 2.45-2.40 (m, 1H), 1.84-1.81 (m, 1H), 1.64-1.58 (m, 2H), 1.32-1.25 (m, 2H), 0.76-0.73 (m, 4H). Chiral SFC: CO₂ and MeOH containing 0.2% methanolic ammonia over a CHIRALPAK^{®} IG column (4.6 × 100 mm 5 µm), retention time = 3.19 min.

**Compound 1116:** LCMS: (M+H)⁺ = 418; purity = 100% (214 nm); retention time = 1.42 min. Method A2 ¹H NMR: (400 MHz, CDCl₃) δ 7.23-7.15 (m, 6H), 6.99 (d, *J=* 4.8 Hz, 2H), 6.14 (s, 1H), 4.67 (d, *J=* 6.4 Hz, 1H), 3.87 (d, *J=* 6.4 Hz, 1H), 3.73-3.69 (m, 3H), 3.34-3.28 (m, 1H), 2.95-2.68 (m, 6H), 2.31-2.27 (m, 1H), 1.92-1.90 (m, 1H), 1.68-1.64 (m, 2H), 1.56-1.45 (m, 2H), 0.77-0.75 (m, 4H). Chiral SFC: CO₂ and MeOH containing 0.2% methanolic ammonia over a CHIRALPAK^{®} IG column (4.6 × 100 mm 5 µm), retention time = 6.17 min.

Compounds 1117 and 1118 were prepared analogously. The two diastereomers were separated by chiral SFC eluting with CO₂ and MeOH containing 0.2% methanolic ammonia over a CHIRALPAK^{®} IG column (4.6 × 100 mm 5 µm) to give **compound 1117** (10 mg) and **compound 1118** (8.4 mg).

**Compound 1117:** LCMS: (M+H)⁺ = 403; purity = 100% (214 nm); retention time = 1.277 min. Method C ¹H NMR (400 MHz, CD₃OD) δ 12.29 - 11.75 (m, 1H), 8.47 (d, J = 2.4 Hz, 1H), 8.37 - 8.23 (m, 1H), 7.61 (dd, J = 27.0, 1.5 Hz, 2H), 7.24 - 7.13 (m, 3H), 6.54 (s, 1H), 3.84 - 3.69 (m, 2H), 3.42 - 3.31 (m, 1H), 3.18 - 3.11 (m, 1H), 2.98 - 2.54 (m, 7H), 1.89 - 1.81 (m, 1H), 1.81 - 1.68 (m, 1H), 1.69 - 1.54 (m, 2H), 1.40 (d, J = 10.6 Hz, 1H), 1.21 (d, J = 17.8 Hz, 1H). Chiral SFC: CO₂ and n-hexane:EtOH (1:4) containing 0.1% DEA over a CHIRALPAK^{®} IG column (4.6 × 250 mm 5 µm), retention time = 11.10 min.

**Compound 1118:** LCMS: (M+H)⁺ = 403; purity = 100% (214 nm); retention time = 1.246 min. Method C ¹H NMR (400 MHz, CD₃OD) δ 8.46 (d, J = 2.4 Hz, 1H), 8.35 - 8.26 (m, 1H), 7.60 (dd, J = 26.1, 1.5 Hz, 2H), 7.23 - 7.12 (m, 4H), 6.56 (s, 1H), 3.88 - 3.70 (m, 2H), 3.41 - 3.27 (m, 1H), 3.18 - 3.11 (m, 1H), 2.92 (dd, J = 9.6, 5.6 Hz, 1H), 2.87 - 2.54 (m, 6H), 1.84 (dd, J = 5.9, 2.9 Hz, 1H), 1.81 - 1.71 (m, 1H), 1.69 - 1.58 (m, 2H), 1.39 (s, 1H), 1.21 (d, J = 18.8 Hz, 2H). Chiral SFC: CO₂ and n-hexane:EtOH (1:4) containing 0.1% DEA over a CHIRALPAK^{®} IG column (4.6 × 250 mm 5 µm), retention time = 16.51 min.

Compounds 1119 and 1120 were prepared analogously. The two diastereomers were separated by chiral SFC eluting with CO₂ and MeOH containing 0.2% methanolic ammonia over a CHIRALPAK^{®} IG column (4.6 × 100 mm 5 µm) to give **compound 1119** (11 mg) and **compound 1120** (10 mg).

**Compound 1119:** LCMS: (M+H)⁺ = 445; purity = 100% (214 nm); retention time = 1.332 min. Method A2 ¹H NMR (400 MHz, CD₃OD) δ 7.41 (d, J = 8.7 Hz, 2H), 7.17 - 7.02 (m, 6H), 6.35 (s, 1H), 3.78 (t, J = 7.1 Hz, 3H), 3.73 - 3.64 (m, 1H), 3.42 - 3.32 (m, 1H), 3.18 - 3.10 (m, 1H), 2.89 - 2.62 (m, 6H), 2.56 - 2.43 (m, 3H), 2.05 (dt, J = 15.3, 7.6 Hz, 2H), 1.97 - 1.82 (m, 1H), 1.80 - 1.56 (m, 2H), 1.46 - 1.33 (m, 1H), 1.32 - 1.14 (m, 2H). Chiral SFC: CO₂ and EtOH containing 0.1% DEA over a CHIRALPAK^{®} IG column (4.6 × 250 mm 5 µm), retention time = 27.82 min.

**Compound 1120:** LCMS: (M+H)⁺ = 445; purity = 100% (214 nm); retention time = 1.477 min. Method C ¹H NMR (400 MHz, CD₃OD) δ 7.41 (d, J = 8.7 Hz, 2H), 7.17 - 7.04 (m, 6H), 6.33 (s, 1H), 3.88 - 3.75 (m, 3H), 3.72 - 3.64 (m, 1H), 3.38 (ddd, J = 13.1, 8.5, 5.0 Hz, 1H), 2.90 - 2.62 (m, 7H), 2.47 (t, J = 8.1 Hz, 2H), 2.10 - 2.01 (m, 2H), 1.84 (dd, J = 6.0, 3.0 Hz, 1H), 1.75 - 1.62 (m, 3H), 1.40 (s, 1H), 1.26 - 1.15 (m, 2H). Chiral SFC: CO₂ and EtOH containing 0.1% DEA over a CHIRALPAK^{®} IG column (4.6 × 250 mm 5 µm), retention time = 34.21 min.

Compounds 1123 and 1124 were prepared analogously. The two diastereomers were separated by chiral SFC eluting with CO₂ and n-hexane:EtOH (1:1) containing 0.1% DEA over a CHIRALPAK^{®} IC column (4.6 × 250 mm 5 µm) to give **compound 1123** (16.9 mg) and **compound 1124** (20.4 mg).

**Compound 1123:** LCMS: (M+H)⁺ = 419; purity = 97.29% (214 nm); retention time = 1.172 min. Method C1 ¹H NMR(400 MHz, DMSO-*d₆*) δ 7.28-7.26 (m, 1H), 7.20-7.16(m, 3H), 6.22 (d, *J* = 6.0 Hz, 1H), 5.43 (d, *J* = 6.4 Hz, 1H), 4.03 (t, *J* = 7.6Hz, 1H), 3.85 (t, *J* = 7.2 Hz, 1H), 3.77-3.67 (m, 4H), 3.03(s, 3H), 3.02-2.98 (m, 2H), 2.83-2.80 (m, 3H), 2.62-2.57 (m, 5H), 1.72-1.66 (m, 2H), 1.53-1.49 (m, 2H), 1.35-1.32 (m, 1H). Chiral SFC: CO₂ and n-hexane:EtOH (1:4) containing 0.1% DEA over a CHIRALPAK^{®} IG column (4.6 × 250 mm 5 µm), retention time = 12.83 min. Chiral SFC: CO₂ and n-hexane:EtOH (1:1) containing 0.1% DEA over a CHIRALPAK^{®} IC column (4.6 × 250 mm 5 µm), retention time = 23.34 min.

**Compound 1124:** LCMS: (M+H)⁺ = 419; purity = 95.41% (214 nm); retention time = 1.169 min. Method C1 ¹H NMR(400 MHz, DMSO-*d₆*) δ 7.29-7.26 (m, 1H), 7.21-7.18 (m, 3H), 6.23 (d, *J* = 6.0Hz, 1H), 5.45 (d, *J=* 10.0 Hz, 1H), 4.00 (t, *J=* 7.6Hz, 1H), 3.85 (t, *J=* 7.2 Hz, 1H), 3.73-3.67 (m, 4H), 3.02(s, 3H), 3.02-2.98 (m, 2H), 2.83-2.80 (m, 3H), 2.62-2.51 (m, 5H), 1.78-1.66 (m, 2H), 1.53-1.49 (m, 2H), 1.35-1.32 (m, 1H). Chiral SFC: CO₂ and n-hexane:EtOH (1:4) containing 0.1% DEA over a CHIRALPAK^{®} IG column (4.6 × 250 mm 5 µm), retention time = 42.16 min. Chiral SFC: CO₂ and n-hexane:EtOH (1:1) containing 0.1% DEA over a CHIRALPAK^{®} IC column (4.6 × 250 mm 5 µm), retention time = 15.86 min.

### Example 6: Synthesis of Compound 1107 and 1108

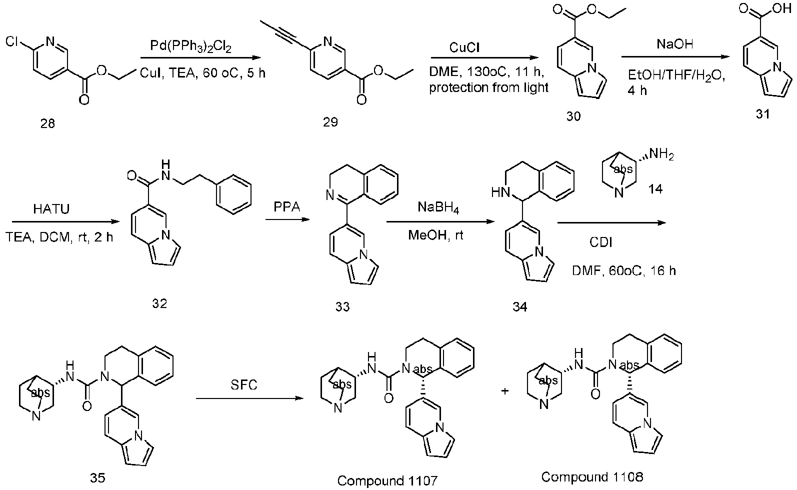

Step 1: To a sealed tube containing ethyl 6-chloronicotinate **28** (1.0 g, 5.39 mmol), Cul (0.154 g, 0.81 mmol), Pd(PPh₃)₂Cl₂ (0.189 g, 0.27 mmol) and anhydrous TEA (1.5 mL, 10.78 mmol) was added prop-1-yne (16.16 mL, 16.16 mmol) (1 mol/L in DMF). The mixture was stirred at 60 °C for 3 h. After cooling, the reaction mixture was poured into water and extracted with three 30 mL portions of EtOAc. The combined organic layers were washed with brine, dried over Na₂SO₄, filtered and concentrated. The crude compound was purified by silica gel column chromatography eluting with a gradient of Petroleum Ether:ethyl acetate, from 50:1 to 20:1 to give ethyl 6-prop-1-ynylnicotinate **29** (0.81 g, 4.3 mmol). LCMS: (M+H)⁺ =190; Purity 96% (UV 254 nm); Retention time =1.1.64min.

Step 2: To a solution of ethyl 6-prop-1-ynylnicotinate **29** (0.897 g, 4.74 mmol) and TEA (5.27 g, 37.9 mmol) in N, N-dimethylacetamide (20 mL) under argon atmosphere was added CuCI (0.48, 4.74 mmol). The mixture was stirred at 130 °C with protection from light for 8 hrs. After cooling, the reaction was diluted with 50 mL of DCM and passed through a plug of celite. The filtrate was washed with saturated aqueous NH₄Cl solution. The organic layer was dried over Na₂SO₄ and concentrated. The crude compound was purified by column chromatography, eluting with Petroleum Ether:ethyl acetate (30:1) to give ethyl indolizine-6-carboxylate **30** (228 mg, 1.2 mmol) . LCMS: (M+H)⁺ = 190; purity = 99 % (254 nm); retention time = 1.85 min. ¹H NMR: (400 MHz, CDCl₃) δ 8.74 (d, *J* = 1.2 Hz, 1H), 7.39 (d, *J* = 2.0 Hz, 1H), 7.35 (d, J = 9.6 Hz, 1H), 7.18-7.16 (m, 1H), 6.89-6.87 (m, 1H), 6.47 (d, J = 4.0 Hz, 1H), 4.38 (dd, J = 7.2, 14.4 Hz, 2H), 1.40 (t, J = 7.2 Hz, 3H).

Step 3: The compound ethyl indolizine-6-carboxylate **30** (228 mg, 1.21 mmol) was suspended in EtOH (1 mL) and water (1 mL). Then KOH (101 mg, 1.81 mmol) was added at room temperature. The reaction was stirred for 3 h. LCMS showed the starting material remained and trace desired compound detected. THF (1 mL) was added and the reaction was stirred at room temperature for 16 h. LCMS showed the starting material was consumed. The reaction mixture was concentrated to remove the organic solvent and then acidified by 1 N HCl to adjust to pH = 4. The resulting precipitate was collected by filtration and dried to give indolizine-6-carboxylic acid **31** (183 mg, 1.13 mmol). LCMS: (M+H)⁺ = 162; purity = 100% (214 nm); retention time = 1.05 min.

Step 4: To a solution of indolizine-6-carboxylic acid **31** (183 mg, 1.13 mmol) and TEA (0.32 mL, 2.27 mmol) in DCM (5 mL) was added HATU (518 mg, 1.36 mmol). After stirring for 10 min, the 2-phenylethanamine (165 mg, 1.36 mmol) was added and the reaction was stirred at room temperature for 2 h. The reaction was concentrated and the residue was purified by column chromatography, eluting with Petroleum Ether:ethyl acetate (4:1) to give N-phenethylindolizine-6-carboxamide **32** (290 mg, 1.097 mmol). LCMS: (M+H)⁺ = 265; purity = 100% (214 nm); retention time = 1.726 min.

Step 5: The N-phenethylindolizine-6-carboxamide **32** (240 mg, 0.91 mmol) and PPA was added to a 50 mL round-bottom flask. The reaction mixture was heated to 150 °C for 1 h. After cooling to 100 °C, the reaction mixture was poured into ice water and basified by NaOH. The mixture was extracted with four 30 mL portions of DCM. The combined organic layers were dried over Na₂SO₄, filtered and concentrated. The residue was purified by flash silica gel column chromatography eluting with a gradient of Petroleum Ether:ethyl acetate, from 10:1 to 5:1) to give 1-(indolizin-6-yl)-3,4-dihydroisoquinoline **33** (150 mg, 0.61 mmol). LCMS: (M+1)⁺ = 247; purity = 95.08 (214 nm); retention time = 1.871 min.

Step 6: To a solution of 1-(indolizin-6-yl)-3,4-dihydroisoquinoline **33** (150 mg, 0.61 mmol) in MeOH (2 mL) was added NaBH₄ (46 mg, 1.22 mmol). The reaction was stirred at room temperature for 1 h. The reaction was poured into water and extracted with three 20 mL portions of DCM. The combined organic layers were dried over Na₂SO₄, filtered and concentrated to give 1-(indolizin-6-yl)-1,2,3,4-tetrahydroisoquinoline **34** (144 mg, 0.58 mmol) suitable for next step without further purification. LCMS: (M+H)⁺ =249; purity = 93.85% (214 nm); retention time = 1.316 min.

Step 7: To a solution of 1-(indolizin-6-yl)-1,2,3,4-tetrahydroisoquinoline **34** (144 mg, 0.58 mmol), (S)-quinuclidin-3-amine HCl salt **14** (173 mg, 0.87 mmol) and TEA (0.41 mL, 2.9 mmol) in DMF (5 mL) was added CDI (188 mg, 1.16 mmol). The reaction mixture was stirred at 60 °C for 16 h. The reaction mixture was purified by preparative HPLC to give 1-(indolizin-6-yl)-N-((S)-quinuclidin-3-yl)-3,4-dihydroisoquinoline-2(1H)-carboxamide **35** (141 mL, 0.35 mmol) . LCMS: (M+H)⁺ = 401; purity = 93.37% (214 nm); retention time = 1.382 min.

Step 8: The 1-(indolizin-6-yl)-N-((S)-quinuclidin-3-yl)-3,4-dihydroisoquinoline-2(1H)-carboxamide 35 (141 mL, 0.35 mmol) was separated by SFC over a CHIRALPAK IG column 4.6 × 100mm, 5 µm; mobile phase: n-hexane (0.1%DEA):EtOH (0.1%DEA); column temperature: 39.3 °C; CO₂ flow rate: 2.2; Co-solvent flow rate: 1.8; Co-solvent%: 45; Wavelength: 214 nm & 254 nm to give **compound 1107** (17.7 mg, 0.04mmol) and **compound 1108** (34.9 mg, 0.087 mmol).

**Compound 1107:** LCMS: (M+H)⁺ = 401; purity = 100% (214 nm); retention time = 1.409 min. Method A2 ¹H NMR: (400 MHz, CD₃OD) δ 8.44 (s, 1H), 7.49 (s, 1H), 7.19-7.08 (m, 6H), 6.57-6.50 (m, 2H), 6.29 (s, 1H), 6.22 (d, *J* = 3.6 Hz, 1H), 4.03-3.99 (m, 1H), 3.79-3.73 (m, 1H), 3.53-3.43 (m, 1H), 3.40-3.33 (m, 1H), 3.16-2.72 (m, 7H), 2.07-1.81 (m, 4H), 1.67-1.60 (m, 1H). Chiral SFC: CO₂ and MeOH containing 0.2% methanolic ammonia over a CHIRALPAK^{®} IG column (4.6 × 100 mm 5 µm), retention time = 3.18 min.

**Compound 1108:** LCMS: (M+H)⁺ = 401; purity = 97.5% (214 nm); retention time = 1.41 min. Method A2 ¹H NMR: (400 MHz, CD₃OD) δ 7.48 (s, 1H), 7.23-7.08 (m, 7H), 6.57-6.49 (m, 2H), 6.29 (s, 1H), 6.21 (d, *J=* 3.6 Hz, 1H), 3.82-3.77 (m, 2H), 3.76-3.31(m, 1H), 3.17-3.11 (m, 1H), 2.93-2.51 (m, 8H), 1.85-1.62 (m, 1H). Chiral SFC: CO₂ and MeOH containing 0.2% methanolic ammonia over a CHIRALPAK^{®} IG column (4.6 × 100 mm 5 µm), retention time = 7.67 min.

### Example 7: Synthesis of Compounds 1121 and 1122

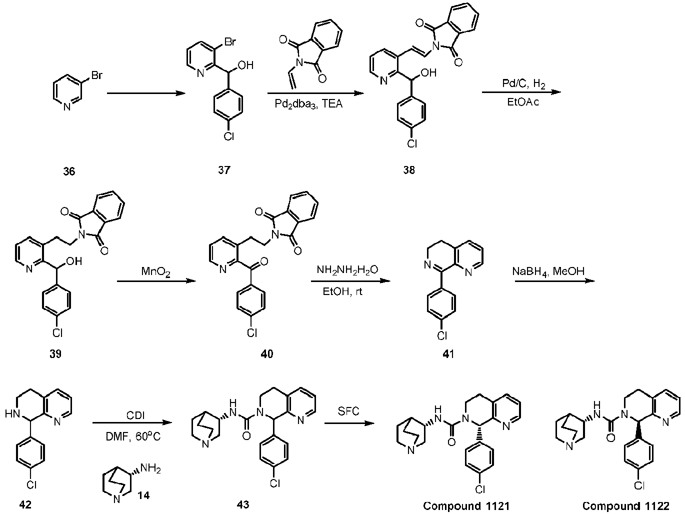

Step 1: To a solution of 3-bromopyridine **36** (5 g, 31.6 mol) in dry THF (60 mL) was added dropwise TMPMgCl LiCI (38 mL, 38 mmol) at 0°C. After addition, the reaction was stirred for 30 min at the temperature, then a solution of 4-chlorobenzaldehyde (0.49 g, 3.48 mmol) in dry THF (5 mL) was added dropwise at 0°C. The reaction was stirred at room temperature for 1 h. The reaction was poured into water and extracted with three 50 mL portions of EtOAc. The combined organic layers were dried over Na₂SO₄, filtered and concentrated. The crude residue was purified by silica gel column chromatography eluting with a gradient of Petroleum Ether:ethyl acetatefrom 20:1 to 10:1 to give (3-bromopyridin-2-yl)(4-chlorophenyl)methanol **37** (3.376 g, 11.3 mmol). LCMS: (M+H)⁺ =298; Purity 100% (UV 254 nm); Retention time =1.79 min.

Step 2: To a solution of (3-bromopyridin-2-yl)(4-chlorophenyl)methanol **37** (3.376 g, 11.3 mmol) , 2-vinylisoindoline-1,3-dione (2.35 g, 13.57 mmol) and TEA in DMF (50 mL) was added Pd₂dba₃ (518 mg, 0.57 mmol) and CyJohnPhos (396 mg, 1.13 mmol). The reaction mixture was purged with N₂ atmosphere and heated to 100 °C for 4 h. The reaction mixture was filtered and the filtrate was concentrated. The residue was suspended in EtOAc (30 mL) and stirred for 10 min. The solid was collected and dried to give (E)-2-(2-(2-((4-chlorophenyl)(hydroxy)methyl)pyridin-3-yl)vinyl)isoindoline-1,3-dione **38** (1.78 g, 4.58 mmol). LCMS: (M+H)⁺ = 391; purity = 95 % (254 nm); retention time = 1.484 min.

Step 3: To a suspension of (E)-2-(2-(2-((4-chlorophenyl)(hydroxy)methyl)pyridin-3-yl)vinyl)isoindoline-1,3-dione **38** (1.68 g, 4.3 mmol) in EtOAc (30 mL) was added 5% Pd/C (0.5 g). The reaction was evacuated and then refilled with H₂. The reaction was stirred at room temperature for 16 h. The reaction was filtered and concentrated to give 2-(2-(2-((4-chlorophenyl)(hydroxy)methyl)pyridin-3-yl)ethyl)isoindoline-1,3-dione **39** (1.68 g, 4.3 mmol). This material was used in the next reaction without further purification. LCMS: (M+H)⁺ = 393; purity = 100% (254 nm); retention time = 1.807 min.

Step 4: To a solution of 2-(2-(2-((4-chlorophenyl)(hydroxy)methyl)pyridin-3-yl)ethyl)isoindoline-1,3-dione **39** (1.68 g, 4.3 mmol) in DCM (30 mL) was added MnO₂ (3.718 g, 42.8 mmol). The reaction was stirred at room temperature for 16 h. Additional MnO₂ (3.17 g, 42.8 mmol) was added and the reaction stirred for 8 h. The reaction mixture was filtered and the filtrate was concentrated to give the 2-(2-(2-(4-chlorobenzoyl)pyridin-3-yl)ethyl)isoindoline-1,3-dione **40** (1.6 g, 4.1 mmol). This material was used in the next reaction without further purification. LCMS: (M+H)⁺ = 391; purity = 97.61% (254 nm); retention time = 1.836 min.

Step 5: To a suspension of 2-(2-(2-(4-chlorobenzoyl)pyridin-3-yl)ethyl)isoindoline-1,3-dione **40** (1.6 g, 4.1 mmol) in EtOH (50 mL) was added 85% H₂NNH₂ H₂O (0.6 mL 16.4 mmol). The reaction mixture was stirred at room temperature for 16 h. The reaction mixture was filtered and the filtrate was concentrated. The crude residue was purified by flash silica gel column chromatography eluting with Petroleum Ether:ethyl acetate (1:1) to 8-(4-chlorophenyl)-5,6-dihydro-1,7-naphthyridine **41** (0.3 g, 1.2 mmol). LCMS: (M+H)⁺ = 243; purity = 99.48 (254 nm); retention time = 1.242 min.

Step 6: To a solution of 8-(4-chlorophenyl)-5,6-dihydro-1,7-naphthyridine **41** (0.3 g, 1.2 mmol in MeOH (5 mL) was added NaBH₄ (94 mg, 2.47 mmol). The reaction was stirred at room temperature for 2 h. The reaction was concentrated and the residue was dissolved in DCM (50 mL). The solution was washed with water, brine, dried over Na₂SO₄ and concentrated to give 8-(4-chlorophenyl)-5,6,7,8-tetrahydro-1,7-naphthyridine **42** (0.3 g, 1.22 mmol). This material was used in the next reaction without further purification. LCMS: (M+H)⁺ = 245; purity = 90.6% (214 nm); retention time = 1.510 min.

Step 7: To a solution of 8-(4-chlorophenyl)-5,6,7,8-tetrahydro-1,7-naphthyridine **42** (0.3 g, 1.22 mmol), (S)-quinuclidin-3-amine HCl salt **14** (366 mg, 1.84 mmol) and TEA (0.85 mL, 6.13 mmol) in DMF (6 mL) was added CDI (398 mg, 2.45 mmol). The reaction mixture was stirred at 60 °C for 16 h. The reaction mixture was purified by preparative HPLC to give 8-(4-chlorophenyl)-N-((S)-quinuclidin-3-yl)-5,6-dihydro-1,7-naphthyridine-7(8H)-carboxamide **43** (220 mg, 0.56 mmol). LCMS: (M+H)⁺ = 397; purity = 100% (254nm); retention time = 1.276 min.

Step 8: The 8-(4-chlorophenyl)-N-((S)-quinuclidin-3-yl)-5,6-dihydro-1,7-naphthyridine-7(8H)-carboxamide **43** (120 mg, 0.3 mmol) was separated by SFC (Instrument: SFC-80 (Thar, Waters); Column: CHIRALPAK AD 20 × 250 mm, 10 µm (Daicel); Column temperature: 35 ºC; Mobile phase: CO₂:Methanol containing 0.2% methanolic ammonia) = 30:70; Flow rate: 80 mL/min; Back pressure: 100 bar; Detection wavelength: 214 nm; Cycle time:6 min; Sample solution:0.12 g dissolved in 15 mL methanol; Injection volume: 4.5 mL) to give **compound 1121** (43.5 mg) and **compound 1122** (37.8 mg).

**Compound 1121:** LCMS: (M+H)⁺ = 397; purity = 100% (214 nm); retention time = 1.269 min. Method A2 ¹H NMR: (400 MHz, CD₃OD) δ 8.41 (d, *J* = 4.4 Hz, 1H), 7.74 (d, *J*= 7.6 Hz, 1H), 7.37-7.31 (m, 3H), 7.19 (d, *J* = 8.0 Hz, 2H), 6.55 (s, 1H), 3.98-3.89 (m, 2H), 3.44-3.37 (m, 1H), 3.28-3.22 (m, 1H), 3.10-3.02 (m, 1H), 2.96-2.67 (m, 6H), 1.93-1.42 (m, 5H). Chiral SFC: CO₂ and EtOH containing 1% methanolic ammonia over a CHIRALPAK^{®} AD-H column (4.6 × 100 mm 5 µm), retention time = 2.30 min. Chiral SFC: CO₂ and MeOH containing 0.2% methanolic ammonia over a CHIRALPAK^{®} IG column (4.6 × 100 mm 5 µm), retention time = 2.38 min.

**Compound 1122:** LCMS: (M+H)⁺ = 397; purity = 100% (214 nm); retention time = 1.276 min. Method A2 ¹H NMR: (400 MHz, CD₃OD) δ 8.41 (d, *J=* 4.8 Hz, 1H), 7.74 (d, *J* = 7.6 Hz, 1H), 7.37-7.31 (m, 3H), 7.18 (d, *J=* 8.4 Hz, 2H), 6.57 (s, 1H), 3.98-3.87 (m, 2H), 3.43-3.36 (m, 1H), 3.26-3.21 (m, 1H), 3.12-3.04 (m, 1H), 2.93-2.79 (m, 5H), 2.68-2.62 (m, 1H), 1.95-1.46 (m, 5H). Chiral SFC: CO₂ and EtOH containing 1% methanolic ammonia over a CHIRALPAK^{®} AD-H column (4.6 × 100 mm 5 µm), retention time = 4.44 min. Chiral SFC: CO₂ and MeOH containing 0.2% methanolic ammonia over a CHIRALPAK^{®} IG column (4.6 × 100 mm 5 µm), retention time = 4.43 min.

### Example 8: Synthesis of Compound 1050

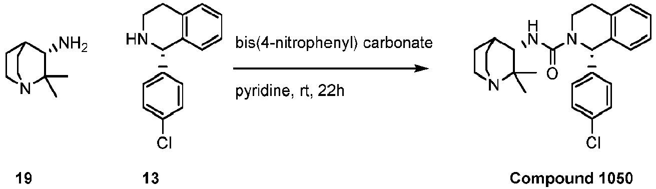

Under an argon atmosphere, a suspension of (S)-2,2-dimethylquinuclidin-3-amine **19** (76 mg, 0.492 mmol) and bis(4-nitrophenyl) carbonate (150 mg, 0.492 mmol) in pyridine (5 mL) was stirred at room temperature for 4 hours. Then, (S)-1-(4-chlorophenyl)-1 ,2,3,4-tetrahydroisoquinoline **13** (120 mg, 0.492 mmol) was added and stirring was continued for 18 hours. The mixture was poured into a 1:1 mixture of ice and saturated potassium carbonate (30 mL) and extracted with two 25 mL portions of ethyl acetate. The combined organic layers were dried over sodium sulfate and concentrated under reduced pressure. The residue was purified by silica flash chromatography eluting with a gradient of chloroform:7M NH₃ in methanol = 1:0 to 9:1] to obtain **Compound 1050** (63 mg, 0.149 mmol) after lyophilisation from acetonitrile/water. LCMS: 100%, retention time = 2.77 min., (M+H)⁺ = 424.2 (method A). ¹H NMR (400 MHz, Chloroform-*d*) δ 7.31 - 7.17 (m, 8H), 6.31 (s, 1H), 4.79 (d, J = 8.3 Hz, 1H), 3.77 - 3.56 (m, 3H), 3.31 - 3.16 (m, 2H), 2.99 - 2.81 (m, 2H), 2.79 - 2.66 (m, 2H), 1.72 - 1.53 (m, 3H), 1.40 (s, 3H), 1.37 - 1.28 (m, 2H), 1.25 (s, 3H).

### Example 9: Synthesis of Compound 1125

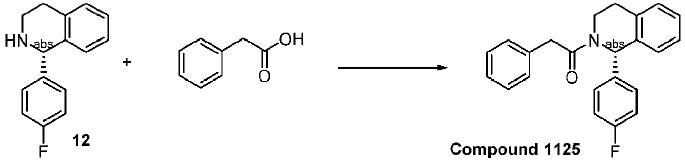

2-phenylacetic acid (100 mg, 0.74 mmol, 1 equiv) was dissolved in DMF (2 mL) and then (S)-1-(4-fluorophenyl)-1,2,3,4-tetrahydroisoquinoline **12** (167 mg, 0.74 mmol, 1.0 equiv), HATU (422 mg, 1.11 mmol, 1.5 equiv) and TEA (225 mg, 2.22 mmol, 3.0 equiv) were added. The mixture was stirred at 25 °C for 1 hour. Water (20 mL) was added and the mixture was extracted with three 10 mL portions of ethyl acetate. The combined organic layers were washed with three 10 mL portions of brine, dried and concentrated *in vacuo* to give crude product. The crude product was purified by reverse phase liquid chromatography (Mobile Phase: A:H₂O (10mM NH₄HCO₃) B: MeCN Gradient: 5%-95% B in 1.2 min Flow Rate : 2.0 ml/min Column : XBridge C18 50 × 4.6 mm,3.5 µm Oven Temperature: 40 °C UV 214 nm,MASS 100-1000) to give (S)-1-(1-(4-fluorophenyl)-3,4-dihydroisoquinolin-2(1H)-yl)-2-phenylethanone, **compound 1125** (77.6 mg). LCMS: (M+H)⁺ = 346; purity = 100% (214 nm); retention time = 2.004 min. Method C2. ¹H NMR (400 MHz, MeOD) δ 7.33- 7.27 (m, 2H), 7.27- 7.21 (m, 5H), 7.21-7.16 (m, 3H), 7.07 (d, J = 6.9 Hz, 1H), 7.01 (t, J = 8.8 Hz, 2H), 6.86 (s, 1H), 3.99- 3.86 (m, 3H), 3.40 (ddd, J = 13.8, 10.6, 5.0 Hz, 1H), 2.78- 2.61 (m, 2H). Chiral SFC: CO₂ and MeOH containing 0.2% ammonia over CHIRALPAK^{®} IG column (4.6 × 100 mm 5 µm), retention time = 1.78 min, 98.83%.

### Reference Example 4: Synthesis of Compounds 1130 and 1131

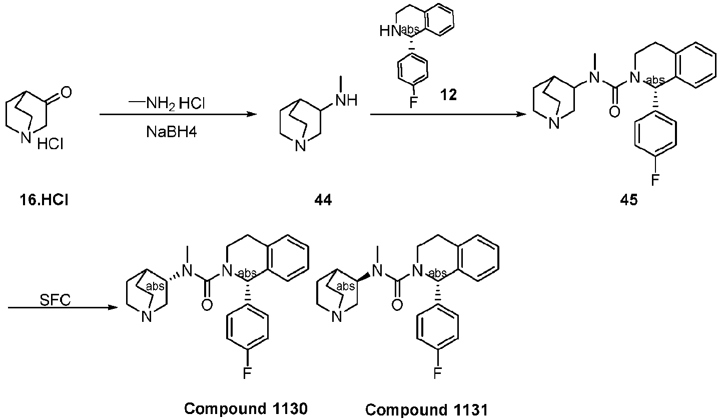

Step 1: To a solution of quinuclidin-3-one hydrochloride (161 mg, 1 mmol) in MeOH (10 mL) was added acetic acid (0.5 mL) and methanamine hydrochloride (80 mg, 1.2 mmol). The mixture was stirred at room temperature for 2 hours. Then NaBH₄ (76 mg, 2.0 mmol) was added and the mixture was stirred at room temperature for 2 hours more. To the mixture was added NaOH (5% a.q, 50 mL) and it was extracted with three 30 mL portions of ethyl acetate. The combined organic layers were washed with three 20 mL portions of brine, dried and concentrated *in vacuo* to give N-methylquinuclidin-3-amine **44** (137 mg).

Step 2: To a solution of (S)-1-(4-fluorophenyl)-1,2,3,4-tetrahydroisoquinoline **12** (222 mg, 0.98 mmol) in DMF (4 mL) was added N-methylquinuclidin-3-amine **44** (137 mg, 0.98 mmol), CDI (486 mg, 3 mmol) and TEA (304 mg, 3 mmol). The mixture was stirred at 70 °C for 20 minutes. The mixture was cooled to 25°C and 20mL of water was added. Then the mixture was extracted with three 20 mL portions of ethyl acetate. The combined organic layers were washed with three 20 mL portions of brine, dried and concentrated *in vacuo* to give crude product. The crude product was purified by preparative reverse phase chromatography (Mobile Phase: A:H₂O (10 mM NH₄HCO₃) B: MeCN Gradient: 5%-95% B in 1.2 min Flow Rate : 2.0 mL/min Column: XBridge C18 50 × 4.6 mm, 3.5 µm Oven Temperature: 40 °C UV 214 nm,MASS 100-1000) to give (1S)-1-(4-fluorophenyl)-N-methyl-N-(quinuclidin-3-yl)-3,4-dihydroisoquinoline-2(1H)-carboxamide **45** (150 mg).

The diastereomers were separated by chiral SFC eluting with CO₂/MeOH containing 0.2% methanolic ammonia over an EnantioPak^{®} IG column (4.6 × 100 mm 5 µm) to give **Compound 1130** (10.9 mg, retention time = 16.337 min) and **Compound 1131** (21.8 mg, retention time = 19.017 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1130:** LCMS: (M+H)⁺ = 394; purity = 100% (214 nm); retention time = 1.829 min. Method C2 ¹H NMR (400 MHz, DMSO-*d₆*) δ 7.24 (dd, J = 3.6, 2.1 Hz, 2H), 7.22-7.15 (m, 3H), 7.15- 7.08 (m, 3H), 6.19 (s, 1H), 3.80 (ddd, J = 12.8, 5.7, 2.9 Hz, 1H), 3.27-3.14 (m, 3H), 3.02 -2.91 (m, 2H), 2.81 (dt, J = 7.8, 4.2 Hz, 1H), 2.66 (s, 3H), 2.62- 2.56 (m, 3H), 2.22 (dd, J = 14.6, 4.1 Hz, 1H), 1.93 (d, J = 2.9 Hz, 1H), 1.70- 1.61 (m, 1H), 1.60- 1.50 (m, 1H), 1.42 (d, J = 3.0 Hz, 1H), 1.24 (d, J = 7.6 Hz, 1H).

**Compound 1131:** LCMS: (M+H)⁺ = 394; purity = 99.16% (214 nm); retention time = 1.811 min. Method C2 ¹H NMR (400 MHz, DMSO-*d₆*) δ 7.27- 7.20 (m, 2H), 7.18 (dd, J = 9.4, 4.4 Hz, 1H), 7.14 (d, J = 7.3 Hz, 4H), 7.06 (d, J = 7.5 Hz, 1H), 6.25 (s, 1H), 3.87- 3.78 (m, 1H), 3.24- 3.15 (m, 2H), 3.11 (d, J = 4.0 Hz, 1H), 2.99 (dd, J = 14.0, 8.3 Hz, 1H), 2.89-2.79 (m, 2H), 2.61 (d, J = 12.8 Hz, 6H), 2.17 (dd, J = 14.0, 3.0 Hz, 1H), 1.95 (d, J = 2.9 Hz, 1H), 1.68- 1.54 (m, 2H), 1.43 (d, J = 3.1 Hz, 1H), 1.28-1.27 (brs, 1H).

### Reference Example 5: Alternate Synthesis of Compound 1012

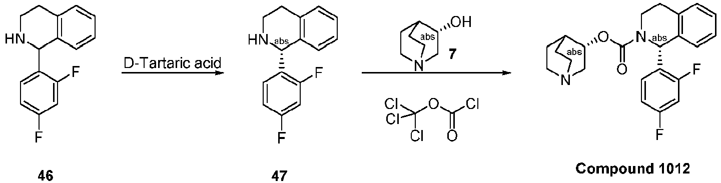

Step 1: To a solution of racemic 1-(2,4-difluorophenyl)-1,2,3,4-tetrahydroisoquinoline **46** (60 g, 0.244 mmol) in isopropanol (1 L) was added dropwise a solution of D-Tartaric acid (45 g, 0.3 mmol) in isopropanol (1 L) at room temperature. The mixture was stirred at room temperature overnight. The precipitate was filtered and the cake was washed with two 100 mL portions of isopropanol to give a solid . The solid was added into isopropanol (2 L) and heated to 100 °C. Water was added dropwise (200 mL) at 100 °C until the solid was dissolved. The mixture was allowed to crystallize at room temperature overnight. The precipitate was isolated by filtration and the cake was washed with two 100 mL portions of isopropanol to give a solid (40 g). A second recrystallization from isopropanol and water (~3/1, 100 °C to room temperature overnight) afforded 30 g of a solid. The solid was dissolved in water (200 mL), alkalized with NaOH (20% aq.) to pH 11 and extracted with two 200 mL portions of ethyl acetate. The combined organic layers were washed with brine (250 mL), dried and concentrated *in vacuo* to give 15 g of (R)-1-(2,4-difluorophenyl)-1,2,3,4-tetrahydroisoquinoline **47.** Chiral SFC: CO₂/MeOH containing 0.2% ammonia over CHIRALPAK^{®} AY-H column (4.6 × 250 mm 5 µm), retention time = 4.257 min), 100% ee.

Step 2: To a solution of (S)-quinuclidin-3-ol 7 (3.11 g, 24 mmol) in MeCN (200 mL) was added trichloromethyl carbonochloridate (3.6 g, 24 mmol) and the mixture was stirred at room temperature for 2 h. The mixture was concentrated to give a solid. The solid was dissolved in 9 mL of DMF and (R)-1-(2,4-difluorophenyl)-1,2,3,4-tetrahydroisoquinoline **47** (3 g, 12 mmol), TEA (4.8 g, 48 mmol) was added. The mixture was stirred at 35 °C overnight. The mixture was cooled to 0 °C and water (1 L) was added. The mixture was extracted with three 300 mL portions of ethyl acetate. The combined organic was washed with three 300 mL portions of brine, dried and concentrated *in vacuo* to give crude product. The crude product was purified by column chromatography eluting with petroleum ether:ethyl acetate (3:1) to give 5 g of **compound 1012.**

**Compound 1012:** LCMS: (M+H)⁺ = 399; purity = 100% (214 nm); retention time = 1.84 min. Method E ¹H NMR (400 MHz, DMSO-*d₆*) δ 7.34- 7.10 (m, 5H), 7.02 (t, J = 7.1 Hz, 2H), 6.37 (s, 1H), 4.58-4.56 (brs, 1H), 3.99 (d, J = 12.8 Hz, 1H), 3.06 (dd, J = 14.3, 8.4 Hz, 1H), 2.88-2.86 (m, 2H), 2.79- 2.51 (m, 6H), 1.86-1.84 (m, 1H), 1.54-1.53 (brs, 1H), 1.39-1.38 (m, 1H), 1.23-1.22 (brs, 2H). Chiral SFC: CO₂/MeOH containing 0.2% ammonia over CHIRALPAK^{®} IG column (4.6 × 100 mm 5 µm), retention time = 4.09 min), 100%.

### Reference Example 6: Synthesis of Compounds 1136 and 1137

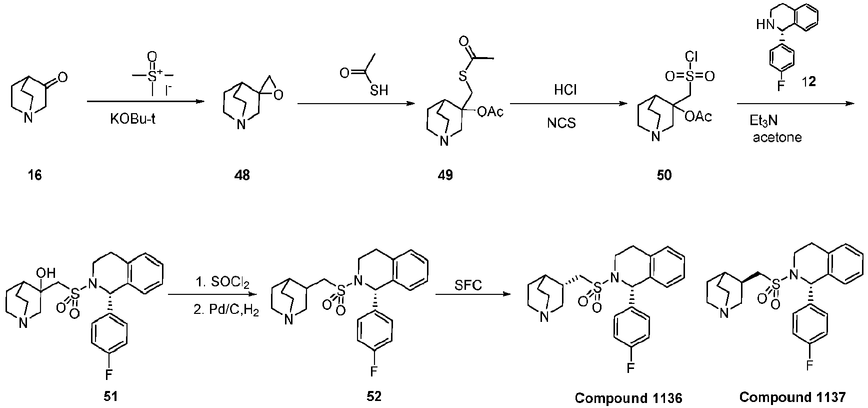

Step 1: A mixture of the hydrochloride salt of 3-quiniclidinone **16** (12.0 g, 80 mmol) and trimethylsulfoxonium iodide (21.g, 100 mmol) in dimethylsulfoxide (9.1 g, 63 mmol) was cooled to 0-5 °C in an ice/water bath under nitrogen atmosphere. A solution of potassium tert-butoxide (20.1 g, 179 mmol) in dimethylsulfoxide (500 mL) was added dropwise over 45 minutes. The mixture was warmed gradually to room temperature and stirred for an additional 16 hours at room temperature. After cooling to 0-5°C (ice/water bath) the mixture was poured into an ice/water mixture (25 g) and then sodium chloride (15 g) was added. The mixture was stirred for 30 minutes and extracted with three 400 mL portions of toluene. The combined toluene phases were dried over sodium sulfate, filtered and evaporated to obtain the epoxide of 3-methylenequinuclidine **48** (6.0 g). The product was used in the next step neat or as toluene solution after the extraction without further purification. LCMS: (M+H)⁺ = 140 (UV 214 nm); Retention time = 0.99 min. Method B

Step 2: A solution of the epoxide of 3-methylenequiniclidine **48** (5.4 g, 39 mmol) in toluene (100 mL) was cooled to 0-5°C (ice/water bath). Thiol acetic acid was added dropwise over 10-15 minutes. The mixture was stirred at 0-5°C for 30 minutes and then allowed to come to room temperature. After stirring at room temperature for 2 hours the formed precipitate was filtered and washed with two 100 mL portions of toluene to give the 3-((acetylthio)methyl) quinuclidin-3-yl acetate **49** (7.7 g). LCMS: (M+H)⁺ = 258 (UV 214 nm); Retention time =2.05 min. Method B

Step 3: To a mixture of 2M HCl (17.5 mL) and MeCN (87.7 mL) was added NCS (35.1 g, 0.26 mol) and then the mixture was cooled to 10 °C. A solution of 3-((acetylthio)methyl) quinuclidin-3-yl acetate **49** (2.57 g, 10 mmol) in MeCN (17.5 mL) was added dropwise to the above mixture, keeping the temperature below 20 °C. The resulting solution was stirred below 20 °C for 10 min, and then diluted with isopropyl ether (100 mL). The organic layer was washed with three 50 mL portions of aqueous NaCl (12%), and concentrated *in vacuo.* The residue was purified by flash silica gel column chromatography eluting with n-hexane:EtOAc, 4:1 to obtain 3-((chlorosulfonyl)methyl) quinuclidin-3-yl acetate **50** (750mg). LCMS: (M+H)⁺ = 282 (UV 214 nm); Retention time =1.304 min. Method B

Step 4: A mixture of (S)-1-(4-fluorophenyl)-1,2,3,4-tetrahydroisoquinoline **12** (227 mg, 1 mmol) and triethylamine (303 mg, 3 mmol) in tetrahydrofuran (40 mL) was cooled to 0 °C, and then 3-((chlorosulfonyl)methyl) quinuclidin-3-yl acetate **50** (281 mg, 1 mmol) was added dropwise. The mixture was stirred at room temperature for 2h. The tetrahydrofuran was removed *in vacuo* and the mixture was diluted with 40 mL of water. It was extracted with three 50 mL portions of dichloromethane:methanol (20:1), then dried with Na₂SO₄. The mixture was filtered, and the solvent was evaporated to give 300 mg of 3-((((S)-1-(4-fluorophenyl)-3,4-dihydroisoquinolin-2(1H)-yl) sulfonyl) methyl) quinuclidin-3-ol **51.**

Step 5: A solution of 3-((((S)-1-(4-fluorophenyl)-3,4-dihydroisoquinolin-2(1H)-yl) sulfonyl) methyl) quinuclidine **51** (430 mg, 1 mmol) in CH₃CN (20 mL) was cooled to 0-5°C (ice/water bath). SOCl₂ was added dropwise over 10-15 minutes. The mixture was stirred at 0-5°C for 30 minutes and then allowed to come to room temperature. After stirring at room temperature for 2 hours, the mixture was concentrated *in vacuo* to give the crude (S)-3-(((1-(4-fluorophenyl)-3,4-dihydroisoquinolin-2(1H)-yl)sulfonyl) methyl)-1- azabicyclic [2.2.2]oct-2-ene, which was used directly in the next reaction. The crude product was taken up in CH₃OH (20 mL), Pd/C was added and the mixture hydrogenated under 1 atm H₂ (balloon). After stirring at room temperature overnight, the mixture was filtered and the solvent was evaporated to give crude product. The crude product was purified by preparative reverse phase HPLC (Mobile Phase: A:H₂O (10 mM NH₄HCO₃) B: MeCN Gradient: 5%-95% B in 1.2 min, Flow Rate: 2.0 mL/min Column: XBridge C18 50 × 4.6 mm, 3.5 µm oven temperature: 40 °C UV 214 nm, MASS 100-1000) to give (1S)-1-(4-fluorophenyl)-N-methyl-N-(quinuclidin-3-yl)-3,4-dihydroisoquinoline-2(1H)-carboxamide **52** (150 mg).

The diastereomers were separated by chiral SFC eluting with CO₂/MeOH containing 0.2% methanolic ammonia over an CHIRALPAK^{®} IG column (4.6 × 100 mm 5 µm) to give **compound 1136** (12.5 mg, retention time = 2.47 min) and **compound 1137** (12 mg, retention time = 2.88 min). Stereochemical assignment of (S) at tetrahydroisoquinoline is absolute based on starting materials, stereochemical assignment at quinuclidine is assigned based on chromatographic elution order as compared to diastereomers of related analogues of known configuration.

**Compound 1136:** LCMS: (M+H)⁺ = 415; purity = 100% (214 nm); retention time = 1.820 min. Method B ¹H NMR (400 MHz, DMSO-*d₆*) δ 7.33- 7.13 (m, 7H), 7.11 (t, J = 7.3 Hz, 1H), 6.03 (s, 1H), 3.71-3.70 (m, 1H), 3.33- 3.26 (m, 1H), 3.08- 2.94 (m, 3H), 2.83-2.82 (m, 2H), 2.68-2.53 (m, 3H), 2.45- 2.33 (m, 1H), 1.77 (d, J = 11.9 Hz, 1H), 1.49- 1.32 (m, 3H), 1.30- 1.06 (m, 3H).

**Compound 1137:** LCMS: (M+H)⁺ = 415; purity = 100% (214 nm); retention time = 2.859 min. Method B ¹H NMR (400 MHz, DMSO-*d₆*) δ 7.30- 7.14 (m, 7H), 7.09 (d, J = 7.6 Hz, 1H), 6.04 (s, 1H), 3.70 (dd, J = 10.5, 3.0 Hz, 1H), 3.33- 3.26 (m, 1H), 2.99-2.96 (m, 3H), 2.86- 2.72 (m, 2H), 2.57 (dd, J = 16.5, 8.5 Hz, 3H), 2.25 (dd, J = 12.9, 5.8 Hz, 1H), 1.87 (d, J = 7.7 Hz, 1H), 1.65 (d, J = 2.0 Hz, 1H), 1.52- 1.38 (m, 2H), 1.38- 1.13 (m, 3H).

Compounds 1140, 1141, 1142, and 1143 were prepared analogously. The diastereomers were separated by chiral SFC eluting with CO₂/MeOH containing 0.2% methanolic ammonia over an EnantioPak^{®} AS column (4.6 × 100 mm 5 µm) to give **compound 1140** (5.0 mg, retention time = 2.67 min), **compound 1141** (6.2 mg, retention time = 3.27 min), **compound 1142** (7.8 mg, retention time = 3.43 min) and **compound 1143** (6.5 mg, retention time = 3.47 min). Stereochemical assignment of (S) or (R) at quinuclidine site is absolute based on elution order comparison to related analogs of known configuration. Stereochemical assignment at 1-position of the tetrahydroisoquinoline is assigned based on chromatographic elution order as compared to diastereomers of related analogues of known configuration. As such, these stereochemical assignments are best approximations of absolute stereochemistry. Analytical data shown below is specific to each isomer.

**Compound 1140:** LCMS: (M+H)⁺ = 397; purity = 100% (214 nm); retention time = 1.328 min. Method A1 ¹H NMR (400 MHz, MeOD) δ 7.32- 7.12 (m, 7H), 7.11- 7.05 (m, 1H), 6.92 (d, J = 7.7 Hz, 1H), 5.97 (s, 1H), 3.72 (ddd, J = 13.6, 6.4, 2.1 Hz, 1H), 3.31 (ddd, J = 13.7, 11.1, 4.6 Hz, 1H), 3.00 (ddd, J = 17.2, 11.0, 6.6 Hz, 1H), 2.88- 2.80 (m, 2H), 2.77- 2.67 (m, 2H), 2.57 (dt, J = 12.3, 8.8 Hz, 2H), 2.24 (ddd, J = 13.7, 6.4, 2.1 Hz, 1H), 2.01- 1.94 (m, 1H), 1.64 (d, J = 2.7 Hz, 1H), 1.52 (ddd, J = 10.0, 8.2, 4.9 Hz, 1H), 1.47- 1.38 (m, 2H), 1.36-1.28 (m, 1H), 1.26- 1.16 (m, 2H).

**Compound 1141:** LCMS: (M+H)⁺ = 397; purity = 100% (214 nm); retention time = 1.527 min. Method A ¹H NMR (400 MHz, MeOD) δ 7.28- 7.14 (m, 7H), 7.12- 7.05 (m, 1H), 6.92 (d, J = 7.7 Hz, 1H), 5.96 (s, 1H), 3.77- 3.69 (m, 1H), 3.32 (ddd, J = 13.7, 11.2, 4.6 Hz, 1H), 2.95 (ddd, J = 23.7, 12.3, 8.1 Hz, 2H), 2.86- 2.71 (m, 3H), 2.60 (td, J = 14.3, 5.3 Hz, 3H), 2.46 (dd, J = 12.9, 5.5 Hz, 1H), 1.91 (d, J = 3.9 Hz, 1H), 1.47 (dd, J = 6.1, 2.7 Hz, 1H), 1.40 (d, J = 2.7 Hz, 1H), 1.27-1.25 (m, 4H).

**Compound 1142:** LCMS: (M+H)⁺ = 397; purity = 100% (214 nm); retention time = 1.520 min. Method C ¹H NMR (400 MHz, DMSO-*d₆*) δ 7.30- 7.14 (m, 7H), 7.09 (d, J = 7.6 Hz, 1H), 6.04 (s, 1H), 3.70 (dd, J = 10.5, 3.0 Hz, 1H), 3.33- 3.26 (m, 1H), 2.99-2.97 (m, 3H), 2.86- 2.72 (m, 2H), 2.57-2.55 (m, 3H), 2.25 (dd, J = 12.9, 5.8 Hz, 1H), 1.87 (d, J = 7.7 Hz, 1H), 1.65 (d, J = 2.0 Hz, 1H), 1.52-1.38 (m, 2H), 1.38- 1.13 (m, 3H).

**Compound 1143:** LCMS: (M+H)⁺ = 397; purity = 100% (214 nm); retention time = 1.527 min. Method C ¹H NMR (400 MHz, MeOD) δ 7.27- 7.13 (m, 7H), 7.12- 7.05 (m, 1H), 6.91 (d, J = 7.7 Hz, 1H), 5.98 (s, 1H), 3.72 (ddd, J = 13.7, 6.5, 1.8 Hz, 1H), 3.31 (ddd, J = 13.7, 11.2, 4.6 Hz, 1H), 3.00 (ddd, J = 17.3, 11.1, 6.5 Hz, 1H), 2.88- 2.79 (m, 2H), 2.76- 2.67 (m, 2H), 2.62- 2.57 (m, 2H), 2.27 (ddd, J = 13.7, 6.4, 2.1 Hz, 1H), 2.04- 1.94 (m, 1H), 1.65 (dd, J = 5.5, 2.8 Hz, 1H), 1.54 (ddd, J = 9.8, 8.1, 4.9 Hz, 1H), 1.49- 1.39 (m, 2H), 1.36- 1.29 (m, 1H), 1.26- 1.18 (m, 2H).

### Reference Example 7: Synthesis of Compounds 1144 and 1145

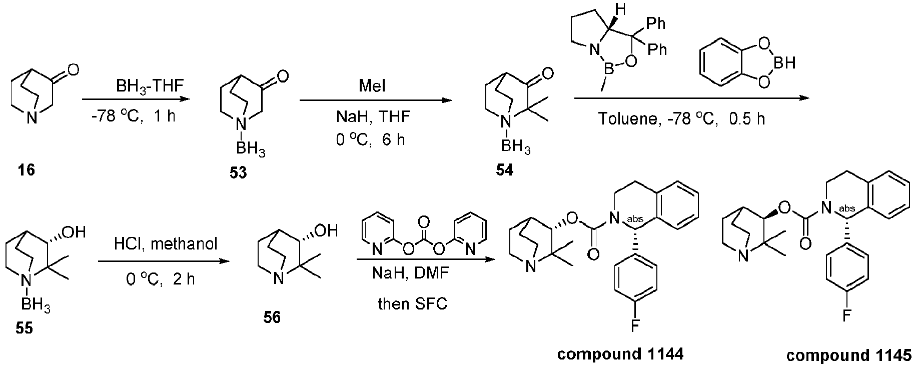

Step 1: Under an argon atmosphere, a solution of quinuclidin-3-one **16** (30 g, 240 mmol) in THF was cooled to -78 °C. Borane dimethyl sulfide complex (1M in THF) (288 mL, 288 mmol) was added and the mixture was stirred at -78 °C for 20 minutes. MeOH (100 mL) was added and the mixture was allowed to warm up to room temperature. The reaction mixture was concentrated under reduced pressure and the residue was purified by column chromatography eluting with petroleum ether:ethyl acetate = 3:1 to give 21 g of quinuclidin-3-one borane complex **53.** ¹H NMR (400 MHz, CDCl₃) δ: 3.54 (brs, 2H), 3.31-3.23 (m, 2H), 3.20-3.12 (m, 2H), 2.70-2.67 (m, 1H), 2.21 - 2.08 (m, 4H), 2.00-1.25 (m, 3H).

Step 2: To a mixture of quinuclidin-3-one borane complex **53** (21 g, 151 mmol) in THF (500 mL) was added sodium hydride (5.4 g, 226 mmol) at 0 °C. The mixture was stirred at 0 °C for 0.5 h under N₂, then iodomethane (32.1 g, 226 mmol) was added dropwise and the mixture was stirred for 2 h. Sodium hydride (5.4 g, 226 mmol) was added at 0 °C, the reaction was stirred at 0 °C for 0.5 h and iodomethane (32.1 g, 226 mmol) was added dropwise. The mixture was stirred at room temperature for 2 h. The reaction was quenched with NH₄Cl solution and concentrated *in vacuo.* The crude was purified with column chromatography (Petroleum Ether:ethyl acetate = 5:1) to give 13 g of 2,2-dimethylquinuclidin-3-one borane complex **54.** ¹H NMR (400 MHz, CDCl₃) δ: 3.54-3.42 (m, 2H), 3.23-3.15 (m, 2H), 2.63-2.60 (m, 1H), 2.15 - 1.97 (m, 4H), 1.90-1.18 (m, 3H), 1.56-1.55 (brs, 6H).

Step 3: A 50% solution of catechol borane in toluene (17 mL, 179 mmol) was added dropwise over 40 min to a yellow solution of 2,2-dimethylquinuclidin-3-one borane complex **54** (8.6 g, 51.5 mmol) and (R)-1-methyl-3,3-diphenylhexahydropyrrolo[1,2-c][1,3,2]oxazaborole (5.6 g, 20.6 mmol) in anhydrous toluene (400 mL) stirring at -50 °C. The reaction mixture was stirred for 20 min following the completion of the addition and then left in a refrigerator (-20 °C) for 2 days. The reaction mixture was diluted with EtOAc (300 mL) and 100 mL of 1M Na₂CO₃, and vigorously stirred for 30 min. The organic layer was washed with four 50 mL portions of a solution of Na₂CO₃ solution by vigorously stirring for 15 min with each wash. The organic layer was dried over MgSO₄, filtered and concentrated to give the crude product as yellow solid which was purified by flash silica gel chromatography using a gradient of 0 to 40% EtOAc:hexane as eluent to afford 4 g of crude (S)-2,2-dimethylquinuclidin-3-ol borane complex **55.** ¹H NMR (400 MHz, CDCl₃) δ 3.68 (d, J = 2.4 Hz, 1H), 3.36-3.19 (m, 2H), 2.96-2.87 (m, 2H), 2.12-2.05 (m, 2H), 1.84 - 1.69 (m, 4H), 1.46 (d, J = 3.6 Hz, 6H), 1.80-1.00 (m, 3H).

Step 4: To a solution of (S)-2, 2-dimethylquinuclidin-3-ol borane complex **55** (4 g, 23.7 mmol) in methanol (20 mL) was added 4 N HCl in dioxane (30 mL, 0.16 mmol) at 0 °C. The mixture was stirred at 0 °C for 1 hour and allowed to warm to room temperature and stirred for 3 h. The mixture was cooled to 0 °C and concentrated *in vacuo* to remove HCl, alkalized to pH = 8 with Na₂CO₃ (10% aq.) and extracted with three 100 mL portions of dichloromethane:methanol (10:1). The combined organic layers were washed with brine (50 mL), dried and concentrated *in vacuo* to give 1.2 g of (S)-2,2-dimethylquinuclidin-3-ol **56.** ¹H NMR (400 MHz, CDCl₃) δ: 3.71 (d, J = 2.8 Hz, 1H), 3.46-3.37 (m, 2H), 3.25-3.08 (m, 2H), 2.18-2.11 (m, 2H), 1.86 - 1.80 (m, 2H), 1.64-1.57 (m, 1H), 1.49 (d, J = 12 Hz, 6H).

Step 5: To a solution of (S)-1-(4-fluorophenyl)-1,2,3,4-tetrahydroisoquinoline **12** (397 mg, 1.75 mmol) in DMF (2 mL) was added dipyridin-2-yl carbonate (400 mg, 1.85 mmol) and 2,2-dimethylquinuclidin-3-ol **56** (407 mg, 2.6 mmol). The mixture was heated to 80 °C and NaH (60%, 105 mg, 2.6 mmol) was added. The mixture was stirred at 80 °C for 12 hours. The mixture was cooled to 25°C and water (20 mL) was added. The mixture was extracted with three 20 mL portions of ethyl acetate. The combined organic layers were washed with three 20 mL portions of brine, dried and concentrated *in vacuo* to give crude product. The crude product was purified by preparative reverse phase HPLC (Mobile Phase: A:H₂O (10 mM NH₄HCO₃) B:MeCN Gradient: 5%-95% B in 1.2min flow rate: 2.0 mL/min column : XBridge C18 50 × 4.6 mm, 3.5 µm oven temperature: 40 °C UV 214 nm, MASS 100-1000) to give (1S)-2,2-dimethylquinuclidin-3-yl-1-(4-fluorophenyl)-3,4-dihydroisoquinoline-2(1H)-carboxylate (30 mg).

The diastereomers were separated by chiral SFC eluting with CO₂/MeOH containing 0.2% methanolic ammonia over an CHIRALPAK^{®} AD-H column (4.6 × 100 mm 5 µm), collecting the major 2 peaks to give **compound 1144** (4.5 mg, retention time = 0.99 min) and **compound 1145** (4.6 mg, retention time= 2.0 min). Stereochemical assignment of (S) at the 1-position of the tetrahydroisoquinoline is absolute based on starting materials, stereochemical assignment at quinuclidine attachement is assigned based on chromatographic elution order as compared to diastereomers of related analogues of known configuration.

**Compound 1144:** LCMS: (M+H)⁺ = 409; purity = 100% (214 nm); retention time = 1.939 min. Method C ¹H NMR (400 MHz, MeOD) δ 7.28-7.05 (m, 5H), 7.00- 6.85 (m, 3H), 6.40 - 6.10 (m, 1H), 4.51 (d, J = 3.0 Hz, 1H), 3.92 (s, 1H), 3.09 - 2.98 (m, 2H), 2.85 (d, J = 9.9 Hz, 1H), 2.80- 2.74 (m, 1H), 2.60 (d, J = 8.6 Hz, 2H), 1.88 (d, J = 3.0 Hz, 1H), 1.80 - 1.69 (m, 1H), 1.66 - 1.58 (m, 2H), 1.38- 1.29 (m, 1H), 1.27-1.22 (brs, 4H), 1.21-1.17 (brs, 2H), 1.15-1.05 (m, 1H)

**Compound 1145:** LCMS: (M+H)⁺ = 409; purity = 99.72% (214 nm); retention time = 1.850 min. Method C ¹H NMR (400 MHz, MeOD) δ 7.19 - 7.08 (m, 5H), 7.05 - 6.95 (m, 1H), 6.93 (t, J = 8.7 Hz, 2H), 6.30 - 6.15 (m, 1H), 4.52 (d, J = 2.4 Hz, 1H), 3.90 (s, 1H), 3.31 (s, 1H), 3.19 - 3.12 (m, 1H), 3.05 (d, J = 14.9 Hz, 1H), 2.86 (ddd, J = 15.6, 9.7, 5.7 Hz, 1H), 2.73 (d, J = 17.0 Hz, 1H), 2.69 - 2.55 (m, 2H), 1.95 - 1.82 (m, 1H), 1.64 (dd, J = 9.3, 4.4 Hz, 2H), 1.40 - 1.30 (m, 1H), 1.25 (s, 3H), 1.20 - 1.17(brs, 1H), 1.07 (s, 3H).

Compounds 1148 and 1149 were prepared analogously. The diastereomers were separated by chiral SFC eluting with CO₂/MeOH containing 0.2% methanolic ammonia over an EnantioPak^{®} AS column (4.6 × 100 mm 5 µm) to give **compound 1148** (4.0 mg, retention time = 11.329 min) and **compound 1149** (4.0 mg, retention time = 12.114 min). Stereochemical assignment of (S) at quinuclidine is absolute based on starting materials, stereochemical assignment at 1-position of the tetrahydroisoquinoline is assigned based on chromatographic elution order as compared to diastereomers of related analogues of known configuration.

**Compound 1148:** LCMS: (M+H)⁺ = 427; purity = 100% (214 nm); retention time = 1.665 min. Method C ¹H NMR (400 MHz, MeOD) δ 7.14 - 7.05 (m, 4H), 6.95 (s, 3H), 6.41 (s, 1H), 4.48 - 4.38 (m, 1H), 4.05 - 3.88 (br, 1H), 3..68 - 3.35 (m, 1H), 3.05 - 2.95 (br, 1H), 2.80 (s, 3H), 2.65 - 2.51 (br, 2H), 1.86 - 1.84 (m, 1H), 1.79 - 1.71 (m, 1H), 1.65 - 1.55 (m, 2H), 1.42 - 1.35 (m, 1H), 1.23 (s, 3H), 1.16 - 1.01 (m, 3H).

**Compound 1149:** LCMS: (M+H)⁺ = 427; purity = 100% (214 nm); retention time = 1.665 min. Method C ¹H NMR (400 MHz, MeOD) δ 7.14 - 7.12 (m, 2H), 7.11 - 7.03 (m, 2H), 7.01 - 6.97 (m, 2H), 6.82 - 6.75 (br, 1H), 6.44 (s, 1H), 4.48 - 4.41 (m, 1H), 3.99 - 3.88 (m, 1H), 3.51 - 3.42 (m, 1H), 3.21 - 3.14 (br, 1H), 3.12 - 2.95 (m, 1H), 2.92 - 2.87 (m, 2H), 2.59 - 2.51(m, 2H), 1.75 - 1.51 (br, 3H), 1.35 - 1.32 (m, 1H), 1.28 - 1.18 (m, 4H), 1.04 (s, 3H).

### Reference Example 8: Synthesis of Compounds 1146 and 1147

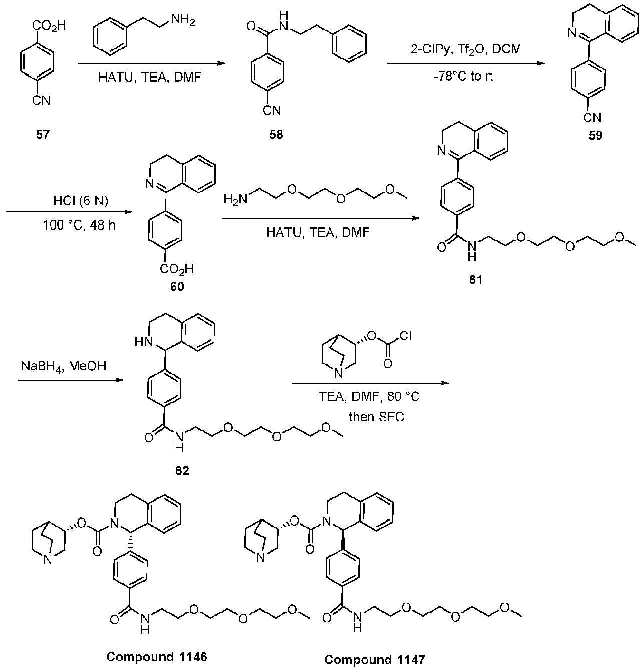

Step 1: TEA (4.7 mL, 34 mmol) was added to the mixture of 4-cyanobenzoic acid 57 (2.5 g, 17 mmol), 2-phenylethanamine (2.059 g, 17 mmol) and HATU (7.75 g, 20.4 mmol) in DMF (30 mL) at 0 °C. The mixture was stirred at room temperature for 2 hours. The mixture was diluted with water (120 mL) and extracted with four 100 mL portions of ethyl acetate. The combined organic layers were washed with brine (50 mL), dried and concentrated *in vacuo* to give 3.99 g of 4-cyano-*N*-phenethylbenzamide **58.** LCMS: (M+H)⁺ = 251 (UV 214 nm); Retention time =1.46 min. Method A1 Step 2: Tf₂O (1.5 mL, 9.6 mmol) in DCM (10 mL) was added dropwise to a stirred mixture of 4-cyano-*N*-phenethylbenzamide **58** (2 g, 8 mmol) and 2-chloropyridine (0.9 mL, 8.8 mmol) in DCM (30 mL) at -78 °C. The reaction mixture was slowly warmed to ambient temperature. The mixture was alkalized with NaOH (10% aq.) to pH = 11 and water (30 mL) was added. The aqueous layer was extracted with four 100 mL portions of dichloromethane. The combined organic layers were washed with brine (50 mL), dried and concentrated *in vacuo* to give 1 g of 4-(3,4-dihydroisoquinolin-1-yl)benzonitrile **59.** LCMS: (M+H)⁺ = 233 (UV 214 nm); Retention time =1.21 min. Method A1

Step 3: The solution of 4-(3,4-dihydroisoquinolin-1-yl)benzonitrile **59** (800 mg, 3.4 mmol) in HCl (12 N) (8 mL) and H₂O (8 mL) was stirred at 100 °C for 48 hours. The solution was cooled to 25 °C and concentrated *in vacuo* to give 860 mg of 4-(3,4-dihydroisoquinolin-1-yl)benzoic acid **60.** LCMS: (M+H)⁺ = 252 (214 nm); retention time = 1.24 min. Method A1

Step 4: TEA (0.55 mL, 4 mmol) was added to the mixture of 4-(3,4-dihydroisoquinolin-1-yl)benzoic acid **60** (500 mg, 2 mmol), 2-(2-(2-methoxyethoxy)ethoxy)ethanamine (390 mg, 2.4 mmol) and HATU (908 mg, 2.4 mmol) in DMF (6 mL) at 0 °C. The mixture was stirred at room temperature for 2 hours. The mixture was diluted with 40 mL of water and extracted with four 50 mL portions of ethyl acetate. The combined organic layers were dried with Na₂SO₄ and concentrated to give 700 mg of 4-(3,4-dihydroisoquinolin-1-yl)-*N-*(2-(2-(2-methoxyethoxy)ethoxy)ethyl)benzamide **61.** LCMS: (M+H)⁺ = 397 (214 nm); retention time = 1.34 min. Method A1

Step 5: NaBH₄ (57 mg, 1.5 mmol) was added to the solution of 4-(3,4-dihydroisoquinolin-1-yl)-*N*-(2-(2-(2-methoxyethoxy)ethoxy)ethyl)benzamide **61** (300 mg, 0.7 mmol) in MeOH (4 mL) at 0 °C. The reaction mixture was stirred at ambient temperature for 2 hours. The mixture was diluted with 20 mL of water and extracted with three 50 mL portions of ethyl acetate. The combined organic layers were dried with Na₂SO₄ and concentrated to give 200 mg of *N*-(2-(2-(2-methoxyethoxy)ethoxy)ethyl)-4-(1,2,3,4-tetrahydroisoquinolin-1-yl)benzami-de **62.** LCMS: (M+H)⁺ = 399 (214 nm); retention time = 1.26 min. Method A1

Step 6: TEA (0.35 mL, 2.5 mmol) was added to the solution of *N*-(2-(2-(2-methoxyethoxy)ethox-y)ethyl)-4-(1,2,3,4-tetrahydroisoquinolin-1-yl)benzamide **62** (200 mg, 0.5 mmol) in DMF (4 mL). After 10 min, (*S*)-quinuclidin-3-yl carbonochloridate (95 mg, 0.5 mmol) was added to the mixture. The mixture was stirred at 80 °C overnight. The mixture was diluted with water (20 mL) and extracted with three 20 mL portions of dichloromethane:methanol (20:1). The combine organic layers were washed with brine (20 mL), dried and concentrated *in vacuo* to give crude product. The crude product was purified by preparative reverse phase HPLC (Mobile Phase: A:H₂O (10 mM NH₄HCO₃) B:MeCN Gradient: 33%-63% B in 8.0min, flow rate: 30.0 mL/min column : XBridge C18 OBD 21.2 × 250 mm, 10 µm, oven temperature: 40 °C UV 214 nm, MASS: 100-1000) to give the desired product (80 mg).

The diastereomers were separated by chiral SFC eluting with CO₂/EtOH containing 1% methanolic ammonia over an EnantioPak^{®} AD column (20 × 250 mm 10 µm) to give **compound 1146** (15.6 mg, retention time = 1.77 min) and **compound 1147** (14.2 mg, retention time = 3.09 min). Stereochemical assignment of (S) at quinuclidine is absolute based on starting materials, stereochemical assignment at 1-position of the tetrahydroisoquinoline is assigned based on chromatographic elution order as compared to diastereomers of related analogues of known configuration.

**Compound 1146:** LCMS: (M+H)⁺ = 552; purity = 97.5% (214 nm); retention time = 1.323 min. Method A1 ¹H NMR (400 MHz, CD₃OD) δ 7.67 (d, *J=* 8.0 Hz, 2H), 7.23-7.21 (brs, 2H), 7.16-7.08 (m, 3H), 7.02 (d, *J* = 7.2 Hz, 1H), 6.31-6.12 (m, 1H), 4.70-4.68 (brs, 1H), 3.87 (dt, *J* = 12.8, 4.2 Hz, 1H), 3.56-3.47 (m, 8H), 3.45 (t, *J* = 4.2 Hz, 2H), 3.39-3.32 (m, 3H), 3.18 (s, 3H), 3.15-3.09 (m, 1H), 2.89-2.59 (m, 7H), 1.95-1.94 (brs, 1H), 1.84-1.59 (m, 2H), 1.52 -1.50(brs, 1H), 1.42- 1.28 (m, 1H).

**Compound 1147:** LCMS: (M+H)⁺ = 552; purity = 100% (214 nm); retention time = 1.322 min. Method A1 ¹H NMR (400 MHz, CD₃OD) δ 7.67-7.65 (brs, 2H), 7.27 (d, *J* = 7.2 Hz, 2H), 7.16-7.02 (m, 4H), 6.32-6.12 (m, 1H), 4.72-4.66 (m, 1H), 3.83 (s, 1H), 3.55-3.47 (m, 8H), 3.44 (t, *J=* 4.2 Hz, 2H), 3.39-3.31 (m, 3H), 3.17 (s, 3H), 3.12-3.10 (brs, 1H), 2.91-2.38 (m, 7H), 1.95-1.94 (brs, 1H), 1.82- 1.58 (m, 2H), 1.52-1.50 (brs, 1H), 1.38-1.36 (brs, 1H).

### Example 10: Synthesis of Compounds 1151 and 1152

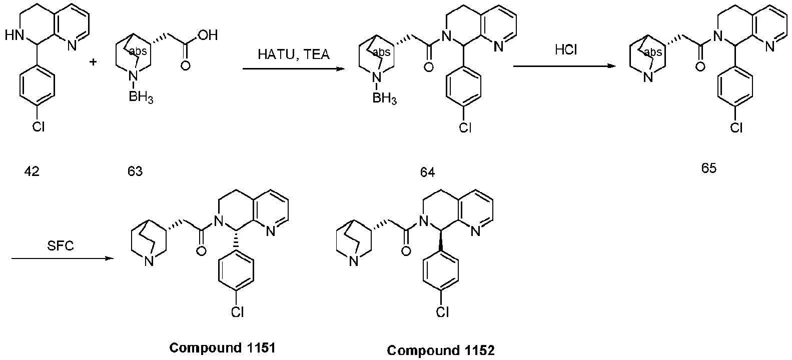

Step 1: (S)-(3-(carboxymethyl)-1-ammoniobicyclo[2.2.2]octan-1-yl)trihydroborate **63** (63 mg, 0.344 mmol) was dissolved in DMF (1 mL) and to the mixture was added 8-(4-chlorophenyl)-5,6,7,8-tetrahydro-1,7-naphthyridine **42** (84 mg, 0.344 mmol), HATU (196 mg, 0.52 mmol) and TEA (104 mg, 1.03 mmol). The mixture was stirred at 25 °C for 1 hour. To the mixture was added water (20 mL) and the resulting mixture was extracted with three 10 mL portions of ethyl acetate. The combined organic layers were washed with three 10 mL portions of brine, dried and concentrated *in vacuo* to give ((3S)-3-(2-(8-(4-chlorophenyl)-5,6-dihydro-1,7-naphthyridin-7(8H)-yl)-2-oxoethyl)-1-ammoniobicyclo[2.2.2]octan-1-yl)trihydroborate **64.** LCMS: (M+H)⁺ = 410; purity = 60.75% (214 nm); retention time = 1.780 min. Method C

Step 2: ((3S)-3-(2-(8-(4-chlorophenyl)-5,6-dihydro-1,7-naphthyridin-7(8H)-yl)-2-oxoethyl)-1-ammoniobicyclo[2.2.2]octan-1-yl)trihydroborate **64** (110 mg) was dissolved in MeOH (5 mL) and to the mixture was added 1,4-dioxane/HCl (5 mL). The mixture was stirred at 25 °C for 1 hour, then concentrated *in vacuo* to give a white solid. It was dissolved in water (10 mL) and alkalized with NaOH (20% aq.) to pH 11 and extracted with three 10 mL portions of ethyl acetate. The combined organic layers were washed three times with brine, dried and concentrated *in vacuo* to give crude product. The crude product was purified by liquid preparation method (Mobile Phase: A: H₂O (10mM NH₄HCO₃) B:MeCN Gradient: 5%-95% B in 1.2 min Flow Rate: 2.0 mL/min, Column: XBridge C18 50*4.6 mm,3.5 µm, Oven Temperature: 40 °C UV 214, MASS100-1000) to give 1-(8-(4-chlorophenyl)-5,6-dihydro-1,7-naphthyridin-7(8H)-yl)-2-((S)-quinuclidin-3-yl)ethanone **65** (80 mg).

The enantiomers were separated by chiral SFC eluting with *n*-hexane containing 0.1% DEA): EtOH containing 0.1% DEA) = 50:50 over a CHIRALPAK^{®} IG (4.6*100 mm 5µm) to give **compound 1151** (8.9 mg) and **compound 1152** (5.1 mg).

***Compound 1151:*** LCMS: (M+H)⁺ = 396; purity = 100% (214 nm); retention time = 1.314 min. Method A2 ¹H NMR (400 MHz, DMSO-*d₆*) δ 8.43 (d, J = 3.6 Hz, 1H), 7.69 (d, J = 7.6 Hz, 1H), 7.36 (d, J = 8.5 Hz, 2H), 7.30 (dd, J = 7.7, 4.8 Hz, 1H), 7.14 (d, J = 8.4 Hz, 2H), 6.63 (s, 1H), 4.04-3.92 (m, 1H), 3.47-3.29 (m, 4H), 3.07- 2.95 (m, 2H), 2.86-2.78 (m, 1H), 2.69-2.62 (m, 3H), 2.20 (dd, J = 13.3, 6.1 Hz, 1H), 2.04-1.95 (m, 1H), 1.71-1.62 (brs, 2H), 1.58-1.43 (m, 3H). Chiral SFC: CO₂/EtOH containing 0.1% ammonia over CHIRALPAK^{®} IG column (4.6*250mm 5µm), retention time = 23.398 min, 98% ee.

***Compound 1152:*** LCMS: (M+H)⁺ = 396; purity = 99.6% (214 nm); retention time = 1.302 min. Method A2 ¹H NMR (400 MHz, DMSO-*d₆*) δ 8.43 (d, J = 4.0 Hz, 1H), 7.70 (d, J = 7.8 Hz, 1H), 7.36 (d, J = 8.4 Hz, 2H), 7.31 (dd, J = 7.6, 4.7 Hz, 1H), 7.13 (d, J = 8.4 Hz, 2H), 6.63 (s, 1H), 4.02-3.92 (m, 1H), 3.06- 2.91 (m, 2H), 2.83 (d, J = 16.7 Hz, 1H), 2.74- 2.58 (m, 5H), 2.56 (dd, J = 10.5, 7.5 Hz, 2H), 2.22 (dd, J = 14.0, 5.9 Hz, 1H), 2.05- 1.97 (brs, 1H), 1.72 -1.61 (m, 1H), 1.58- 1.46 (m, 3H), 1.33-1.26 (m, 1H). Chiral SFC: CO₂/EtOH containing 0.1% ammonia over CHIRALPAK^{®} IG column (4.6*250mm 5µm), retention time = 36.160 min, 99.78% ee.

Compounds 1153, 1154, 1155, 1156, 1157, 1158, 1159, 1160, 1161, 1162, 1163, and 1164 were prepared analogously.

For Compounds 1153 and 1154, the diastereomers were separated by chiral SFC eluting with CO₂/MeOH containing 0.2% methanolic ammonia over an EnantioPak^{®} AD column (4.6*100 mm 5µm) to give **Compound 1153** (23.5 mg, retention time = 1.21 min) and **Compound 1154**(19.6 mg, retention time = 2.31 min). Stereochemical assignment of (S) at quinuclidine is absolute based on starting materials, stereochemical assignment at the 1-position of the tetrahydroisoquinoline is assigned based on chromatographic elution order as compared to diastereomers of related analogues of known configuration.

**Compound 1153:** LCMS: (M+H)⁺ = 427; purity = 90% (214 nm); retention time = 1.711 min. Method A ¹H NMR (400 MHz, CD₃OD) δ 7.29-6.94 (m, 7H), 6.33 (brs, 1H), 4.61 (d, *J=* 2.4 Hz, 1H), 4.00 (brs, 1H), 3.42-3.22 (m, 2H), 3.19-3.09 (m, 1H), 3.00-2.91 (m, 1H), 2.89-2.79 (m, 1H), 2.78-2.62 (m, 2H), 1.97-1.95 (brs, 1H), 1.83-1.80 (brs, 1H), 1.72-1.70 (brs, 2H), 1.42-1.12 (m, 7H).

**Compound 1154:** LCMS: (M+H)⁺ = 427; purity = 99% (214 nm); retention time = 1.472 min. Method A1 ¹H NMR (400 MHz, CD₃OD) δ 7.31-6.98 (m, 7H), 6.33 (brs, 1H), 4.63 (d, *J=* 2.4 Hz, 1H), 4.00-3.98 (brs, 1H), 3.44-3.42 (brs, 1H), 3.29-3.27 (brs, 1H), 3.21-3.12 (m, 1H), 3.02-2.92 (m, 1H), 2.89-2.79 (m, 1H), 2.78-2.63 (m, 2H), 1.99-1.98 (brs, 1H), 1.85-1.84 (brs, 1H), 1.79-1.67 (m, 2H), 1.42-1.40 (brs, 1H), 1.36 (s, 3H), 1.18(s, 3H).

For compounds 1155 and 1156, the diastereomers were separated by chiral SFC eluting with CO₂/MeOH containing 0.2% methanolic ammonia over an EnantioPak^{®} IG column (4.6*100mm 5µm) to give **Compound 1155** (6.4 mg, retention time = 15.816 min) and **Compound 1156** (7.5 mg, retention time = 21.804 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1155:** LCMS: (M+H)⁺ = 384; purity = 100% (214 nm); retention time = 1.210 min. Method A1 ¹H NMR (400 MHz, CD₃OD) δ 7.31-7.25 (m, 1H), 7.24-7.19 (m, 2H), 7.17 (d, *J=* 7.2 Hz, 1H), 5.09 (d, *J=* 4.8 Hz, 1H), 4.69-4.64 (m, 1H), 4.29 (dt, *J=* 12.8, 4.0 Hz, 1H), 3.21 (dd, J= 8.4 Hz, 1H), 3.06-2.92 (m, 3H), 2.90-2.74 (m, 7H), 2.68 (d, *J=* 14.4 Hz, 1H), 2.37 (d, *J=* 15.6 Hz, 1H), 2.01-2.00 (brs, 1H), 1.90-1.88 (brs, 1H), 1.82-1.73 (m, 1H), 1.68-1.58 (m, 1H), 1.55-1.44 (m, 1H), 1.32-1.24 (m, 3H).

**Compound 1156:** LCMS: (M+H)⁺ = 384; purity = 98% (214 nm); retention time = 1.215 min. Method A1 ¹H NMR (400 MHz, CD₃OD) δ 7.32-7.26 (m, 1H), 7.23-7.19 (m, 2H), 7.17 (d, *J=* 7.6 Hz, 1H), 5.09 (d, *J=* 5.2 Hz, 1H), 4.69-4.64 (m, 1H), 4.29 (dt, J= 12.0, 4.0 Hz, 1H), 3.23 (dd, J= 8.4 Hz, 1H), 3.06-2.93 (m, 3H), 2.90-2.76 (m, 7H), 2.69 (d, *J=* 14.8 Hz, 1H), 2.37 (d, *J=* 15.6 Hz, 1H), 2.02-2.00 (brs, 1H), 1.93-1.91 (brs, 1H), 1.83-1.73 (m, 1H), 1.68-1.58 (m, 1H), 1.55-1.46 (m, 1H), 1.32-1.24 (m, 3H).

For compounds 1157 and 1158, the diastereomers were separated by chiral SFC eluting with CO₂/MeOH containing 0.2% methanolic ammonia over an IG column(4.6*100mm, 5um) to give **Compound 1157** (19.4 mg, retention time = 1.92 min) and **Compound 1158**(15.2 mg, retention time = 2.69 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1157:** LCMS: (M+H)⁺ =403; purity = 100% (214 nm); retention time = 1.312 min. Method A2 ¹H NMR (400 MHz, DMSO-*d₆*) δ 13.06 (s, 1H), 8.02 (s, 1H), 7.50 (d, J = 8.7 Hz, 1H),7.45 -7.33 (m, 1H), 7.32- 7.22 (m, 3H), 7.22-7.10 (m, 2H), 6.38 (s, 1H), 4.70-4.58 (s, 1H), 4.07 - 3.86 (m, 1H), 3.09 (dd, J = 14.3, 8.5 Hz, 1H), 2.96-2.80 (m, 2H), 2.71-2.70 (brs, 2H), 2.60 (d, J = 6.3 Hz, 3H), 1.98-1.88 (brs, 1H), 1.77-1.52 (m, 2H), 1.51-1.43 (m, 1H), 1.28- 1.19 (m, 2H).

**Compound 1158:** LCMS: (M+H)+ =403; purity = 100% (214 nm); retention time = 1.314 min. Method A2 ¹H NMR (400 MHz, DMSO-*d₆*) δ 13.07 (s, 1H), 8.02 (s, 1H), 7.51 (d, J = 8.5 Hz, 1H), 7.47-7.29 (m, 2H), 7.28-7.22 (brs, 2H), 7.21-7.15 (brs, 2H), 6.37 (s, 1H), 4.70- 4.63 (m, 1H), 3.91 (dt, J = 13.0, 5.0 Hz, 1H), 3.15-3.00 (brs, 1H), 2.98-2.80 (m, 2H), 2.77- 2.54 (m, 4H), 2.48-2.32 (m, 1H), 1.92 (d, J = 2.8 Hz, 1H), 1.78-1.67 (brs, 1H), 1.63-1.53 (m, 1H), 1.52-1.41 (brs, 1H), 1.27- 1.20 (m, 2H).

For compounds 1159 and 1160, the diastereomers were separated by chiral SFC eluting with CO₂/MeOH containing 0.2% methanolic ammonia over an AD-H column (4.6*100mm, 5µm) to give **Compound 1159** (20 mg, retention time = 2.92 min) and **Compound 1160** (15 mg, retention time = 2.30 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1159:** LCMS: (M+H)⁺ = 434; purity = 100% (214 nm); retention time = 1.539 min. Method C ¹H NMR (400 MHz, DMSO-*d₆*) δ 7.84-7.82 (m, 2H), 7.27 (t, J = 40.1 Hz, 5H), 6.33 (s, 1H), 4.63-4.61 (brs, 1H), 3.89-3.86 (brs, 1H), 3.48-3.45 (brs, 1H), 3.05 (d, J = 7.1 Hz, 1H), 2.89-2.86 (brs, 2H), 2.77 (s, 3H), 2.63 (d, J = 32.7 Hz, 5H), 1.89-1.86 (brs, 1H), 1.59 -1.58(brs, 1H), 1.49 (d, J = 40.9 Hz, 2H), 1.31- 1.15 (m, 2H).

**Compound 1160:** LCMS: (M+H)⁺ = 434; purity = 100% (214 nm); retention time = 1.545 min. Method C ¹H NMR (400 MHz, DMSO-*d₆*) δ 7.59-7.55 (m, 2H), 7.22 (d, J = 19.6 Hz, 5H), 6.37 (s, 1H), 4.64-4.62 (brs, 1H), 3.92-3.90 (brs, 1H), 3.39-3.36 (brs, 1H), 3.15-3.02 (m, 1H), 2.88-2.86 (brs, 2H), 2.67-2.65 (brs, 2H), 2.55-2.53 (m, 5H), 1.90-1.88 (brs, 1H), 1.64-1.63 (brs, 1H), 1.57-1.55 (brs, 1H), 1.46-1.42 (brs, 1H), 1.40-1.31 (m, 1H), 1.26-1.24 (m, 3H).

For compounds 1161 and 1162, the diastereomers were separated by chiral SFC eluting with CO₂/MeOH containing 0.2% methanolic ammonia over an AD-H column (4.6*100mm, 5µm)to give **Compound 1161** (15 mg, retention time = 2.09 min) and **Compound 1162** (15 mg, retention time = 2.59 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1161:** LCMS: (M+H)⁺ = 418; purity = 100% (214 nm); retention time = 1.504 min. Method C ¹H NMR (400 MHz, DMSO-*d₆*) δ 7.57-7.52 (m, 2H), 7.23-7.20 (m, 5H), 6.34 (s, 1H), 4.64-4.62 (brs, 1H), 3.93-3.83 (m, 1H), 3.44-3.42 (brs, 1H), 3.04-3.02 (brs, 1H), 2.88 (d, J = 14.4 Hz, 2H), 2.59-2.56 (m, 6H), 1.92-1.90 (brs, 1H), 1.72-1.70 (brs, 1H), 1.58-1.56 (brs, 1H), 1.47-1.45 (brs, 1H), 1.39- 0.80 (m, 4H).

**Compound 1162:** LCMS: (M+H)+ = 418; purity = 100% (214 nm); retention time = 1.492 min. Method C ¹H NMR (400 MHz, DMSO-*d₆*) δ 7.85-7.82 (m, 2H), 7.24-7.20 (brs, 5H), 6.29 (s, 1H), 4.64 -4.62(brs, 1H), 3.85-3.83 (brs, 1H), 3.47-3.45 (brs, 1H), 2.91 (s, 3H), 2.81- 2.54 (m, 6H), 2.33-3.32 (brs, 1H), 1.91-1.90 (brs, 1H), 1.68-1.66 (brs, 1H), 1.57-1.55 (brs, 1H), 1.42-1.40 (m, 1H), 1.37- 1.08 (m, 3H).

For compounds 1163 and 1164, the diastereomers were separated by chiral SFC eluting with EtOH containing 1% methanolic ammonia over an EnantioPak^{®} AD-H column (4.6*100mm 5µm) to give **Compound 1163** (13.2 mg, retention time = 2.31 min) and **Compound 1164** (14.5 mg, retention time = 1.83 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

**Compound 1163:** LCMS: (M+H)⁺ = 420.8; purity = 100% (214 nm); retention time = 1.525 min. Method E ¹H NMR (400 MHz, DMSO-*d₆*) δ 7.29 - 7.13 (m, 3H), 7.11 (s, 1H), 6.78 (d, J = 8.4 Hz, 1H), 6.69-6.52 (m, 2H), 6.21-6.08 (brs, 1H), 4.70-4.58 (brs, 1H), 4.19 (s, 4H), 3.95-3.83 (brs, 1H), 3.28 - 3.20 (m, 1H), 3.15 - 3.03 (m, 1H), 2.89 -2.78 (m, 2H), 2.75-2.66 (m, 2H), 2.61 (dd, J = 13.8, 7.9 Hz, 2H), 1.95-1.86 (brs, 1H), 1.74 - 1.55 (m, 2H), 1.47 (dd, J = 6.0, 2.8 Hz, 1H), 1.36-1.27 (brs, 1H), 1.26-1.20 (brs, 1H).

**Compound 1164:** LCMS: (M+H)⁺ = 420.8; purity = 99.4% (214 nm); retention time = 1.518 min. Method E ¹H NMR (400 MHz, DMSO-*d₆*) δ 7.28 - 7.11 (m, 4H), 6.79 (d, J = 8.3 Hz, 1H), 6.75-6.53 (m, 2H), 6.19-6.08 (m, 1H), 4.69 - 4.59 (m, 1H), 4.19 (s, 4H), 3.86 (dt, J = 12.6, 5.0 Hz, 1H), 3.31 - 3.21 (m, 1H), 3.07 (dd, J = 14.5, 8.1 Hz, 1H), 2.92-2.79 (m, 2H), 2.75-2.65 (m, 2H), 2.63 - 2.55 (m, 2H), 1.95-1.86 (brs, 1H), 1.79-1.68 (brs, 1H), 1.58 (dt, J = 9.3, 6.6 Hz, 1H), 1.53-1.42 (brs, 1H), 1.37- 1.29 (m, 1H), 1.26-1.22 (brs, 1H).

### Reference Example 9: Synthesis of Compounds 1165 and 1166

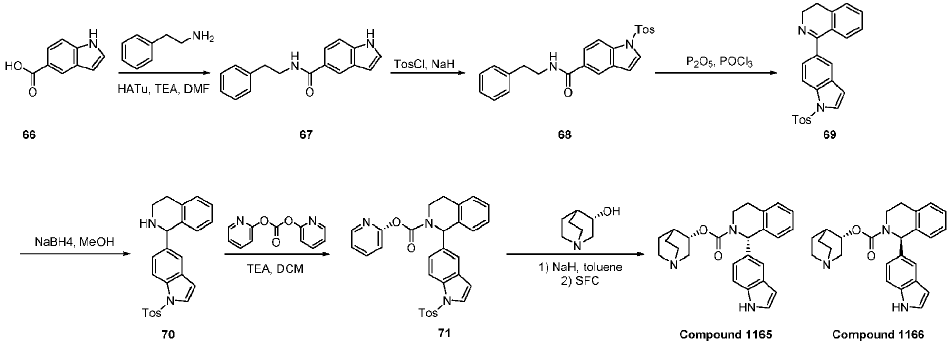

Step 1: To a solution of 1H-indole-5-carboxylic acid **66** (3.0 g, 18.6 mmol) in DMF (30 mL) was added 2-phenylethanamine (3.4 mL, 27.2 mmol), HATU (14.0 g, 36.8 mmol) and TEA (10.2 mL, 73.8 mmol) at 0°C. The mixture was stirred for 5 hours at room temperature. Water (200 mL) was added and the phases were separated. The organic phase was washed with brine (20 mL) and dried over Na₂SO₄. The solvent was evaporated to give N-phenethyl-1H-indole-5-carboxamide **67** (1.27 g). LCMS: (M+H)⁺ = 265; Purity 91% (UV 254 nm); Retention time = 1.76 min.

Step 2: To a solution of N-phenethyl-1H-indole-5-carboxamide **67** (1.27 g, 4.8 mmol) in DMF (15 mL) was added NaH (230 mg, 9.6 mmol) at 0 °C. The mixture was stirred at room temperature for 0.5 h, followed by adding *p*-toluenesulfonyl chloride (1.0 g, 5.28 mmol) dropwise. The mixture was stirred at room temperature for 12 h. Water (60 mL) was added and the phases were separated. The organic phase was washed with brine (20 mL) and dried over Na₂SO₄. The crude was purified with column chromatography to give N-phenethyl-1-tosyl-1H-indole-5-carboxamide **68** (1.5 g). LCMS: (M+H)⁺ = 419; Purity 59% (UV 254 nm); Retention time =2.00 min.

Step 3: To a solution of N-phenethyl-1-tosyl-1H-indole-5-carboxamide **68** (1.0 g, 2.39 mmol) in POCl₃ (20 mL) was added P₂O₅ (679 mg, 4.78 mmol). The mixture was heated under reflux for 4 hours. The mixture was cooled to 25 °C and quenched with ice water (100 mL), alkalized with NaOH (10% aq.) to pH =11 and extracted with three 100 mL portions of ethyl acetate. The combined organic layers were washed with three 100 mL portions of brine, dried and concentrated *in vacuo* to give the crude product. The material was purified by SiO₂ column chromatography, eluting with petroleum ether:ethyl acetate (3:1) to give 1-(1-tosyl-1H-indol-5-yl)-3,4-dihydroisoquinoline **69** (700 mg). LCMS: (M+H)⁺ = 401; purity = 90 % (254 nm); retention time = 1.67 min.

Step 4: To a solution of 1-(1-tosyl-1H-indol-5-yl)-3,4-dihydroisoquinoline **69** (700 mg, 1.75 mmol) in methanol (15 mL) was added NaBH₄ (133 mg, 3.50 mmol) at 0 °C. The mixture was stirred for 1 h at 0 °C. Water (50 mL) was added to quench the reaction. The mixture was extracted with dichloromethane/MeOH (10:1), washed with brine (50 mL) dried over Na₂SO₄ and concentrated *in vacuo.* The crude (720 mg) was used in the next step without further purification. LCMS: (M+H)⁺ = 403; purity = 97 % (254 nm); retention time = 1.78 min.

Step 5: To a solution of 1-(1-tosyl-1H-indol-5-yl)-1,2,3,4-tetrahydroisoquinoline **70** (753 mg, 1.87 mmol) in dichloromethane (15 mL) was added dipyridin-2-yl carbonate (526 mg, 2.44 mmol) and TEA (566 mg, 5.61 mmol). The mixture was stirred at room temperature for 3 hours. The mixture was cooled to 25°C and water was added (40 mL). The mixture was extracted with three 20 mL portions of dichloromethane. The combined organic layers were washed three times with brine, dried and concentrated *in vacuo.* The crude (980 mg) was used in the next step without further purification. LCMS: (M+Na)⁺ = 523; purity = 92 % (254 nm); retention time = 1.83 min.

Step 6: To a solution of pyridin-2-yl 1-(1-tosyl-1H-indol-5-yl)-3,4-dihydroisoquinoline-2(1H)-carboxylate **71** (800 mg, 1.53 mmol) in toluene (10 mL) was added (S)-2,2-dimethylquinuclidin-3-ol (194 mg, 1.20 mmol) and NaH (73 mg, 3.02 mmol). The mixture was stirred at 60 °C for 12 hours. The mixture was cooled to 25°C and water was added (60 mL). The mixture was extracted with three 60 mL portions of ethyl acetate. The combined organic layers were washed three times with brine, dried and concentrated *in vacuo* to give crude product (100 mg).

The diastereomers were separated by chiral SFC eluting with CO₂/EtOH containing 1% methanolic ammonia over an EnantioPak^{®} SC column (4.6*100mm 5µm) to give **Compound 1165** (30 mg, retention time = 2.24 min) and **Compound 1166**(40 mg, retention time = 2.81 min). Stereochemical assignment of (*S*) at quinuclidine is absolute based on starting materials, stereochemical assignment at 1 position of the tetrahydroisoquinoline is assigned based on chromatographic elution order as compared to diastereomers of related analogues of known configuration.

***Compound 1165:*** LCMS: (M+H)⁺ = 402; purity = 100% (214 nm); retention time = 1.49 min. ¹H NMR: (400 MHz, CDCl₃) δ 8.27 (s, 1H), 7.39-7.34 (m, 2H), 7.26-7.11 (m, 6H), 6.48 (t, *J=* 2.0 Hz, 2H), 4.89 (t, *J=* 4.0 Hz, 1H), 4.21-4.11(m, 1H), 3.38-3.33 (m, 2H), 3.02-2.84 (m, 7H), 2.23 (s, 1H), 1.84-1.66 (m, 3H), 1.53-1.49 (m, 1H).

***Compound 1166:*** LCMS: (M+H)⁺ = 402; purity = 100% (214 nm); retention time = 1.49 min. ¹H NMR: (400 MHz, CDCl₃) δ 8.44-8.26 (m, 1H), 7.39 (s, 2H), 7.24-7.14 (m, 6H), 6.57-6.21 (m, 2H), 5.11 (s, 1H), 4.65 (bs, 1H), 4.08-3.95 (m, 1H), 3.60-3.23 (m, 6H), 3.07-2.90 (m, 3H), 2.47 (s, 1H), 2.19-2.14 (m, 1H), 1.92-1.80 (m, 2H).

### Reference Example 10: Synthesis of Compounds 1165 and 1166

Step 1: To a solution of pyridin-2-yl 1-(4-cyclopropoxyphenyl)-3,4-dihydroisoquinoline-2(1H)-carboxylate (420 mg, 1.09 mmol) in toluene (10 mL) was added (S)-2,2-dimethylquinuclidin-3-ol (152 mg, 1.20 mmol) and NaH (36 mg, 1.50 mmol). The mixture was stirred at 60 °C for 12 hours. The mixture was cooled to 25 °C and water was added (20 mL). The mixture was extracted with three 20 mL portions of ethyl acetate. The combine organic layers were washed three times with brine, dried and concentrated *in vacuo* to give the crude product. The crude product was separated by chiral SFC eluting with CO₂/EtOH containing 0.5% methanolic ammonia over an EnantioPak^{®} AD column (20*250mm 10µm) to give compound 1167 (18 mg, retention time = 1.09 min) and compound 1168 (35 mg, retention time = 3.46 min ).

Stereochemical assignment of (*S*) at quinuclidine is absolute based on starting materials, stereochemical assignment at 1 position of the tetrahydroisoquinoline is assigned based on chromatographic elution order as compared to diastereomers of related analogues of known configuration.

***Compound 1167:*** LCMS: (M+H)⁺ = 446.9; purity = 100% (214 nm); retention time = 1.71 min. ¹H NMR: (400 MHz, CD₃OD) δ 7.24-6.87 (m, 8H), 6.35-6.18 (m, 1H), 4.70-4.64 (m, 1H), 4.04-4.02 (m, 1H), 3.77-3.75 (m, 1H), 3.50-3.42 (m, 1H), 3.30-3.29 (m, 1H), 2.98-2.86 (m, 4H), 2.10 (d, *J=* 2.8 Hz, 1H), 2.00-1.98 (m, 1H), 1.85-1.84 (m, 2H), 1.61-1.54 (m, 1H), 1.43 (s, 3H), 1.31-1.28 (m, 4H), 0.78-0.66 (m, 4H).

***Compound 1168:*** LCMS: (M+H)⁺ = 446.9; purity = 100% (214 nm); retention time = 1.71 min. ¹H NMR: (400 MHz, CD₃OD) δ 7.25-6.97 (m, 8H), 6.33-6.29 (m, 1H), 4.64 (d, *J* = 2.8 Hz, 1H), 4.02 (d, *J* = 8.4 Hz, 1H), 3.77-3.74 (m, 1H), 3.43-3.37 (m, 1H), 3.22-3.19 (m, 1H), 2.97-2.73 (m, 4H), 2.03 (d, *J* = 4.8 Hz, 1H), 1.79-1.76 (m, 3H), 1.48-1.47 (m, 1H), 1.38 (s, 3H), 1.31 (s, 1H), 1.20 (s, 3H), 0.78-0.66 (m, 4H).

### Reference Example 11: Synthesis of Compounds 1174 and 1175

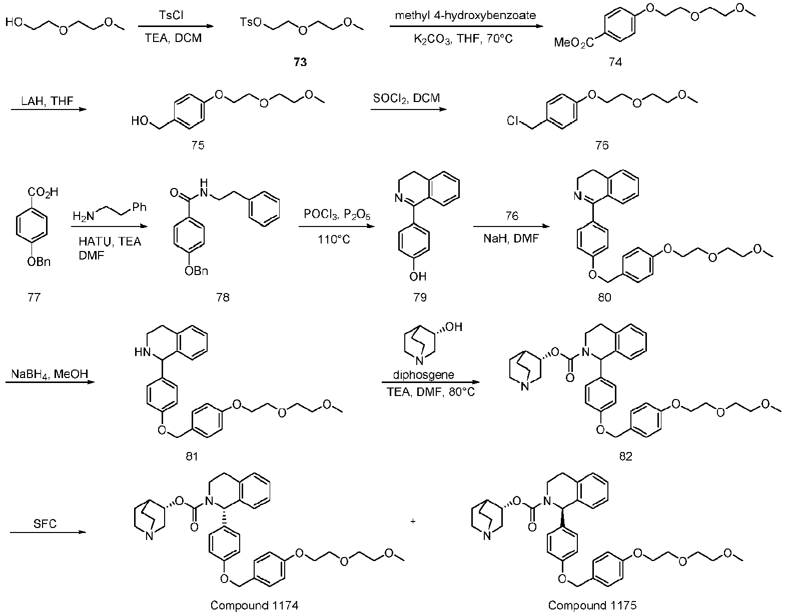

Step 1: TEA (4.6 mL, 33.3 mmol) was added to a solution of 2-(2-methoxyethoxy)ethanol (2 g, 16.6 mmol) in dichloromethane (50 mL) at 0 °C. The mixture was stirred at room temperature for 30 min. Then TsCI (3.809 g, 20 mmol) was added to the reaction mixture. The mixture was stirred at room temperature overnight. The mixture was diluted with water (50 mL) and extracted with three 100 mL portions of dichloromethane. The combined organic layers were washed with brine (50 mL), dried and concentrated *in vacuo* to give 4 g of 2-(2-methoxyethoxy)ethyl 4-methylbenzenesulfonate 73 as colorless oil.

Step 2: K₂CO₃ (3.023 g, 21.9 mmol) was added to a mixture of 2-(2-methoxyethoxy)ethyl 4-methylbenzenesulfonate **73** (2 g, 7.3 mmol) and methyl 4-hydroxybenzoate (1.331 g, 8.7 mmol) in THF (100 mL). The reaction mixture was stirred at 70 °C for 24 hours. The mixture was diluted with water (50 mL) and extracted with three 100 mL portions of ethyl acetate. The combine organic layers were washed with brine (50 mL), dried and concentrated *in vacuo.* The crude was purified by chromatography (petrol ether/ ethyl acetate = 2:1) to give 2.2 g of methyl 4-(2-(2-methoxyethoxy)ethoxy)benzoate **74** as yellow oil. LCMS: (M+NH₄)⁺ = 272 (UV 214 nm); Retention time = 1.40 min.

Step 3: LAH in THF (10 mL, 10.4 mmol) was added to the solution of methyl 4-(2-(2-methoxyethoxy)ethoxy)benzoate **74** (2.2 g, 8.6 mmol) in THF (10 mL) at 0 °C. The reaction mixture was stirred at ambient temperature for 2 hours. The reaction was quenched with NH₄Cl (20 mL). The aqueous layer was extracted with three 100 mL portions of ethyl acetate. The combined organic layers were washed with brine (50 mL), dried and concentrated *in vacuo* to give 1.45 g of (4-(2-(2-methoxyethoxy)ethoxy)phenyl)methanol **75** as yellow oil. LCMS: (M+Na)⁺ = 249 (UV 214 nm); Retention time = 1.22 min.

Step 4: SOCl₂ (2.3 mL, 32 mmol) was added to the solution of (4-(2-(2-methoxyethoxy)ethoxy)phenyl)methanol **75** (1.45 g, 6.4 mmol) in dichloromethane (20 mL) at 0 °C. The reaction mixture was stirred at ambient temperature for 2 hours. The mixture was concentrated *in vacuo* to give 900 mg of 1-(chloromethyl)-4-(2-(2-methoxyethoxy)ethoxy)benzene **76** as colorless oil. LCMS: (M+H₃O)⁺ = 263 (UV 214 nm); Retention time = 1.56 min.

Step 5: TEA (2.8 mL, 20 mmol) was added to the mixture of 4-(benzyloxy)benzoic acid (2.28 g, 10 mmol), 2-phenylethanamine (1.21 g, 10 mmol) and HATU (4.56 g, 12 mmol) in DMF (30 mL) at 0 °C. The mixture was stirred at room temperature for 2 hours. The mixture was diluted with water (120 mL) and extracted with four 100 mL portions of ethyl acetate. The combined organic layers were washed with brine (50 mL), dried and concentrated *in vacuo* to give 3 g of 4-(benzyloxy)-N-phenethylbenzamide **78** as a white solid.

Step 6: POCl₃ (20 mL) was added to 4-(benzyloxy)-N-phenethylbenzamide **78** (3 g, 9 mmol) and P₂O₅ (1.542 g, 10.9 mmol) at 0 °C. The reaction mixture was stirred at 115°C for 3 hours. The reaction mixture was poured into cold water. The mixture was alkalized with NaOH (10% aq.) to pH 11. The aqueous layer was extracted with three 100 mL portions of dichloromethane. The combined organic layers were washed with brine (50 mL), dried and concentrated *in vacuo.* The crude was purified by chromatography (petrol ether:ethyl acetate = 1:1) to give 1.2 g of 4-(3,4-dihydroisoquinolin-1-yl)phenol **79** as a yellow solid. LCMS: (M+H)⁺ = 224 (UV 214 nm); Retention time =1.15 min.

Step 7: NaH (97 mg, 4 mmol) was added to the solution of 4-(3,4-dihydroisoquinolin-1-yl)phenol **79** (600 mg, 2.7 mmol) in DMF (10 mL) at 0 °C. The reaction mixture was stirred at 0°C for 30 min. Then 1-(chloromethyl)-4-(2-(2-methoxyethoxy)ethoxy)benzene **76** (789 mg, 3.2 mmol) was added to the mixture. The reaction mixture was stirred at ambient temperature for 2 hours. The reaction was quenched with aqueous NH₄Cl (5 mL). The mixture was diluted with 40 mL of water and extracted four 80 mL portions of ethyl acetate. The combined organic layers were dried with Na₂SO₄ and concentrated to give 900 mg of 1-(4-(4-(2-(2-methoxyethoxy)ethoxy)benzyloxy)phenyl)-3,4-dihydroisoquinoline **80** as yellow oil. LCMS: (M+H)⁺ = 432 (214 nm); retention time = 1.45 min.

Step 8 NaBH₄ (158 mg, 4.2 mmol) was added to the solution of 1-(4-(4-(2-(2-methoxyethoxy)ethoxy)benzyloxy)phenyl)-3,4-dihydroisoquinoline **80** (900 mg, 2.1 mmol) in MeOH (10 mL) at 0 °C. The reaction mixture was stirred at ambient temperature for 2 hours. The mixture was diluted with 20 mL of water and extracted by three 50 mL portions of ethyl acetate. The combined organic layers were dried with Na₂SO₄ and concentrated to give 600 mg of 1-(4-(4-(2-(2-methoxyethoxy)ethoxy)benzyloxy)phenyl)-1,2,3,4-tetrahydroisoquinoline **81** as yellow oil. LCMS: (M+H)⁺ = 434 (214 nm); retention time = 1.53 min.

Step 9: TEA (0.64 mL, 4.6 mmol) was added to the solution of 1-(4-(4-(2-(2-methoxyethoxy)ethoxy)benzyloxy)phenyl)-1,2,3,4-tetrahydroisoquinoline **81** (400 mg, 0.9 mmol) in DMF (4 mL). After 10 min, (S)-quinuclidin-3-yl carbonochloridate (262 mg, 1.4 mmol) in DMF (2 mL) formed in situ from (S)-quinuclidine-3-ol and diphosgene was added to the mixture. The resulting mixture was stirred at 80 °C for 4 hours. The mixture was diluted with water (30 mL) and extracted with three 50 mL portions of dichloromethane:methanol (20:1). The combined organic layers were washed with brine (20 mL), dried and concentrated *in vacuo* to give crude product. The crude product was purified by HPLC method [(Mobile Phase: A: H₂O (10 mM NH₄HCO₃) B:MeCN Gradient: 55%-85% B in 12.0 min, Flow Rate : 30.0 mL/min Column: XBridge C18 OBD 21.2*250mm, 10 µm, Oven Temperature: 40 °C UV 214, MASS:100-1000] to give the desired product (200mg).

The diastereomers were separated by chiral SFC eluting with CO₂/MeOH containing 0.2 methanol ammonia over an EnantioPak^{®} AD column (20*250mm 10µm) to give compound 1174 (83.8 mg, retention time = 4.54 min) and compound 1175(43.6 mg, retention time = 2.74 min). Stereochemical assignment of (S) at quinuclidine is absolute based on starting materials, stereochemical assignment at 1 position of the tetrahydroisoquinoline is assigned based on chromatographic elution order as compared to diastereomers of related analogues of known configuration.

***Compound 1174:*** LCMS: (M+H)⁺ = 587; purity = 100% (214 nm); retention time = 1.535 min. Method A1 ¹H NMR (400 MHz, CD₃OD) δ 7.32 (d, *J=* 8.4 Hz, 2H), 7.24-7.02 (m, 6H), 6.96-6.87 (m, 4H), 6.39-6.12 (m, 1H), 4.96 (s, 2H), 4.85-4.77 (m, 1H), 4.11 (t, *J=* 4.8 Hz, 2H), 4.01 (dt, *J=* 13.2, 4.8 Hz, 1H), 3.83 (t, *J=* 4.8 Hz, 2H), 3.71-3.66 (m, 2H), 3.59-3.50 (m, 3H), 3.37 (s, 3H), 3.31-3.22 (m, 1H), 2.99-3.72 (m, 7H), 2.08 (s, 1H), 1.95-1.91 (m, 1H), 1.82-1.72 (m, 1H), 1.69-1.59 (m, 1H), 1.53-1.49 (m, 1H).

***Compound 1175:*** LCMS: (M+H)⁺ = 587; purity = 95% (214 nm); retention time = 1.536 min. Method A1 ¹H NMR (400 MHz, CD₃OD) δ 7.31 (d, *J* = 8.8 Hz, 2H), 7.24-7.01 (m, 6H), 6.90-6.84 (m, 4H), 6.36-6.14 (m, 1H), 4.93 (s, 2H), 4.82-4.76 (m, 1H), 4.10 (t, *J=* 4.8 Hz, 2H), 3.96 (dt, *J* = 12.8, 4.2 Hz, 1H), 3.81 (t, *J* = 4.8 Hz, 2H), 3.70-3.66 (m, 2H), 3.58-3.40 (m, 3H), 3.36 (s, 3H), 3.22 (dd, *J* = 14.4, 8.4 Hz, 1H), 2.99-3.58 (m, 7H), 2.03 (s, 1H), 1.87-1.83(m, 1H), 1.78-1.69 (m, 1H), 1.69-1.54 (m, 1H), 1.52-1.43 (m, 1H).

4.6 × 100 mm 5 µmCellular Assays: To measure the potency of compounds, a progranulin induction cellular assay in mouse primary microglia (pMG), primary cortical neurons, and BV-2 cell lines is used. BV-2 cells are split the day before plating into a 96well plate format at approximately 80%. Cells should be plated the day before and allowed for 1 hour attachment period and for 16 hour incubation. Levels of progranulin secreted into the cell culture medium or retained in the cell lysate can be quantified using an ELISA-based readout and measurement of secreted mouse PGRN in the medium was assessed by the methodology published by Ghidoni et al. 2012. Standard ELISA kits to measure PGRN are available from vendors such as Adipogen, R&D, and Biovendor.

### Example 11: Biological Assays

In vivo Assays: A mouse ELISA protocol to detect progranulin in brain, plasma, or cerebrospinal fluid (CSF) can be used, with GRN +/- mice or GRN +/+ mice (available from TACONIC). The mouse is administered a compound and the amount of progranulin in the brain is assessed after a specific amount of time. Mice treated with a test compound or compounds are compared to control mice which are not treated with the compound. Treatment can be done with a single or multiple dosing of compounds. Control samples are assigned a relative value of 100%.

Other *in vivo* assays can be performed using a GRN +/- and GRN +/+ rats, non-human primates (e.g., monkey, dog) using a similar protocol.

Treatment with the test compound increases the progranulin secretion relative to the control is at least about 110%, at least about 130%, at least about 150%, at least about 180%, at least about 200%, at least about 250%, or at least about 300%.

Some specific biological data is shown in the below Table, where PRGN v1 and PRGN v2 are shown in µM and represent EC₅₀ values as determined by 8 point binding curves in replicate. Compound potency was determined by measuring progranulin secretion from BV-2 (mouse microglia) cells. BV-2 cells were grown in RPMI with 2 mM L-glutamine supplemented with 10% fetal bovine serum. On the day of the assay, cells were plated in 96-well plates at a density of 30,000 cells/well in RPMI (Roswell Park Memorial Institute media) /L-glutamine supplemented with 2% Fetal clone serum II (plating media) and left to adhere for 1 hour. Following attachment to the plate, media was replaced with treatment (8-point curves in half-log dilutions starting at 10 µM) in plating media and incubated for 16 hours. Secreted progranulin was measured from a 1:10 dilution of the conditioned media using the R&D Systems mouse progranulin ELISA kit (cat# DY2557) and following the manufacturer's instructions. Assay results are reported in the below Table as PGRN v1 and PGRN v2, and were obtained using the same protocol in different assays.

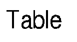

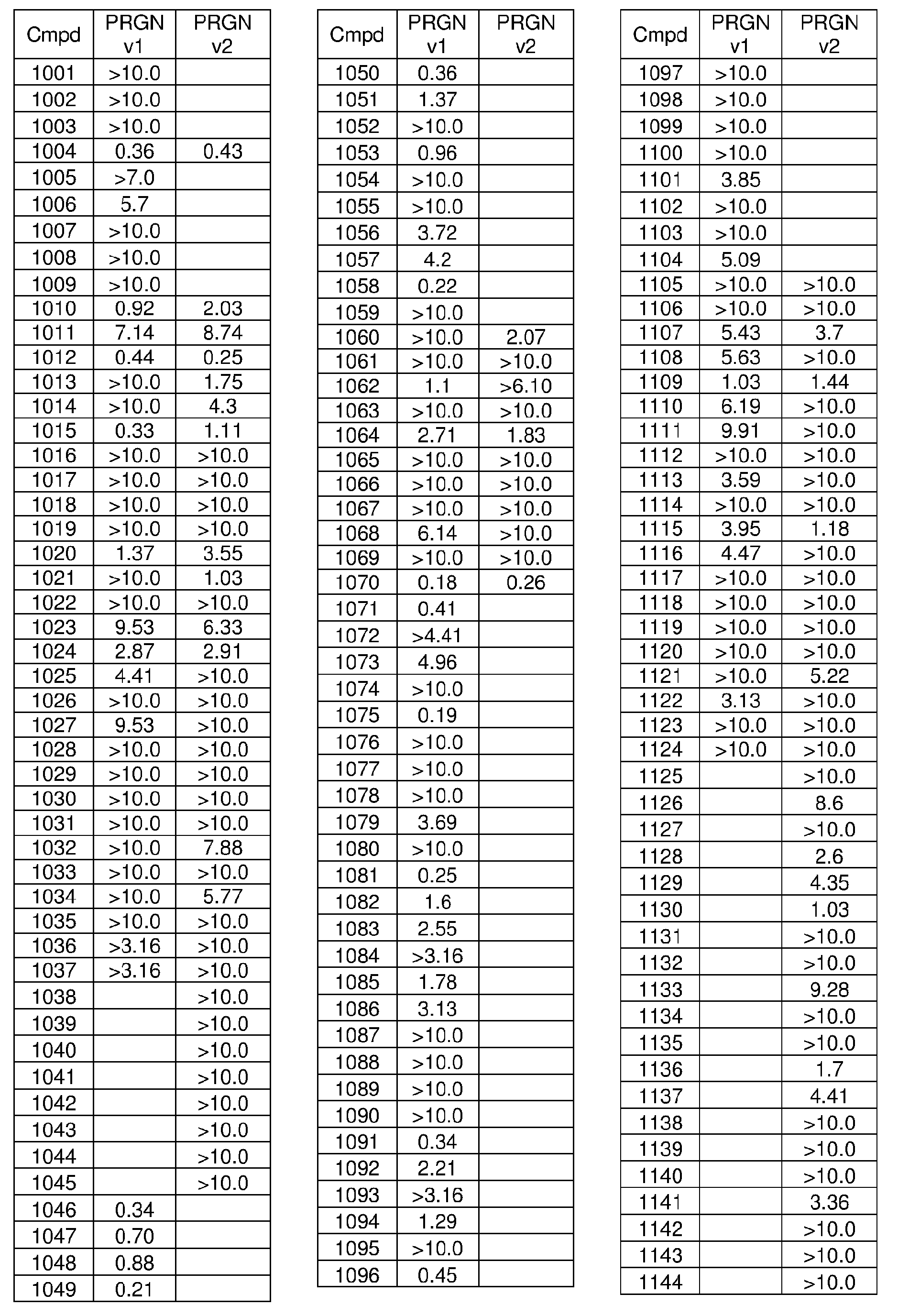

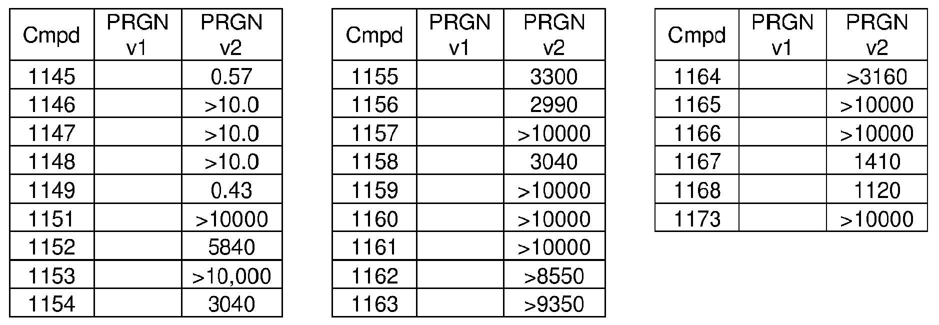

In view of the many possible embodiments to which the principles of the disclosure may be applied, it should be recognized that the illustrated embodiments are only examples and should not be taken as limiting the scope of the invention.

## Claims

1. A compound, or pharmaceutically acceptable salt thereof, having a structure of Formula (IA): 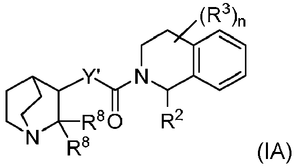 wherein
Y' is CH₂ or NH;
R² is C₆₋₁₀aryl, 5-10 membered heteroaryl comprising 1-4 ring heteroatoms selected from N, O, and S, or 5-12 membered monocyclic or bicyclic carbocycle or heterocycle, wherein the heterocycle comprises 1-4 ring heteroatoms selected from N, O, and S, and R² is optionally substituted with 1-3 R³ groups;
each R³, when present, is independently selected from C₁₋₆alkyl, C₀₋₃ alkylene-halo, O-C₁₋₃ alkylene-halo, C₀₋₃ alkylene-CN, C₀₋₃ alkylene-NR⁵₂, C₀₋₆ alkylene- OR⁵, C₀₋₆ alkylene-C(O)OR⁷, C(O)N(R⁷)₂, SO₂R⁶, O-C₀₋₆alkylene-Ar, oxo, and C₀₋₆alkylene-Ar;
Ar is 3-8-membered carbocycle or heterocycle, wherein the heterocycle comprises 1-4 ring heteroatoms selected from N, O, and S; C₆₋₁₀aryl; or 5-10 membered heteroaryl comprising 1-4 ring heteroatoms selected from N, O, and S and Ar is optionally substituted with 1-3 groups independently selected from halo, C₁₋₆alkyl, C₁₋₆haloalkyl, C₁₋₆alkoxy, C₁₋₆haloalkoxy, CN, and SO₂C₁₋₃alkyl;
each R⁵ is independently H, C₁₋₆alkyl, or C(O)C₁₋₆alkyl;
each R⁶ is independently C₁₋₆alkyl, C₁₋₆haloalkyl, or Ar;
each R⁷ is independently H or C₁₋₆alkyl;
each R⁸ is independently H or Me, or both R⁸ taken together with the carbon to which they are attached form a cyclopropyl ring; and
n is 0-2;or wherein the compound has a structure selected from: 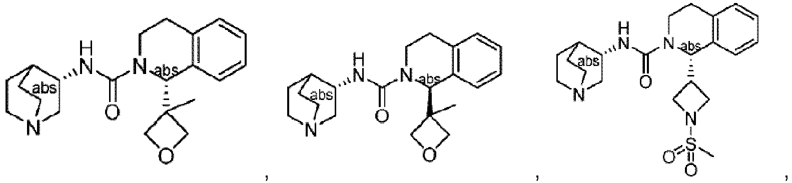 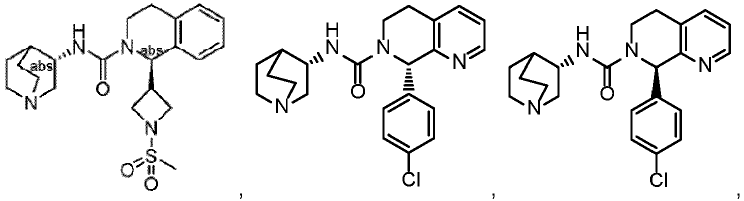 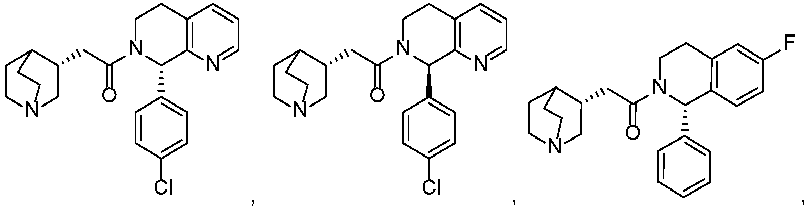 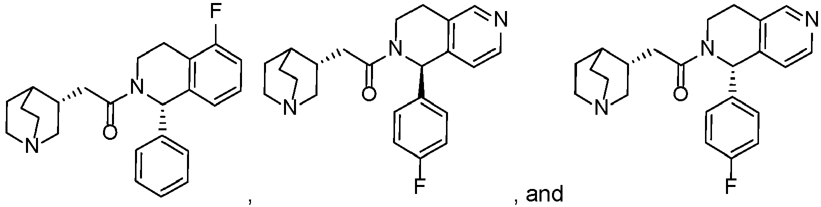 or a pharmaceutically acceptable salt thereof.

2. The compound or salt of claim 1, wherein R² is substituted with 1-3 R³, and each R³ is independently selected from F, CI, OH, OMe, OiPr, OBn, O-cyclopropyl, CF₃, OCF₃, CN, SO₂Me, SO₂-cyclopropyl, SO₂iPr, oxo, imidazolyl, triazolyl, pyrrolidinyl, pyrrolidinonyl, thiadiazolyl, methyl-thiadiazolyl, trifluoromethyl- thiadiazolyl, oxadiazolyl, methyl-oxadiazolyl, trifluoromethyl- oxadiazolyl, and phenyl.

3. The compound or salt of claim 1 or 2, wherein R² comprises phenyl and is optionally substituted with 1-2 groups selected from fluoro and chloro.

4. The compound or salt of any one of claims 1 to 3, wherein R² is selected from the group consisting of 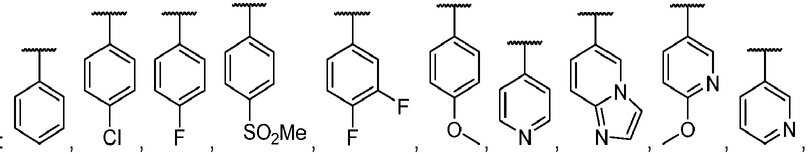 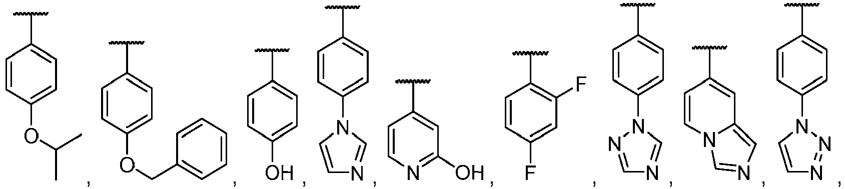 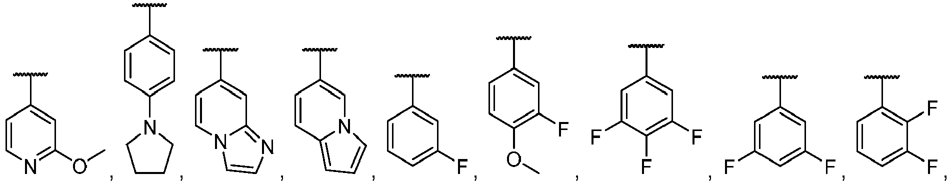 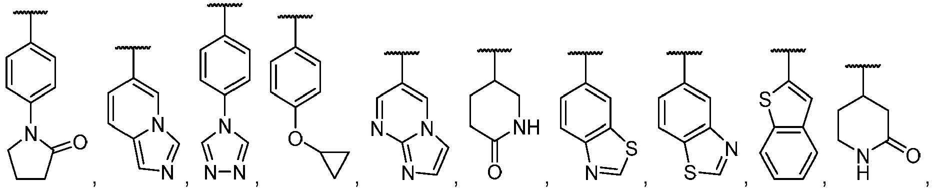 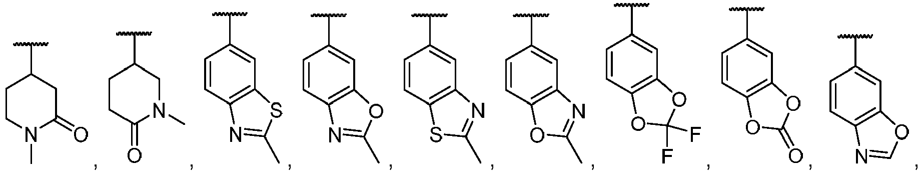 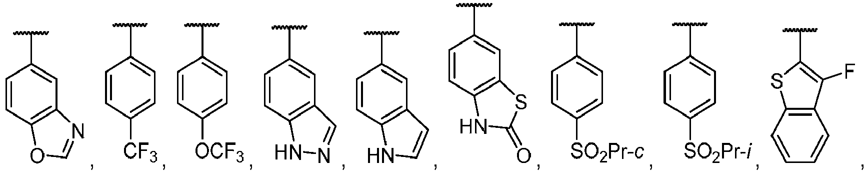 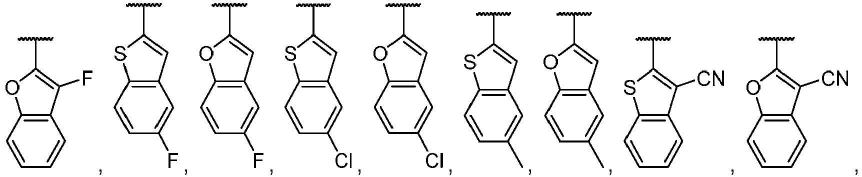 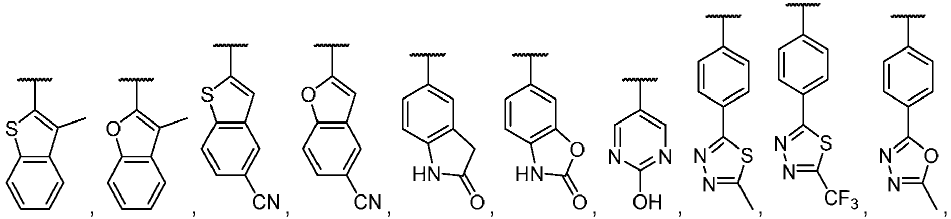 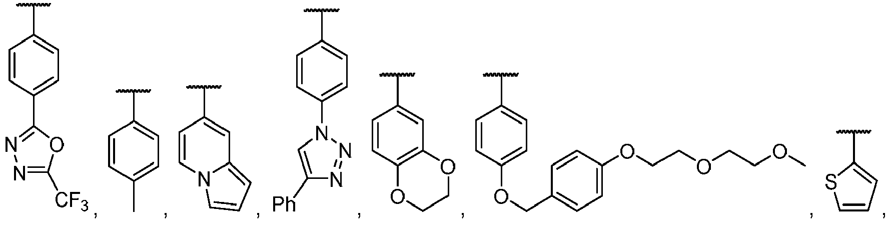 or 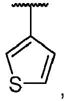 preferably wherein R² is:

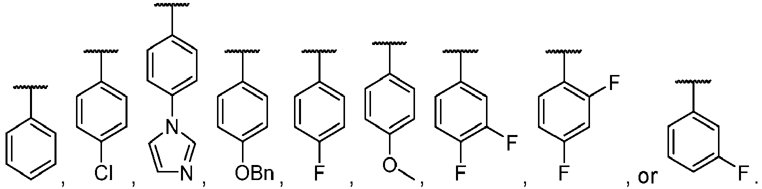

5. The compound or salt of any one of claims 1 to 4, either wherein one R⁸ is H and the other Me; or wherein each R⁸ is Me.

6. The compound or salt of any one of claims 1 to 4, wherein both R⁸ together with the carbon to which they are attached form a cyclopropyl ring.

7. The compound or salt of any one of claims 1 to 6, wherein n is 0.

8. The compound or salt of any one of claims 1 to 6, wherein n is 1 and R³ is F or Cl.

9. The compound or salt of claim 1, having a structure as shown in Table A, preferably as shown in Table B:

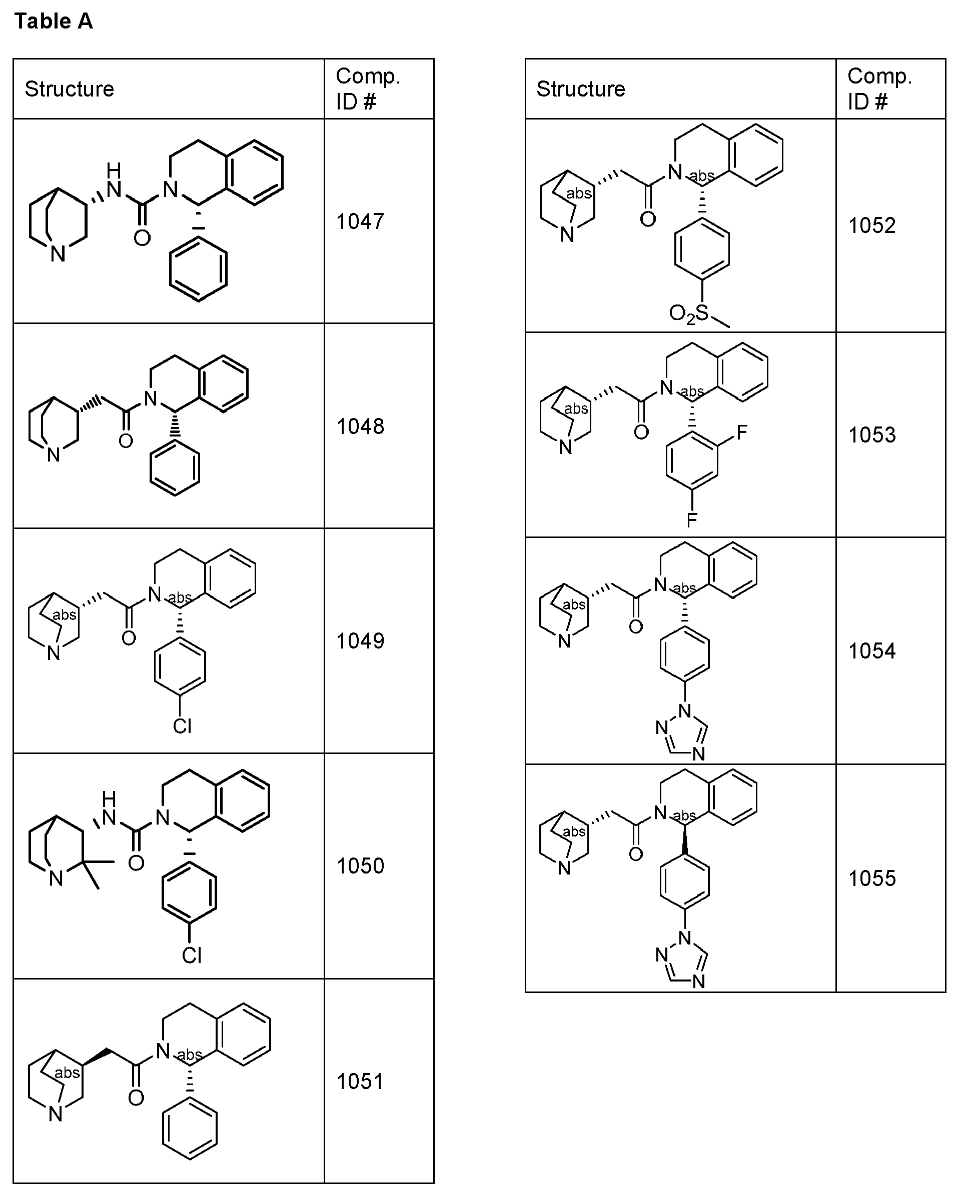

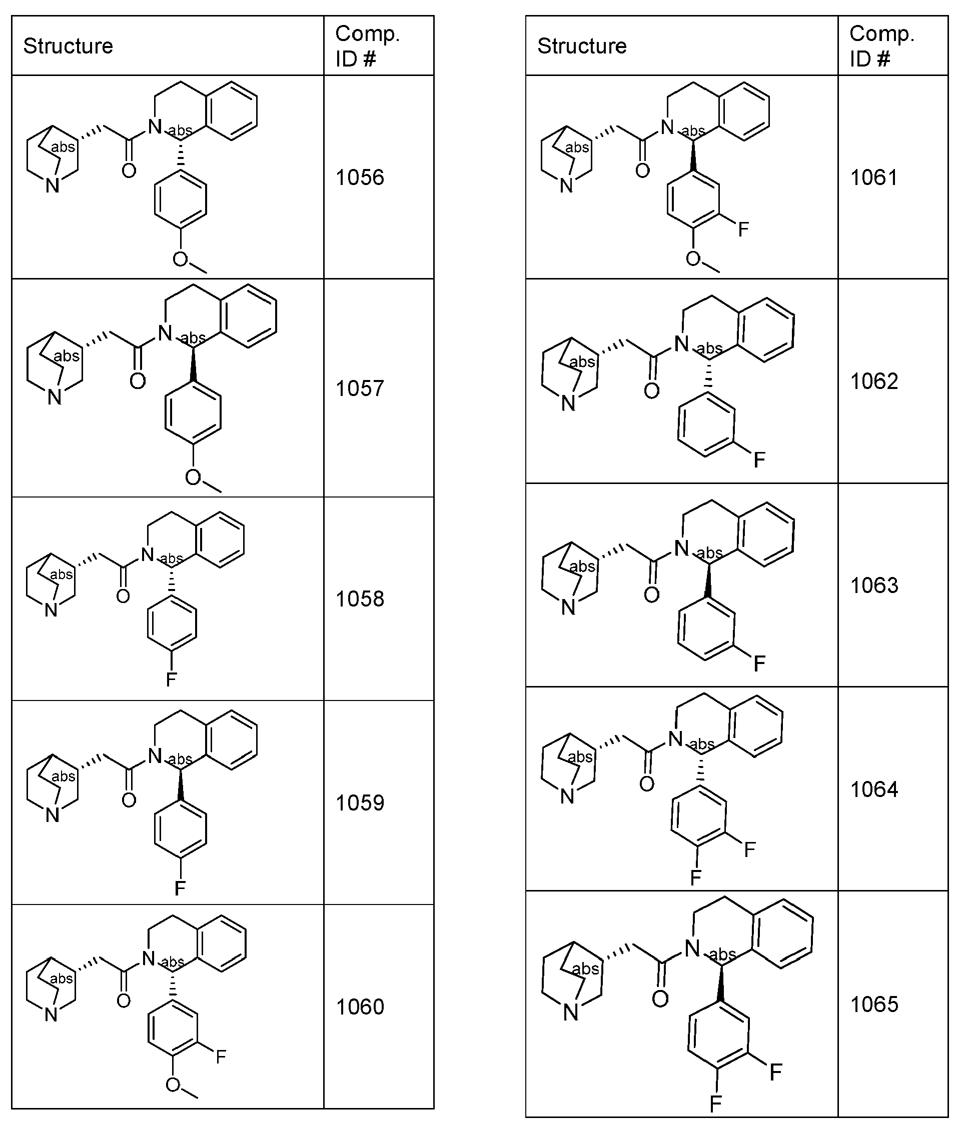

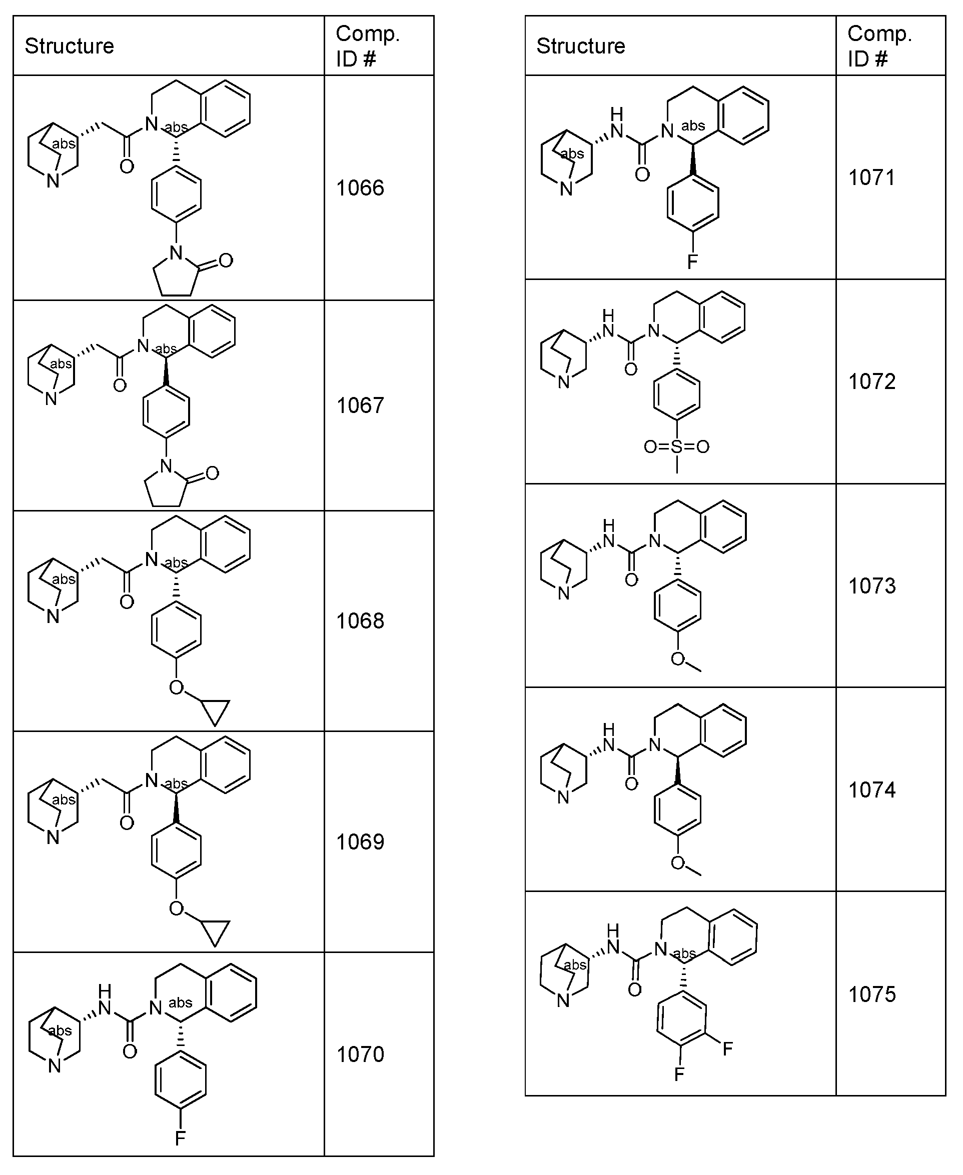

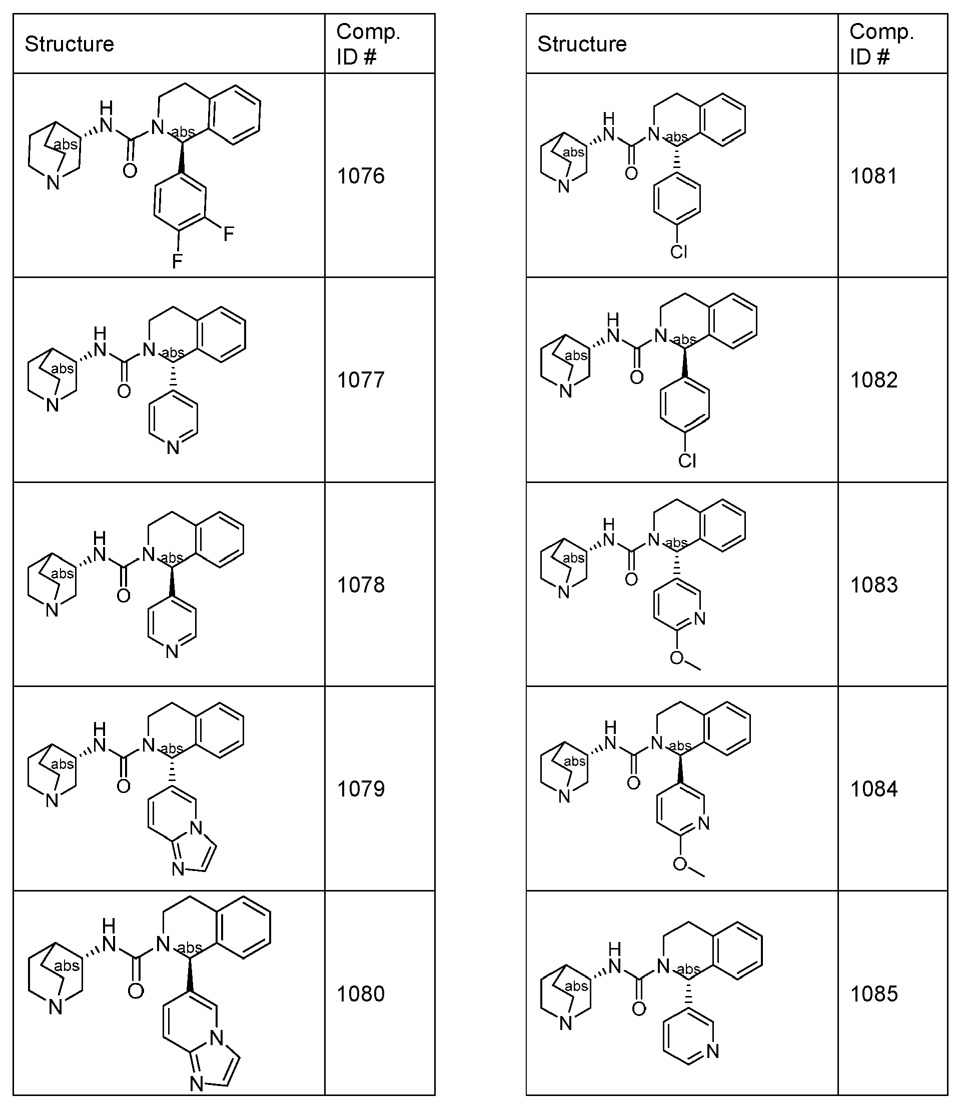

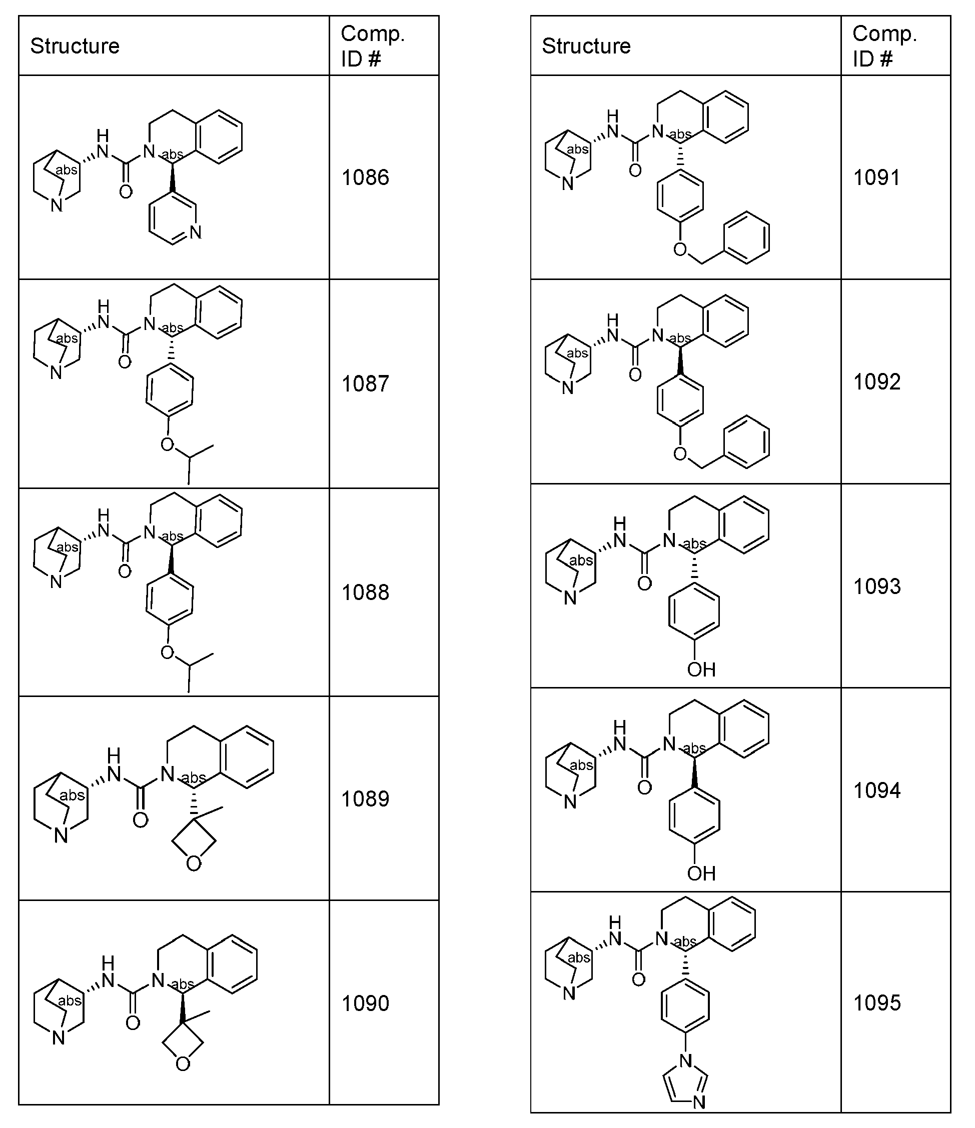

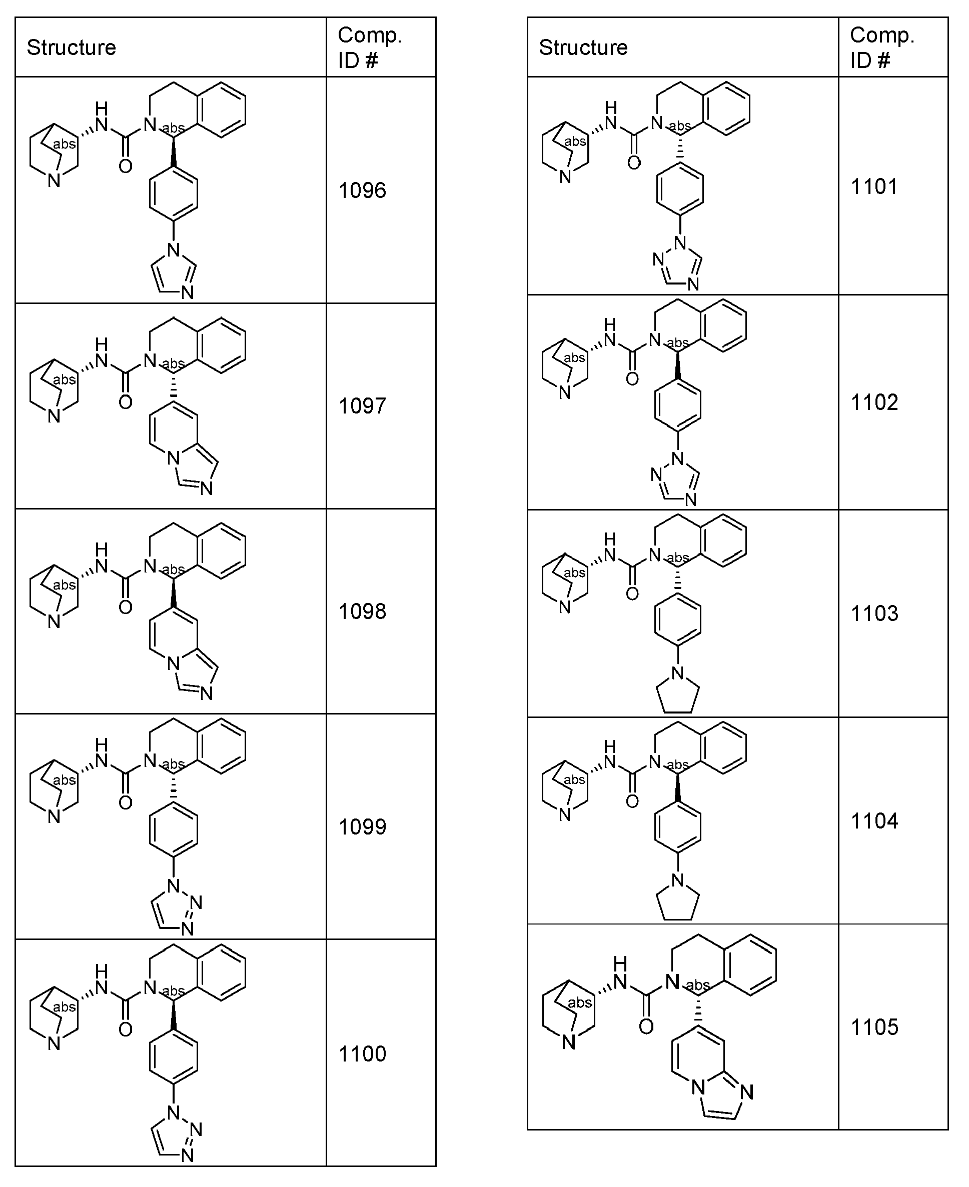

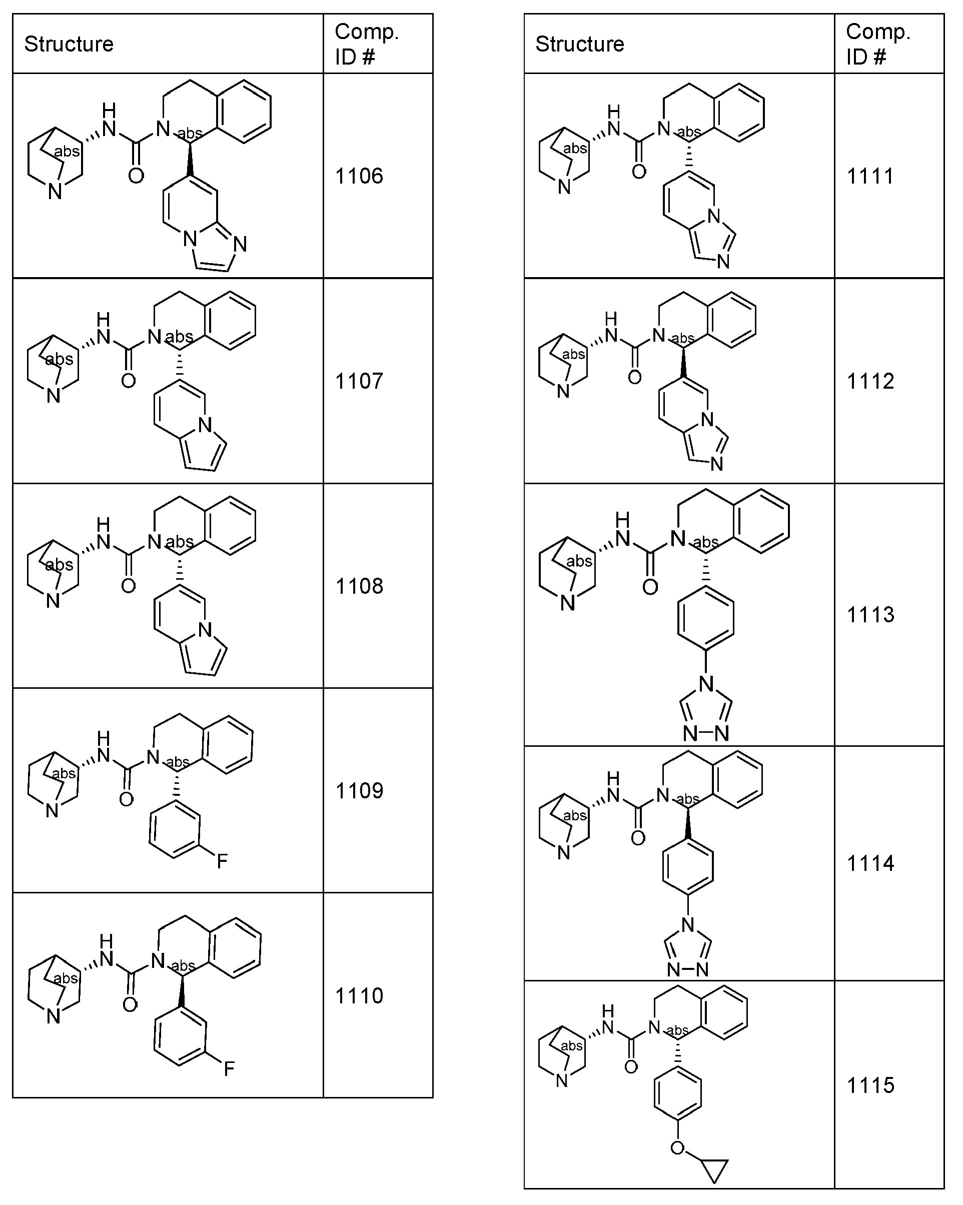

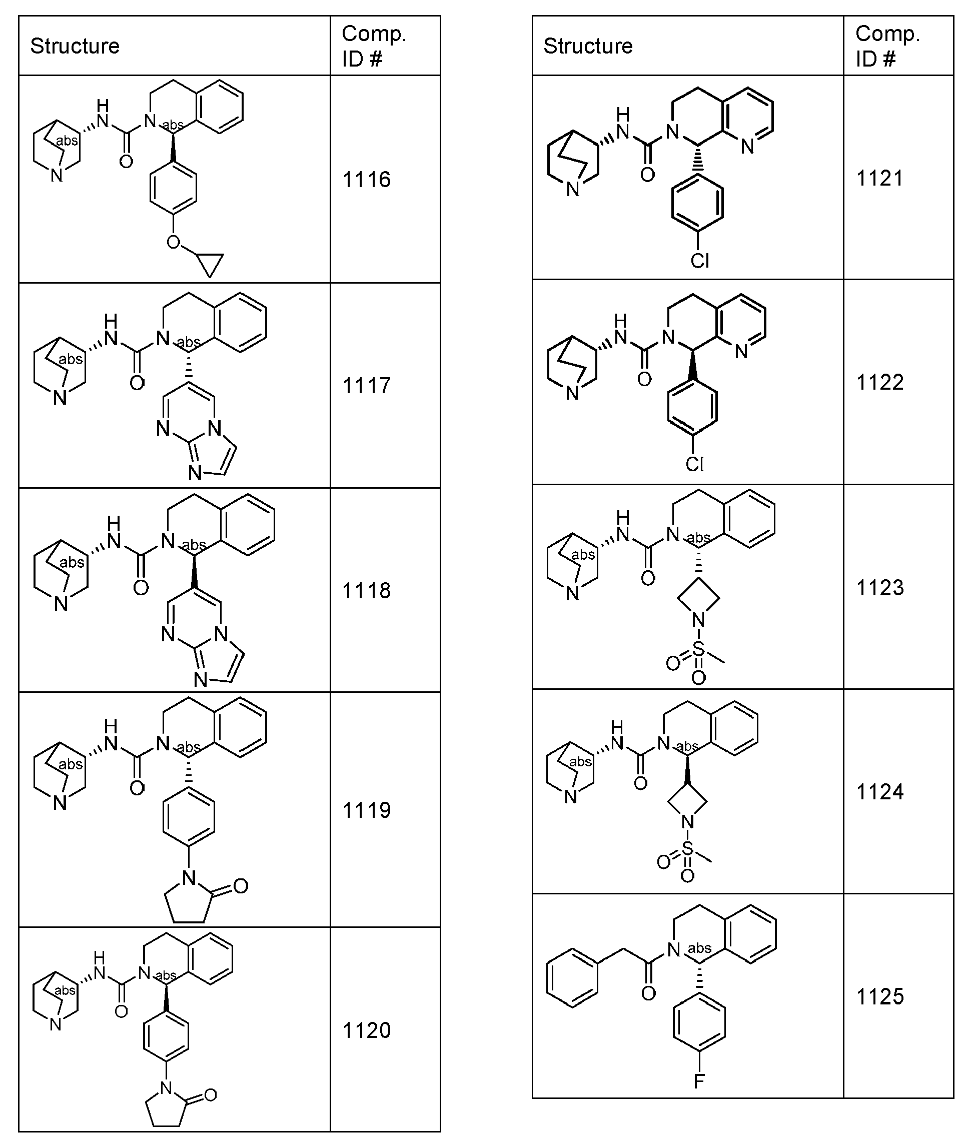

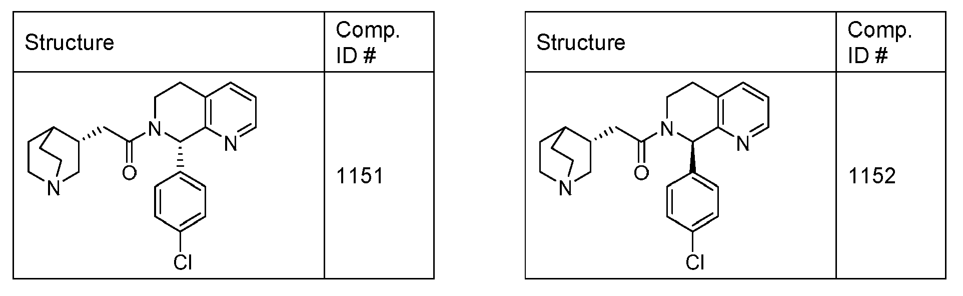

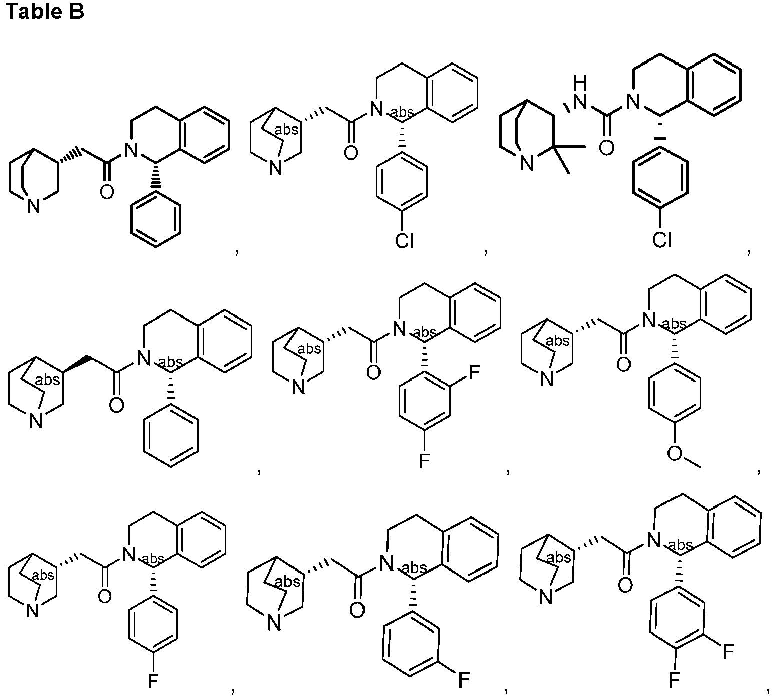

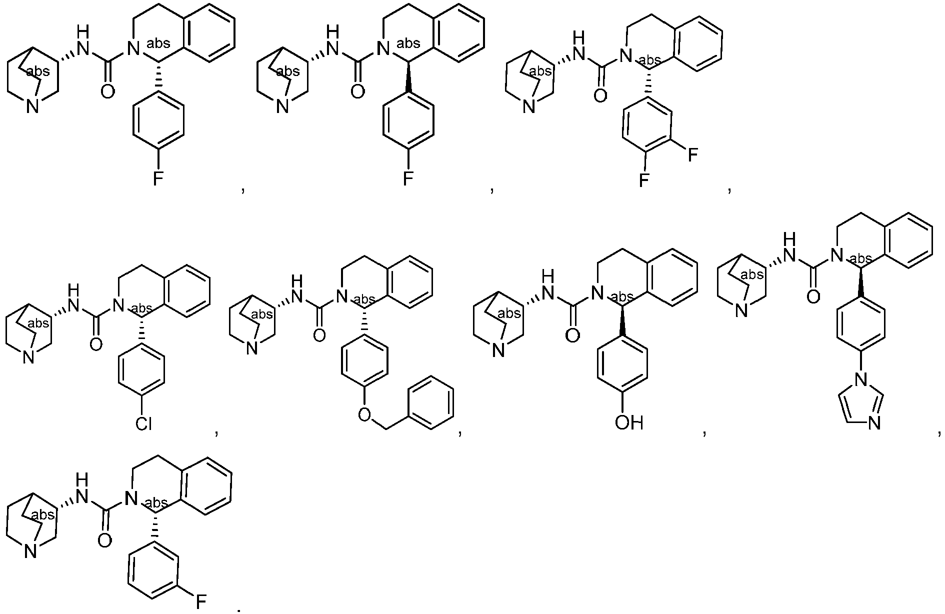

10. The compound or salt of claim 1, having a structure selected from the group consisting of 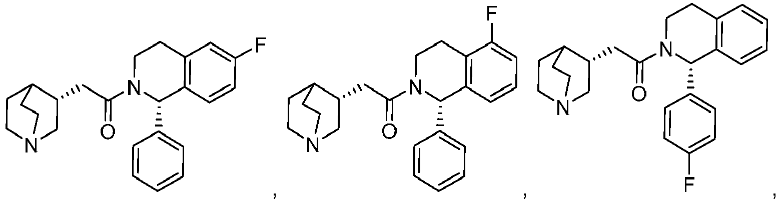 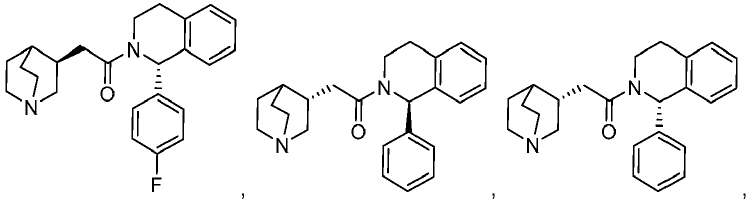 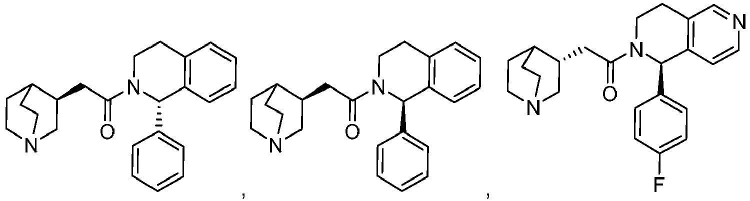 , and

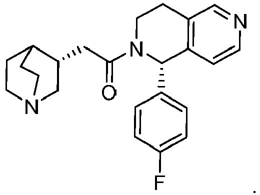

11. The compound or salt of any one of claims 1 to 10 in the form of a salt.

12. A pharmaceutical composition comprising the compound or salt of any one of claims 1 to 11 and a pharmaceutically acceptable excipient.

13. The compound or salt of any one of claims 1 to 11 for use in the treatment of a progranulin-associated disorder.

14. The compound or salt for use of claim 13, wherein the progranulin-associated disorder is Alzheimer's disease (AD), Parkinson's disease (PD), Amyotrophic lateral sclerosis (ALS), Frontotemporal dementia (FTD), Frontotemporal dementia -Granulin subtype (FTD-GRN), Lewy body dementia (LBD), Prion disease, Motor neuron diseases (MND), Huntington's disease (HD), Spinocerebellar ataxia (SCA), Spinal muscular atrophy (SMA), a lysosomal storage disease, a disease associated with inclusions and/or misfunction of C9orf72, TDP-43, FUS, UBQLN2, VCP, CHMP28, and/or MAPT, an acute neurological disorder, glioblastoma, or neuroblastoma.

15. The compound or salt for use of claim 14, wherein
(i) the lysosomal storage disease is Paget disease, Gaucher's disease, Nieman's Pick disease, Tay-Sachs Disease, Fabry Disease, Pompes disease, or Naso-Hakula disease;
(ii) the acute neurological disorder is stroke, cerebral hemorrhage, traumatic brain injury or head trauma;
(iii) the progranulin-associated disorder is Frontotemporal dementia (FTD); or
(iv) the progranulin-associated disorder is Frontotemporal dementia -Granulin subtype (FTD-GRN).

## Patentansprüche

1. Verbindung oder pharmazeutisch annehmbares Salz davon mit einer Struktur der Formel (IA): 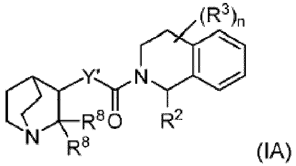 worin:
Y' CH₂ oder NH ist;
R² C₆₋₁₀-Aryl, 5- bis 10-gliedriges Heteroaryl mit 1 bis 4 Ring-Heteroatomen, die aus N, O und S ausgewählt sind, oder ein 5- bis 12-gliedriger monozyklischer oder bizyklischer Carbocyclus oder Heterocyclus ist, wobei der Heterocyclus 1 bis 4 Ring-Heteroatome umfasst, die aus N, O und S ausgewählt sind, und R² gegebenenfalls mit 1 bis 3 R³-Gruppen substituiert ist;
die R³, sofern vorhanden, jeweils unabhängig voneinander aus C₁₋₆-Alkyl, C₀₋₃-Alkylen-halogen, O-C₁₋₃-Alkylenhalogen, C₀₋₃-Alkylen-CN, C₀₋₃-Alkylen-NR⁵₂, C₀₋₆-Alkylen-OR⁵, C₀₋₆-Alkylen-C(O)OR⁷, C(O)N(R⁷)₂, SO₂R⁶, O- C₀₋₆-Alkylen-Ar, Oxo und C₀₋₆-Alkylen-Ar ausgewählt sind;
Ar ein 3- bis 8-gliedriger Carbocyclus oder Heterocyclus, wobei der Heterocyclus 1 bis 4 Ringheteroatome umfasst, die aus N, O und S ausgewählt sind, oder 5- bis 10-gliedriges Heteroaryl ist, das 1 bis 4 Ringheteroatome umfasst, die aus N, O und S ausgewählt sind, und Ar gegebenenfalls mit 1 bis 3 Gruppen substituiert ist, die jeweils unabhängig voneinander aus Halogen, C₁₋₆-Alkyl, C₁₋₆-Halogenalkyl, C₁₋₆-Alkoxy, C₁₋₆-Halogenalkoxy, CN und SO₂C₁₋₃-Alkyl ausgewählt sind;
die R⁵ jeweils unabhängig voneinander H, C₁₋₆-Alkyl oder C(O)C₁₋₆-Alkyl sind;
die R⁶ jeweils unabhängig voneinander C₁₋₆-Alkyl, C₁₋₆-Halogenalkyl oder Ar sind;
die R⁷ jeweils unabhängig voneinander H oder C₁₋₆-Alkyl sind;
die R⁸ jeweils unabhängig voneinander H oder Me sind oder die beiden R⁸ zusammen mit dem Kohlenstoff, an den sie gebunden sind, einen Cyclopropylring bilden; und
n = 0 bis 2 ist;
oder wobei die Verbindung eine aus den folgenden ausgewählte Struktur aufweist: 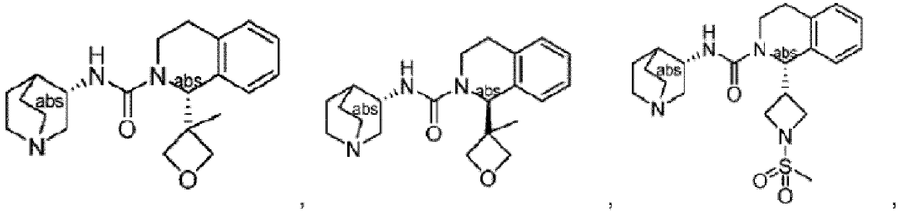 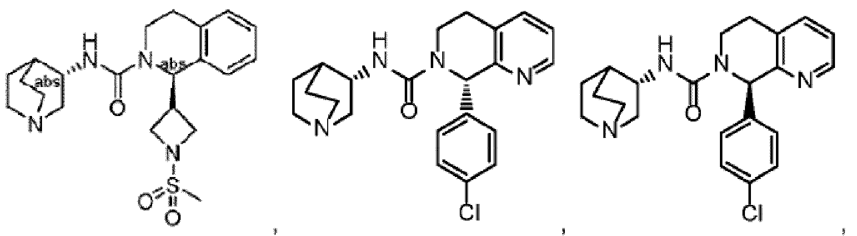 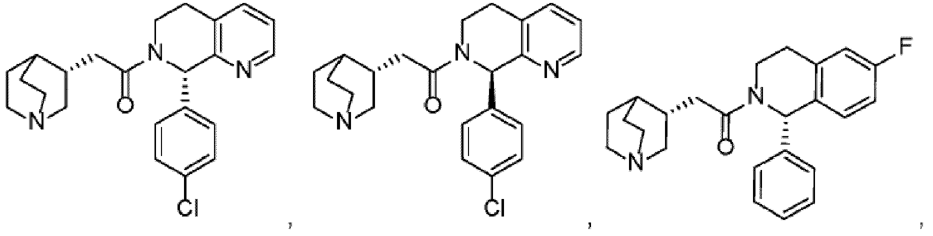 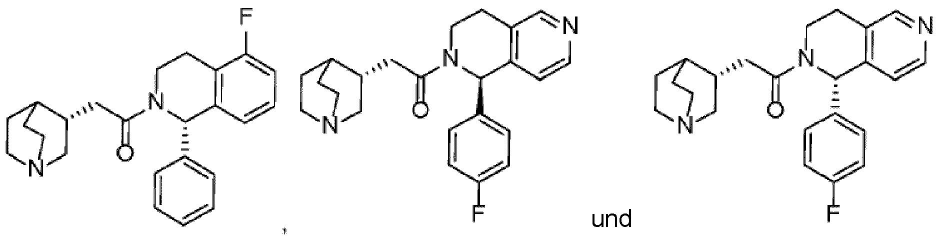
oder ein pharmazeutisch annehmbares Salz davon.

2. Verbindung oder Salz nach Anspruch 1, worin R² mit 1 bis 3 R³ substituiert ist und die R³ jeweils unabhängig aus F, CI, Oh, OMe, OiPr, OBn, O-Cycloproypl, CF₃, OCF₃, CN, SO₂Me, SO₂-Cyclopropyl, SO₂iPr, Oxo, Imidazolyl, Triazolyl, Pyrrolidinyl, Pyrrolidinonyl, Thiadiazolyl, Methylthiadiazolyl, Trifluormethylthiadiazolyl, Oxadiazolyl, Methyloxadiazolyl, Trifluormethyloxadiazolyl und Phenyl ausgewählt sind.

3. Verbindung oder Salz nach Anspruch 1 oder 2, worin R² Phenyl umfasst und gegebenenfalls mit 1 bis 2 Gruppen substituiert ist, die aus Fluor und Chlor ausgewählt sind.

4. Verbindung oder Salz nach einem der Ansprüche 1 bis 3, worin R² aus der aus Folgendem bestehenden Gruppe ausgewählt ist: 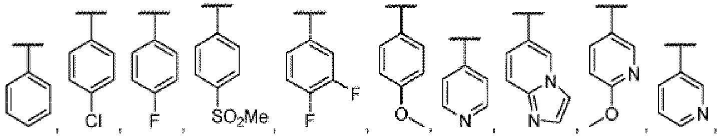 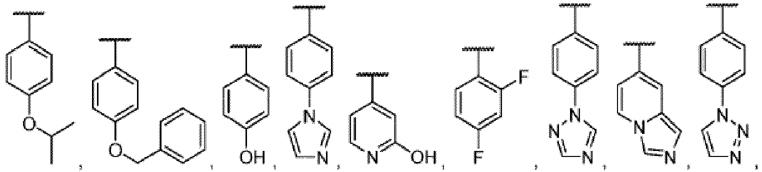 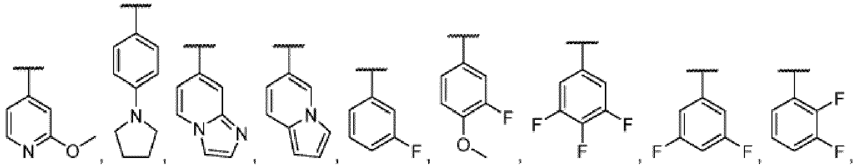 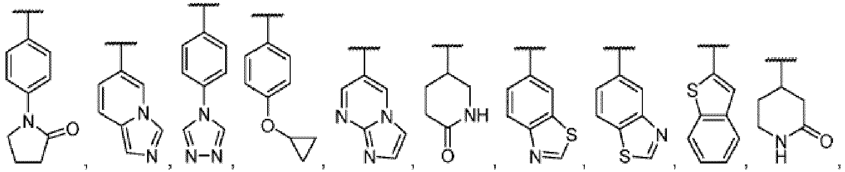 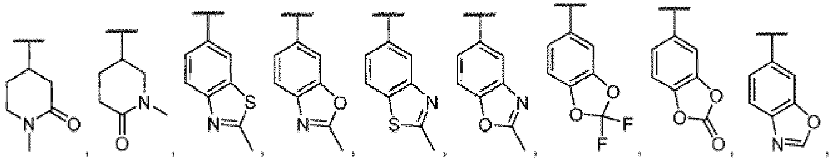 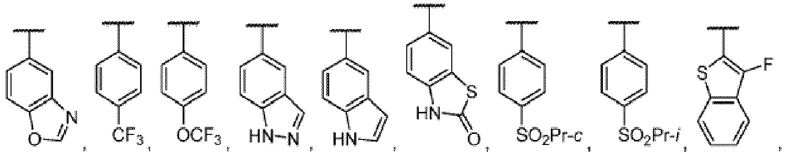 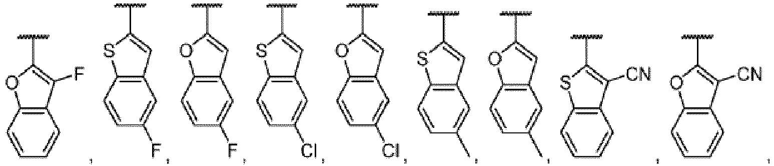 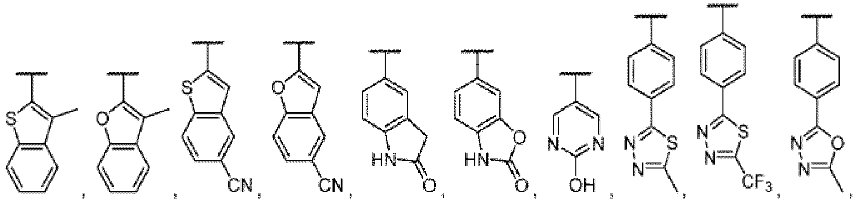 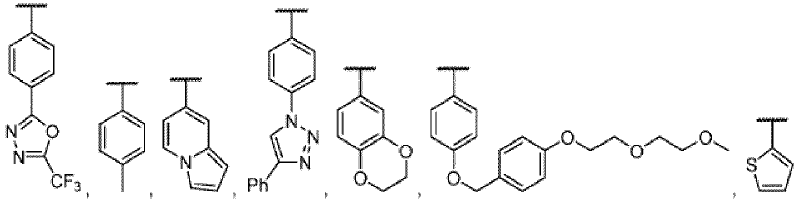 oder 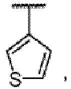 worin R² vorzugsweise Folgendes ist:

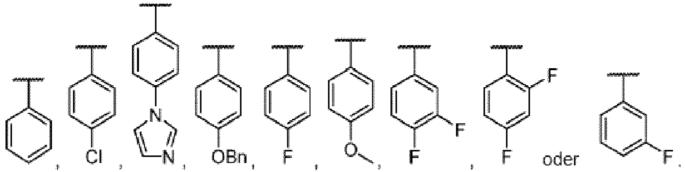

5. Verbindung oder Salz nach einem der Ansprüche 1 bis 4, worin entweder ein R⁸ H und das andere Me ist; oder worin jedes R⁸ Me ist.

6. Verbindung oder Salz nach einem der Ansprüche 1 bis 4, worin beide R⁸ zusammen mit dem Kohlenstoff, an den sie gebunden sind, einen Cyclopropylring bilden.

7. Verbindung oder Salz nach einem der Ansprüche 1 bis 6, worin n = 0 ist.

8. Verbindung oder Salz nach einem der Ansprüche 1 bis 6, worin n = 1 ist und R³ F oder Cl ist.

9. Verbindung oder Salz nach Anspruch 1 mit einer Struktur wie in Tabelle A dargestellt, vorzugsweise wie in Tabelle B dargestellt:

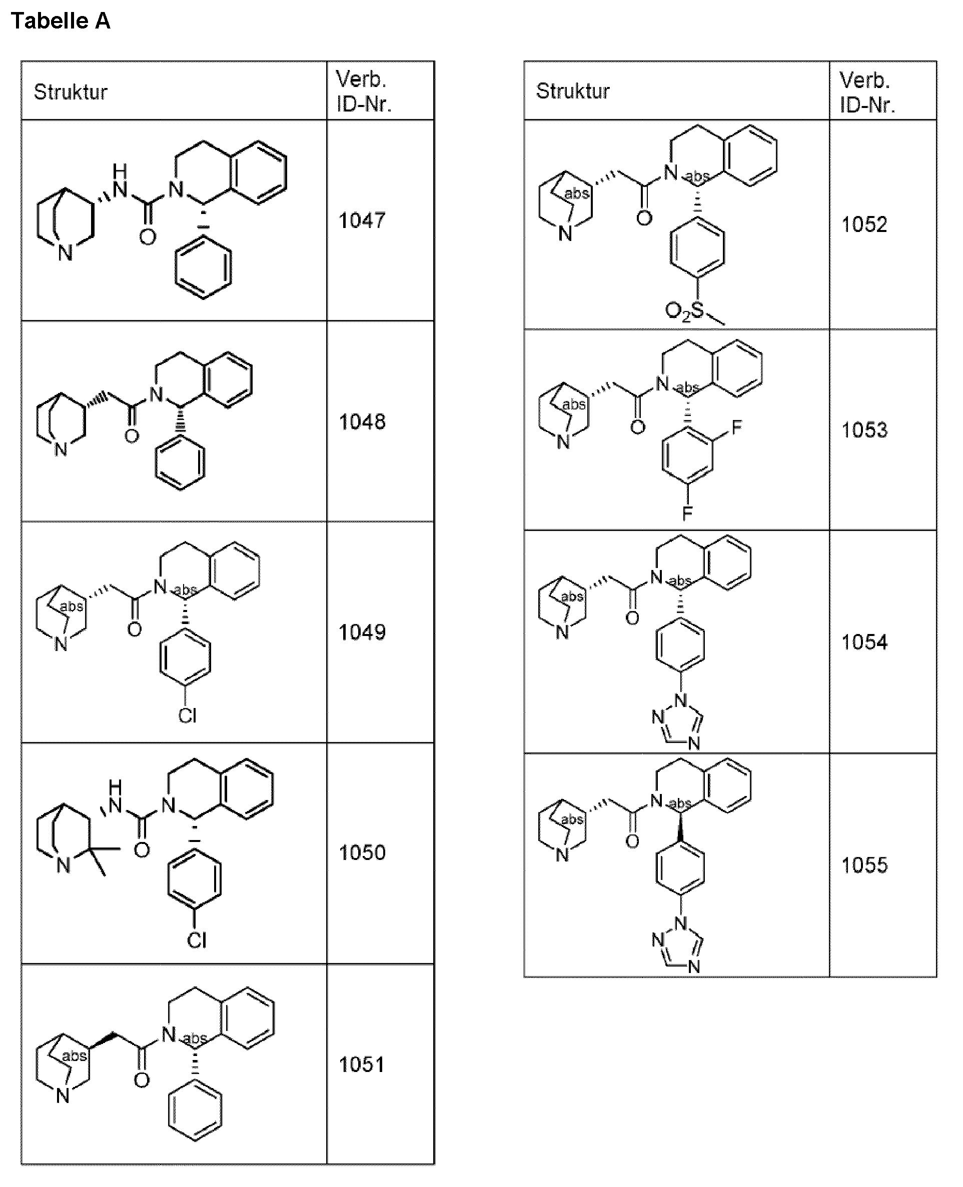

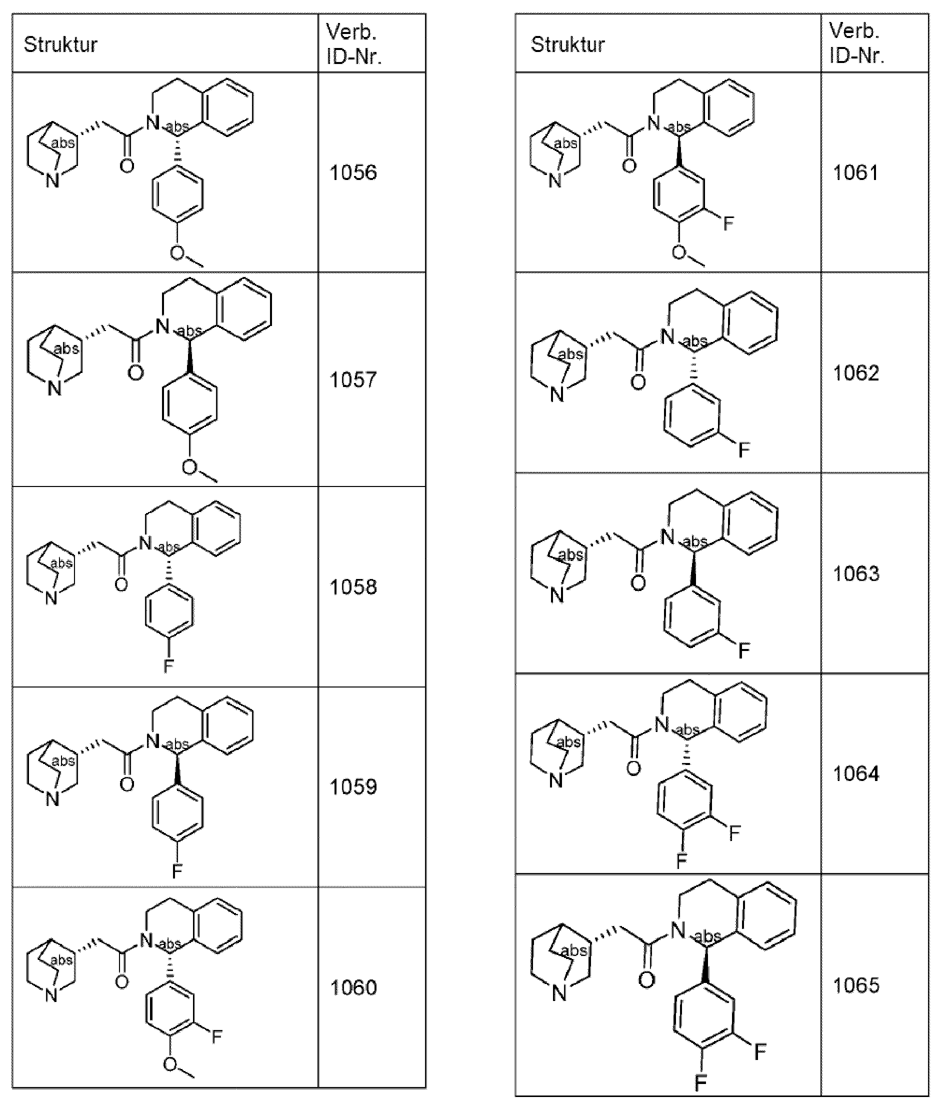

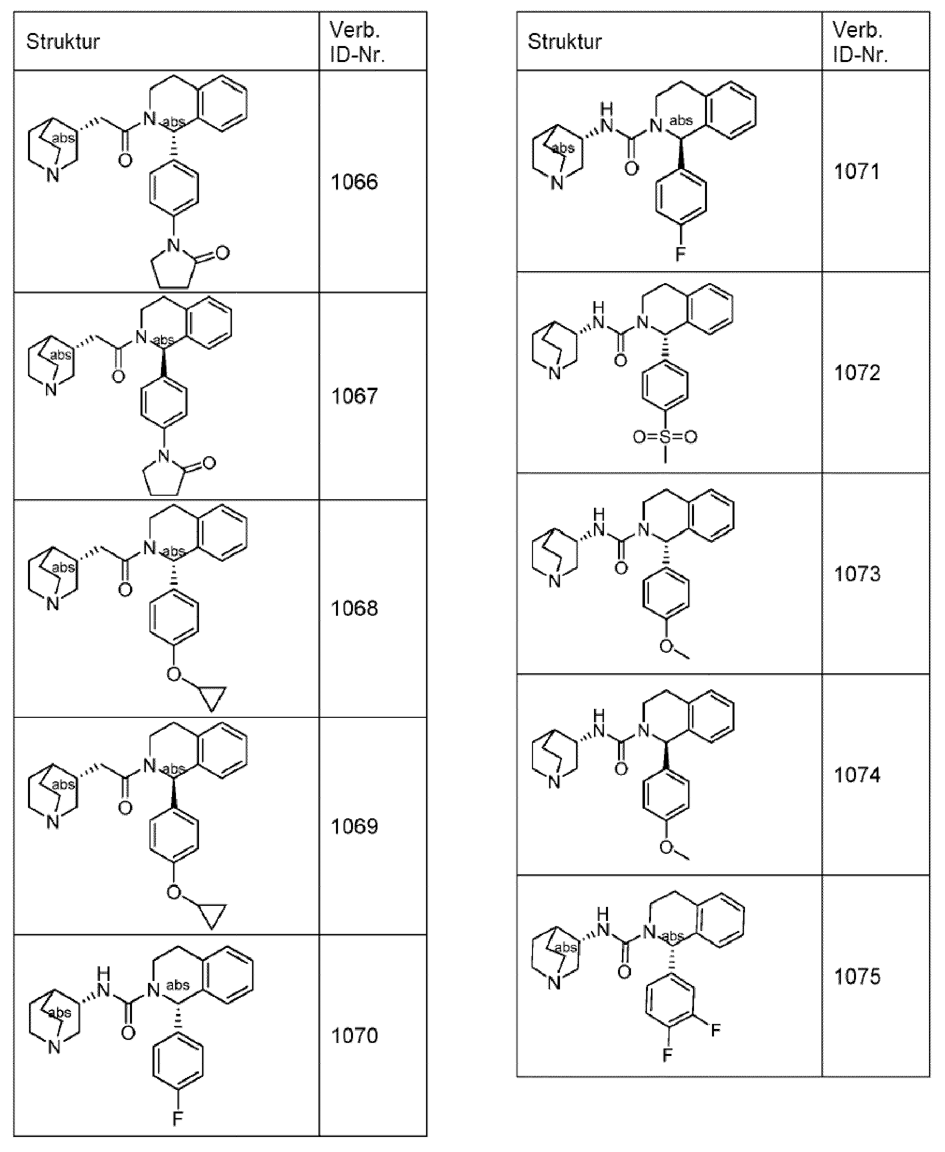

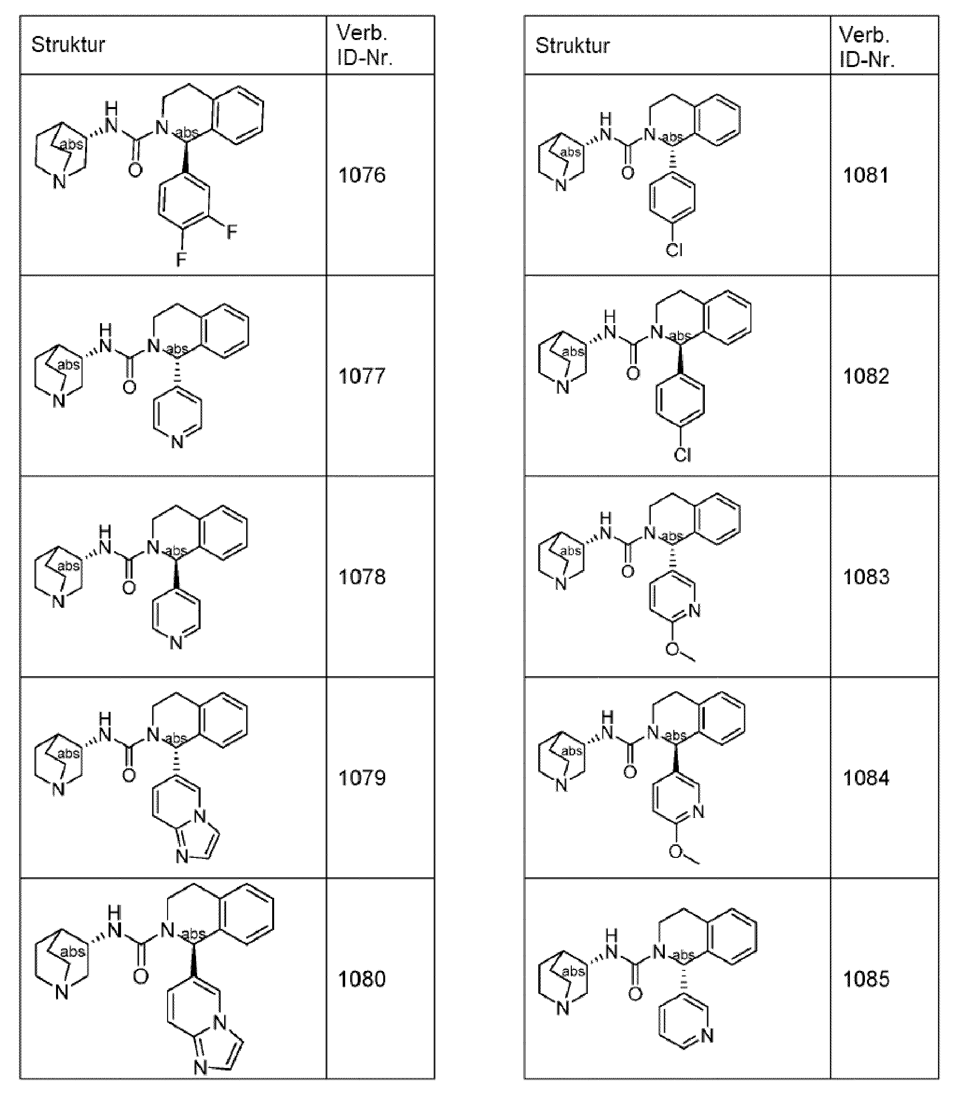

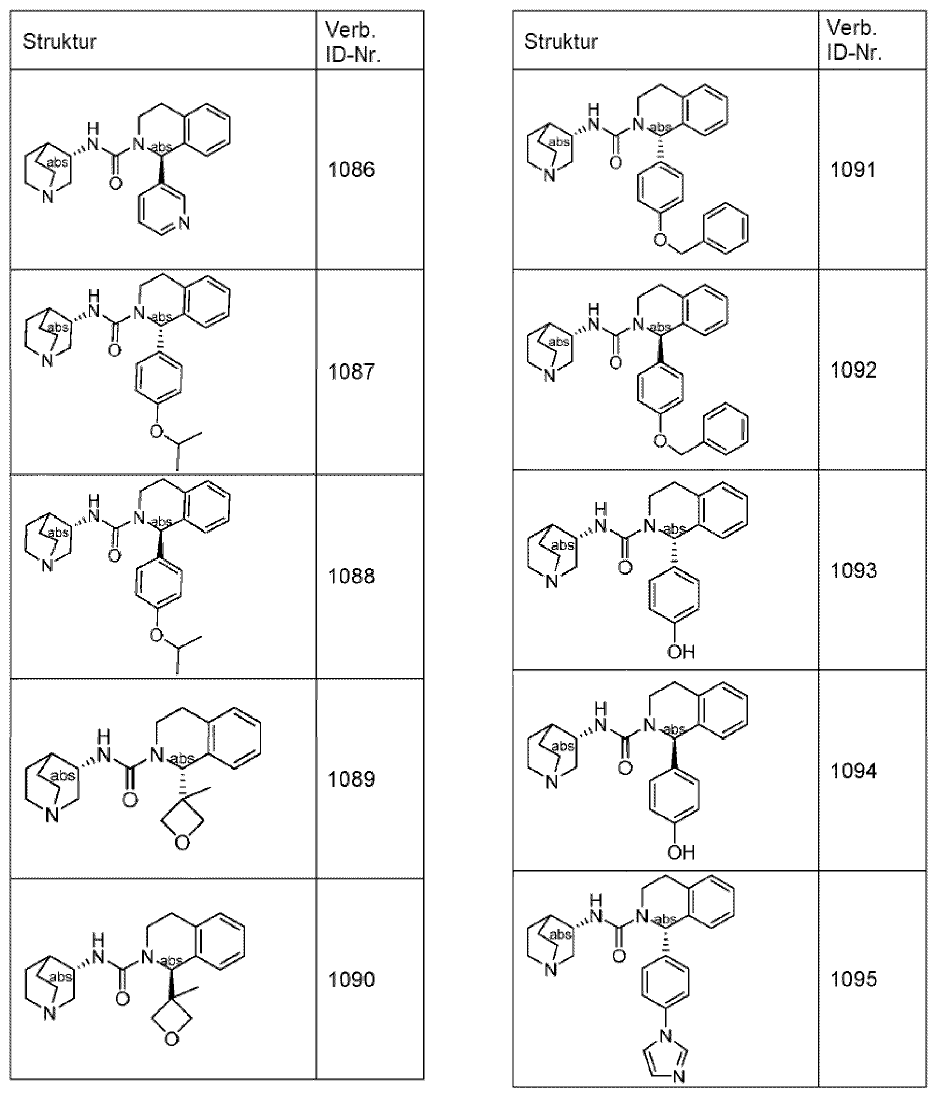

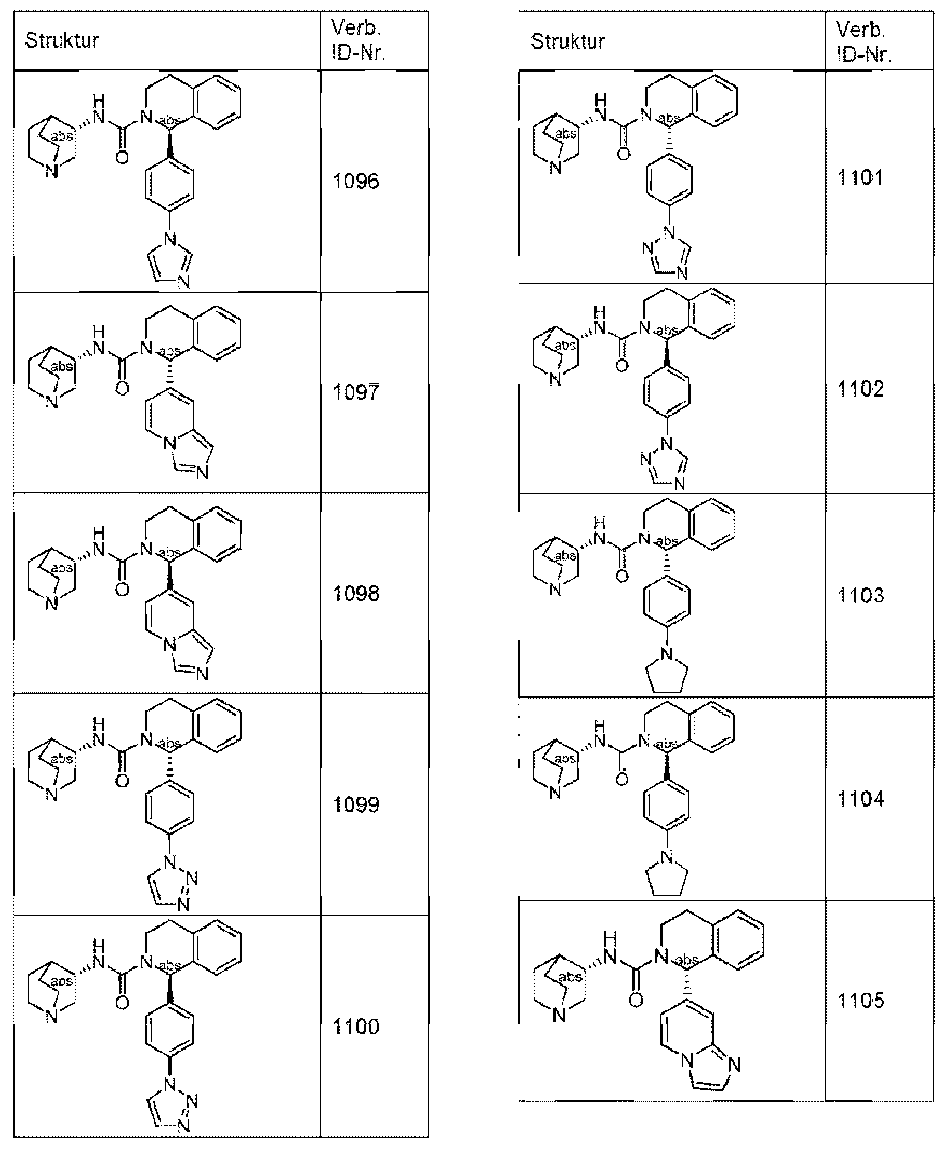

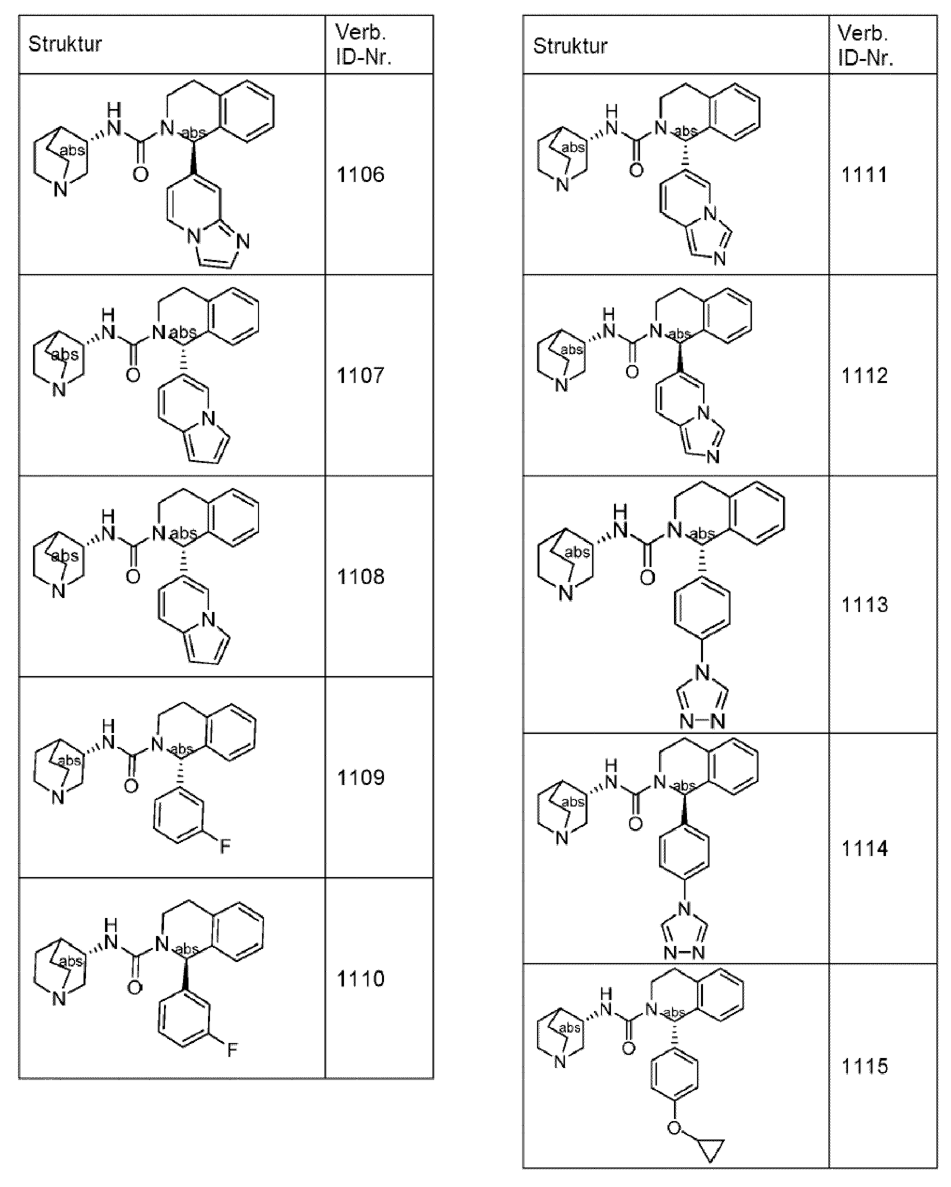

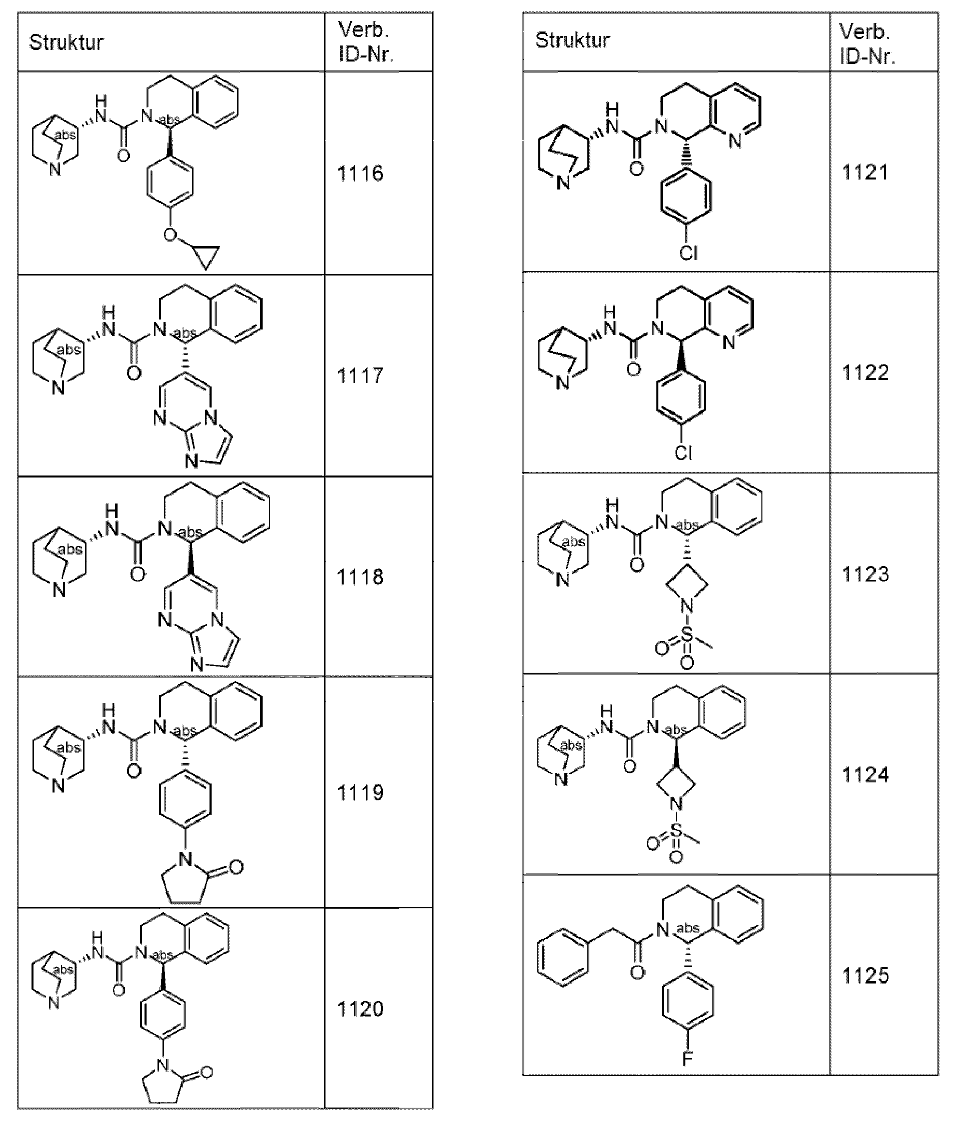

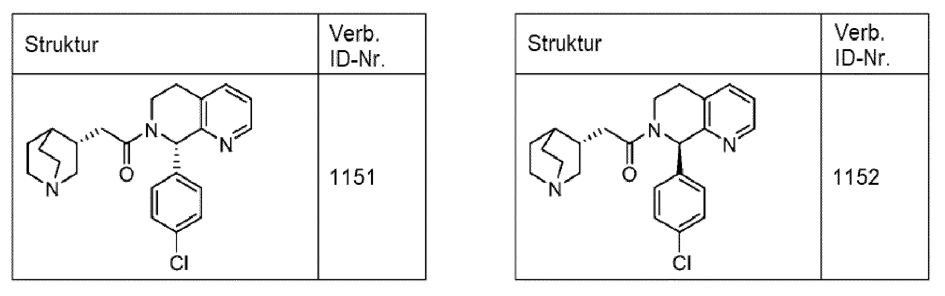

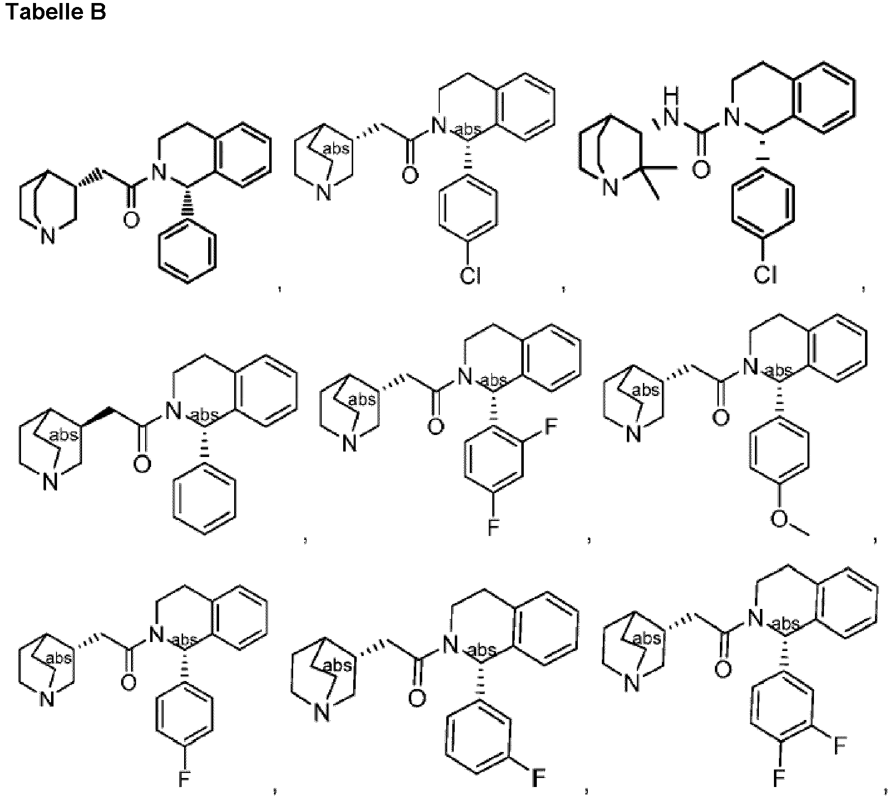

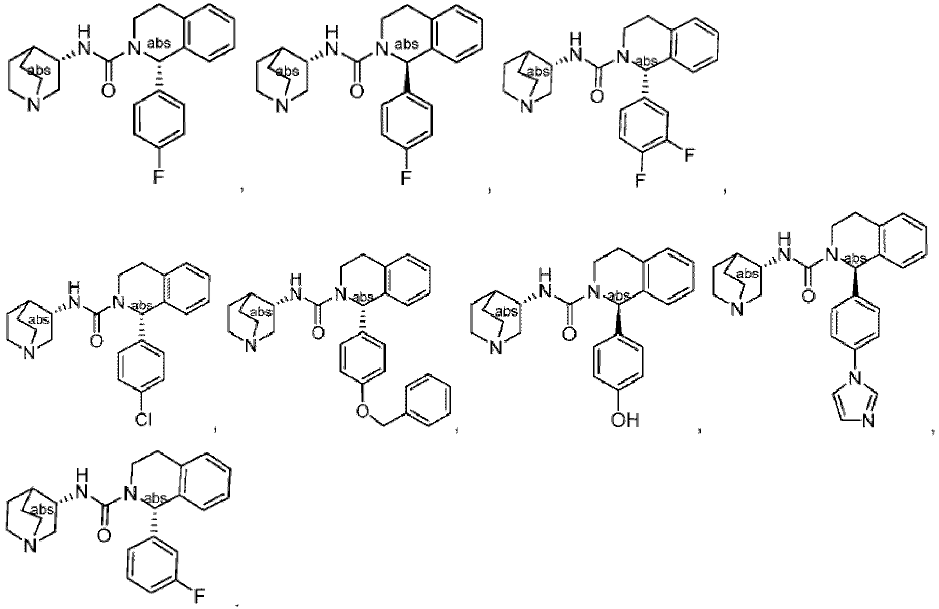

10. Verbindung oder Salz nach Anspruch 1 mit einer aus der aus Folgendem bestehenden Gruppe ausgewählten Struktur:

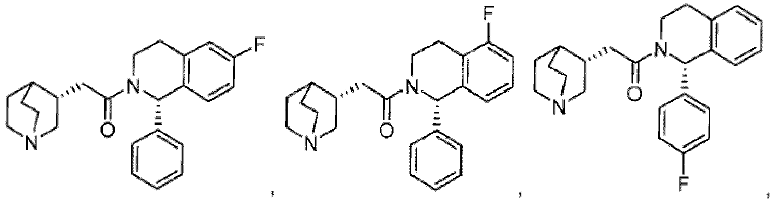

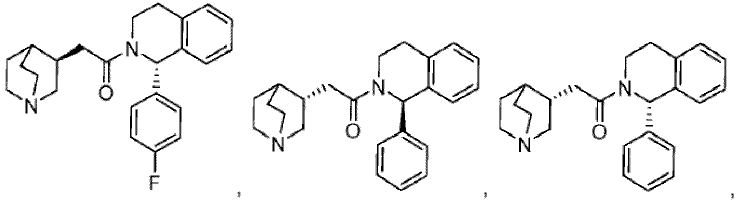

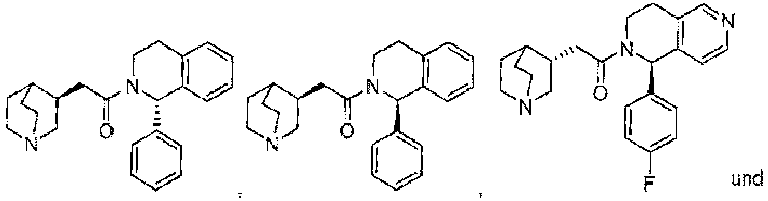

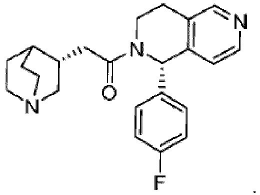

11. Verbindung oder Salz nach einem der Ansprüche 1 bis 10 in Form eines Salzes.

12. Pharmazeutische Zusammensetzung, die eine Verbindung oder ein Salz nach einem der Ansprüche 1 bis 11 und einen pharmazeutisch annehmbaren Exzipienten umfasst.

13. Verbindung oder Salz nach einem der Ansprüche 1 bis 11 zur Verwendung bei der Behandlung einer mit Progranulin zusammenhängenden Störung.

14. Verbindung oder Salz zur Verwendung nach Anspruch 13, wobei die mit Progranulin zusammenhängende Störung Alzheimer-Krankheit (AK), Parkinson-Krankheit (PK), amyotrophe Lateralsklerose (ALS), frontotemporale Demenz (FTD), frontotemporale Demenz - Granulin-Subtyp (FTD-GRN), Lewy-Body-Demenz (LBD), eine Prionenerkrankung, eine Motoneuronerkrankung (MND), Chorea Huntington (CH), spinozerebelläre Ataxie (SCA), spinale Muskelatrophie (SMA), eine lysosomale Speichererkrankung, eine mit Inklusionen und/oder Fehlfunktionen von C9orf72, TDP-43, FUS, UBQLN2, VCP, CHMP28 und/oder MAPT zusammenhängende Erkrankung, eine akute neurologische Störung, ein Glioblastom oder ein Neuroblastom ist.

15. Verbindung oder Salz zur Verwendung nach Anspruch 14, wobei:
(i) die lysosomale Speichererkrankung Morbus Paget, Morbus Gaucher, Morbus Nieman-Pick, Morbus Tay-Sachs, Morbus Fabry, Morbus Pompe oder Naso-Hakula-Krankheit ist;
(ii) die akute neurologische Störung ein Schlaganfall, eine Hirnblutung, eine traumatische Hirnverletzung oder ein Kopftrauma ist;
(iii) die mit Progranulin zusammenhängende Störung frontotemporale Demenz (FTD) ist; oder
(iv) die mit Progranulin zusammenhängende Störung frontotemporale Demenz - Granulin-Subtyp (FTD-GRN) ist.

## Revendications

1. Composé, ou sel pharmaceutiquement acceptable de celui-ci, ayant une structure de Formule (IA) : 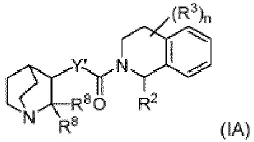 dans lequel
Y' est un CH₂ ou un NH ;
R² est un aryle en C₆₋₁₀, un hétéroaryle de 5 à 10 chaînons comprenant 1 à 4 hétéroatomes de noyau choisis parmi un N, un O et un S, ou un carbocycle ou un hétérocycle monocyclique ou bicyclique de 5 à 12 chaînons, dans lequel l'hétérocycle comprend 1 à 4 hétéroatomes de noyau choisis parmi un N, un O et un S, et R² est optionnellement substitué par 1 à 3 groupes R³ ;
chaque R³, le cas échéant, est indépendamment choisi parmi un alkyle en C₁₋₆, un alkylène en C₀₋₃-halogéno, un O-alkylène en C₁₋₃-halogéno, un alkylène en C₀₋₃-CN, un alkylène en C₀₋₃-NR⁵₂, un alkylène en C₀₋₆-OR⁵, un alkylène en C₀₋₆-C(O)OR⁷, un C(O)N(R⁷)₂, un SO₂R⁶, un O-alkylène en C₀₋₆-Ar, un oxo et un alkylène en C₀₋₆-Ar ;
Ar est un carbocycle ou un hétérocycle de 3 à 8 chaînons, dans lequel l'hétérocycle comprend 1 à 4 hétéroatomes de noyau choisis parmi un N, un O et un S ; un aryle en C₆₋₁₀ ; ou un hétéroaryle de 5 à 10 chaînons comprenant 1 à 4 hétéroatomes de noyau choisis parmi un N, un O et un S et Ar est optionnellement substitué par 1 à 3 groupes indépendamment choisis parmi un halogène, un alkyle en C₁₋₆, un halogénoalkyle en C₁₋₆, un alcoxy en C₁₋₆, un halogénoalcoxy en C₁₋₆, un CN et un SO₂-alkyle en C₁₋₃ ;
chaque R⁵ est indépendamment un H, un alkyle en C₁₋₆ ou un C(O)-alkyle en C₁₋₆ ;
chaque R⁶ est indépendamment un alkyle en C₁₋₆, un halogénoalkyle en C₁₋₆ ou un Ar ;
chaque R⁷ est indépendamment un H ou un alkyle en C₁₋₆ ;
chaque R⁸ est indépendamment un H ou un Me, ou les deux R⁸ pris conjointement avec le carbone auquel ils sont fixés forment un noyau cyclopropyle ; et
n vaut de 0 à 2 ; ou dans lequel le composé a une structure choisie parmi : 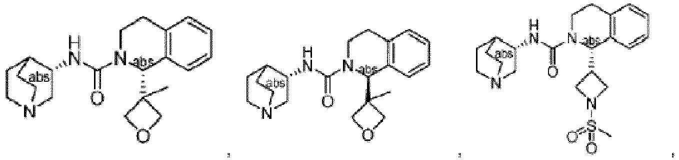 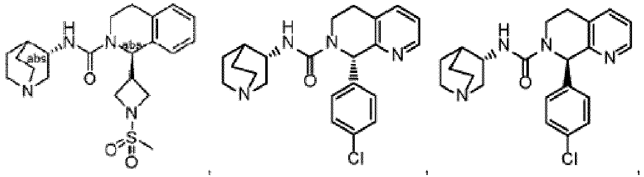 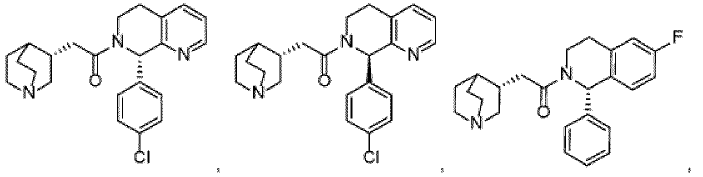 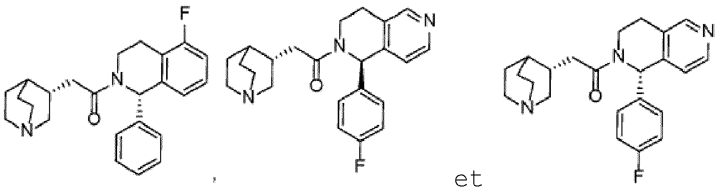 ou un sel pharmaceutiquement acceptable de celui-ci.

2. Composé ou sel selon la revendication 1, dans lequel R² est substitué par 1 à 3 R³, et chaque R³ est indépendamment choisi parmi un F, un Cl, un OH, un OMe, un OiPr, un OBn, un O-cyclopropyle, un CF₃, un OCF₃, un CN, un SO₂Me, un SO₂-cyclopropyle, un SO₂iPr, un oxo, un imidazolyle, un triazolyle, un pyrrolidinyle, un pyrrolidinonyle, un thiadiazolyle, un méthyl-thiadiazolyle, un trifluorométhyl-thiadiazolyle, un oxadiazolyle, un méthyl-oxadiazolyle, un trifluorométhyl-oxadiazolyle et un phényle.

3. Composé ou sel selon la revendication 1 ou 2, dans lequel R² comprend un phényle et est optionnellement substitué par 1 à 2 groupes choisis parmi un fluoro et un chloro.

4. Composé ou sel selon l'une quelconque des revendications 1 à 3, dans lequel R² est choisi dans le groupe constitué de : 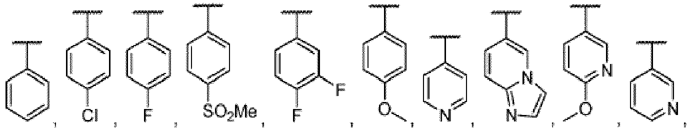 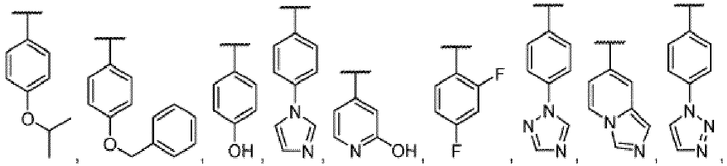 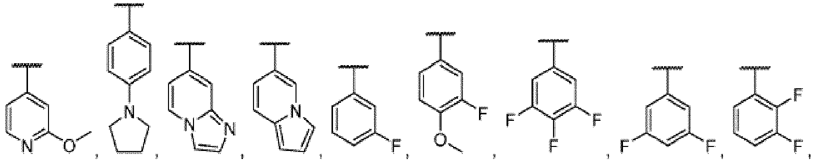 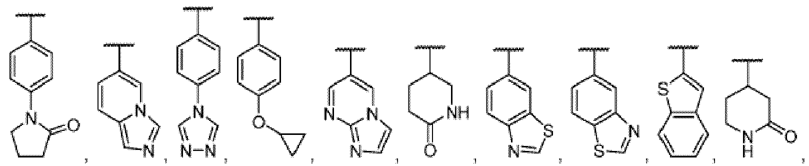 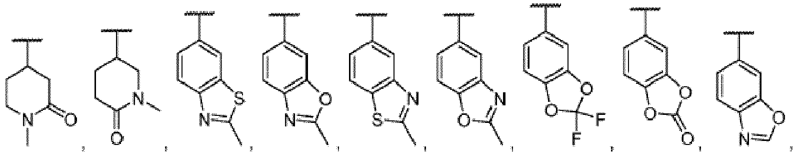 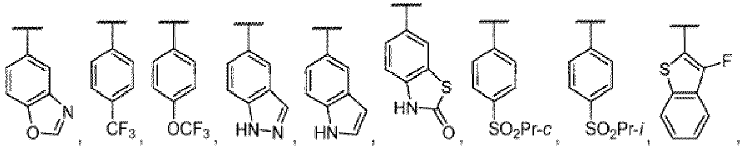 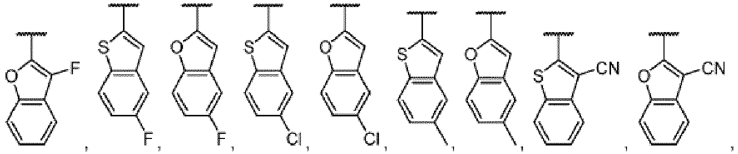 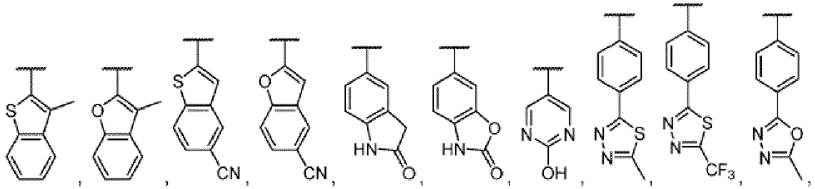 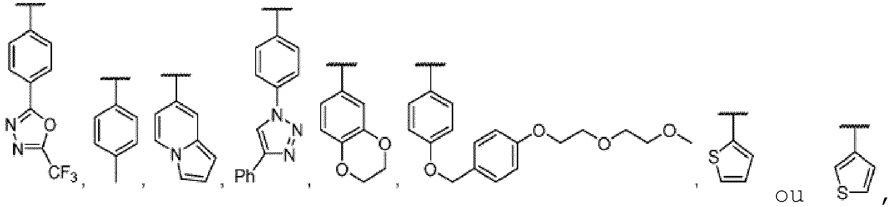 de préférence dans lequel R² est :

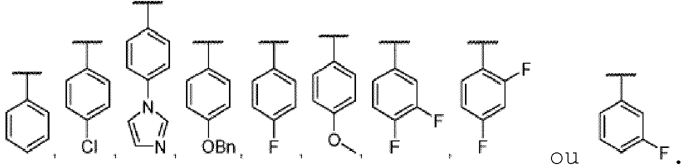

5. Composé ou sel selon l'une quelconque des revendications 1 à 4, soit dans lequel un R⁸ est un H et l'autre un Me ; soit dans lequel chaque R⁸ est un Me.

6. Composé ou sel selon l'une quelconque des revendications 1 à 4, dans lequel les deux R⁸ conjointement avec le carbone auquel ils sont fixés forment un noyau cyclopropyle.

7. Composé ou sel selon l'une quelconque des revendications 1 à 6, dans lequel n vaut 0.

8. Composé ou sel selon l'une quelconque des revendications 1 à 6, dans lequel n vaut 1 et R³ est un F ou un Cl.

9. Composé ou sel selon la revendication 1, ayant une structure telle que montrée dans le Tableau A, de préférence telle que montrée dans le Tableau B :

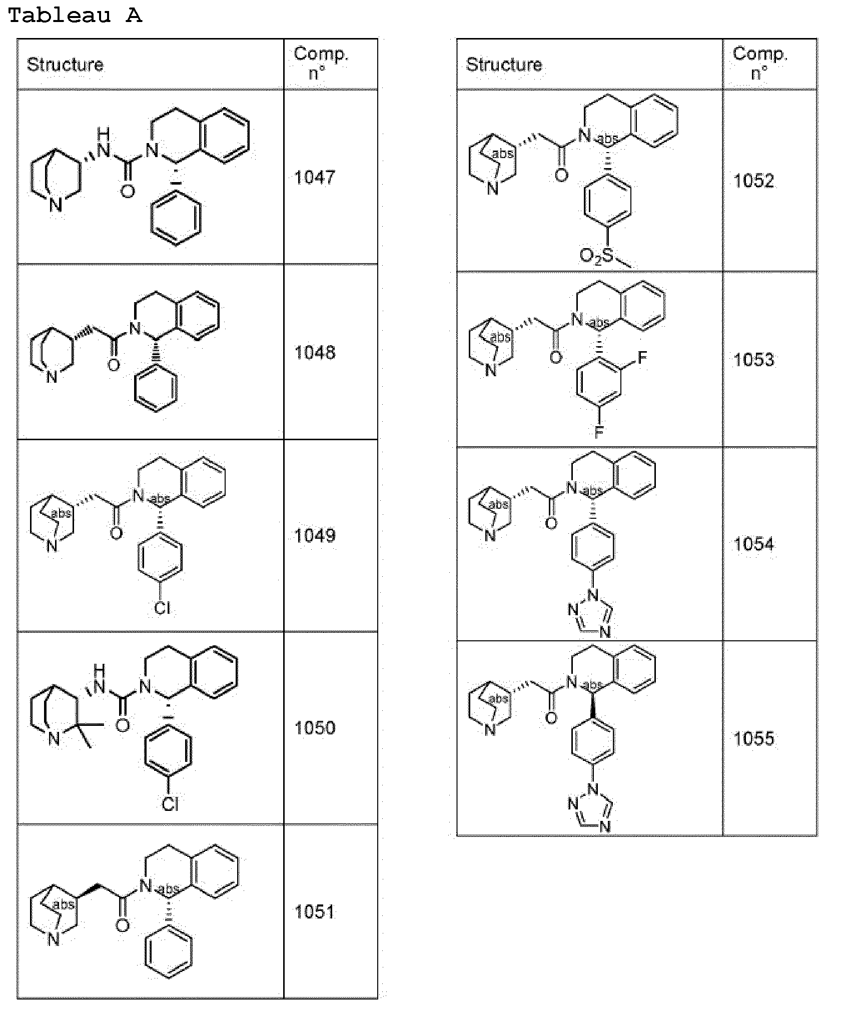

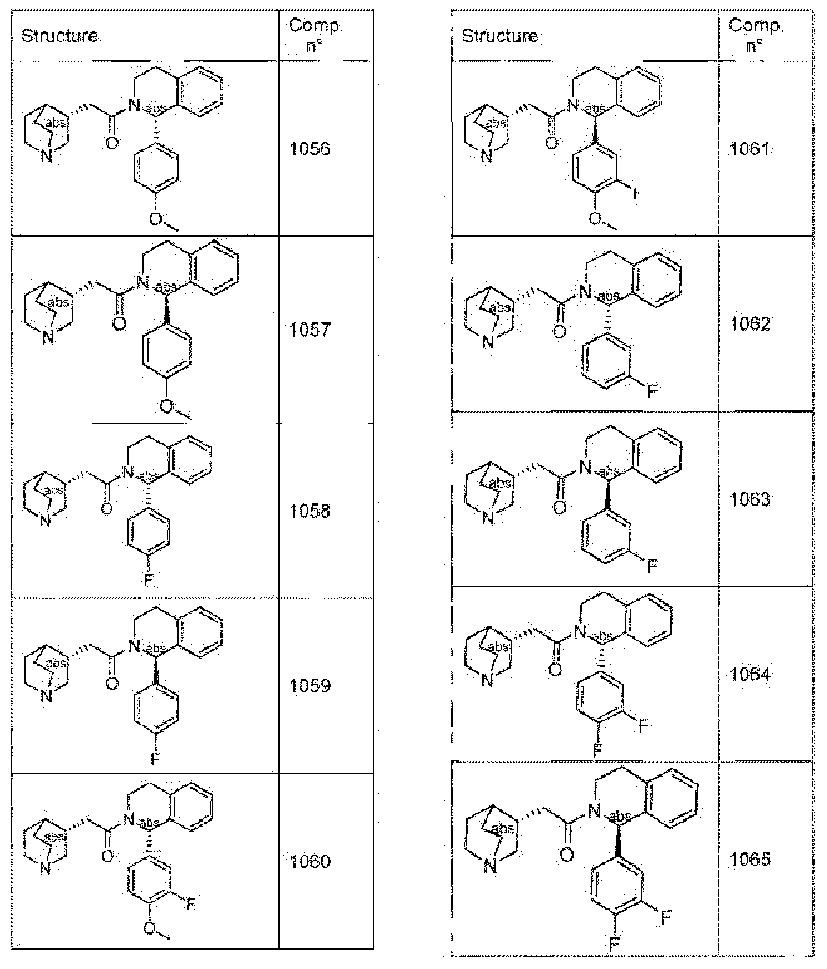

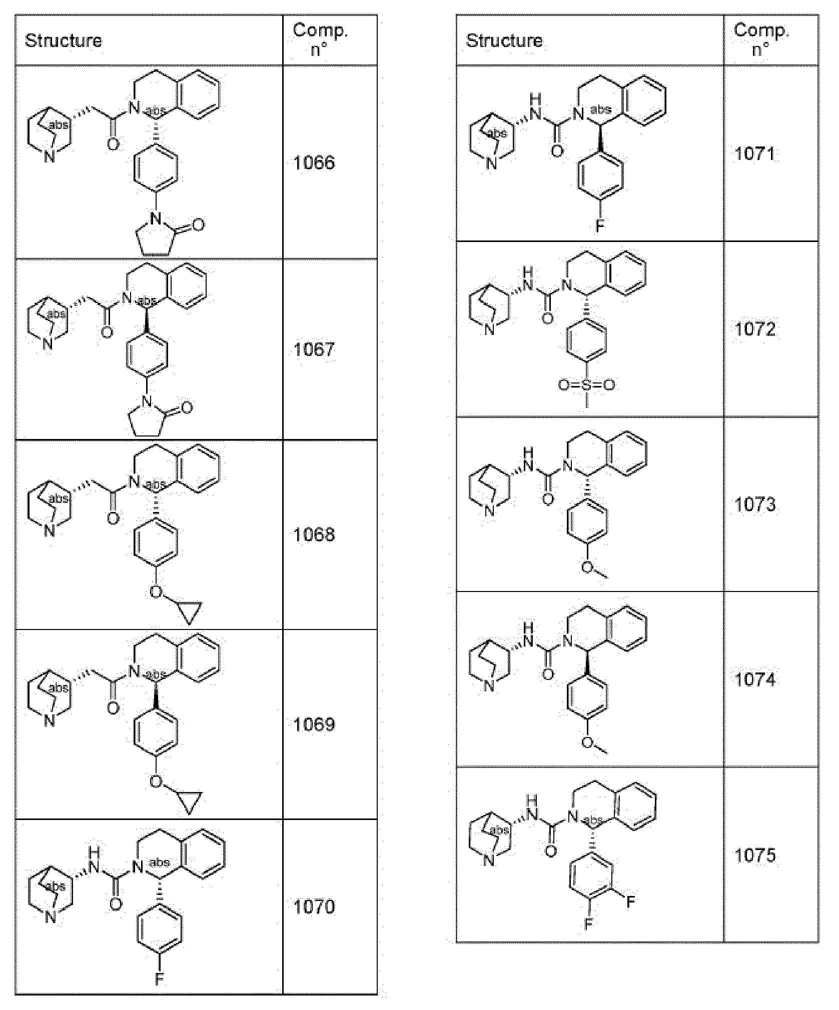

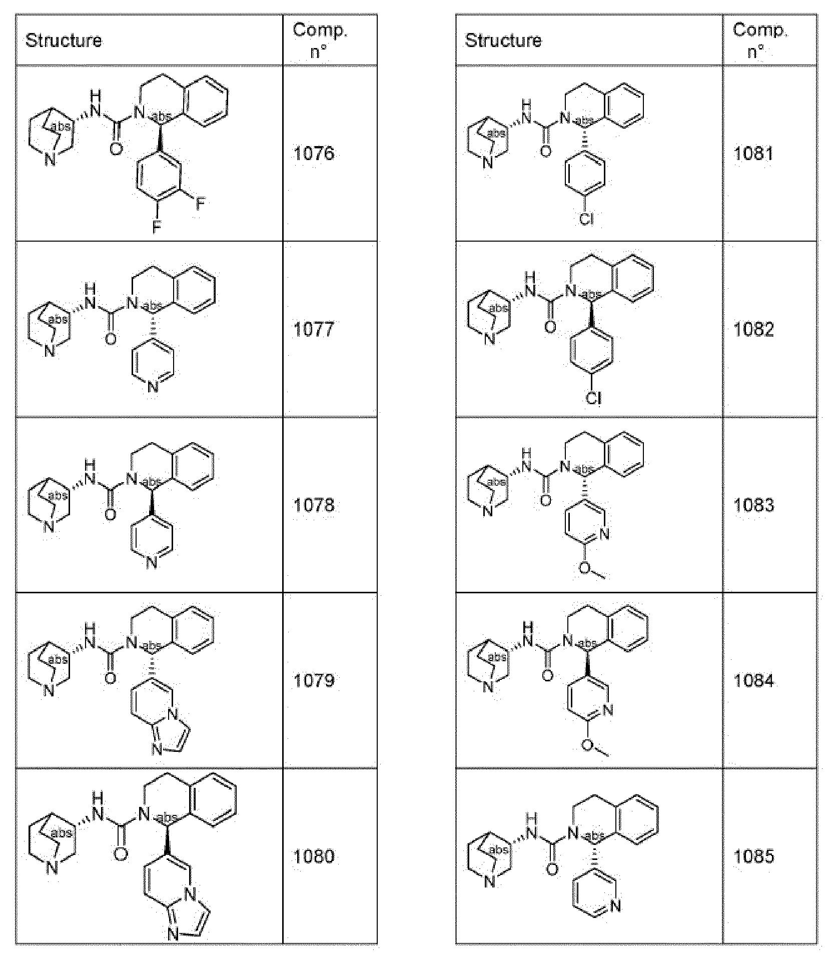

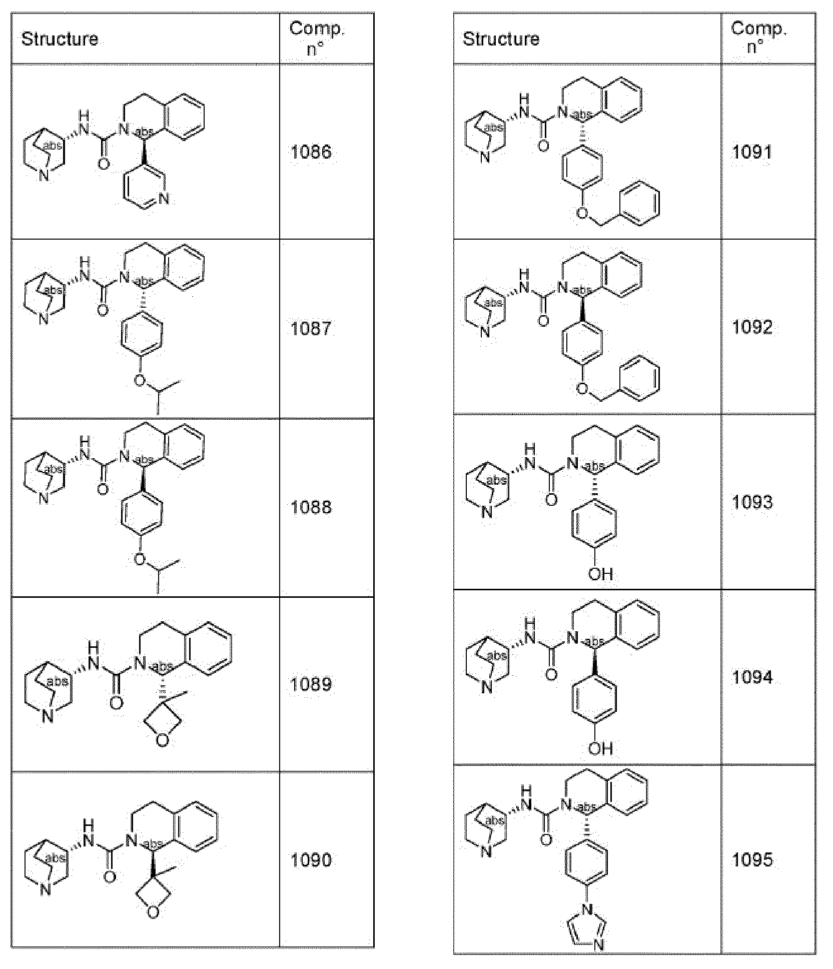

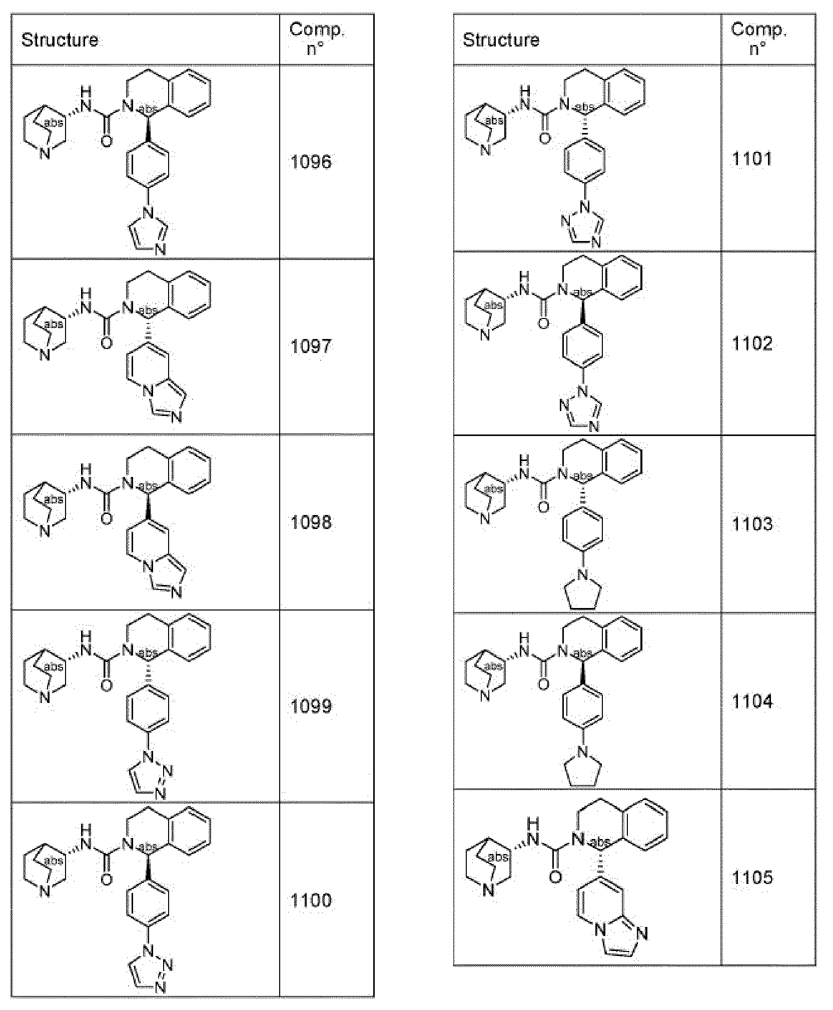

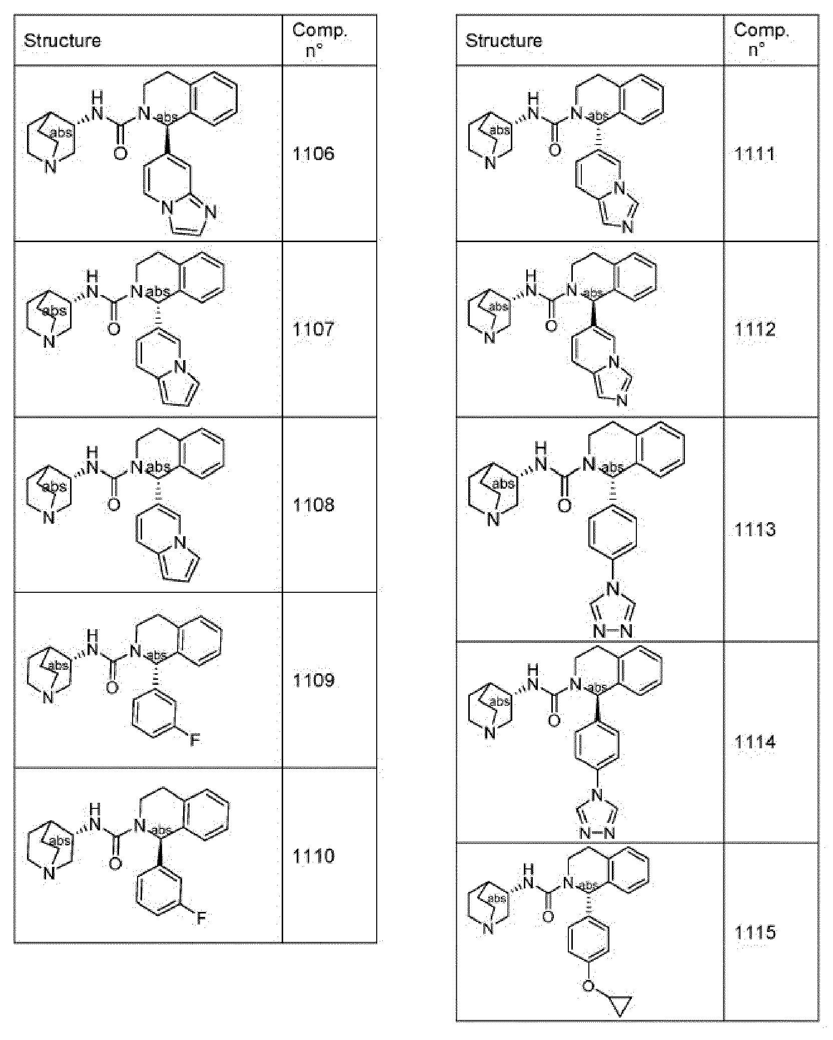

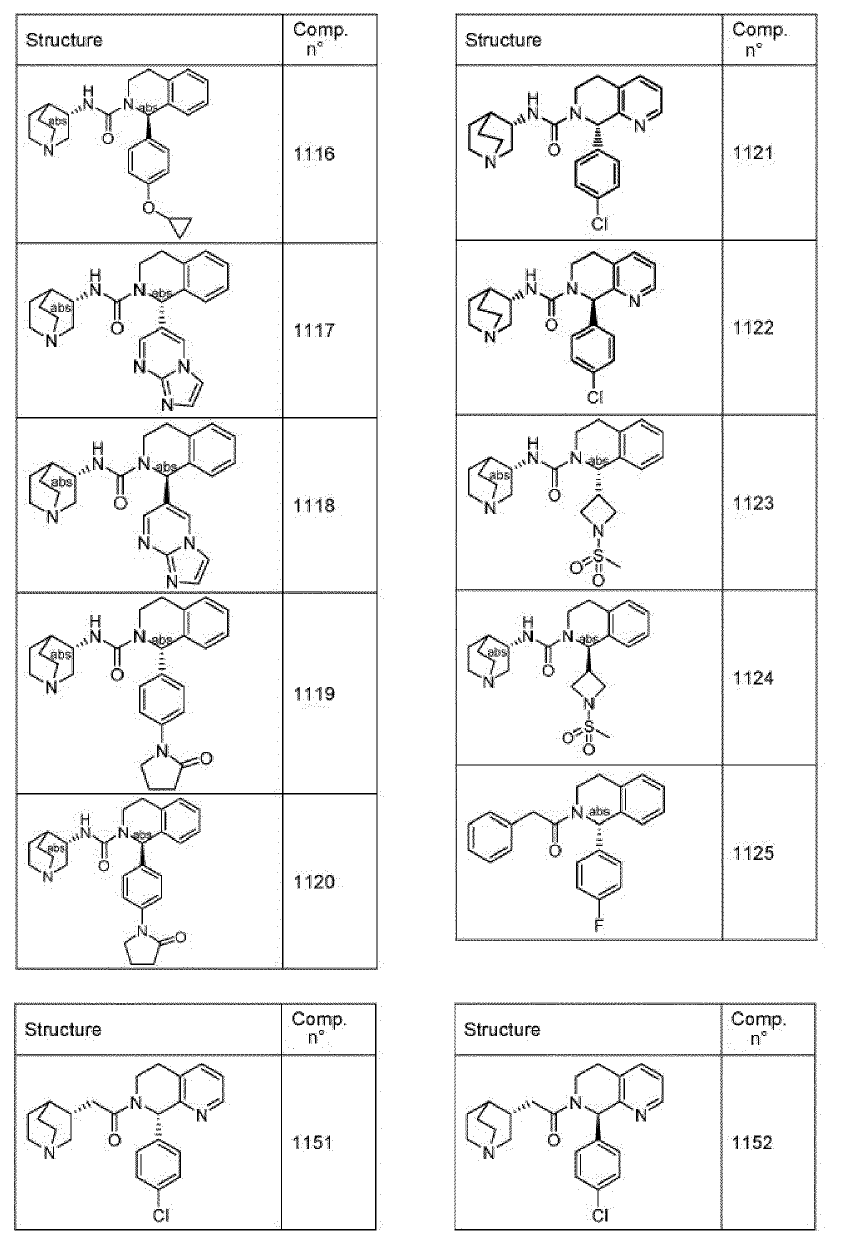

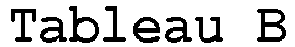

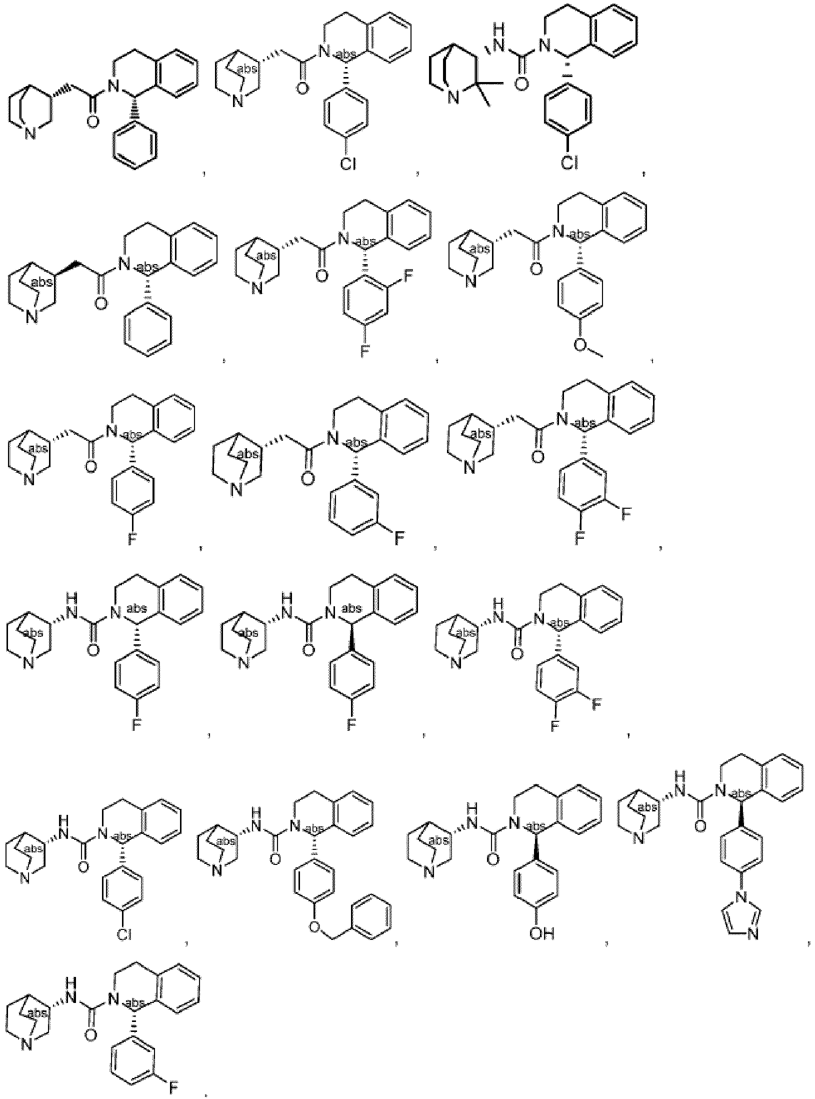

10. Composé ou sel selon la revendication 1, ayant une structure choisie dans le groupe constitué de 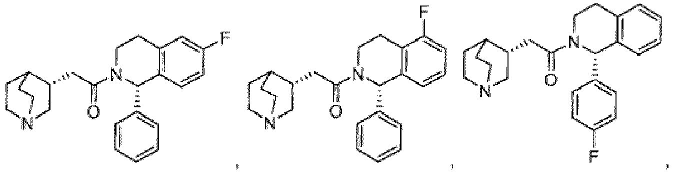 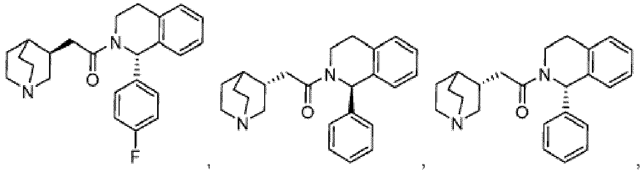 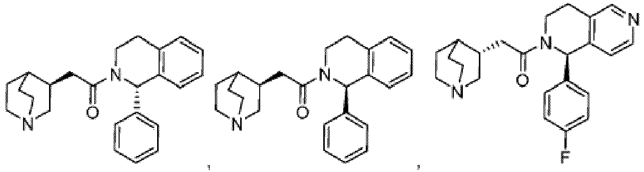 et

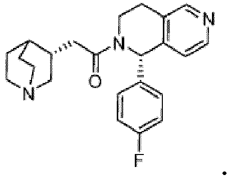

11. Composé ou sel selon l'une quelconque des revendications 1 à 10 sous la forme d'un sel.

12. Composition pharmaceutique comprenant le composé ou le sel selon l'une quelconque des revendications 1 à 11 et un excipient pharmaceutiquement acceptable.

13. Composé ou sel selon l'une quelconque des revendications 1 à 11 pour une utilisation dans le traitement d'un trouble associé à la progranuline.

14. Composé ou sel pour une utilisation selon la revendication 13, dans lequel le trouble associé à la progranuline est la maladie d'Alzheimer (AD), la maladie de Parkinson (PD), la sclérose latérale amyotrophique (ALS), la démence frontotemporale (FTD), la démence frontotemporale - sous-type granuline (FTD-GRN), la démence à corps de Lewy (LBD), la maladie à prion, les maladies des neurones moteurs (MND), la maladie de Huntington (HD), l'ataxie spinocérébelleuse (SCA), l'amyotrophie spinale (SMA), une maladie lysosomale, une maladie associée à des inclusions et/ou à un dysfonctionnement de C9orf72, TDP-43, FUS, UBQLN2, VCP, CHMP28, et/ou MAPT, un trouble neurologique aigu, le glioblastome ou le neuroblastome.

15. Composé ou sel pour une utilisation selon la revendication 14, dans lequel
(i) la maladie lysosomale est la maladie de Paget, la maladie de Gaucher, la maladie de Niemann-Pick, la maladie de Tay-Sachs, la maladie de Fabry, la maladie de Pompe ou la maladie de Nasu-Hakola ;
(ii) le trouble neurologique aigu est un accident vasculaire cérébral, une hémorragie cérébrale, une lésion cérébrale traumatique ou un traumatisme crânien ;
(iii) le trouble associé à la progranuline est la démence frontotemporale (FTD) ; ou
(iv) le trouble associé à la progranuline est la démence frontotemporale - sous-type granuline (FTD-GRN).
